(19) Europäisches Patentamt
European Patent Office
Office européen des brevets

(11) EP 3 321 371 A1

(12) **EUROPEAN PATENT APPLICATION**

(43) Date of publication:
**16.05.2018 Bulletin 2018/20**

(51) Int Cl.:
***C12Q 1/18*** *(2006.01)* ***G01N 33/50*** *(2006.01)*

(21) Application number: **17159022.7**

(22) Date of filing: **02.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **09.11.2016 US 201615347285**

(71) Applicants:
- **Zhang, Ying**
  **Ellicott City, MD 21043 (US)**
- **Feng, Jie**
  **Baltimore, MD 21211 (US)**
- **Yee, Rebecca**
  **Baltimore, MD 21201 (US)**
- **Zhang, Shuo**
  **Baltimore, MD 21232 (US)**
- **Zhang, Wenhong**
  **Shanghai 200051 (CN)**
- **Wang, Xiaodong**
  **70191 Stuttgart (DE)**

(72) Inventors:
- **ZHANG, Ying**
  **Ellicott City, MD Maryland 21043 (US)**
- **FENG, Jie**
  **Baltimore, MD Maryland 21211 (US)**
- **YEE, Rebecca**
  **Baltimore, MD Maryland 21201 (US)**
- **ZHANG, Wenhong**
  **Shanghai, Shanghai 200051 (CN)**
- **ZHANG, Shuo**
  **Baltimore, MD Maryland 21232 (US)**

(74) Representative: **J A Kemp**
**14 South Square**
**Gray's Inn**
**London WC1R 5JJ (GB)**

(54) **NOVEL METHODS FOR RAPID DRUG SUSCEPTIBILITY TESTING AND DRUG SCREENS FOR GROWING AND NON-GROWING CELLS OF PROKARYOTIC AND EUKARYOTIC ORIGIN**

(57) The presently disclosed subject matter provides methods, compositions, and kits for extremely rapid assessment of drug susceptibility of growing as well as non-growing cells of prokaryotic and also eukaryotic origins, without the need for organism growth, which is in contrast to the current growth-dependent methods.This growth-independent new methodology achieves extremely rapid drug susceptibility testing results previously not possible. Methods for rapidly identifying compounds with inhibitory activity against both growing and non-growing cells in real-time continuous manner with previously unachievable efficiency in drug screens using the novel methodology are also provided. Cell cultures are incubated with a drug or test agent and with a staining mixture containing a first fluorescent dye indicative the emission of which is indicative of live cells (e.g. SYBR Green I) and a second fluorescent dye indicative emitting a different color, the emission of which is indicative of dead cells (e.g. Propidium iodide). The ratio of the intensities of the emitted fluorescence of the dyes is indicative of the percentage live and dead cells in the culture.

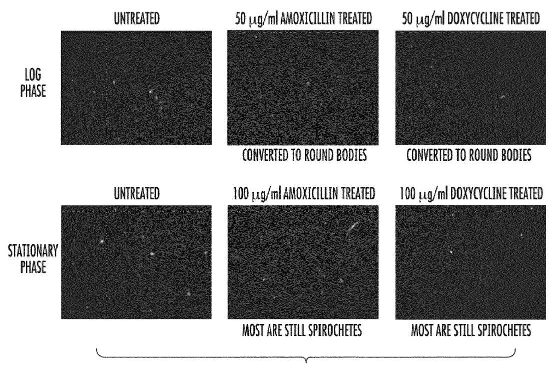


*Fig. 1*

EP 3 321 371 A1

**Description**

BACKGROUND AND INTRODUCTION

A rising public health concern is the increasing emergence and spread of antibiotic resistant bacteria and innovative approaches for prompt detection of antibiotic resistance is critical in saving patient lives and reducing the spread of antibiotic resistant organisms. To combat antibiotic resistance, healthcare providers are encouraged to minimize the unnecessary usages of antibiotics. Hence, laboratory diagnostic tests are imperative in providing healthcare providers knowledge to tailor the appropriate treatment to each individual. However, currently, the common drug susceptibility tests all rely on growth of the organisms and take at least 1-2 days for non-fastidious organisms [CLSI, 2016] and can be up to 1 month for fastidious slow growing organisms like *M. tuberculosis.* [CLSI, 2011] before any information can be determined about the pathogen, putting the patient's life as risk with potential worsened disease outcome and increase spread of antibiotic resistant organisms.

The current antibiotic susceptibility tests (AST) such as the Kirby-Bauer Disk Diffusion test or broth dilution method to determine a drug's minimum inhibitory concentration (MIC) provide accurate results, but the major drawback of these tests is the lengthy time (at least 16 hours) required until results are produced even for non-fastidious organisms [CLSI, 2016]. AST methods utilizing MALDI-TOF mass spectrometry (MS) which was originally used for microorganism identification, has been developed but such methods produced varying AST results and were unable to detect resistance among highly resistant organisms such as *E. coli, K. pneumoniae, P. aeruginosa,* and also methicillin-resistant *S. aureus.* While the development of the automated VITEK®2 produced high sensitivity for AST, the test still takes about 12 hours to get results as it is dependent on the growth of the test organism [Machen et al., 2014]. In the case of tuberculosis, a leading infectious killer worldwide, where multidrug-resistant TB (MDR-TB) and extensively drug-resistant TB (XDR-TB) poses an increasing challenge for TB control, a period of 2-6 weeks is required to obtain drug susceptibility testing results, which increases the risk of development and spread of drug resistant TB [Zignol et al., 2012]. Additionally, the current drug susceptibility test (DST) for pyrazinamide, a unique sterilizing persister drug that shortens treatment from 9-12 months to 6 months, include MGIT 960 or BacT/ALERT systems at pH 6.0 [Aragon et al., 2007], which are unreliable and subject to frequent false resistance problem and thus not routinely performed [Zhang and Mitchison, 2003; Hewlett et al., 1995; Chedore et al., 2010].

To expedite the process of antimicrobial susceptibility testing, the present inventors have developed a novel approach using SYBR Green I/Propidium Iodide (PI) staining viability assay. The SYBR Green I dye is commonly used in molecular biology to stain nucleic acids and was used for viability assessment for some bacteria by flow cytometry [Barbesti et al., 2000] but has not been commonly used for bacterial viability assessment [Feng et al., 2014] or used for AST. SYBR Green I is a permeable dye that stains all cells green whereas PI is an impermeant dye that stains only dead or damaged cells with a compromised cell membrane [Feng et al., 2014]. Hence, the green/red fluorescence ratio as easily measured by fluorescence microscopy or fluorescence microplate readers represents the viability of a cell population. As the assay is not growth-dependent, it eliminates a bottleneck in current AST.

The inventors of the present application have developed a novel, growth-independent antibiotic susceptibility test that can produce rapid and robust results less than 24 hours for slow and fast growing organisms. The present application shows for the first time, the utility of a SYBR Green I and Propidium Iodide (SYBR Green I/PI) assay for extremely rapid detection of antibiotic resistance in representative major pathogens of serious threats including multidrug resistant *Staphylococcus aureus, Escherichia coli, Klebsiella pneumoniae, Acinetobacter baumannii,* and *Mycobacterium tuberculosis.* The new SYBR Green/PI AST could identify antibiotic resistance in less than 30 minutes for the fast growing bacteria using different antibiotics for both bactericidal and bacteriostatic agents. The power of this novel SYBR Green I/PI methodology was further demonstrated for the extremely rapid detection of drug resistance in slow growing *M. tuberculosis* within 16 hrs using isoniazid and pyrazinamide resistance as examples, thus overcoming the limitation of the current culture-based time-consuming AST which takes 2-6 weeks.

The optimized SYBR Green I/PI assay generated rapid and robust results in concordance with traditional AST methods when tested on various clinically derived strains and a panel of drugs used for treatment in the clinic. The SYBR Green I/PI assay overcomes not only the inefficiency of conventional culture based AST or drug susceptibility testing (DST) but also the limitations and compromised sensitivity of molecular testing methods.

There is an increasing antibiotic resistance crisis ongoing and new drugs and antibiotics are urgently needed to combat these life threatening antibiotic resistant infections. Our newly developed SYBR Green I/PI AST testing methodology can also be adapted for extremely efficient and versatile real-time continuous drug screens in high throughput format, which is unlike the conventional drug screens at a fixed time point.

These findings change the concept of antibiotic susceptibility testing because the assay is a viability based, growth-independent method that can significantly reduce the time for the current growth-based AST. The SYBR Green I/PI AST testing methodology may revolutionize current drug susceptibility testing as well as drug screens for different organisms or cells of prokaryotic and eukaryotic origin, and may play a major role in saving lives and improving patient

outcomes in a timely manner, as well as decreasing transmission of increasing antibiotic resistant organisms.

SUMMARY

The presently disclosed subject matter provides methods, compositions, and kits for extremely rapid assessment of drug susceptibility of growing as well as non-growing cells of prokaryotic and also eukaryotic origins, without the need for organism growth, which is in contrast to the current growth-dependent methods.This growth-independent new methodology achieves extremely rapid drug susceptibility testing results previously not possible Methods for rapidly identifying compounds with inhibitory activity against both growing and non-growing cells in drug screens using the novel methodology are also provided.

In one aspect, the presently disclosed subject matter provides methods for assessing the viability or assessing the drug susceptibility of cells, such as antimicrobial susceptibility. The cells may be eukaryotic or prokaryotic cells and include, for example, spirochetal organisms, such as from the *Borrelia* genus, and more preferably, the *Borrelia burgdorferi* species, and related organisms, and other bacteria, including, but not limited to, *Staphylococcus aureus, Escherichia coli, Klebsiella pneumoniae, Acinetobacter baumannii,* and *Mycobacterium tuberculosis.*

In one aspect of the present invention there is provided a method for assessing the susceptibility of cells to at least one drug or test agent, the method comprising:

(a) obtaining or establishing a cell culture comprising said cells;
(b) incubating the cell culture with a staining mixture comprising: (i) a first agent which emits fluorescence of a first color that is indicative of live cells in the culture, and (ii) a second agent which emits fluorescence of a second color that contrasts from the first color and is indicative of dead cells in the culture;
(c) calculating a ratio of the intensity of emitted fluorescence of the first color to the intensity of emitted fluorescence of the second color; and
(d) assessing the viability or susceptibility of the cells in the culture to said at least one drug or test agent, wherein the ratio calculated in (c) is indicative of the percentage of live cells and dead cells in the culture.

The methods of the present invention may further comprise the step of contacting or exposing the cells to at least one dose of at least one drug or test agent.

Where it is desired to assess drug susceptibility the methods of the present invention may comprise the step of incubating the cells with a drug or agent that kills or inhibits the growth of the organism or cells. The present invention also provides for one or more test agents to be assayed for inhibitory activity, such as in a drug screen which may be optionally be performed in a real-time continuous manner and/or in a high throughput format.

The present invention also provides kits which may be used to carry out the methods of the present invention invention. In at least some embodiments of the present invention, there is a provided a kit for rapid screening or susceptibility testing of at least one candidate agent that is capable of inhibiting growth or survival of cells of eukaryotic or prokaryotic origin wherein the kit comprises:

(a) optionally a population of the cells of eukaryotic or prokaryotic origin or a
culture thereof;
(b) a staining mixture comprising: (i) a first agent which emits fluorescence of a first color that is indicative of live cells(e.g. SYBR Green I), and (ii) a second agent (e.g. propidium iodide) which emits fluorescence of a second color that contrasts from the first color and is indicative of dead cells, wherein when the staining mixture is incubated with the cells or culture thereof a calculated ratio of the intensity of emitted fluorescence of the first color to the intensity of emitted fluorescence the second color is indicative of the percentage of live cells in the population or culture thereof; and
(c) optionally instructions, such as instructions for using the cells in (a) and the staining mixture in (b) to screen for at least one candidate agent that is capable of inhibiting growth or survival of the cells.

Suitably the cells or organisms which are assayed with the methods or kits of the present invention are selected from the group consisting of: all cells of prokaryotic (bacteria and archaea) and also eukaryotic origin, including but not limited to cells of Gram-positive bacteria such as *Staphylococcus aureus,* and Gram-negative bacteria such as *Escherichia coli, Klebsiella pneumoniae,* and *Acinetobacter baumannii,* and mycobacteria such as *Mycobacterium tuberculosis,* and other microbial species, and eukaryotic cells such as fungal cells, plant cells, parasite cells, animal cells, or cancer cells.

In at least some embodiments of the methods described herein, the method comprises assessing the drug (e.g. antibiotic) susceptibility of the cells or organism being assayed to at least one agent by incubating the culture of step (a) under suitable conditions without the need for the test organism to grow. This enables a substantial reduction

of time from days and weeks needed for current drug susceptibility testing to extremely short times of just minutes or hours with the methodology described herein.

The methods of the present invention preferably utilize higher drug concentrations than the normal or usual drug concentrations needed for inhibiting organism or cell growth as used in current conventional drug susceptibility testing.

In at least some embodiments of the methods described herein, the method is performed in a high-throughput format, such as for drug screens.

In at least some embodiments of the methods described herein, the method is performed in a high-throughput format, such as for drug screens.

In at least some embodiments of the methods described herein, the method is performed in a real-time continuous manner optionally in a high-throughput format, such as for drug screens. As such, the methods of the present invention may be more versatile and efficient than the conventional drug screens typically done at a single time point.

Certain aspects of the presently disclosed subject matter having been stated hereinabove, which are addressed in whole or in part by the presently disclosed subject matter, other aspects will become evident as the description proceeds when taken in connection with the accompanying Examples and Figures as best described herein below.

BRIEF DESCRIPTION OF THE FIGURES

Having thus described the presently disclosed subject matter in general terms, reference will now be made to the accompanying Figures, which are not necessarily drawn to scale, and wherein:

FIG. 1 shows representative morphological changes of *Borrelia* cells following treatment with antibiotics;

FIG. 2 shows a comparison of methods for assaying *B. burgdorferi* viability in a 96-well plate;

FIG. 3 shows a linear relationship between the percentage of live *B. burgdorferi* and the green/red fluorescence ratio of a SYBR Green/propidium iodide (PI) assay ([1]BSK-H medium was removed in the washing steps prior to the LIVE/DEAD assay);

FIG. 4 shows the *B. burgdorferi* B31 strain with commonly used viability assays MTT, XTT, fluoroscein diacetate assay (FDA), the commercially available LIVE/DEAD BackLight assay, and the presently disclosed SYBR Green I/PI assay;

FIG. 5A, FIG. 5B, FIG. 5C, and FIG. 5D show the *B. burgdorferi* B31 strain observed with: (FIG. 5A) the fluorescent microscopy LIVE/DEAD BacLight stain; (FIG. 5B) SYBR Green/PI stain; (FIG. 5C) FDA stain; and (FIG. 5D) the *B. burgdorferi* biofilm stained by SYBR Green/PI;

FIG. 6 shows a SYBR Green/PI assay showing correlation with direct microscope counting in an antibiotic exposure ([1]Samples were stained with LIVE/DEAD BacLight kit);

FIG. 7 shows a representative drawing of the Yin-Yang model of bacterial persisters and latent infections where it is proposed to target both growing and non-growing bacterial populations for more effective treatment of difficult to cure or persistent bacterial infections and even cancer (Zhang, 2014);

FIG. 8A, FIG. 8B, and FIG. 8C show: (FIG. 8A) a growth curve of *B. burgdorferi* strain B31 *in vitro;* (FIG. 8B) representative images of log phase (3 day culture) and stationary phase (7 day culture) of the *B. burgdorferi* B31 strain observed with fluorescent microscopy using SYBR Green I/PI stain; the arrows indicate multiple morphological forms of *B. burgdorferi* in stationary phase; and (FIG. 8C) susceptibility of log phase (3 days) and stationary phase (7 days) *B. burgdorferi* to 50 $\mu$M drugs after 5-day treatment. The percentages of residual live cells were determined by SYBR Green I/PI assay;

FIG. 9 shows the screening of a FDA-approved drug library (2,000 compounds) on stationary phase *Borrelia* persisters. *In vitro* activity of some effective antibiotics against stationary phase *B. burgdorferi* (cultured for 7 days) is shown;

FIG. 10A, FIG. 10B, FIG. 10C, and FIG. 10D show representative images of the stationary phase of *B. burgdorferi* strain B31treated by: (FIG. 10A) daptomycin; (FIG. 10B) cefoperazone; (FIG. 10C) tetracycline; and (FIG. 10D) drug-free control. Treatment was followed by staining with SYBR Green I/PI;

FIG. 11 shows representative images of the stationary phase of *B. burgdorferi* strain B31 treated by carbomycin (left) and clofazimine (right);

FIG. 12 shows antibiotic minimum inhibitory concentrations (MICs) of some persister-active antibiotics for *B. burgdorferi* strain B31;

FIG. 13A, FIG. 13B, and FIG. 13C show representative images of: (FIG. 13A) 3-day-old log; (FIG. 13B) 7-day-old stationary; and (FIG. 13C) 10-day-old stationary phase *B. burgdorferi* cultures. The *B. burgdorferi* cultures of varying ages were stained with SYBR Green I/PI assay and observed under the microscope (400 $\times$ magnification). The arrows indicate the spirochete (s), round body (r), and microcolony (m) forms of *B. burgdorferi* in stationary phase cultures;

FIG. 14 shows the effect of drugs (50 μg/mL) and combinations on stationary phase *Borrelia.* Susceptibility of stationary phase *B. burgdorferi* to drugs alone and their combinations after 5 days treatment. G/R: Green/Red ratio. Bracketed values: microscope counting percentages of residual viable cell. Dox: doxcycline; Amox: amoxillin; Cef-P: cefoperazone; Cef-T:ceftriaxone; MTZ: metronidazone; CFZ: clofazimine; MCZ: miconazole; PMB: polymyxin B;

FIG. 15 shows representative drug combinations against *Borrelia* biofilm. Images captured with epi-fluorescence inverted microscope (20X magnification). Drug concentration, 50 μg/mL;

FIG. 16 shows the activity of representative drug combinations against *Borrelia* biofilm. Fluorescence intensity and area of image were calculated by Image Pro Plus software;

FIG. 17A and FIG. 17B show the effect of antibiotics alone and in combinations on aggregated microcolony form and planktonic forms of *B. burgdorferi.* Stationary phase *B. burgdorferi* culture (10-day old) was treated with 10 μg/mL drugs (labeled on the image) for 7 days followed by staining by SYBR Green I/PI assay. Green cells indicate live cells whereas red cells indicate dead cells: (FIG. 17A) the *B. burgdorferi* aggregated microcolony (MC) form was more resistant to different antibiotics or their combinations than the planktonic form (round body and spirochetal form) (PT) as observed by fluorescence microscopy at 400 × magnification; and (FIG. 17B) susceptibility of the *B. burgdorferi* microcolony form to antibiotics and antibiotic combinations was assessed by fluorescence microscopy at 200 × magnification. The luminance of an individual RB is much weaker than that of a microcolony, which made the individual cells hard to observe when the microcolonies were being examined. Abbreviation: Dox, doxycycline; CefP, cefoperazone; Cfz, clofazimine; Dap, daptomycin; Smx, sulfamethoxazole; Cab, carbencillin; Car, carbomycin;

FIG. 18A, FIG. 18B, FIG. 18C, FIG. 18D, FIG. 18E, FIG. 18F, FIG. 18G, FIG. 18H, FIG. 18I show subculture (15 days) of 10-day-old *B. burgdorferi* stationary phase culture treated with different antibiotics alone or in combinations. Representative images were taken with fluorescence microscopy (400 × magnification) using SYBR Green I/PI staining. Only Dox+Dap+CefP completely killed all forms including the microcolony form *of B. burgdorferi* persisters as shown by lack of any viable green spirochetal form after 15 day subculture. Abbreviation: Dox, doxycycline; CefP, cefoperazone; Cfz, clofazimine; Dap, daptomycin; Smx, sulfamethoxazole;

FIG. 19A, FIG. 19B, FIG. 19C, FIG. 19D show microscopy demonstrating round body formation in the presence of amoxicillin and subsequent reversion to spirochetal form of *B. burgdorferi* during subculture: (FIG. 19A) 5-day old *B. burgdorferi* culture consisting primarily of spirochetal form; (FIG. 19B) coccoid round body forms formed from *B. burgdorferi* spirochetes upon treatment with amoxicillin (50 μg/mL) for 3 days; (FIG. 19C) reversion of round body form of *B. burgdorferi* from (FIG. 19B) to spirochetal form after 5 day subculture in fresh BSK-medium; and (FIG. 19D) 7-day old stationary phase *B. burgdorferi* treated with 100 μg/mL amoxicillin for 3 days;

FIG. 20 shows exposure of 5-day old spirochetes and amoxicillin-induced round body form of *B. burgdorferi* (5 days) to 50 μM doxycycline, cefuroxime, and ceftriaxone for 5 days. The percentages of residual live cells were determined by SYBR Green I/PI assay followed by fluorescence microscopy counting;

FIG. 21A, FIG. 21B, FIG. 21C, FIG. 21D, FIG. 21E, FIG. 21F, FIG. 21G, FIG. 21H, FIG. 21I show representative images of amoxicillin-induced round body form of *B. burgdorferi* (6- day old culture induced with 50 μg/mL amoxicillin for 72 hours) treated with different antibiotics (50 μM) for 7 days followed by staining with SYBR Green I/PI assay and fluorescence microscopy;

FIG. 22A, FIG. 22B, FIG. 22C, FIG. 22D, FIG. 22E, FIG. 22F, FIG. 22G, FIG. 22H, FIG. 22I, FIG. 22J, FIG. 22K, FIG. 22L, FIG. 22M, FIG. 22N, FIG. 22O, FIG. 22P show the effect of antibiotics alone or in combinations on stationary phase *B. burgdorferi* microcolonies. Stationary phase culture of *B. burgdorferi* (10-day old) was treated with 10 μg/mL drugs alone or in combinations (labeled on the image) for 7 days followed by staining with SYBR Green I/PI assay. Green cells indicate live cells whereas red cells indicate dead cells. Abbreviation: Dox, doxycycline; CefP, cefoperazone; Art, Artemisinin; Dap, daptomycin; CefM, cefmetazole; Scp, sulfachlorpyridazine;

FIG. 23A, FIG. 23B, FIG. 23C, FIG. 23D, FIG. 23E, FIG. 23F, FIG. 23G, FIG. 23H, FIG. 23I show subculture (20 days) of the amoxicillin-induced round body form of *B. burgdorferi* (6-day old culture induced with 50 μg/mL amoxicillin for 72 hours) treated with different antibiotics alone or in combinations. Representative images were taken with fluorescence microscopy using SYBR Green I/PI staining. Only Dox+Dap+CefP completely killed all round body form of *B. burgdorferi* persisters as shown by lack of any viable green spirochetal form after 20-day subculture (FIG. 23G). Abbreviation: Dox, doxycycline; CefP, cefoperazone; Dap, daptomycin; Art: artemisinin; Scp, sulfachlorpyridazine;

FIG. 24 shows representative images of stationary phase *B. burgdorferi* treated with different compounds (50 μM) followed by staining with SYBR Green I/PI assay. Abbreviation: DOX: doxycycline, AMO: amoxicillin, DAP: daptomycin, DAU: daunomycin, NOG: nogalamycin, PYR: pyrromycin, RHO: Rhodomycin A, CHA: chaetochromin, PRO: prodigiosin, MIT: mitomycin, NAN: nanaomycin, DAC: dactinomycin, EMO: emodin;

FIG. 25 shows representative images of stationary phase *B. burgdorferi* strain B31 treated with different compounds (20 μM) followed by staining with SYBR Green I/PI assay. Abbreviation: DOX: doxycycline, DAP: daptomycin, DAU: daunomycin, NOG: nogalamycin, PYR: pyrromycin, RHO: Rhodomycin A, CHA: chaetochromin, PRO: prodigiosin, NAN: nanaomycin;

FIG. 26 the percentage of live cells and the green/red fluorescence ratio from the SYBR Green I/PI viability assay. Known proportions of isopropyl killed (30 minutes) and live (A) *Staphylococcus aureus* (USA300), (B), *Klebsiella pneumoniae* (Isolate 7), (C) *Escherichia coli* (W3110 (D) *Acinetobacter baumannii* and (E) *Mycobacterium tuberculosis* (H37Ra) were stained with SYBR Green I/PI and measured using a fluorescence plate reader. A linear regression line was determined. (F) Fluorescence microscopy image showing known proportions (0%, 50%, 100%) of live (i-iii) *Staphylococcus aureus* (USA300) and (iv-vi) *Klebsiella pneumoniae* (Isolate 7) stained with SYBR Green I/PI reveal red (dead) and green (live) ratios in concordance with the known proportions of live and killed organisms from representative Gram-positive (*S. aureus*) and Gram-negative (*K. pneumoniae*) bacteria.

FIG. 27A, FIG. 27B, FIG. 27C, FIG. 27D, FIG. 27E, and FIG. 27F show the results of *S. aureus* USA300 strain (FIG. 27A, FIG. 27B, and FIG. 27C) and *K. pneumoniae* Isolate 7 strain (FIG. 27D, FIG. 27E, and FIG. 27F) observed with fluorescence microscopy after SYBR Green/PI staining of different viable proportions. Live cells are stained in green and dead cells treated with 70% isopropyl alcohol for 1 hr are stained in red;

Fig. 28. SYBR Green I/PI can monitor the dynamics of resistant and sensitive strains during antibiotic drug exposure. After following the antibiotic susceptibility testing protocol for broth microdilution methods as described in Clinical and Laboratory Standards Institutes, application of SYBR Green/PI staining to the bacterial cultures showed that both SYBR Green/PI and OD600 reading indicate that the MIC of chloramphenicol against (A) S. *aureus* USA300 is 8 wing the antibiotic susceptibility testing pr*E. coli* W3110 is 4 8 wing the antibiotic susceptibility testing protocol for broth microdilution meover the course of 1.5 hrs revealed (C) *S. aureus* strain Newman was sensitive to kanamycin compared to resistant strain CA127 and (D) *E. coli* strain W3110 sensitive to ampicillin showed significant decrease in the green/red fluorescence ratio as early as 30 minutes compared to resistant strain KTE181.

FIG. 29 shows the antimicrobial susceptibility testing results for *S. aureus* strains as determined by the traditional Kirby-Bauer Disk Diffusion method. Abbreviation: Gen, gentamicin; Kan, kanamycin; Tet, tetracycline; Ery, erythromycin; Rif, rifampicin; Cip, ciprofloxacin; Cm, chloramphenicol; --, did not test; S, sensitive; R, resistant; IR, intermediate resistance;

FIG. 30 shows that the SYBR Green/PI stain can distinguish between R (resistant) and S (sensitive) strains of *S. aureus* against various antibiotics in one and a half hours. Treatment of varying concentrations of bacteriostatic and bactericidal antibiotics (0 μg/mL, 5 μg/mL, 30 μg/mL, 50 μg/mL, and 100 μg/mL) were added to overnight *S. aureus* samples diluted to 1:500 in tryptic soy broth. After incubation with antibiotics for 1.5 hours, SYBR Green/PI staining was performed and distinguished the strains and their respective susceptibility categories. All susceptibility results were in concordance with results from the Kirby-Bauer disk diffusion test;

FIG. 31 shows the quantitative cut-off that can be used to distinguish susceptible and resistant strains of *S. aureus.* Using the green and red fluorescence values determined from the fluorescence microtiter plate reader, the proportion of killed cells was calculated with the formula $(LD_{treated} - LD_{untreated})/ LD_{untreate}$, where LD is equal to the ratio of live (green fluorescence) and dead (red fluorescence) cells with treatment of 100 μg/mL of the respective drugs. The cut-offs distinguishing resistant and susceptible strains for the different antibiotics tested were established by averaging all the values (i.e. proportion of killed cells) from the respective susceptibility categories for all susceptible and resistant strains that were tested;

FIG. 32 shows that the SYBR Green/PI stain can distinguish between R (resistant) and S (sensitive) strains of *S. aureus* against various antibiotics in 30 minutes. Treatment of varying concentrations of bacteriostatic and bactericidal antibiotics (0 μg/mL to 400 μg/mL) were added to overnight *S. aureus* samples diluted to 1:25 in tryptic soy broth. After incubation with antibiotics for 30 minutes, SYBR Green/PI staining was performed and distinguished the strains and their respective susceptibility categories. All susceptibility results were in concordance with results from the Kirby-Bauer disk diffusion test;

FIG. 33 shows the antimicrobial susceptibility testing results for *E. coli* K-12 lab strain (W3110) and *E. coli* clinical strains as determined by the traditional Kirby-Bauer Disk Diffusion method. Abbreviation: Cm, chloramphenicol; Cip, ciprofloxacin; Trim, trimethoprim; Gen, gentamicin; Kan, kanamycin; Tet, tetracycline; Amp, ampicillin; Sm, streptomycin; S, sensitive; R, resistant; IR, intermediate resistance;

FIG. 34 shows that SYBR Green/PI stain can distinguish between R (resistant), IR (intermediate resistant), S (susceptible) strains of *E. coli* against various antibiotics in 1.5 hr. Treatment of varying concentrations of bacteriostatic and bactericidal antibiotics (0 μg/mL, 5 μg/mL, 30 μg/mL, 50 μg/mL, and 100 μg/mL) were added to overnight *E. coli* samples diluted to 1:500 in tryptic soy broth. After incubation with antibiotics for 1.5 hours, SYBR Green/PI staining was performed and distinguished the strains. All the susceptibility results were in concordance with results from the Kirby-Bauer disk diffusion test;

FIG. 35 shows the quantitative cut-off that can be used to distinguish susceptible and resistant strains of *E. coli.* Using the green and red fluorescence values determined from the fluorescence microtiter plate reader, the proportion of killed cells was calculated with the formula $(LD_{treated} - LD_{untreated})/ LD_{untreated}$, where LD is equal to the ratio of Live (green fluorescence) and Dead (red fluorescence) cells. The cut-offs distinguishing resistant and susceptible strains for the different antibiotics at the designated concentrations were established by averaging all the values

(i.e. proportion of killed cells) from the respective susceptibility categories for all susceptible and resistant strains that were tested. N.D = not determined;

FIG. 36 shows that SYBR Green/PI stain can distinguish between R (resistant) and S (susceptible) strains of *E. coli* against various antibiotics in 30 minutes. Treatment of varying concentrations of bacteriostatic and bactericidal antibiotics (0 μg/mL, 25 μg/mL, 50 μg/mL, 100 μg/mL, and 200 μg/mL) were added to overnight *E. coli* samples diluted to 1:25 in tryptic soy broth. After incubation with antibiotics for 30 minutes, SYBR Green/PI staining was performed and distinguished the strains. All the susceptibility results were in concordance with results from the Kirby-Bauer disk diffusion test;

FIG. 37 shows that SYBR Green/PI stain can distinguish between R (resistant) and S (susceptible) strains of *K. pneumoniae* against various antibiotics in 30 minutes. Treatment of varying concentrations of antibiotics (0 μg/mL, 25 μg/mL, 50 μg/mL, 100 μg/mL, and 200 μg/mL) were added to overnight *E. coli* W3110 and *K. pneumoniae* (Isolate 7) samples diluted to 1:25 in tryptic soy broth. After incubation with antibiotics for 30 minutes, SYBR Green/PI staining was performed and distinguished the strains. All the susceptibility results were in concordance with results from the Kirby-Bauer disk diffusion test;

FIG. 38 shows that the SYBR Green/PI stain can detect a similar trend in the number of *E. coli* persisters surviving antibiotic exposure compared to traditional colony forming unit (CFU) enumeration. The decrease in the level of persisters after exposure with the combination of polymycin B and gentamicin starting at Day 3 can be detected by both CFU enumeration and also green/red fluorescence ratio from SYBR Green/PI staining;

FIG. 39 shows linear relationship between *M. tuberculosis* H37Ra viability and Green/Red fluorescence ratio of the SYBR Green I/PI assay. Emission spectra of suspensions of various proportions of live and isopropyl alcohol-killed *M. tuberculosis* H37Ra were obtained, and the Green/Red fluorescence ratios were calculated for each proportion of live/dead cells. The line is a least-square fit of the relationship between percentage of live bacteria and Green/Red fluorescence ratio performed as described;

FIG. 40 shows representative images of *M. tuberculosis* H37Ra WT and PZA resistant mutants P5, P2 culture (20 day old), observed with fluorescence microscopy using SYBR Green I/PI stain. H37Ra WT and PZA resistant mutants were treated with 2 mg/mL PZA overnight (b, f, j). Salicylic acid (c, g, k) and acetic acid (d, h, 1) were used as enhancer to help increase the activity of PZA; and

FIG. 41 Drug susceptibility testing of M. tuberculosis against first-line tuberculosis drugs using SYBR Green I/PI assay. (A) M. tuberculosis H37Ra and INH resistant mutants I2, I4 (10 day old) were treated with 10, 100, 500 and 1000 f bactericidal drugs kanamysistant mutants I2 and I4 were determined to be resistant using 500 and 1000 μg/ml INH. (B) M. tuberculosis parental strain H37Ra and PZA resistant mutants (P5 and P2) (20 day old) were treated overnight with PZA (2 mg/ml). Treatment with salicylic acid (40 μg/ml) and acetic acid (8 μM) strongly increased the efficacy SYBR Green I/PI in detecting PZA resistance in mutants P5 and P2 compared to parental strain H37Ra. (Student's t-test, * = $p < 0.05$, ** = $p < 0.005$)

FIG. 42. SYBR Green I/PI assay reveals killing of bactericidal and bacteriostatic antibiotics in 30 minutes. SYBR Green I/PI assay reveals killing between sensitive and resistant strains after treatment with increasing concentrations of bactericidal drugs kanamycin for S. aureus (A) and ampicillin for E. coli (B). Upon treatment with increasing concentrations of bacteriostatic drugs erythromycin for S. aureus (C) and trimethoprim for E. coli (D), SYBR Green I/PI assay was still able to tell sensitive from resistant strains when administered at different doses for both sensitive and resistant strains. (Studentf bactericidal and bacteriostatic antibiotics in 30 minutes. SYBR Green I/PI assay reveals killing between sensitive and re

FIG. 43. Representative images of active drugs identified in PZA drug combination screen on a 20 day old stationary phase *M. tuberculosis* H37Ra culture at acid pH 5.5. A 20 day old *M. tuberculosis* H37Ra stationary phase culture diluted in acid pH 5.5 7H9 medium without ADC to $10^{6-7}$ bacilli/ml was treated with 50 μM drugs (labeled on the image) combined with 200 μM PZA for 7 days followed by staining with SYBR Green I/PI assay and epifluorescence microscopy (40 × magnification). Green cells indicate live cells whereas red cells indicate dead cells.

DETAILED DESCRIPTION

The presently disclosed subject matter now will be described more fully hereinafter with reference to the accompanying Figures, in which some, but not all embodiments of the presently disclosed subject matter are shown. Like numbers refer to like elements throughout. The presently disclosed subject matter may be embodied in many different forms and should not be construed as limited to the embodiments set forth herein; rather, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions and the associated Figures. Therefore, it is to be understood that the presently disclosed subject matter is not to be limited to the specific embodiments disclosed and that modifications and other embodiments are intended to be included

within the scope of the appended claims.

I. METHODS FOR IDENTIFYING INHIBITORY ACTIVITY AND DRUG SUSCEPTIBILITY IN EUKARYOTIC AND PROKARYOTIC CELLS INCLUDING ANTI-PESISTER ACTIVITY AND ANTIMICROBIAL ACTIVITY IN *BORRELIA BURGDORFERI* AND OTHER BACTERIA

The presently disclosed subject matter relates to methods for assessing the viability of cells, methods for assessing the susceptibility of cells to drugs or candidate drugs such as candidate antimicrobial agents, methods for screening for at least one agent that inhibits the growth or survival of cells, methods for inhibiting the growth and/or survival of cells, and related compositions and kits that can be used to perform or implement the methods. As discussed in more detail elsewhere herein, the cells may be of eukaryotic or prokaryotic origin and may, for example, be bacteria which may be replicating or non-replicating cells. Non-limiting examples including replicating and/or non-replicating persister forms of *B. burgdorferi, Staphylococcus aureus, Escherichia coli, Klebsiella pneumoniae, Acinetobacter baumannii,* and *Mycobacterium tuberculosis.*

One aspect of the invention provides a method for assessing the viability or susceptibility of cells to at least one drug or test agent. The cells may be cells of eukaryotic or prokaryotic origin. The cells may be from a selected organism which may be a single-celled or multicellular organism. By way of example, the cells may be from a single individual and may for example be cells from a single human (e.g. a cancer patient, such as cells from a biopsy or tumor of said patient), or from a collection or group of individuals (e.g. a group of individuals suffering from a common disease or disorder such as cancer or a disease or disorder mediated by an infectious agent such as bacteria or other microbes). Cells from a selected organism may include microbial cells such as bacteria or yeast cells and accordingly in some embodiments the selected organism is bacteria and there is therefore accordingly provided a method for assessing the drug susceptibility of bacterial cells which may optionally be pathogenic bacteria. The selected organism may be bacteria of a selected genus such as *Staphylococcus, Escherichia, Klebsiella, Acinetobacter or Mycobacterium tuberculosis* and the selected organism may, for example, be *Staphylococcus aureus, Escherichia coli, Klebsiella pneumoniae, Acinetobacter baumannii,* and *Mycobacterium tuberculosis. In some embodiments the bacteria may be a bacterial isolate or sub-species (e..g from one of the aforementioned species)*. Optionally, where the cells are bacteria or other microbes or infectious agents the cells may originate (including be derived from) a single individual or host or from multiple individuals or hosts.

A cell culture comprising the cells to be tested is obtained or established. The cells may be isolated cells such as isolated cells from a selected organism (e.g. bacteria) which may optionally be from a selected species or genus. Accordingly in one embodiment the method is a method for assessing the drug susceptibility of cells from a selected organism wherein the method comprises obtaining or establishing a culture comprising isolated cells from the selected organism e.g. bacterial cells such as the above-mentioned bacterial species and genera, or as otherwise mentioned herein, or other cells of prokaryotic or eukaryotic origin including, for example, yeast cells, fungal cells, parasite cells, or cancer cells.

The methods of the present invention may further comprise incubating the cells with a staining mixture comprising: (i) a first agent which emits fluorescence of a first color that is indicative of live cells in the culture, and (ii) a second agent which emits fluorescence of a second color that contrasts from the first color and is indicative of dead cells in the culture. In some embodiments the first fluorescent compound is, for example, SYBR Green I that identifies live cells, and the second fluorescent compound is, for example, propidium iodide (PI) that identifies dead or non-viable cells.

The methods of the present invention may be used to assess drug susceptibility or to screen one or more test agents for inhibitory activity. Accordingly, the methods of the present invention may comprise the step of exposing or contacting the cells or organisms to be assayed with at least one dose of at least one drug or test agent. The cells (or organisms e.g. bacteria) are then typically incubated with the at least one drug or test agent.

An important feature of the methodology of the present invention is that cells may be incubated without the requirement for growth of the cells or organism. Accordingly, the cells may be incubated under non-growth conditions and do not require (and may therefore omit) extended culturing or incubation times to allow for growth of the cells or organism; this greatly increases the rapidity of the present methods and assays. Accordingly, in some embodiments the methods of the present invention comprise the step of incubating the cells with a drug or agent that kills or inhibits the growth of the cells / organism for a period of time without the requirement for organism growth. The period of time may range from minutes to hours or days depending on the organism. The drug(s) or agent(s) being tested may also influence the period of time due to e.g. the rate of drug uptake, the rate of drug action, and the metabolic status of the cell.

In preferred embodiments the period of incubation of the cells with the drug or agent (which may be a combination of drugs or agents) is less than about 17 hours, 16 hours, 15 hours, 14 hours, 13 hours, 12 hours, 11 hours, 10 hours, 9 hours, 8 hours, 7 hours, 6 hours, 5 hours, 4 hours, 3 hours, 2 hours or 1 hour but is optionally at least about 5, 10, 15, 20, 25, 30, 35, 40, 45 or 50 minutes.

In some embodiments the period of incubation may be less than about 55, 50, 45, 40, 35, 30, 25, 20, 15, 10, or 5 minutes. In some embodiments the period of incubation is in the range of about 5 to 10, 5 to 15, 5 to 20, 5 to 25, 5 to 30, 5 to 35, 5 to 40, 5 to 50 or 5 to 60 minutes; about 10 to 15, 10 to 20, 10 to 25, 10 to 30, 10 to 35, 10 to 40, 10 to 50 or 10 to 60 minutes; about 15 to 20, 15 to 25, 15 to 30, 15 to 35, 15 to 40, 15 to 50 or 15 to 60 minutes; about 25 to 30, 25 to 35, 25 to 40, 25 to 50 or 25 to 60 minutes; about 5, 10, 15, 20, 25, 30, 35, 40, 50 or 60 minutes to 1.5 hours; 5, 10, 15, 20, 25, 30, 35, 40, 50 or 60 minutes to 2 hours; 5, 10, 15, 20, 25, 30, 35, 40, 50 or 60 minutes to 3 hours; 5, 10, 15, 20, 25, 30, 35, 40, 50 or 60 minutes to 4 hours; 5, 10, 15, 20, 25, 30, 35, 40, 50 or 60 minutes to 6 hours; 5, 10, 15, 20, 25, 30, 35, 40, 50 or 60 minutes to 8 hours; 5, 10, 15, 20, 25, 30, 35, 40, 50 or 60 minutes to 10 hours; 5, 10, 15, 20, 25, 30, 35, 40, 50 or 60 minutes to 12 hours; 5, 10, 15, 20, 25, 30, 35, 40, 50 or 60 minutes to 14 hours; 5, 10, 15, 20, 25, 30, 35, 40, 50 or 60 minutes to 15 hours; 5, 10, 15, 20, 25, 30, 35, 40, 50 or 60 minutes to 16 hours; or 5, 10, 15, 20, 25, 30, 35, 40, 50 or 60 minutes to 17 hours. As mentioned herein, the methods of the present invention may advantageously be used for real-time and continuous monitoring for both drug susceptibility testing and drug screens. This enables real time data to be obtained and enables the more rapid identification of sensitive or resistant cells or organisms.

Accordingly, the period of incubation can be a real-time continuous period, such as from 5 minutes to 16 hours, rather than a limited single time point, for all cells.

The incubation with the drug / agent (e.g. test agent such as where a library of compounds is screened to identify inhibitory activity) may be performed at the same time as incubating the fluorescent agents (i.e. the staining mixture) or afterwards. The steps of incubation with the staining mix and the drug / agent may be performed simultaneously, separately or sequentially and may therefore be overlapping or non-overlapping. Optionally, the method further comprises determining a breakpoint (cut off) or range for the at least one drug.

The methods of the present invention may employ higher drug concentrations than the normal or usual drug concentrations needed for inhibiting organism / cell growth or survival in the current conventional drug susceptibility tests and screens. The typical drug concentrations that are found to distinguish susceptible and resistant cells using the novel growth-independent SYBR Green I/PI methodology described herein range from 10-10,000 fold of conventional growth based MIC (minimum inhibitory concentration). The high drug concentrations required are necessary to achieve the short time needed to determine the susceptibility or resistance of a particular strain or cell in comparison with sensitive and resistant controls. Multiple drug concentrations within the above range may also be used to rapidly determine the drug susceptibility of the cells being assayed. Accordingly, n a preferred embodiment of the methods described herein the cells are incubated with the at least one drug or test agent at a concentration range from 10-10,000 fold of conventional growth based MIC. In some embodiments the concentration range may be from 15-7500 fold; 20-5000; 50-2500 fold of conventional growth based MIC. In some embodiments the concentration range may be at least 10, 50, 100, 250, 500, 1000, 2500, 5000, 7500 fold of convention growth based MIC.

In some embodiments of the methods of the present invention, the method is a method for assessing the susceptibility of cells to at least one drug wherein step (b) comprises: (b)(i) incubating the cells with at least one dose of said at least one drug for a period of exposure without the need for growth of said cells; and (b)(ii) incubating the drug-treated cells with said staining mixture. As mentioned above, incubation of the cells is growth independent and therefore the cells may be incubated under non-growth conditions thereby enabling the methods of the invention to be performed at a much greater speeds than methods which require a period of growth of the cells.

In some embodiments of the methods of the present invention, the method is a method for assessing susceptibility of cells to at least one test agent wherein step (b) comprises: (b)(i) incubating the cells with at least one dose of said at least one test agent for a period of exposure without the need for growth of said cells; (b)(ii) incubating the test agent-treated cells with said staining mixture. Such a method may be used as a drug screen, the drug screen may suitably be used to identify compounds or drug candidates (candidate agents) that are capable of inhibiting growth or survival of cultured or growing cells as well as for non-growing dormant or persister cells of prokaryotic or eukaryotic origin. In some embodiments of the methods described herein, the method includes conducting a microscopic counting rescreen to confirm that at least one test agent is a candidate agent for inhibiting growth or survival of the cells. As mentioned above, incubation of the cells is growth independent and therefore the cells may be incubated under non-growth conditions thereby enabling the methods of the invention to be performed at a much greater speeds than methods which require a period of growth of the cells.

As mentioned above, the steps of incubation with the staining mix and the drug / agent may be performed simultaneously, separately or sequentially and and therefore steps (b)(i) and (b)(ii) may be performed together or separately and may be overlapping or non-overlapping. Where steps (b)(i) and (b)(ii) are performed separately or sequentially then the cells are exposed to the at least one drug or test agent first and then the cells are exposed to the staining mixture. Accordingly, the methods may comprise exposing the cells to the at least one drug or test agent first and then after said exposure adding the first and second fluorescent agents (e.g. SYBR Green I and the propidium iodide). Thus, the first and second fluorescent agents (e.g. SYBR Green I and the propidium iodide) can be used after drug /test agent exposure or together with drug /test agent exposure over a period of time and, as discussed elsewhere herein, optionally

in a continuous, real time and/or high throughput manner.

In at least some embodiments, the methods and drug screens of the present invention can be continuous over a period of time. This is different from conventional drug screens of a defined time- or end-point, e.g. 3 day or 7 day, but with the new methods described herein drug susceptibility or drug action in drug screens can be assessed at multiple or differing time points, in a continuous, real-time and/or high throughput manner. Accordingly, in some embodiments a method of the present invention may comprise taking readings of the fluorescence at multiple time points so that drug susceptibility of the cells is assessed at different time points: this may allow for the effects of a drug or test agent (including combinations of drugs or test agents) to be monitored over time as well as allowing for the testing or identification of drug candidates sequentially over a period of time ranging from hours, days and weeks.

In some embodiments of the present invention, the methodology of the present invention may advantageously be used for real-time continuous monitoring in DST with the present invention allowing one to monitor drug sensitivity or resistance to a test drug or agent in a continuous real-time manner. As mentioned above, the current DST and drug screens typically use a fixed time point for assessing the results, and the advantage of the novel methodology described herein (e.g. the novel SYBR Green I/PI methodology) allows the DST to be done in a more quantitative and more real-time continuous manner. This enables the identification of susceptible or resistant cells (e.g. strains) in extremely rapid time e.g. in as little as 5 to 30 minutes.

In some embodiments of the present invention, the methodology of the present invention may be used to provide for real-time continuous monitoring and identification of drug candidates in drug screens. The current drug screens are typically done with a fixed time point, which produce very limited information and small number of hits or drug candidates. With the novel methodology described herein (e.g. the novel SYBR Green I/PI methodology), one is able to identify multiple active "hits" without having to terminate the drug screening experiment. This enables the identification of different hits at different time points within a defined time period, typically ranging from minutes to hours to days or weeks as indicated above, in a continuous, real time and/or high throughput manner. This will be important for identifying drugs that target different cell populations with different metabolic status so as to identify drug candidates that can inhibit or kill the very heterogeneous population of cells of prokaryotic or eukaryotic origin.

Incubating the cell culture in step (b) preferably comprises incubating the cell culture under suitable conditions. Suitable conditions may comprise incubating the cells at an appropriate cell density. An appropriate cell density may be defined by the sensitivity of the fluorescence viability assay (e.g. in the range of 101-9 cells/ml, 102-9 cells/ml, 103-9 cells/ml, 104-9 cells/ml, 105-9 or 106-9 cells/ml, 101-8 cells/ml, 102-8 cells/ml, 103-8 cells/ml, 104-8 cells/ml, or 105-8 cells/ml) for drugs and test agents tested and the fluorescent indicator dyes used. The present invention provides growth-independent assays or methods. Accordingly, incubating step (b) can be performed without the need for cell growth and such growth may therefore be omitted from the methods of the present invention. The growth independent nature of the assays described herein substantially reduces the time from days and weeks needed for current drug susceptibility testing to extremely short time of just minutes or hours. The growth independent nature of the assays described herein substantially reduces the time needed for current growth based drug screens to extremely short time of just minutes or hours. Since the methods of the present invention are growth independent it will accordingly be understood that incubation of the cells under suitable conditions need not require growth conditions and therefore the suitable conditions may comprise growth or non-growth conditions.

In some embodiments of the methods of the present invention, the step of incubating the culture with the staining mixture and/or drug or test agent is performed for a period between about 5 to 30 minutes; for a period between about 10 to 20 minutes; or for a period of approximately 15 minutes. Optionally, the step of incubating with the staining mixture and/or drug or test agent is performed in the dark.

In some embodiments of the present disclosure, including the methods and kits disclosed herein, the first color emitted by the first fluorescent agent is green and the second color as emitted by the second fluorescent agent is red or orange. In at least some embodiments, the first agent is SYBR Green I and/or the second agent is propidium iodide. Suitably, a non-toxic concentration of the first and second fluorescent agents may be chosen whilst still achieving adequate staining results. Thus, the first and second fluorescent dyes SYBR Green I/PI can be used at an appropriate concentration that is non-toxic while still staining live cells green for SYBR Green I or dead cells red for PI. In this way the fluorescent agents or dyes (the terms may be used interchangeably) can be added simultaneously with the drug or test agent together for real-time continuous monitoring of drug susceptibility or drug screens in a dynamic manner to obtain more quantitative data on drug susceptibility and in drug screens. Alternatively, the SYBR Green I/PI dyes can be added after drug exposure or incubation for determining the results of sensitivity or resistance of the organism in DST or the activity of the drug or compound in real-time drug screens.

In some embodiments of the present invention, the SYBR Green I is present in the culture in a concentration range of between about 0.1x and about 100x and propidium iodide is present in the culture in a range of between about 0.1 mM and about 5 mM. In still further embodiments, the concentration of SYBR Green I in the culture is about 10x and the concentration of propidium iodide is about 2mM. The concentrations of SYBR Green I and propidium iodide can vary depending on the particular organism or cell types or in DST or in drug screens, or when used simultaneously or

sequentially with drugs or test agents. In addition, the incubation time with the staining mix can vary from typically 10-60 minutes up to hours or days depending on the particular organism or cell types or in DST or in drug screens, or when used simultaneously or sequentially with drugs or test agents.

In some embodiments of the methods of the present invention, the fluorescence intensity of the culture and staining mixture is determined at 535 nm, which measures green fluorescence, and 635 nm, which measures red fluorescence.

The methodology provided by the present invention is growth-independent which is completely distinct from the current growth based DST. Thus, in the methodology of the present invention the cells may be incubated without the need for the test cells / test organism to grow. This may substantially reduce the time, from days and weeks needed for current drug susceptibility testing, to extremely short times of just minutes or hours with the actual time required depending upon the incubation conditions, the cells used and the drugs tested. Where the effects of multiple drugs or test agents are assessed on a continuous basis then the total incubation time may be increased accordingly so as to allow for the assessment of drug susceptibility or drug action and identification of multiple drug candidates over time in real-time on a continuous or sequential basis.

The growth-independence of the methods described herein also allows a much wider variety of cells to be assayed then conventional drug screens or DSTs since the methods of the present invention can be applied to non-growing or non-replicating cells such as dormant or persister cells from different pathogenic microbes of prokaryotic and eukaryotic origin, such as persister cells of pathogenic bacteria including mycoplasma, chlamydia, spirochetal organisms, anaplasma, ehrlichia, rickettsia, coxiella, bartonella, fungi, yeasts, parasites (babesia, malaria, toxoplasma etc), as well as other cells which cannot be grown in culture or can only be grown with difficulty or isolated directly from host tissues without culture as in cancer tissues from patients. The methods of the present invention may therefore find particular utility as regards cell types which are typically hard to assay for drug susceptibility or to use in drug screens using conventional methods. In addition to persister cells, the methods of the present invention may particularly lend themselves to cells which cannot be grown in culture (or cannot readily be grown in culture) and to slow growing cells such as various *Mycobacterium* spp. such as *M. tuberculosis, M. leprae,* and nontuberculous mycobacteria such as *M. avium, M. intracellulare, M. kansasii.* Further examples of cells in this context may include stem cells, pluripotent or multipotent cells, cancer cells, cancer stem cells, and fastidious cells.

The subject matter of the present disclosure provides a new algorithm of defining resistance and susceptibility using the methods described herein and the ratio of the intensity of emitted fluorescence of the first color to the intensity of emitted fluorescence of the second color (e.g. the ratio of SYBR Green and PI). for rapid drug susceptibility testing and also drug screens in real-time manner that are not case with the conventional and current methods, for all cells of prokaryotic and eukaryotic origin.

Subsequent to incubation, the methods of the present invention may comprise the step of calculating a ratio of the intensity of emitted fluorescence of the first color to the intensity of emitted fluorescence of the second color (e.g. the ratio of SYBR Green and PI). This ratio may then be used to assess the viability of the cells in the culture with the ratio being indicative of the percentage of live and dead cells in the culture. In this way the susceptibility of the cells to a drug or test agent may be assessed, such as in a rapid drug sensitivity test or rapid screen of test agents to identify one or more drug or candidate agents which may exhibit useful inhibitory activity. In preferred embodiments, a fluorescence reader may be linked to a computer with a software that will calculate the green/red fluorescence ratios of test organism, optionally with sensitive and resistant organisms as controls and drug free controls, to determine the susceptibility and resistance of a test organism.

In at least some embodiments of the present invention, the susceptibility (or resistance) of the cells may be assessed by comparing the viability of the cells in the culture in the presence of the at least one drug or test agent with the viability of cells in a control culture which lacks, or has not been exposed to, the at least one drug or test agent.

In at least some embodiments of the present invention, the cells are assessed as susceptible or resistant to the at least one drug or test agent according to whether the ratio in step (b) remains the same or decreases or increases after the period of exposure to the at least one dose of the at least one drug or test agent.

In at least some embodiments of the present invention, the cells are assessed as susceptible to the at least one drug or test agent if the ratio in step (b) remains the same or decreases after the period of exposure to the at least one dose of the at least one drug or test agent, and wherein the cells are assessed as resistant to the at least one drug or test agent if the ratio in step (b) increases after the period of exposure to the at least one dose of the at least one drug or test agent.

In at least some embodiments of the present invention, susceptibility or resistance is assessed by comparison of the ratio with a control, such as known resistant and/or known sensitive cells (e.g. a known resistant or sensitive bacterial strain), or a control which lacks, or has not been exposed to, the at least one drug or test agent.

In some embodiments of the methods described herein the cells are assessed as resistant or susceptible to the at least one drug or test agent by comparison to sensitive control cells and/or resistant control cells, for example based on a difference between the ratio of the fluorescence of the first agent over the fluorescence of the second agent

of the cells and that of sensitive control cells and/or a resistant control cells for the at least one dose of the at least one drug or test agent. Susceptibility or resistance may be assessed by comparing the ratios of the control cells of known sensitive and known resistant controls with and without the at least one test agent or drug to define susceptibility or resistance of a particular test strain to a particular test agent or drug. The test agent or drug may, for example, be any antibiotic currently used in treatment of infections caused by microbes (bacteria, fungi, protozoa, parasites, etc,) or could be any anticancer agent, antimicrobial agent, or antifungal agent etc.

Cells may be assessed as resistant to the at least one drug or test agent if the ratio in step (b) is increased or does not decrease in comparison with said sensitive control cells after the period of exposure to the at least one dose of the at least one agent, and the cells may be assessed as susceptible to the at least one drug or test agent if the ratio in step (b) is decreased or does not increase in comparison with said resistant control cells after the period of exposure to the at least one dose of the at least one agent.

In some embodiments a method of the present disclosure is performed in real time and/or in a high-throughput format. A high-throughput format is particularly envisaged in the case of drug susceptibility testing or drug screens which may suitably be used to identify agents with activity against growing and non-growing cells of prokaryotic and eukaryotic origin. In some embodiments, the high-throughput format uses at least one multi-well microplate. Non-limiting examples of suitable multi-well microplates include, without limitation, a 6-well microplate, a 24-well microplate, a 96-well microplate, a 384-well microplate, and a 1536-well microplate. In still other embodiments, the multi-well microplate comprises a 96-well microplate.

Unexpectedly, it has been found that the presently disclosed methods, in some embodiments, can be used without a washing step and for high-throughput screens. That is, the methods can be used to assess viability, susceptibility of cells to drugs and test agents, and in drug screening directly in a high-throughput real-time manner and/or in the absence of washing. Accordingly yet another advantage is that the methods described herein do not require a washing step after drug incubation to obtain results, as multiple readings (e.g. of plates) can be taken directly without a washing step. This not only saves time but also prevents loss of small numbers of cells during the washing step. Thus, in at least some embodiments of the methods described herein readings are taken directly without the incubated cells being first washed to remove drug to obtain results over a period of time in real-time manner

Yet another advantage which may be achieved with the methods described herein is that the cells do not need to be recultured or regrown after drug exposure before results can be obtained and, so consequently, in at least some embodiments of the present invention the cells are not recultured or regrown after drug incubation.

This novel methodology of the present invention allows more accurate and extremely rapid DST and more drug candidates to be identified in a real-time manner over a period of time without having to stop the experiment or introducing a washing step to remove drugs or test agents to allow for regrowth of the surviving cells in current growth based drug screens.

In some embodiments of the methods and kits described herein, the culture comprises a stationary phase culture which may optionally comprise non-replicating persister cells. In some embodiments, the stationary phase culture may comprise morphological variant forms selected from the group consisting of L-forms or cell wall defective forms, round bodies, planktonic and aggregated biofilm forms..

As used herein, the term "cell culture" or "culture" includes a reference to a population of cells in culture. Since the methods of the present invention are growth independent the culture conditions need not be conducive for growth and the cells in the culture need not be growing cells, and may therefore be growing or non-growing cells. The cell culture can be found in any type of container, such as a flask, a tube, a microwell plate, array, and the like. In some embodiments, the culture may be a cell suspension The cells may have different phases of growth such as a lag phase, log phase and stationary phase. In some embodiments, a cell culture in "stationary phase" means that the cells in the culture have an approximately equal growth rate and death rate. As used herein, the term "growing forms" of cells generally refers to cells that are in lag or log phase and not in stationary phase. In some embodiments, the stationary phase cell culture has been grown for approximately 7 days or more. In other embodiments, the stationary phase cell culture comprises non-replicating persister cells. By "non-replicating persister cells," it is meant cells that enter a state in which they stop replicating and exhibit tolerance to drugs, such as the ability to tolerate antibiotics in the case of bacteria. The term "culturable cells" includes a reference to cells which are capable of being maintained in culture (but which are not necessarily capable of growing or replicating in culture) and to cells which are capable of growing or replicating in culture; both of these cells types may be employed in the methodology of the present invention.

The methodology of the present invention is applicable to all prokaryotic and eukaryotic cells. The cells used in the methods and kits of the present invention may therefore be selected accordingly. The cells may be of prokaryotic (bacteria and archaea) or eukaryotic origin and may be selected from the group consisting of: bacteria; Gram-positive bacteria such as *Staphylococcus aureus*; Gram-negative bacteria such as *Escherichia coli, Klebsiella pneumoniae,* and *Acinetobacter baumannii*; mycobacteria such as *Mycobacterium tuberculosis*; bacteria from the *Borrelia* genus (e.g., replicating and/or non-replicating persister forms of *B. burgdorferi);* bacteria from a selected genus and wherein the selected genus is optionally selected from the group consisting of *Borrelia, Staphylococcus, Escherichia, Klebsiella,*

*Acinetobacter,* and *Mycobacterium*; microbial cells of eukaryotic or origin; eukaryotic cells such as fungal or yeast cells, plant cells, parasite cells, cancer cells; animal cells including but not limited to vertebrate cells and invertebrate cells, mammalian cells, mouse cells, rabbit cells, rat cells, guinea pig cells, fish cells, amphibians cells, reptile cells, bird cells, monkey cells, hamster cells, non-human primate (e.g. monkeys, apes, gibbons, chimpanzees, orangutans, macaques and the like) cells, and human cells; cancerous or infected animal cells; bovine cells (e.g., cattle, oxen, and the like); ovine cells (e.g., sheep and the like), caprine cells (e.g. goats and the like); porcine cells (e.g., pigs, hogs, and the like); equine cells (e.g., horses, donkeys, zebras, and the like); feline, canine, rodent or lagomorph cells; cells from animal disease models (e.g., rats or mice used in experiments, and the like); tumor cells; cells from a human or non-human animal suffering from a cell proliferative disorder e.g. a neoplastic disorder; cells of a pathogen, e.g., a multicellular pathogen or microorganism such as a pathogenic bacterium, pathogenic fungus, or a pathogenic protist (e.g., a Plasmodium cell); cells derived from a cell line e.g. CHO cells, HeLa cells. The cells may be replicating and/or non-replicating persister or dormant forms of any of the aforementioned cells; cancerous (e.g. breast cancer, prostate cancer, colon cancer, pancreatic cancer, lung cancer, leukemia etc), diseased, infected (e.g. with an antibiotic or antimicrobial refractory infection), or precancerous cells of any of the aforementioned cells. Also, included are cell-lines, stem cells, pluripotent, multipotent or oligopotent cells, cancer stem cells, cancer cells from cancer tissues of patients, fastidious cells and transformed cells of any of the aforementioned cells or cells otherwise described herein. In some embodiments the cells are not cancer cells. In other embodiments the cells are not bacterial cells. In some embodiments the cells are not microbial cells. In some embodiments the cells are not infectious or pathogenic organisms.

In some embodiments, the bacteria are spirochete organisms such as *Treponema spp. scuh as T. pallidum* and Borrelia spp. such as *Borrelia burgdorferi, B. afzelii, B. garinii, B. miyamotoi. Borrelia* is a genus of bacteria of the *Spirochete* phylum. The *Borrelia burgdorferi* sensu lato complex includes at least 18 genospecies. Non-limiting examples of bacteria in this genus include *B. burgdorferi, B. garinii, B. afzelii, B. americana, B. carolinensis, B. lusitaniae, B. japonica, B. miyamotoi* and *B. sinica.* In some embodiments, the bacteria are *Borrelia burgdorferi.* In some embodiments, the bacteria comprise replicating forms of *Borrelia burgdorferi,* non- replicating persister forms of *Borrelia burgdorferi,* and combinations of replicating forms of *Borrelia burgdorferi,* non-replicating persister forms of *Borrelia burgdorferi.* In some embodiments, the *Borrelia burgdorferi* comprise a morphological form selected from the group consisting of round bodies, planktonic, spirochete form, a spheroplast form (L-forms), a cystic or round body form, a microcolony form, a biofilm-like and biofilm form, and combinations thereof. In other embodiments of the invention the cells are preferably not growing *Borrelia burgdorferi* cells, and more preferably are not growing *Borrelia* cells. In other embodiments of the invention the cells are preferably not *Borrelia burgdorferi* cells, and more preferably are not *Borrelia* cells.

Whilst various of the methods and kits and other aspects or embodiments which are described herein have been disclosed in relation to bacterial cells, such as *B. burgdorferi* or bacteria from a selected few representative species such as *E. coli, S. aureus, M. tuberculosis etc,* such references are intended to be exemplary as it is envisaged that the present invention, including the methods and kits described herein, would be equally applicable to other cells including other prokaryotic cells as well as eukaryotic cells. The teachings herein may therefore be read accordingly so that they are not so restricted but extend to other cells such as other prokaryotic or eukaryotic cells.

In some embodiments, the presently disclosed subject matter provides a method for assessing the viability or antimicrobial susceptibility of bacteria from a selected genus, the method comprising: (a) establishing a bacterial culture comprising isolated bacteria from the selected genus; (b) incubating the bacterial culture with a staining mixture comprising: (i) a first agent which emits fluorescence of a first color that is indicative of live bacterial cells in the culture, and (ii) a second agent which emits fluorescence of a second color that contrasts from the first color and is indicative of dead bacterial cells in the culture; and (c) calculating a ratio of the intensity of emitted fluorescence of the first color to the intensity of emitted fluorescence the second color; and (d) assessing the viability of the bacteria in the culture, wherein the ratio calculated in (c) is indicative of the percentage of live bacteria in the culture. Optionally, the method may further comprise:

(i) assessing the susceptibility of bacteria from a selected genus to at least one antimicrobial agent by incubating the culture of step (a), optionally under suitable conditions for bacterial growth to occur, with at least one dose of at least one antimicrobial agent and assessing the susceptibility of bacteria from the selected genus to at least one antimicrobial agent by calculating the ratio in step (b) without the need for bacterial growth, after a period of exposure to the at least one dose of the at least one antimicrobial agent, wherein the bacteria are assessed as susceptible to the at least one antimicrobial agent if the ratio in step (b) remains the same or decreases after the period of exposure to the at least one dose of the at least one antimicrobial agent, and wherein the bacteria are assessed as resistant to the at least one antimicrobial agent if the ratio in step (b) increases after the period of exposure to the at least one dose of the at least one agent; or

(ii) identifying a candidate agent that is capable of inhibiting growth or survival of bacteria from the selected genus by contacting the culture of step (a) with a test agent and assessing a viability of the bacteria in the culture in the presence of the test agent as compared to the viability of the bacteria in a control culture which lacks the test agent.

In some embodiments, the presently disclosed subject matter and methods provide a new SYBR Green I/propidium iodide (PI) (also termed SYBR Green/PI) assay based on a green fluorescence to red fluorescence ratio for rapid viability assessment of eukaryotic or prokaryotic cells such as bacteria, such as those from the *Borrelia* genus or a genus selected from the group consisting of *Staphylococcus, Escherichia, Klebsiella, Acinetobacter,* and *Mycobacterium.* This assay is superior to other assays commonly used for measuring the viability and for rapid drug susceptibility testing of *B. burgdorferi,* and other bacteria and cells, such as the current commercially available LIVE/DEAD BacLight viability assay (Invitrogen, Carlsbad, CA). The term "viability assay" as used herein refers to an assay to determine the ability of cells to maintain or recover viability, such as the ability to grow.

In at least some embodiments a method of the present invention may comprise the following steps: (a) obtaining a culture comprising the cells to be assayed (which as discussed herein may be eukaryotic or prokaryotic, growing or non-growing etc); (b) performing a viability assay of the cells in the culture by using a SYBR Green I/Propidium Iodide assay based on a ratio of green fluorescence, indicative of live cells, to red fluorescence, indicative of dead cells, comprising: (i) mixing the culture with a staining mixture comprising SYBR Green I and propidium iodide; (ii) allowing the culture and staining mixture to incubate in the dark; (iii) determining the fluorescence intensity of the culture and staining mixture at 535 nm, which measures green fluorescence, and 635 nm, which measures red fluorescence; and (iv) calculating the ratio of green fluorescence to red fluorescence; and wherein a ratio of green fluorescence to red fluorescence of more than approximately 7 means that the cells are viable.

In some embodiments, the presently disclosed subject matter provides a method for assessing the viability of bacteria from a selected genus, the method comprising: (a) obtaining a culture comprising bacterial cells from the selected genus; (b) performing a viability assay of the bacterial cells in the culture by using a SYBR Green I/Propidium Iodide assay based on a ratio of green fluorescence, indicative of live bacterial cells, to red fluorescence, indicative of dead bacterial cells, comprising:

(i) mixing the culture with a staining mixture comprising SYBR Green I and propidium iodide; (ii) allowing the culture and staining mixture to incubate in the dark; (iii) determining the fluorescence intensity of the culture and staining mixture at 535 nm, which measures green fluorescence, and 635 nm, which measures red fluorescence; and (iv) calculating the ratio of green fluorescence to red fluorescence; and wherein a ratio of green fluorescence to red fluorescence of more than approximately 7 means that the bacterial cells are viable.

In some embodiments of the present disclosure including the methods disclosed herein, the first color emitted by the first fluorescent agent is green and the second color as emitted by the second fluorescent agent is red or orange. In at least some embodiments, the first agent is SYBR Green I and the second agent is propidium iodide. In further embodiments, the SYBR Green I is present in the culture in a concentration range of between about 0.1x and about 100x and propidium iodide is present in the culture in a range of between about 0.1 mM and about 5 mM. In still further embodiments, the concentration of SYBR Green I in the culture is 10x and the concentration of propidium iodide is 2mM.

In some embodiments, the culture in the methods and kits of the present invention may comprise a BSK-H medium. In other embodiments, the step of incubating the culture with the mixture is performed for about 5, 10, 15, 25, or 30 minutes. In further embodiments, the step of incubating is performed in the dark.

In some embodiments, the method is performed in a high-throughput format, e.g., for drug screening. Unexpectedly, it has been found that the presently disclosed methods, in some embodiments, can be used without a washing step and the need for subsequent cell growth for high-throughput screens. That is, the methods can be used to assess viability, susceptibility of bacteria to antimicrobials, and in drug screening directly in a high-throughput manner in the absence of washing.

In some embodiments, the presently disclosed subject matter provides a method for assessing the susceptibility of cells to at least one drug (e.g. antimicrobial agent), the method comprising: (a) establishing a culture comprising isolated cells to be tested; (b) incubating the culture, optionally under suitable conditions for growth of the cells to occur, with: (i) at least one dose of at least one drug (e.g. antimicrobial agent); and (ii) a staining mixture comprising a first agent which emits fluorescence of a first color that is indicative of live cells in the culture, and a second agent which emits fluorescence of a second color that contrasts from the first color and is indicative of dead cells in the culture, wherein a ratio of the intensity of emitted fluorescence of the first color to the intensity of emitted fluorescence of the second color is indicative of the percentage of live cells in the culture; and (c) assessing the susceptibility of the cells to at least one drug (e.g. antimicrobial agent) by calculating the ratio in (b)(ii) without the need for cell growth, after a period of exposure to at least one dose of at least one drug (e.g. antimicrobial agent), wherein the cells are assessed as susceptible to at least one drug (e.g. antimicrobial agent) if the ratio in (b)(ii) remains the same or decreases after the period of exposure to at least one dose of at least one drug (e.g. antimicrobial agent), and wherein the cells are assessed as resistant to at least one drug (e.g. antimicrobial agent) if the ratio in (b)(ii) increases after the period of exposure to at least one dose of at least one drug (e.g. antimicrobial agent). In at least some embodiments the method (and other methods as described herein) may be performed with no growth, or substantially no growth, of the cells during the

incubation step with the at least one dose of at least drug.

In some embodiments, the presently disclosed subject matter provides a method for assessing the susceptibility of bacteria from a selected genus to at least one antimicrobial agent, the method comprising: (a) establishing a bacterial culture comprising isolated bacteria from the selected genus; (b) incubating the culture, optionally under suitable conditions for bacterial growth to occur, with: (i) at least one dose of at least one antimicrobial agent; and (ii) a staining mixture comprising a first agent which emits fluorescence of a first color that is indicative of live bacteria in the culture, and a second agent which emits fluorescence of a second color that contrasts from the first color and is indicative of dead bacteria in the culture, wherein a ratio of the intensity of emitted fluorescence of the first color to the intensity of emitted fluorescence of the second color is indicative of the percentage of live bacteria in the culture; and (c) assessing the susceptibility of bacteria from the selected genus to at least one antimicrobial agent by calculating the ratio in (b)(ii) without the need for bacterial growth, after a period of exposure to at least one dose of at least one antimicrobial agent, wherein the bacteria are assessed as susceptible to at least one antimicrobial agent if the ratio in (b)(ii) remains the same or decreases after the period of exposure to at least one dose of at least one antimicrobial agent, and wherein the bacteria are assessed as resistant to at least one antimicrobial agent if the ratio in (b)(ii) increases after the period of exposure to at least one dose of at least one agent.

In some embodiments, the presently disclosed subject matter provides a method for screening for a compound that is capable of inhibiting cells which cells may be prokaryotic or eukaryotic cells, the method comprising: (a) obtaining a stationary phase culture that comprises said prokaryotic or eukaryotic cells; (b) contacting the culture with a test compound; (c) performing a viability assay of the cells in the culture by using a SYBR Green I/Propidium Iodide assay based on a ratio of green fluorescence, indicative of live cells, to red fluorescence, indicative of dead cells, comprising: (i) mixing the culture with a staining mixture comprising SYBR Green I and propidium iodide; (ii) allowing the culture and staining mixture to incubate in the dark; (iii) determining the fluorescence intensity of the culture and staining mixture at 535 nm, which measures green fluorescence, and 635 nm, which measures red fluorescence; (iv) calculating the ratio of green fluorescence to red fluorescence; and (v) comparing the ratio of green fluorescence to red fluorescence to the ratio of green fluorescence to red fluorescence of a set of controls with known amounts of live and dead cells to determine the percentage of live cells and dead cells in the culture; and (d) comparing the percentage of the live cells in the culture treated with the test compound to a control under identical conditions, but in the absence of the test compound; wherein a significant decrease in the percentage of the live cells and/or a significant increase in the percentage of dead cells in the culture with the test compound compared to the percentage of the live cells or dead cells in the control under identical conditions, but in the absence of the test compound is indicative that the test compound is capable of inhibiting the cells.

The term "contacting" as used herein refers to any action that results in at least one compound of the presently disclosed subject matter physically contacting at least one cell (e.g. bacterial cell) or the environment in which at least one cell (e.g. bacterial cell) resides (e.g., a culture medium).

In some embodiments, the presently disclosed subject matter provides a method for screening for a compound that is capable of inhibiting bacteria from a selected genus, the method comprising: (a) obtaining a stationary phase bacterial culture that comprises bacterial cells from the selected genus; (b) contacting the culture with a test compound; (c) performing a viability assay of the bacterial cells in the culture by using a SYBR Green I/Propidium Iodide assay based on a ratio of green fluorescence, indicative of live bacterial cells, to red fluorescence, indicative of dead bacterial cells, comprising: (i) mixing the culture with a staining mixture comprising SYBR Green I and propidium iodide; (ii) allowing the culture and staining mixture to incubate in the dark; (iii) determining the fluorescence intensity of the culture and staining mixture at 535 nm, which measures green fluorescence, and 635 nm, which measures red fluorescence; (iv) calculating the ratio of green fluorescence to red fluorescence; and (v) comparing the ratio of green fluorescence to red fluorescence to the ratio of green fluorescence to red fluorescence of a set of controls with known amounts of live and dead bacterial cells to determine the percentage of live bacterial cells and dead bacterial cells in the culture; and (d) comparing the percentage of the live bacterial cells in the culture treated with the test compound to a control under identical conditions, but in the absence of the test compound; wherein a significant decrease in the percentage of the live bacterial cells and/or a significant increase in the percentage of dead bacterial cells in the culture with the test compound compared to the percentage of the live bacterial cells or dead bacterial cells in the control under identical conditions, but in the absence of the test compound is indicative that the test compound is capable of inhibiting the bacterial cells.

In some embodiments of the methods described herein, the method comprises determining a minimum inhibitory concentration breakpoint or cut-off for at least one drug or agent (e.g at least one antimicrobial agent). In some embodiments of the present invention, the first color is green and the second color is red or orange. In some embodiments of the present invention, the first agent is SYBR Green I and the second agent is propidium iodide. In some embodiments of the present invention, the concentration of SYBR Green I in the culture is about 10x and the concentration of propidium iodide is about 2mM. In some embodiments of the present invention, the culture further comprises a BSK-H medium.

In some embodiments, the presently disclosed subject matter provides a method for identifying a candidate agent that is capable of inhibiting growth or survival of cells which cells may be prokaryotic or eukaryotic cells, the method

comprising: (a) establishing a culture comprising isolated cells to be tested; (b) contacting the culture with a test agent; (c) assessing a viability of the cells in the culture in the presence of the test agent as compared to the viability of the cells in a control culture which lacks the test agent for drug screens on non-growing cells in continuous time lapse manner over different time span and rapid drug susceptibility testing in a growth independent manner such that susceptibility to a test agent can be rapidly determined without relying on cell growth as in conventional drug or antibiotic susceptibility testing, wherein assessing the viability of the cells in the culture comprises: (i) incubating the culture with a staining mixture comprising a first agent which emits fluorescence of a first color that is indicative of live cells, and a second agent which emits fluorescence of a second color that contrasts from the first color and is indicative of dead cells; (ii) calculating a ratio of the intensity of emitted fluorescence of the first color to the intensity of emitted fluorescence the second color, wherein the ratio is indicative of the percentage of live cells in the culture; and (d) identifying the test agent as a candidate agent that is capable of inhibiting growth or survival of the prokaryotic or eukaryotic cells if the calculated ratio for the culture is decreased relative to a similarly calculated ratio for the control culture. As used herein, a "similarly calculated ratio" means that the ratio for the control culture is calculated in the same manner as the ratio calculated for the cell culture (e.g. bacterial culture where the cells are bacterial cells).

In some embodiments, the presently disclosed subject matter provides a method for identifying a candidate agent that is capable of inhibiting growth or survival of bacteria from a selected genus, the method comprising: (a) establishing a culture comprising isolated bacteria from the selected genus; (b) contacting the culture with a test agent; (c) assessing a viability of the bacteria in the culture in the presence of the test agent as compared to the viability of the bacteria in a control culture which lacks the test agent for drug screens on non-growing persisters in continuous time lapse manner over different time span and rapid antimicrobial susceptibility testing in a growth independent manner such that susceptibility to a test agent can be rapidly determined without relying on bacterial growth as in conventional antibiotic susceptibility testing, wherein assessing the viability of the bacteria in the culture comprises: (i) incubating the culture with a staining mixture comprising a first agent which emits fluorescence of a first color that is indicative of live bacteria, and a second agent which emits fluorescence of a second color that contrasts from the first color and is indicative of dead bacteria; (ii) calculating a ratio of the intensity of emitted fluorescence of the first color to the intensity of emitted fluorescence the second color, wherein the ratio is indicative of the percentage of live bacteria in the culture; and (d) identifying the test agent as a candidate agent that is capable of inhibiting growth or survival of bacteria from the selected genus if the calculated ratio for the culture is decreased relative to a similarly calculated ratio for the control culture.

In some embodiments, the presently disclosed subject matter provides a method for identifying a candidate agent that is capable of inhibiting growth or survival of of cells which cells may be prokaryotic or eukaryotic cells, the method comprising: (a) establishing a culture comprising isolated cells to be tested; (b) contacting the culture with a test agent; (c) assessing a viability of the cells in the culture in the presence of the test agent as compared to the viability of the cells in a control culture which lacks the test agent for drug screens on non-growing cells in continuous time lapse manner over different time span and rapid drug susceptibility testing in a growth independent manner such that susceptibility to a test agent can be rapidly determined without relying on cell growth as in conventional drug or antibiotic susceptibility testing, wherein assessing the viability of the cells in the culture comprises: (i) incubating the culture with a staining mixture comprising a first agent which emits fluorescence of a first color that is indicative of live cells, and a second agent which emits fluorescence of a second color that contrasts from the first color and is indicative of dead cells; (ii) calculating a ratio of the intensity of emitted fluorescence of the first color to the intensity of emitted fluorescence the second color, wherein the ratio is indicative of the percentage of live cells in the culture; and (d) identifying the test agent as a candidate agent that is capable of inhibiting growth or survival of cells from the selected genus if the calculated ratio for the culture is decreased relative to a similarly calculated ratio for the control culture..

In some embodiments, the presently disclosed subject matter provides a method for identifying a candidate agent that is capable of inhibiting growth or survival of bacteria from a selected genus, the method comprising: (a) establishing a culture comprising isolated bacteria from the selected genus; (b) contacting the culture with a test agent; (c) assessing a viability of the bacteria in the culture in the presence of the test agent as compared to the viability of the bacteria in a control culture which lacks the test agent for drug screens on non-growing persisters in continuous time lapse manner over different time span and rapid antimicrobial susceptibility testing in a growth independent manner such that susceptibility to a test agent can be rapidly determined without relying on bacterial growth as in conventional antibiotic susceptibility testing, wherein assessing the viability of the bacteria in the culture comprises: (i) incubating the culture with a staining mixture comprising a first agent which emits fluorescence of a first color that is indicative of live bacteria, and a second agent which emits fluorescence of a second color that contrasts from the first color and is indicative of dead bacteria; (ii) calculating a ratio of the intensity of emitted fluorescence of the first color to the intensity of emitted fluorescence the second color, wherein the ratio is indicative of the percentage of live bacteria in the culture; and (d) identifying the test agent as a candidate agent that is capable of inhibiting growth or survival of bacteria from the selected genus if the calculated ratio for the culture is decreased relative to a similarly calculated ratio for the control culture.

In some embodiments of the methods described herein, the method includes conducting a microscopic counting rescreen to confirm that at least one test agent is a candidate agent for inhibiting growth or survival of bacteria.

As used herein, the terms "inhibit", "inhibits", or "significant decrease" means to decrease, suppress, attenuate, diminish, or arrest, for example the growth and/or survival of cells (e.g. bacteria) in a culture or in a subject, by at least 10%, 20%, 30%, 40%, 50%, 60%, 70%, 80%, 90%, 95%, 98%, 99%, or even 100% compared to an untreated control culture or subject. Inhibiting "survival" of cells (e.g. bacteria) in this context refers to killing cells or reducing live cell count. In some embodiments, the growth of the cells is inhibited by more than approximately 50%. In other embodiments, the percentage of live cells in the culture after the treatment with the test compound is less than approximately 50% compared to the percentage of live cells in the control under identical conditions, but in the absence of the test compound. In still other embodiments, the stationary phase culture comprises non-growing cells such as non-replicating persister cells. Further, as used herein, the term "significant increase" means an increase by at least 10%, 20%, 30%, 40%, 50%, 60%, 70%, 80%, 90%, 95%, 98%, 99%, or even 100%.

In accordance with the present invention, the term "drug" and like expressions as used herein generally includes a reference to compounds or agents (the terms may be used synonymously) which are capable of inhibiting growth and/or survival of cultured or growing cells and/or inhibiting growth and/or survival of non-growing cells such as dormant or persister cells. Accordingly, it will be understood that the methods and kits of the present disclosure relate to static agents and cidal agents and the present invention provides methods for screening or identifying such activity and compounds, and for assessing susceptibility of cells to such agents. As will be understood from the disclosures herein, inhibitory activity, such as static or cidal activity, may be in relation to cultured or growing cells or in relation to growth non-growing dormant or persister cells. Unless the context indicates otherwise, the term "drug" and similar expressions may include test compounds or agents (see below) and candidate agents. Accordingly, the term may refer to a variety or agents or compounds which may be assayed using the methods described herein such as in the the DSTs and screening methods of the present invention. In some embodiments, the methods of the present invention may comprise assaying the effects of a combination of drugs or test compounds / agents. Thus, in at least some embodiments the terms "at least one drug" and "at least one test agent / compound" may include a reference to where a combination of drugs / agents is being tested. In this way synergistic or effective combinations of drugs or test agents may be identified by the methods of the present invention.

As used herein, the term "synergistic" (and for the avoidance of doubt, grammatical variants thereof) includes a reference to circumstances under which the biological activity of a combination of a compound and at least one additional therapeutic agent is greater than the sum of the biological activities of the respective agents when administered individually. The methodology of the present invention can be used for drug combination screens, such as shown in Example 7. Accordingly, in some embodiments of the methods of the present invention may be used to identify one or more drug candidates or agents that synergize with one or more known drugs or other agents. Unless the context indicates otherwise, the terms "drug", "antibiotic", "antimicrobial", "antifungal", "antiparasitic", "anticancer", "test agent", "agent", "test compound", "candidate drug", "candidate agent", "candidate compound", "inhibitor" and the like (and for the avoidance of doubt, grammatical variants thereof) may be used interchangeably herein.

The term "test compound" (used synonymously with agent herein) as used herein can be any compound or drug that is desirable to test for inhibitory activity such as its capability of inhibiting growth and/or survival of cultured or growing cells and/or inhibiting growth and/or survival of non-growing cells such as dormant or persister cells, e.g. anti-persister activity. The term "anti-persister activity" means that the compound has inhibitory activity against those cells that might still persist in a culture or subject after contact or administration with a course of antibiotics. The persistent cells may evade host immune clearance and result in chronic persistent infection in a subject. The methodology of the present invention works for both cidal and static agents and the methods of the present invention may accordingly be used to screen for, or identify susceptibility to, cidal and static. The test compound may be a known compound, such as an identified drug found to be effective in at least one disease or disorder, or may be an unknown compound that is not known to be effective in any disease or disorder. In some embodiments, the test compound is a known compound that has been approved by a health regulatory agency (e.g., FDA or EMA) for an indication other than treating chronic persistent Lyme disease. In some embodiments, the test compound is a compound that is known to exhibit antibiotic activity against bacteria other than those from the *Borrelia* genus. In some embodiments, the test compound is a known compound that has not previously been reported to exhibit inhibitory activity against non-growing or non-replicating forms of the cells. In some embodiments, the test compound is a known compound that has not previously been reported to exhibit inhibitory activity against non-growing or dormant forms of the cells. In some embodiments, the test compound is a known compound that has not previously been reported to exhibit antibiotic activity against non-replicating persister forms of bacteria.

As used herein, the term "antibiotic" refers to a compound that has the ability to kill or inhibit the growth of bacteria. In some embodiments the term "antibiotic" refers to a compound that has the ability to kill or inhibit the growth of bacteria particularly bacteria selected from a genus including, but not limited to, *Borrelia, Staphylococcus, Escherichia, Klebsiella, Acinetobacter,* and *Mycobacterium.* More generally, an antimicrobial is an agent that kills microorganisms or inhibits their growth. Antimicrobials medicines can be grouped according to the microorganisms they act primarily against. For example, antibiotics are used against bacteria. As used herein, the term 'beta-lactam" or "beta-lactam

antibiotic" refers to an antibiotic with a beta-lactam ring as part of its core structure, such as penicillin and penicillin derivatives (penams), cephalosporins (cephems), monobactams, and carbapenems. While these antibiotics work by inhibiting bacterial cell wall biosynthesis, other antibiotics such as protein synthesis inhibitors (tetracyclines, aminoglycosides, macrolides, etc), nucleic acid synthesis inhibitors (fluoroquinolones, rifamycins etc) and antimetabolite sulfa drugs can also be used similarly for antibiotic susceptibility testing with the methodology described herein.

As used herein, the term "cell culture" or "culture" includes a reference to a population of cells in culture. Since the methods of the present invention are growth independent the culture conditions need not be conducive for growth and the cells in the culture need not be growing cells, and may therefore be growing or non-growing cells. In some embodiments a cell culture or culture may refer to the cells being in a medium conducive for growth of the cells and to optionally further the cells growing in the medium. Thus, in some embodiments the term "bacterial culture" or "culture" may refer to bacteria growing in a medium conducive for growth of those bacteria. The bacterial culture can be found in any type of container, such as a flask, a tube, a microwell plate, or array, and the like.

Generally, bacteria have different phases of growth. When a population of bacteria first enters a high-nutrient environment that allows growth, the cells need to adapt to their new environment. The first phase of growth is the lag phase, a period of slow growth when the cells are adapting to the high-nutrient environment and preparing for fast growth. The lag phase has high biosynthesis rates, as proteins necessary for rapid growth are produced. The second phase of growth is the log phase, also known as the logarithmic or exponential phase, in which the bacteria undergo rapid exponential growth. During log phase, nutrients are metabolized at maximum speed until one of the nutrients is depleted and starts limiting growth. The third phase of growth is the stationary phase and is caused by depleted nutrients. In some embodiments, a bacterial culture in "stationary phase" means that the bacteria in the culture have an approximately equal growth rate and death rate. As used herein, the term "growing forms" of bacteria generally refers to bacteria that are in lag or log phase and not in stationary phase. In some embodiments, the stationary phase bacterial culture has been grown for approximately 7 days. In other embodiments, the stationary phase bacterial culture comprises non-replicating persister cells. By "non-replicating persister cells," it is meant bacterial cells that enter a state in which they stop replicating and are able to tolerate antibiotics.

As mentioned elsewhere herein, the methodology of the present invention enables rapid drug susceptibility testing and drug screening which considerably shortens the time required by current methods. Various features and advantages that may be achieved by the present invention will be appreciated from the discussion herein and may include one or more of the following: (1) suitability for use with all cells- prokaryotic and eukaryotic cells which may or may not be capable of being grown in culture; (2) a new algorithm of defining resistance and susceptibility using this method by the ratio of SYBR Green and PI; (3) use of much higher drug concentrations than the normal or usual drug concentrations needed for inhibiting organism growth as used in the current conventional drug susceptibility tests (DST) which is growth-dependent and time-consuming depending upon the growth speed of the test organism; (4) the new test is growth-independent which is completely distinct from the current growth based DST; (5) the new test can be used for drug screens for all cells of prokaryotic and eukaryotic origin to identify new drug candidates in record time without the requirement for growth of cells or the organisms, especially for slow growing organisms like *M. tuberculosis,* which significantly shortens the time needed for results of the screen from weeks or hours; (6) the organisms or cells used in the drug screens can be either growing cells or non-growing dormant cells or persister cells; (7) the new test is much more versatile and can be used for continuous real-time drug screens to identify different drug candidates sequentially over a period of time, ranging from hours, days and weeks without having to stop the screen, in contrast to the current drug screen which is at a fixed single time point.

## II. KITS FOR USE IN IDENTIFYING INHIBITORY ACTIVITY (E.G. ANTI-PERSISTER ACTIVITY) AND DRUG SUSCEPTIBILITY EVALUATION

The presently disclosed subject matter also relates to kits for practising the methods of the presently disclosed subject matter. Thus, one aspect of the present invention provides a kit for use in a method of the present invention. Similarly, the invention provides a method of the present invention which is performed using a kit as described herein. In some embodiments, there is provided a kit for the rapid and growth-independent assessment of drug susceptibility or for the rapid screening of drugs to identify compounds or drug candidates that are capable of inhibiting growth or survival of growing cells as well as non-growing cells of prokaryotic and also eukaryotic origins.

In general, a presently disclosed kit contains some or all of the components, reagents, supplies, and the like to practice a method according to the presently disclosed subject matter. In some embodiments, the term "kit" refers to any intended any article of manufacture (e.g., a package or a container) comprising an effective amount of reagents (e.g. drugs or antibiotics and indicator fluorescent dyes at suitable concentrations) for performing a presently disclosed assay. The kit may optionally include the cells to be assayed (e.g. bacteria from the selected genus) and/or one or more control cells e.g. sensitive and/or resistant control cells for testing. The kits of the present invention may optionally include a set of particular instructions for practising the methods of the presently disclosed subject matter. In other embodiments,

the presently disclosed subject matter provides a kit for screening for a compound that is capable of inhibiting or killing the cells of interest (e.g. bacteria from a selected genus or cancer cells), the kit comprising cells of the type to be screened (e.g. bacterial cells from the selected genus) and reagents for performing a presently disclosed assay.

In some embodiments, the kit is for rapid screening or susceptibility testing of at least one candidate agent that is capable of inhibiting growth or survival of cells of eukaryotic or prokaryotic origin. In at least some embodiments a kit of the present invention comprises a population of cells to be assayed. Optionally the population of cells may be provided in the form of a culture and the cells may be selected from all cells of prokaryotic or eukaryotic origin. In other embodiments of the invention the kit does not comprise the cells to be assayed and in such instances the cells to be assayed may be provided by the user of the kit.

A staining mixture may suitably be comprised in the kits of the present invention wherein the staining mixture comprises: (i) a first agent which emits fluorescence of a first color that is indicative of live cells(e.g. SYBR Green I), and (ii) a second agent (e.g. propidium iodide) which emits fluorescence of a second color that contrasts from the first color and is indicative of dead cells, wherein when the staining mixture is incubated with the cells or culture thereof a calculated ratio of the intensity of emitted fluorescence of the first color to the intensity of emitted fluorescence the second color is indicative of the percentage of live cells in the population or culture thereof.

Accordingly in some embodiments there is provided a kit for rapid screening or susceptibility testing of at least one candidate agent that is capable of inhibiting growth or survival of cells of eukaryotic or prokaryotic origin wherein the kit comprises: (a) optionally a population of the cells of eukaryotic or prokaryotic origin or a culture thereof (alternatively the cells may be provided separately from the kit, such as by the user of the kit); (b) a staining mixture comprising: (i) a first agent which emits fluorescence of a first color that is indicative of live cells(e.g. SYBR Green I), and (ii) a second agent (e.g. propidium iodide) which emits fluorescence of a second color that contrasts from the first color and is indicative of dead cells, wherein when the staining mixture is incubated with the cells or culture thereof a calculated ratio of the intensity of emitted fluorescence of the first color to the intensity of emitted fluorescence the second color is indicative of the percentage of live cells in the population or culture thereof; and (c) optionally instructions, such as instructions for using the cells in (a) and the staining mixture in (b) to screen for at least one candidate agent that is capable of inhibiting growth or survival of the cells / for said rapid screening or drug susceptibility testing.

In at least some embodiments, a kit of the present invention may comprise instructions for one or more of the following:

(i) instructions for use of the kit for use in a method of the present invention;
(ii) instructions for contacting or exposing the cells to be assayed with at least one test agent or drug;
(iii) instructions for incubating the staining mixture with the cells to be assayed;
(iv) instructions for using SYBR Green I in the screen at a concentration of about 10x and using propidium iodide in the screen at a concentration of about 2mM;
(v) instructions for incubating the cells at appropriate density (e.g. about $10^{1-9}$ cells/ml or at about $10^{4-8}$ cells/ml) for the drugs tested and the fluorescent indicator dyes used with an agent that kills or inhibits the growth of the cells without the requirement for cell growth for various times, ranging from minutes to hours, or days or weeks, depending on the conditions of the screen;
(vi) instructions for assessing the viability of the cells to be assayed; or
(vii) instructions for calculating a ratio of the intensity of emitted fluorescence of the first color to the intensity of emitted fluorescence the second color.

Where a kit of the present invention comprises a culture of cells, the culture optionally comprises a stationary phase culture or morphological variant forms such as L-forms (cell wall defective forms), round bodies, microcolonies, or biofilms.. The stationary phase culture may comprise non-growing cells, such as non-replicating dormant or persister cells.

Kits of the present invention may optionally comprise one or or of the following:

(i) at least one drug or test agent to screen or test for its ability to inhibit cell growth or survival;
(ii) at least one microplate (e.g.in 96, or 384, or 1536 well microplate format);
(iii) software for calculating the fluorescence ratios of the test organism / cells and/or for determining the susceptibility and resistance of a test organism / cells using the fluorescence data;
(iv) one or more suitable controls e.g. known resistant and/or known susceptible cells and/or drug-free controls; and/or
(v) one or more buffers to dilute the cells (e.g. bacteria) to appropriate density for testing and/or to dilute one or more other reagents;

In certain embodiments, a kit of the present invention may comprise drugs in different concentrations optionally in a microplate format (e.g. 96-well, 384-well, or 1536-well microplate format).

In some embodiments, the presently disclosed subject matter provides a kit for screening for at least one agent that is capable of inhibiting growth or survival of bacteria from a selected genus, the kit comprising isolated non-replicating persister forms of bacteria from the selected genus and reagents for performing a SYBR Green I/Propidium iodide viability assay.

In some embodiments, the presently disclosed subject matter provides a kit for screening or antimicrobial susceptibility testing of at least one candidate agent that is capable of inhibiting growth or survival of bacteria from a selected genus, the kit comprising: (a) a population of isolated bacteria comprising bacteria from the selected genus (e.g. from the *Borellia* genus) or a culture thereof; (b) a staining mixture comprising: (i) a first agent which emits fluorescence of a first color that is indicative of live bacterial cells, and (ii) a second agent which emits fluorescence of a second color that contrasts from the first color and is indicative of dead bacterial cells, wherein when the staining mixture is incubated with the bacteria population or culture thereof a calculated ratio of the intensity of emitted fluorescence of the first color to the intensity of emitted fluorescence the second color is indicative of the percentage of live bacteria in the population or culture thereof; and (c) instructions for using the bacteria in (a) and the staining mixture in (b) to screen for at least one candidate agent that is capable of inhibiting growth or survival of bacteria from the selected genus.

In some embodiments, the kit further comprises one or more of the following:

(i) at least one test agent to screen for its ability to inhibit the growth or survival of bacteria from a selected genus; the selected genus may optionally be selected from the group consisting of *Borrelia, Staphylococcus, Escherichia, Klebsiella, Acinetobacter,* and *Mycobacterium;*
(ii) instructions for contacting the population of bacteria or population thereof with at least one test agent;
(iii) instructions for incubating the staining mixture with the population of bacteria or culture thereof;
(iv) instructions for assessing the viability of the bacteria in the population or culture thereof;
(v) instructions for calculating a ratio of the intensity of emitted fluorescence of the first color to the intensity of emitted fluorescence the second color.

In some embodiments, the calculated intensity ratio in is indicative of the percentage of live bacteria in the population or culture thereof after a period of exposure to at least one test agent. In particular embodiments, the bacteria are *Borrelia burgdorferi.* In other embodiments, the bacteria are not *Borrelia burgdorferi* and may optionally be selected from the group consisting of *Staphylococcus aureus, Escherichia coli, Klebsiella pneumoniae, Acinetobacter baumannii,* and *Mycobacterium tuberculosis.*

In still other embodiments, the culture comprises a stationary phase culture comprising non-replicating persister cells. In further embodiments, the stationary phase culture comprises at least one morphological form selected from the group consisting of round bodies, planktonic, biofilm and combinations thereof.

In at least some embodiments a kit of the present invention may comprise one or more drugs or test agents to be tested in different concentrations e.g. in a 96-well, 384-well, or 1536-well microplate format; a staining mixture as described herein; and/or one or more buffers e.g. to dilute the cells to appropriate density for testing. In some embodiment the kit may comprise the cells to be tested; in other embodiments the kit does not comprise the cells to be the tested. Optionally the user may provide their own control cells, such as sensitive and/or resistant control cells or cells as controls for testing, whilst in other embodiments the kit may contain the sensitive and/or resistant control cells and/or cells as controls for testing.

In some embodiments, a kit of the present invention is a kit for assessing the viability and sensitivity of cultures of bacteria from a selected genus for at least one agent (e.g., current Lyme disease antibiotics or any new agents) that is capable of inhibiting growth or survival of bacteria from the selected genus, the kit comprising cultures of bacteria from the selected genus, reagents for performing a SYBR Green I/Propidium iodide assay, and optionally at least one test agent.

## III. GENERAL DEFINITIONS

Although specific terms are employed herein, they are used in a generic and descriptive sense only and not for purposes of limitation. Unless otherwise defined, all technical and scientific terms used herein have the same meaning as commonly understood by one of ordinary skill in the art to which this presently described subject matter belongs.

Following long-standing patent law convention, the terms "a," "an," and "the" refer to "one or more" when used in this application, including the claims. Thus, for example, reference to "a subject" includes a plurality of subjects, unless the context clearly is to the contrary (e.g., a plurality of subjects), and so forth.

Throughout this specification and the claims, the terms "comprise," "comprises," and "comprising" are used in a non-exclusive sense, except where the context requires otherwise. Likewise, the term "include" and its grammatical variants are intended to be non-limiting, such that recitation of items in a list is not to the exclusion of other like items that can be substituted or added to the listed items.

For the purposes of this specification and appended claims, unless otherwise indicated, all numbers expressing amounts, sizes, dimensions, proportions, shapes, formulations, parameters, percentages, quantities, characteristics, and other numerical values used in the specification and claims, are to be understood as being modified in all instances by the term "about" even though the term "about" may not expressly appear with the value, amount or range. Accordingly, unless indicated to the contrary, the numerical parameters set forth in the following specification and attached claims are not and need not be exact, but may be approximate and/or larger or smaller as desired, reflecting tolerances, conversion factors, rounding off, measurement error and the like, and other factors known to those of skill in the art depending on the desired properties sought to be obtained by the presently disclosed subject matter. For example, the term "about," when referring to a value can be meant to encompass variations of, in some embodiments, ± 100% in some embodiments ± 50%, in some embodiments ± 20%, in some embodiments ± 10%, in some embodiments ± 5%, in some embodiments ±1%, in some embodiments ± 0.5%, and in some embodiments ± 0.1% from the specified amount, as such variations are appropriate to perform the disclosed methods or employ the disclosed compositions.

Further, the term "about" when used in connection with one or more numbers or numerical ranges, should be understood to refer to all such numbers, including all numbers in a range and modifies that range by extending the boundaries above and below the numerical values set forth. The recitation of numerical ranges by endpoints includes all numbers, e.g., whole integers, including fractions thereof, subsumed within that range (for example, the recitation of 1 to 5 includes 1, 2, 3, 4, and 5, as well as fractions thereof, e.g., 1.5, 2.25, 3.75, 4.1, and the like) and any range within that range.

## EXAMPLES

The following Examples have been included to provide guidance to one of ordinary skill in the art for practicing representative embodiments of the presently disclosed subject matter. In light of the present disclosure and the general level of skill in the art, those of skill can appreciate that the following Examples are intended to be exemplary only and that numerous changes, modifications, and alterations can be employed without departing from the scope of the presently disclosed subject matter. The synthetic descriptions and specific examples that follow are only intended for the purposes of illustration, and are not to be construed as limiting in any manner to make compounds of the disclosure by other methods.

### EXAMPLE 1

### Methods for Identifying Novel Anti-Persister Activity for *Borrelia burgdorferi* Materials and Methods

*Bacterial strain, media and culture: Borrelia burgdorferi* strain B31 was obtained from the American Type Tissue Collection. *Borrelia burgdorferi* was cultured in BSK-H medium (HiMedia Laboratories Pvt. Ltd.), with 6% rabbit serum (Sigma-Aldrich, St. Louis, MO). All culture media were filter-sterilized by 0.2-μm filter. Cultures were incubated in sterile 50-mL closed conical tubes (BD Biosciences, San Diego, CA) at 33 °C without antibiotics. After 7 days, 100-μL stationary-phase *B. burgdorferi* cultures ($1 \times 10^6$ cells) were transferred to 96-well tissue culture microplates for drug screening.

*Microscopy techniques:* Specimens were examined on a Nikon Eclipse E800 microscope equipped with differential interference contrast (DIC) and epi-fluorescent illumination, and recorded with a Spot slider color camera. Cell proliferation assays were performed by directly counting using a bacterial counting chamber (Hausser Scientific Partnership, Horsham, PA) and DIC microscopy. To assay the viability of *B. burgdorferi,* the SYBR Green I/PI assay or LIVE/DEAD BacLight bacterial viability assay was performed. The ratio of live (green) and dead (red) *B. burgdorferi* was calculated by counting these cells using a bacterial counting chamber and epi-fluorescent microscopy.

*Antibiotics and FDA-approved drug library:* Doxycycline, amoxicillin, metronidazole, clofazimine, ketoconazole, miconazole, aspirin, polymyxin B (PMB), and sulfamethoxazole (SMX) (all purchased from Sigma-Aldrich) were dissolved in appropriate solvents (Clinical and Laboratory Standards Institute, 2007) to form stock solutions. All the antibiotic stocks were filter-sterilized by a 0.2-μm filter. The stocks then were pre-diluted into 500-μM pre-diluted stocks and stored at -20 °C.

Each drug in the Johns Hopkins Clinical Compound Library (JHCCL) (Chong et al., 2006) was made into 10-mM stock solutions with DMSO. The stock solutions were arrayed in a total of 24 96-well plates, leaving the first and the last columns in each plate for controls. Each solution in these master plates was diluted with PBS to make 500-μM pre-diluted plates. The first and the last columns in each pre-diluted plate were included as blank controls, doxycycline control, and amoxicillin control. The pre-diluted drug plates were sealed and stored at -20 °C.

*Antibiotic susceptibility test:* To qualitatively determine the effect of the antibiotics, 10 μL of each compound from the pre-diluted plate or pre-diluted stock was added to the *B. burgdorferi* culture in the screening plate. The final volume per well was adjusted to 100 μL. The plates were sealed and placed in a 33 °C incubator for 7 days.

For assaying the live and dead cells in the screening plates, the SYBR Green I/PI assay was used as described in a previous study (Feng, Wang, Shi, et al., 2014). SYBR Green I (10,000 × stock, Invitrogen, Carlsbad, CA) (10 μL)

was mixed with 30 μL propidium iodide (20 mM, Sigma-Aldrich) into 1.0 mL of sterilized $dH_2O$ and mixed thoroughly. Staining mixture (10 μL) was added to each well and mixed thoroughly. The plates at room temperature were incubated in the dark for 15 minutes. With the excitation wavelength at 485 nm, the fluorescence intensities at 535 nm (green emission) and 635 nm (red emission) were measured for each well of the screening plate using a HTS 7000 plus Bio Assay Reader (PerkinElmer Inc., Waltham, MA). Meanwhile the *B. burgdorferi* suspensions (live and 70% isopropyl alcohol-killed) were mixed with five different proportions of live:dead cells (0:10, 2:8, 5:5, 8:2, 10:0) the mixture was added in wells of a 96-well plate. SYBR Green I/PI reagent was then added to each of the five samples and the green/red fluorescence ratios for each proportion of live/dead *B. burgdorferi* were measured using a HTS 7000 plus Bio Assay Reader as described above. The regression equation and regression curve of the relationship between the percentage of live bacteria and green/red fluorescence ratios were obtained with least-square fitting analysis. The regression equation was used to calculate the percentage of live cells in each well of the screening plate. Some effective candidates were further confirmed by epi-fluorescence microscope counting.

*MIC determination:* The standard microdilution method was used to determine the antibiotic minimum inhibitory concentration (MIC) that would inhibit visible growth *of B. burgdorferi* after a 72-hour incubation period (Sapi et al., 2011; Dever et al., 1992; Boerner et al., 1995). *B. burgdorferi* cells ($1 \times 10^5$) were inoculated into each well of a 96-well microplate containing 90 μL fresh BSK-H medium per well. Each diluted antibiotic (10 μL) was added to the culture. All experiments were run in triplicate. The 96-well plate was sealed and placed in the incubator at 33 °C for 5 days. Cell proliferation was assessed using a SYBR Green I/PI assay and a bacterial counting chamber after the incubation.

*Establishment of a stationary phase model for drug screens*: Representative morphological changes of *Borrelia* cells following treatment with some currently used antibiotics is shown in FIG. 1. During the log phase, the *Borrelia* cells treated with amoxicillin and doxycycline adopt cystic or round body forms. During the stationary phase, the *Borrelia* cells treated with the same antibiotic adopt a spirochete form. These morphological variants *of B. burgdorferi* have different antibiotic susceptibilities.

## Results

A comparison of methods for assaying *B. burgdorferi* viability in a 96-well plate showed that the SYBR Green/propidium iodide (PI) assay using the green fluorescence to red fluorescence ratio resulted in a consistent correlation between the percent of live cells and the ratio (FIG. 2). When plotted, this was found to be a linear ratio (FIG. 3).

FIG. 4 shows the *B. burgdorferi* B31 strain with commonly used viability assays MTT, XTT, fluoroscein diacetate assay (FDA), the commercially available LIVE/DEAD BacLight assay, and the presently disclosed SYBR Green I/PI assay. The SYBR Green I/PI assay had a less than 10% error and could be completed in approximately 20 minutes.

FIG. 5A, FIG. 5B, FIG. 5C, and FIG. 5D show the *B. burgdorferi* B31 strain observed with: (FIG. 5A) the fluorescent microscopy LIVE/DEAD BacLight stain; (FIG. 5B) SYBR Green/PI stain; (FIG. 5C) FDA stain; and (FIG. 5D) the *B. burgdorferi* biofilm stained by SYBR Green/PI. The SYBR Green/PI assay showed correlation with direct microscope counting with antibiotic exposure (FIG. 6).

FIG. 7 shows a representative drawing of the Yin-Yang model of bacterial persisters and latent infections (Zhang, 2014). The Yin-Yang model depicts a dynamic and complex bacterial population consisting of growing (Yang, in red) and non-growing populations (Yin, in black) which are in varying metabolic states *in continuum* and can interconvert. In the growing population (Yang), there is a small population of non-growing or slowly growing persisters (Yin), which in turn contain a small number of growing bacteria. The persister population is again heterogeneous and composed of various sub-populations in continuum and includes a varying hierarchy of persisters. In the case of TB, isoniazid (INH) kills growing bacteria (Yang), rifampin (RIF) kills some growing bacteria and slowly growing persisters, whereas pyrazinamide (PZA) kills only persisters. Persisters not killed by antibiotics could revert to replicating forms (reverters) and cause relapse. The current Lyme disease antibiotics doxycycline and amoxicillin or ceftriaxone only kill the growing *Borrelia burgdorferi* bacteria (Yang) and have little or no activity for dormant non-growing *Borrelia burgdorferi* persisters (Yin). The Yin-Yang model proposes targeting both replicating and non-replicating cells for better treatment of both persistent bacterial infections, including Lyme disease. For example, newly identified persister active drugs or antibiotics, such as daptomycin, clofazimine, cefoperazone, carbomycin, sulfa drugs, and/or quinolones, can be used in combination with currently used Lyme disease antibiotics, such as doxycycline, amoxicillin and/or ceftriaxone, which are active against the growing *Borrelia burgdorferi* bacteria (Yang) for more effective treatment of all forms of Lyme disease, especially the chronic and persistent forms of the disease.

For FIG. 8, *B. burgdorferi* culture was grown in BSK-H medium for 7 days, and cell number was determined by microscope counting at different time points. The *B. burgdorferi* growth reached peaks ($5 \times 10^7$ spirochetes/mL) after 5-6 days. The microscope counts of cell density remained relatively constant until 11 days of incubation (FIG. 8A). Studies showed that persistent *B. burgdorferi* might be in different morphological forms, namely round bodies (cysts) and biofilm. These multiple morphological forms of *B. burgdorferi* might have different antibiotic susceptibilities (Brorson et al., 2009; Sapi et al., 2011). By microscope examination, the ratio of round bodies and biofilm-like colonies significantly

increased in the stationary phase *B. burgdorferi* culture (FIG. 8B). These stationary phase cultures may represent a mixed population of different morphological forms. The *B. burgdorferi* stationary phase culture of 7 days was chosen as a persistence model to screen for drugs.

*Evaluation of in vitro antibiotic susceptibility of stationary phase B. burgdorferi:* Previous studies and clinical experiences demonstrated that doxycycline and amoxicillin exhibit bactericidal activity against *B. Burgdorferi* (Hunfeld and Brade, 2006). The conventional antibiotics used for Lyme disease, such as doxycycline and penicillin, do not kill the cystic form of *B. burgdorferi,* yet some studies showed that metronidazole could kill the cystic form of *B. burgdorferi* (Brorson and Brorson, 1999). The presently disclosed subject matter discloses the effect of these frontline drugs (doxycycline, amoxillin and metronidazole) on log phase and stationary phase *B. burgdorferi* and evaluation and susceptibility by the SYBR Green I/PI assay. Treatment with clinical commonly used antibiotics showed these frontline drugs were effective against log phase *B. burgdorferi,* but had little effect against stationary phase *B. burgdorferi* (FIG. 8C). The result of microscope counting correlated with the result of the SYBR Green I/PI assay.

*Screening FDA-approved drug library for effective drugs against dormant B. burgdorferi:* For screening the effect of antibiotics on persister *B. burgdorferi,* stationary phase *B. burgdorferi* were used as a persistence model to screen an FDA-approved drugs library. Meanwhile, doxycycline and amoxicillin were added to each test plate as control drugs. Several measurements for the activity of control drugs revealed that the relative error of using the SYBR Green I/PI assay was less than 15%. Of the 1,514 drugs tested, dozens of antibiotics were considered to have a higher activity than the clinical commonly used antibiotics against the *B. burgdorferi* persisters (Table 1). Epi-fluorescence microscope counting further validated some effective candidates measured by SYBR Green I/PI, and the agreement of them was good with the largest difference less than 20%.

Table 1. Activity of top 27 active hits that had good activity (better than current clinical drugs) against stationary phase *B. burgdorferi* persisters[a]

| Drugs (50 μM) | Residual viable cells[b] | Residual viable cells[c] | Ratio of Green/Red fluoresce | | | | |
|---|---|---|---|---|---|---|---|
| | | | Primary screening | Rescreening | Rescreening | p-value[d] | p-value[e] |
| Control | 93% | 94% | 8.67 | 8.38 | 8.59 | - | - |
| Amoxicillin | 76% | 76% | 7.98 | 7.86 | 7.82 | 1.000000 | 0.2336 |
| Doxycycline | 75% | 67% | 7.62 | 7.35 | 7.58 | 0.233596 | 1.0000 |
| Penicillin G | 75% | 68% | 7.41 | 7.68 | 7.92 | 0.699416 | 0.3987 |
| Tetracycline | 54% | 50% | 7.59 | 6.14 | 7.18 | 0.102366 | 0.1712 |
| Ceftriaxone | 50% | 44% | 6.74 | 6.89 | 6.78 | 0.000182 | 0.0029 |
| Cefuroxime | 49% | 43% | 6.59 | 6.84 | 6.67 | 0.000317 | 0.0029 |
| Clarithromycin | 70% | 65% | 7.70 | 7.36 | 7.59 | 0.038775 | 0.3422 |
| Azithromycin | 77% | 80% | 8.33 | 8.10 | 7.92 | 0.071492 | 0.0703 |
| Daptomycin | 35% | 28% | 6.10 | 6.20 | 6.09 | 0.000008 | 0.0002 |
| Clofazimine | 45% | 32% | 6.56 | 6.23 | 6.02 | 0.000599 | 0.0017 |
| Cefoperazone | 37% | 34% | 6.54 | 6.32 | 6.23 | 0.000126 | 0.0008 |
| Carbomycin | 41% | 37% | 6.37 | 6.81 | 6.32 | 0.001045 | 0.0033 |
| Vancomycin | 48% | 38% | 6.65 | 6.58 | 6.37 | 0.000152 | 0.0011 |
| Cephalothin | 49% | 40% | 6.74 | 6.49 | 6.55 | 0.000133 | 0.0012 |
| Cefotiam | 42% | 43% | 6.41 | 7.55 | 6.21 | 0.000503 | 0.0840 |
| Cefmetazole | - | 43% | 6.80 | 7.38 | 6.00 | 0.045064 | 0.0767 |
| Cefepime | - | 44% | 6.67 | 7.16 | 6.45 | 0.006368 | 0.0162 |
| Amodiaquin | - | 45% | 6.79 | 6.44 | 6.85 | 0.000946 | 0.0040 |
| Control | 93% | 94% | 8.67 | 8.38 | 8.59 | - | - |
| Streptomycin | - | 45% | 6.72 | 6.93 | 6.76 | 0.000175 | 0.0022 |
| Ticarcillin | - | 46% | 6.82 | 6.72 | 6.93 | 0.000163 | 0.0023 |
| Cefonicid | - | 46% | 6.86 | 7.54 | 6.07 | 0.067661 | 0.1130 |
| Piperacillin -tazobactam | 47% | 47% | 7.18 | 6.47 | 6.98 | 0.009594 | 0.0253 |
| Cefdinir | - | 48% | 6.88 | 7.51 | 6.29 | 0.049107 | 0.0911 |
| Ceforanide | - | 48% | 6.89 | 7.49 | 6.33 | 0.043847 | 0.0839 |

(continued)

| Drugs (50 μM) | Residual viable cells[b] | Residual viable cells[c] | Ratio of Green/Red fluoresce | | | | |
|---|---|---|---|---|---|---|---|
| | | | Primary screening | Rescreening | Rescreening | p-value[d] | p-value[e] |
| Cefmenoxime | - | 48% | 6.82 | 7.59 | 6.32 | 0.058674 | 0.1062 |
| Bismuth | - | 48% | 6.94 | 6.82 | 6.92 | 0.000082 | 0.0024 |
| Ceftizoxime | - | 49% | 6.94 | 6.83 | 7.03 | 0.000223 | 0.0036 |
| Ceftibuten | 51% | 49% | 6.81 | 6.78 | 7.27 | 0.004888 | 0.0177 |
| Amphotericin B | - | 50% | 7.14 | 6.88 | 6.87 | 0.000783 | 0.0065 |
| Cefamandole | - | 50% | 6.71 | 7.73 | 6.52 | 0.076304 | 0.1378 |
| Quinine hydrobromide | - | 50% | 7.00 | 6.85 | 6.88 | 0.000124 | 0.0028 |
| Cyclacillin | 51% | 53% | 6.81 | 6.88 | 7.64 | 0.045210 | 0.1052 |
| Collistin | 50% | 54% | 7.15 | 7.26 | 7.23 | 0.000319 | 0.0135 |
| Sulfameter | | 54% | 7.13 | 7.46 | 6.98 | 0.009635 | 0.0451 |
| Tigecycline | 58% | 51% | 6.98 | 7.06 | 6.96 | 0.001557 | 0.0138 |

[a] Stationary phase *B. burgdorferi* (7-day old) cells were treated with drugs for 7 days.
[b]Residual viable *B. burgdorferi* was assayed by epifluorescence microscope counting.
[c]residual viable *B. burgdorferi* was calculated according to the regression equation and ratio of Green/Red fluorescence obtained by SYBR Green I/PI assay.
[d] the p-value of standard T-test for treated group and amoxicillin treated samples.
[e] the p-value of standard T-test for treated group and doxycycline treated samples.

Based on primary screening, some active candidates were selected (with residual viable cells less than 50%) for re-screening by SYBR Green I/PI assay and microscope counting. The re-screen confirmed the result of primary screening.

Several FDA-approved drugs were identified showing good bactericidal activity against stationary phase *B. burgdorferi.* Bactericidal activities of some drugs were significantly higher than that of frontline antibiotics doxycycline or amoxicillin (FIG. 9). For example, daptomycin, clofazimine, carbomycin, some cephalosporin antibiotics (such as cefoperazone, cefotiam and cefepime), streptomycin, and antimalarial antibiotic amodiaquim, showed relatively high bactericidal activities against stationary phase *B. burgdorferi.*

Representative images are shown of the stationary phase *of B. burgdorferi* strain B31treated by daptomycin, cefoperazone, and tetracycline (FIG. 10A, FIG. 10B, FIG. 10C, and FIG. 10D), as well as carbomycin and clofazimine (FIG. 11). Some of these compounds may block protein synthesis. For example, macrolides have greater activity than penicillin or ceftriaxone for *B. burgdorferi* persisters. Carbomycin, a macrolide, showed higher activity against *B. burgdorferi* than other macrolides, such as erythromycin. Other compounds may damage DNA and/or energy, such as clofazimine. Compounds also might block DNA synthesis. Some sulfa drugs, such as sulfameter and sulfisoxazole, were effective against stationary phase *B. burgdorferi*, while sulfamethoxazole also exhibited a low MIC (≤0.2 μg/mL).

Although most drugs did not affect the SYBR Green I/PI assay, some colored compounds caused interference to the SYBR Green I/PI assay. For example, pyrvinium pamoate and doxorubicin showed very high activity by the SYBR Green I/PI assay, but no bactericidal activity was observed by microscopic counting. It was found that these red compounds could make the background red and cause false positive results. Thus, validation by other methods, such as microscopic counting, to confirm the SYBR Green I/PI data is suggested.

*MIC test:* The MICs of some effective antibiotics against stationary phase *B. burgdorferi* were determined by the new SYBR Green I/PI assay and microscope counting. The results obtained from the two methods were the same. The MIC values (FIG. 12) of doxycycline, amoxicillin and metronidazole were in agreement with previous studies (Sapi et al., 2011; Hunfeld and Brade, 2006). Meanwhile it was found that log phase *B. burgdorferi* was very sensitive to carbomycin, cefoperazone, cefotiam and sulfamethoxazole (FIG. 12). On the other hand metronidazole, clofazimine, tazobactam, ketoconazole, miconazole were less potent against multiplying *B. burgdorferi* (FIG. 12).

The presently disclosed subject matter provides a rapid and convenient viability assay (e.g., SYBR Green I/PI) that is suitable for high-throughput screening for identifying new drugs and for rapidly evaluating antibiotic susceptibility of *B. burgdorferi* (Feng, Wang, Shi, et al., 2014). Using this rapid method, a FDA-approved compound library was screened for activity against non-replicating persisters of *B. burgdorferi.* A number of drug candidates were identified that have activity for *Borrelia* persisters.

Daptomycin is a lipopeptide antibiotic used in the treatment of infections caused by Gram-positive organisms. The presently disclosed data showed daptomycin had the highest activity against stationary phase *B. burgdorferi* persisters among all the active hits. Daptomycin could disrupt multiple aspects of bacterial cell membrane function. It inserts into the membrane, and creates pores that allow cells to leak ions, which causes rapid depolarization, resulting in a loss of membrane potential and bacterial cell death (Pogliano et al., 2012). The *B. burgdorferi* cells treated by daptomycin showed almost all red fluorescence as spirochetes (FIG. 10A) after staining. This result indicated the daptomycin could disrupt the cell membrane of *B. burgdorferi,* resulting in propidium iodide permeating into the cell. Microscope counting revealed few spheroplasts after daptomycin treatment; without wishing to be bound to any one particular theory, it is thought that the daptomycin could induce lysis of spheroplast cells.

Macrolides and ketolides were chosen as candidate antibiotics for clinical therapy of Lyme disease in previous studies (Hunfeld and Brade, 2006). Here, it has been found that carbomycin, a 16-membered macrolide, showed higher bactericidal activity against stationary phase *B. burgdorferi* than the classic macrolides, such as erythromycin and roxithromycin. The MIC data (FIG. 12) showed carbomycin also was effective against multiplying *B. burgdorferi.*

Treatment of *B. burgdorferi* with beta-lactams was commonly used therapy in a clinical setting (Hunfeld and Brade, 2006). Beta-lactams might induce round-body propagules of *B. burgdorferi* by disrupting the synthesis of the peptidoglycan layer of cell walls (Kersten et al., 1995). Microscopic examination also showed that round-body propagules (Brorsen et al., 2009) were the majority in the cefoperazone treated stationary phase *B. burgdorferi* (FIG. 10B). According to the MICs measured by previous studies, all beta-lactams showed good activity against multiplying *B. burgdorferi.* The presently disclosed subject matter shows, however, that a difference exists in the effects of beta-lactam antibiotics on stationary phase *B. burgdorferi* cells.

Cefoperazone, a third generation cephalosporin, appears to be the best beta-lactam antibiotic against stationary phase *B. burgdorferi,* followed by some second generation cephalosporins, such as cefotiam, cefmetazole and cefonicid. As in previous studies (Hunfeld and Brade, 2006), first generation cephalosporins showed very limited activity against stationary phase *B. burgdorferi.* It has been found that the activities of cephalosporins against stationary phase *B. burgdorferi* did not completely fit with the classic generation grouping of these antibiotics according to their spectrum of activity against Gram-negative and Gram-positive bacteria. This observation is probably related to the differences of *B. burgdorferi* from common gram-negative or gram-positive bacteria (Bergström and Zückert, 2010). Although beta-lactamase inhibitor tazobactam had a limited effect on multiplying *B. burgdorferi*, piperacillin-tazobactam was active against stationary phase *B. burgdorferi.* Genomic data showed *B. burgdorferi* possessed a gene coding putative beta-lactamase PhnP. Therefore, a beta-lactamase inhibitor might be helpful in reducing the resistance rates of *B. burgdorferi* to beta-lactams. In some embodiments, the optimized combination of beta-lactams and lactamase inhibitor has good activity against *B. burgdorferi* persistence.

Tetracycline antibiotics, especially doxycycline, are used in clinic settings as frontline drugs for Lyme disease. These antibiotics have lower MIC values (Hunfeld and Brade, 2006) and have good activity on multiplying *B. burgdorferi.* Interestingly, it was found that tetracycline had higher activity against stationary phase *B. burgdorferi* than doxycycline. It was observed that most cells were round-body propagules in the stationary phase *B. burgdorferi* treated by tetracycline (FIG. 10C). *B. burgdorferi* could form different morphological shapes in stationary phase or under adverse conditions, while tetracycline antibiotics might be ineffective on some morphological cells, such as round bodies (cysts) cells (Sapi et al., 2011).

In addition, some FDA-approved antibiotics were found to have bactericidal activity against stationary phase *B. burgdorferi* in the presently disclosed subject matter. Clofazimine was originally developed for the treatment of tuberculosis, although now it is commonly used for the treatment of leprosy (Arbiser and Moschella, 1995). The presently disclosed data showed that clofazimine was effective on the stationary phase *B. burgdorferi*, although the MIC of clofazimine is relatively high (6.25 μg/mL). Also, some sulfonamides, such as sulfameter and sulfisoxazole, were found to be effective against stationary phase *B. burgdorferi*, while sulfamethoxazole exhibited low values for MIC (<0.2 μg/mL). These effective antibiotics may be regarded as candidates for further drug combination studies in animal models and clinical investigations.

In summary, a FDA-approved drug library consisting of about 2,000 compounds was screened on stationary phase *B. burgdorferi* enriched in non-replicating persisters using a newly developed SYBR Green I/propidium iodide (PI) assay. A number of drug candidates that have excellent activity against *B. burgdorferi* persisters were identified from existing drugs used for treating other diseases or conditions. These drugs include daptomycin, clofazimine, carbomycin, sulfa drugs like sulfamethoxazole and certain cephalosporins, such as cefoperazone. The presently disclosed subject matter provides methods that can be used to identify novel anti-persister activity against bacteria in the *Borrelia* genus.

EXAMPLE 2

Drug Combinations against *Borrelia burgdorferi* Persisters *In Vitro:* Eradication Achieved by Using Daptomycin, Cefoperazone and Doxycycline

Materials and Methods

*Strain, media and culture:* The strain, media, and culture were obtained and used as in Example I.

*Antibiotics:* Doxycycline (Dox), amoxicillin (Amo), cefoperazone (CefP), clofazimine (Cfz), miconazole (Mcz), polymyxin B (Pmb), sulfamethoxazole (Smx), daptomycin (Dap), carbomycin (magnamycin A), vancomycin, nisin, carbencillin, ofloxacin, tigecycline, hydroxychloroquine, rifampin, and clarithromycin (Sigma-Aldrich) were dissolved in suitable solvents (Clinical and Laboratory Standards Institute, 2007) to obtain stock solutions. The antibiotic stocks were filter-sterilized by 0.2-μm filter except clofazimine, which was dissolved in DSMO (dimethylsulfoxide) and not filtered. Then the stocks were stored at -20°C.

*Microscopy techniques:* Specimens were examined on a Nikon Eclipse E800 microscope equipped with differential interference contrast (DIC) and epi-fluorescence illumination, and recorded with a Spot slider color camera. Cell proliferation assays were performed by direct counting using a bacterial counting chamber (Hausser Scientific Partnership) and DIC microscopy. SYBR Green I/PI assay was performed to assess the viability *of B. burgdorferi.* The ratio of live (green) and dead (red) *B. burgdorferi* was calculated by counting these cells using a bacterial counting chamber and epi-fluorescence microscopy. The three representative images of every sample were captured for quantitative analysis. Image Pro-Plus software was applied for measuring the biomass (fluorescence intensity) of different forms (spirochetes, round body, and microcolony) of *B. burgdorferi* as previously described (Shopov and Williams, 2000).

*Antibiotic exposure assay:* To qualitatively determine the effect of antibiotics, 10 μL of each compound from the pre-diluted plate or pre-diluted stock was added to stationary phase *B. burgdorferi* culture in the 96-well plate. The final volume per well was adjusted to 100 μL at a concentration of 10 μg/mL for each antibiotic. Plates were sealed and placed in a 33°C incubator for 7 days. The SYBR Green I/PI viability assay was used to assess the live and dead cells after antibiotic exposure as described (Feng, Wang, Shi, et al., 2014). Briefly, 10 μL of SYBR Green I (10,000 × stock, Invitrogen) was mixed with 30 μL propidium iodide (PI, 20 mM) into 1.0 mL of sterile $dH_2O$. Then, 10 μL of staining mixture was added to each well and mixed thoroughly. The plates were incubated at room temperature in the dark for 15 minutes followed by plate reading at excitation wavelength at 485 nm and the fluorescence intensity at 535 nm (green emission) and 635 nm (red emission) in microplate reader (HTS 7000 plus Bio Assay Reader, PerkinElmer Inc., USA). With least-square fitting analysis, the regression equation and regression curve of the relationship between percentage of live bacteria and green/red fluorescence ratios was obtained. The regression equation was used to calculate the percentage of live cells in each well of the 96-well plate.

*Subculture of antibiotic-treated B. burgdorferi to assess viability of the organisms:* Seven-day-old *B. burgdorferi* culture ($1\times10^7$ spirochetes/mL) (500 μL) was treated with drugs or drug combinations in Eppendorf tubes. After incubation at 33 °C for 7 days without shaking, the cells were collected by centrifugation and rinsed with 1 mL fresh BSK-H medium followed by resuspension in 500 μL fresh BSK-H medium without antibiotics. Then 50 μL of cell suspension was transferred to 1 mL fresh BSK-H medium for subculture at 33 °C for 2 weeks. Cell proliferation was assessed using SYBR Green I/PI assay and bacterial counting chamber (Hausser Scientific Partnership) by microscopy as described above.

Results

Taking advantage of a newly developed SYBR Green I/PI viability assay, an FDA-approved drug library was recently screened against stationary phase *B. burgdorferi* persisters and 27 drug candidates were identified that individually have higher activity than the currently recommended Lyme antibiotics doxycycline or amoxicillin (Example 1; Feng, Wang, Shi, et al., 2014). Among the top 27 confirmed drug candidates, daptomycin, clofazimine, carbomycin, sulfa drugs, such as sulfamethoxazole, and certain cephalosporins, such as cefoperazone, showed higher activity against *B. burgdorferi* persisters (Feng, Wang, Shi, et al., 2014). Interestingly, some drug candidates, such as daptomycin and clofazimine, with the highest activity against non-growing persisters had poor activity against actively growing *B. burgdorferi* with high MICs, at 12.5-25 μg/mL and 6.25 μg/mL, respectively (Feng, Wang, Shi, et al., 2014). Although these drug candidates active against persisters may not have good activity when used alone due to their poor activity against growing *B. burgdorferi,* it raises the question whether they may be used with another antibiotic, such as doxycycline, that is effective at inhibiting or killing the growing forms of *B. burgdorferi.* Such combinations may yield more effective treatment of Lyme disease.

Experimentally, since a stationary phase culture contains mixed populations of growing and non-growing bacteria that have different morphological variants, such as round bodies and microcolonies, that are tolerant to antibiotics (Feng, Wang, Shi, et al., 2014; Brorson et al., 2009; Sapi et al., 2011), it is most likely that a single drug may not effectively

kill all bacterial populations including morphological variants. In the presently disclosed subject matter, a range of drug combinations was evaluated with the aim to identify optimal drug combinations that are most effective at killing *B. burgdorferi* persisters.

*B. burgdorferi culture possesses different proportions of morphological variants including round body and microcolony forms as the culture ages:* As shown in a previous study (Feng, Wang, Shi, et al., 2014), the stationary phase culture was enriched with morphological variants, such as round body form and biofilm-like aggregated microcolony, form in increasing proportions in contrast to individual spirochetes found in log phase culture (FIG. 13). To more accurately assess the proportion of different morphological variant forms, representative images of each sample taken from cultures of different ages were examined to measure the percentage of different morphological forms of *B. burgdorferi* (Table 2). It was found that the log phase (3-day-old) *B. burgdorferi* culture consisted almost entirely of spirochetal form (96%), with few round body form (4%) and no aggregated microcolony form (FIG. 13A). In the 7-day-old stationary phase culture of *B. burgdorferi,* there were 38% spirochetal form, 23% cystic or round body form, and 39% microcolony form (FIG. 13B). When *B. burgdorferi* stationary phase culture was cultured for 10 days, the percentage of the microcolony form increased to 64%, and the spirochetal form and the round body form were 20% and 16%, respectively (FIG. 13C).

*Persister frequencies in log phase and stationary phase cultures:* Because *B. burgdorferi* does not form colonies easily on agar plates, the conventional method to assay persister frequency after antibiotic exposure by calculating the percentage of bacteria killed by a bacteriocidal antibiotic cannot be applied to *B. burgdorferi.* Therefore, the frequency of *B. burgdorferi* persisters in log phase and stationary phase cultures was assessed using the SYBR Green I/PI viability assay after exposure of the cultures to antimicrobials. *E. coli* culture was used as a control after exposure to antibiotics to validate the SYBR Green I/PI viability assay for persister frequency assessment. The persister frequency of the log phase *E. coli* culture with exposure to 50 μg/mL amoxicillin for 3 hours was 4.4% for the SYBR Green I/PI assay and 0.9% for the CFU assay (Table 1). Using the SYBR Green I/PI assay, the persister frequencies of *B. burgdorferi* ranged from 5-10% for log phase cultures, but ranged 16-27% in stationary phase cultures treated with ceftriaxone, doxycycline or amoxicillin (Table 1). Given that the SYBR Green I/PI viability assay seemed to give about 5 fold (4.4%/0.9%) overestimation of the persister frequency over the CFU assay with the *E. coli* control, the real persister frequencies of *B. burgdorferi* are likely to be in the range of 1-2% for *B. burgdorferi* log phase cultures and 3-5.5% for stationary phase cultures.

*Microcolony form is more tolerant to antibiotics than free-living spirochetal and round body forms:* Previous studies showed that the stationary phase *B. burgdorferi* was more resistant or tolerant to antibiotics than the log phase culture (Feng, Wang, Shi, et al., 2014). In view of the heterogeneity of the morphological variants of the stationary phase culture (FIG. 13B and FIG. 13C), the susceptibility of different variant forms of *B. burgdorferi* to commonly employed antibiotics for Lyme disease (doxycycline, amoxicillin, and ceftriaxone) was determined in a more quantitative manner. Interestingly, it $_{\text{was}}$ found that different variant forms had differing susceptibilities to these antibiotics (Table 2). The log phase culture (3-day-old) primarily consisting of spirochetal form was highly susceptible to these antibiotics, whereas the stationary phase (7- and 10-day old) cultures comprising mainly of round body and biofilm-like microcolony forms were less sensitive to these antibiotics, as shown by increasing proportion of viable cells remaining after the antibiotic exposure (Table 2).

FIG. 14 shows the effect of drugs (50 μg/mL) and combinations on stationary phase *Borrelia.* FIG. 15 shows some promising drug combinations against *Borrelia* biofilm. FIG. 16 shows the activity of drug combinations against *Borrelia* biofilm.

When the 10-day-old stationary phase culture, consisting of mixed populations of spirochetal form in minor portions and round body form and microcolony form in major proportions, was exposed to various antibiotics, it was found that the microcolony form was more tolerant to antibiotics than the free-living spirochetal form and the round body form. Daptomycin at 10 μg/mL, a drug with high activity against *B. burgdorferi* persisters (Feng, Wang, Shi, et al., 2014), killed all planktonic forms (spirochetal and round body) of stationary phase cells (FIG. 17A, Panel h), but could only partially kill the microcolony form of *B. burgdorferi* persisters as shown by the presence of significant numbers of red cells (dead cells) mixed with some green cells (viable cells) in the microcolony (FIG. 17A, Panel h). The other persister active drug cefoperazone (Feng, Wang, Shi, et al., 2014) had weaker activity than daptomycin since it had some activity for the planktonic form cells (52% cells were green cells), but little activity for the microcolony form of persisters where most of the microcolony cells remained as green (live) cells (FIG. 17A, Panel e). In contrast, doxycycline had the least activity against stationary phase *B. burgdorferi* persisters where about 71 % free-living planktonic cells including spirochetal form and round body form were not killed by doxycycline as shown by green (live) cells (FIG. 17A, Panels d, f and j), but the microcolony form was almost all live (FIG. 17A, Panels c, e and i). These findings suggest there is a differential tolerance or resistance in different variant forms of persisters *in vitro* (spirochetal form, round body form and microcolony in increasing order of resistance) to both current Lyme disease antibiotics and also even persister active antibiotics daptomycin and cefoperazone, with the microcolony form being the most tolerant to antibiotics.

*Effect of drug combinations on stationary phase B. burgdorferi persisters:* Despite the powerful anti-persister activity of daptomycin and cefoperazone, they had limited activity to kill the most resistant microcolony form of persisters

at 10 μg/mL (FIG. 17). These findings suggest that these FDA-approved persister drugs may have limited potential if used alone against *B. burgdorferi.* To identify more effective drug combinations that kill different variant forms *of B. burgdorferi* stationary phase persisters, 81 drug combinations were evaluated including FDA-approved drugs on a 10-day-old *B. burgdorferi* culture enriched with microcolony and round body forms at 10 μg/mL of each individual drug (close to or lower than MIC). The results showed that some drug combinations were indeed much more effective than single drugs alone (Table 2). Among them, daptomycin highlighted itself as having the best activity against stationary phase *B. burgdorferi* persisters when combined with other drugs.

Daptomycin (10 μg/mL) alone could not eliminate the microcolonies by itself (FIG. 17A, Panel g), but daptomycin in combination with doxycycline or beta-lactams was very effective against *B. burgdorferi* planktonic persisters and also against microcolonies (Table 2, FIG. 17B). However, daptomycin in combination with doxycycline or cefoperazone produced better bacteriocidal activity for the microcolony form than either of these agents alone or drug combinations without daptomycin, such as doxycycline + cefoperazone or even doxycycline + cefoperazone + sulfamethoxazole, as shown by more red cells (dead cells) being produced after daptomycin drug combinations (FIG. 17B, Panels f, g, i, j, k and 1). Nevertheless, daptomycin used as part of two drug combinations did not completely eradicate microcolony form of persisters (FIG. 17B, Panels f and g). Remarkably, daptomycin as part of a three drug combination using doxycycline and cefoperazone eradicated all microcolonies with only few traces of red (dead) cells left (FIG. 17B, Panels h, i, j, k and 1), whereas other daptomycin-containing three drug combinations using cefoperazone + either carbenicillin or carbomycin or clofazimine still had some red microcolonies remaining after treatment.

In addition to doxycycline and beta-lactams, some clinical drugs, such as vancomycin, ofloxacin, clarithromycin, and hydroxychloroquine, which are not recommended for treating Lyme disease, also exhibited some weak activity on the 10-day-old stationary phase *B. burgdorferi* culture, either alone or in combination with doxycycline and cefoperazone. Rifampin alone did not have significant activity for *B. burgdorferi* persisters, but in combination with doxycycline, amoxicillin, ceftriaxone or cefoperazone had higher activity for *B. burgdorferi* persisters (Table 3). Among all the other non-daptomycin drug combinations, the only two drug combinations that are close to daptomycin drug combinations in killing *B. burgdorferi* persisters were Dox + either CefP or miconazole or sulfamethoxazole (Table 3). In addition, clofazimine showed good activity against stationary phase *B. burgdorferi* persisters when combined with doxycycline and cefoperazone (Table 3). It is worth noting that the activity of carbenicillin, vancomycin, ofloxacin, clarithromycin, tigecycline, nisin, and hydroxychloroquine when combined with doxycycline only marginally enhanced doxycycline activity and their anti-persister activities were not as effective as when they were combined with daptomycin (Table 3).

*Subculture of antibiotic-treated B. burgdorferi:* In a previous study, it was found that daptomycin at 50 μM (equivalent to 81 μg/mL, a high dose to achieve in humans) had remarkable anti-persister activity that seemed to kill all *B. burgdorferi* persisters, as shown by all red cells stained by PI (FIG. 3D in Feng, Wang, Shi, et al., 2014). To confirm that these red cells are indeed dead, a subculture test in fresh BSK-H medium was performed and it was found that indeed these red cells treated with 50 μM daptomycin were dead as they failed to grow in the subculture test as shown by lack of any visible green spirochetes after 15 day subculture (data not shown). Having established the subculture test as a reliable assay for assessing the viability of antibiotic treated cells, the above results obtained with select antibiotics or antibiotic combinations that produced the best bacteriocidal effects against persisters were validated (see FIG. 17).

To do this, a 7-day-old stationary phase *B. burgdorferi* culture was subjected to exposure with select antibiotics and antibiotic combinations for 7 days, followed by subculture in fresh BSK-H medium for 7 days or 15 days. Microscope counting showed that drug-free controls and samples treated with single drug grew in the 7-day subculture. Samples treated with two drug combinations grew more slowly (Table 4). However, after the 7-day subculture, all the three drug combinations, e.g., doxycycline+daptomycin+ either cefoperazone or Smx or Cfz did not show any sign of growth as no visible spirochetal form was observed, whereas other drug combinations all had visible green spirochetes under the microscope. After the 15-day subculture, there were about $1\times10^7$ spirochetes in the control sample and $5\times10^6$ spirochetes in doxycycline or amoxicillin treated samples (Table 4). Interestingly, daptomycin alone, or two drug combinations doxycycline+cefoperazone and doxycycline+daptomycin, or even three drug combination doxycycline+daptomycin+clofazimine, could not sterilize the *B. burgdorferi* persisters, as they all had visible spirochetes growing after subculture (FIG. 18).

However, doxycycline+daptomycin+sulfamethoxazole significantly reduced the number of spirochetes with very few spirochetes being visible after the 15-day subculture (FIG. 18h). By far the best result was achieved with daptomycin in combination with doxycycline and cefoperazone, which killed all *B. burgdorferi* persisters with no viable bacteria observed (FIG. 18i). This is demonstrated by a decrease in the Green/Red fluorescence and lack of any viable green spirochetes, in contrast to samples treated with other drugs alone or drug combinations, which all had higher Green/Red fluorescence and visible green spirochetal bacteria (Table 4, FIG. 18). Importantly, this drug combination could eliminate not only planktonic stationary phase *B. burgdorferi* persisters (spirochetal and round body forms), but also the more resistant biofilm-like microcolonies (Table 4, FIG. 18). Subculturing the sample treated with this drug combination showed no sign of any detectable organisms by microscopy (detection limit < $10^5$) even after 15 days of

subculture (Table 4, FIG. 18i). These findings indicate that the microcolony structures are not eliminated by doxycycline, amoxicillin, persister active drugs alone, two drug combinations or even some three drug combinations, but could be eradicated by the drug combination of doxycycline, cefoperazone and daptomycin.

Table 2. Varying degrees of susceptibility of different forms of *B. burgdorferi* to commonly used Lyme antibiotics

| | Percentage of different forms *of B. burgdorferi*[a] | | | Percentage of residual viable cells[b,c] | | |
|---|---|---|---|---|---|---|
| | Spirochete | Cystic or round body form | Microcolony | Doxycycline | Amoxicillin | Ceftriaxone |
| 3-day log phase cultured | 96% | 4% | 0% | 8% (6.4%) | 23% (9.6%) | 6% (5.8%) |
| 7-day stationary phase culture | 38% | 23% | 39% | 71% (24%) | 80% (25%) | 47% (16%) |
| 10-day stationary phase culture | 20% | 16% | 64% | 80% (~25%) | 83% (~27%) | 70% (~25%) |

a. Percentages of different forms of *B. burgdorferi* were calculated by measuring three representative microscope images with Image Pro-Plus software.
b. Percentages of residual viable *B. burgdorferi* relative to drug-free control after drug treatment were calculated according to the regression equation and ratio of Green/Red fluorescence obtained by SYBR Green I/PI assay as described (Feng, Wang, Shi, et al., 2014). The samples were treated with antibiotics for 7 days before viability was assessed by the SYBR Green I/PI assay.
c. Values in brackets indicate persister frequencies (percentage of live cells after antibiotic treatment). The number of *B. burgdorferi* assayed by epi-fluorescence microscope counting was calibrated using *E. coli* as a control.
d. The log phase culture was obtained by subculture of a stationary phase culture at 1:50 dilution for 3 days in BSK medium.
e. Three hour log phase *E. coli* culture ($1.7 \times 10^8$ cfu/mL) was treated with 50 $\mu$g/mL amoxicillin for 3 hours followed by persister frequency determination.
f. Persister frequency calculated by epi-fluorescence microscope counting after SYBR Green I/PI viability staining.
g. Persister frequency calculated by standard plate colony count assay.

Table 3. Effect of drug combinations on stationary phase *B. burgdorferi* culture[a]

| Live Cell % | C | Mcz | Pmb | Smx | Dap | Cfz[b] | Car | Van | Cab | Ofl | Clar | Tig | Hcq | Rif[b] |
|---|---|---|---|---|---|---|---|---|---|---|---|---|---|---|
| C | 87% | 65% | 77%[c] | 82%[c] | 52% | 73% | 65% | 64% | 67% | 81%[c] | 63% | 67% | 71% | 82%[c] |
| Dox | 72% | 53% | 64% | 71% | 32% | 43% | 59% | 63% | 64% | 60% | 60% | 59% | 68% | 60% |
| Amo | 75%[c] | 51% | 75% | 68% | 41% | 56% | 63% | - | - | - | - | - | - | 60% |
| Ceftriaxone | 68% | 48% | 64% | 64% | 41% | 57% | 63% | - | - | - | - | - | - | 59% |
| Cefoperazone | 64% | 45% | 64% | 65% | 41% | 46% | 60% | 64% | 63% | 62% | 63% | 51% | 53% | 58% |
| Dox+CefP | 59% | 39% | 59% | 37% | **19%** | 43% | 55% | 50% | 59% | 56% | 55% | 51% | 48% | - |
| Dap | 48% | 37% | 35% | **27%** | 35% | **20%** | **26%** | **23%** | **20%** | **27%** | 32% | **23%** | 31% | - |

(continued)

| Live Cell % | C | Mcz | Pmb | Smx | Dap | Cfz[b] | Car | Van | Cab | Ofl | Clar | Tig | Hcq | Rif[b] |
|---|---|---|---|---|---|---|---|---|---|---|---|---|---|---|
| Dap+Dox | 34% | 34% | 33% | **24%** | 32% | **20%** | **23%** | **25%** | **16%** | **20%** | 31% | **21%** | **25%** | - |

a. Ten-day-old stationary phase *B. burgdorferi* culture enriched with micro-colonies was treated with 10 μg/mL drugs alone or in different combinations for 7 days. The percentage of residual viable *B. burgdorferi* was calculated according to the regression equation and ratio of Green/Red fluorescence using the SYBR Green I/PI assay as described (Feng, Wang, Shi, et al., 2014). Direct microscopy counting was employed to verify the results of SYBR Green I/PI assay. The most effective drug combinations as reflected by residual viable cell percentages of less than 30% are shown in bold type. The best drug combinations without daptomycin are underlined. Abbreviations: Dox, doxycycline; Amo, amoxicillin; CefP, cefoperazone; Cfz, clofazimine; Mcz, miconazole; Pmb, polymyxin B; Dap, daptomycin; Smx, sulfamethoxazole; Cab, carbencillin; Car, carbomycin; Van, vancomycin; Ofl, ofloxacin; Clar, clarithromycin; Tig, tigecycline; Hcq, hydroxychloroquine; Rif, rifampin. "-" indicates not determined. C = drug-free control
b. To eliminate the influence of red color of antibiotics, fluorescence data was corrected using antibiotic control.
c. P-values of the standard *t*-test for the all treated group versus the drug-free control were less than 0.01 except the data marked with "c".

Table 4. Subculture tests to assess the viability of drug-treated stationary phase *B. burgdorferi*.[a]

| Drugs[b] | Residual viable cells[c] | G/R ratio immediately after treatment[d] | After 7 day subculture | | After 15 day subculture | |
|---|---|---|---|---|---|---|
| | | | G/R ratio[d] | Spirochetes[e] | G/R ratio[d] | Spirochetes[e] |
| Control | 82% | 7.32 | 7.28 | $5\times10^6$ | 7.38 | $6\times10^6$ |
| Dox | 67% | 6.71 | 6.23 | $9\times10^5$ | 6.89 | $4\times10^6$ |
| Amoxicillin | 80% | 7.16 | 6.32 | $1\times10^6$ | 7.23 | $6\times10^6$ |
| Dox+Dap+CefP | 10% | 5.32 | 4.93 | $<1\times10^5$ | 4.82 | $<1\times10^5$ |
| Dox+Dap+Cfz | 15% | 4.96 | 4.86 | $<1\times10^5$ | 7.38 | $6\times10^6$ |
| Dox+Dap+Smx | 18% | 5.85 | 5.34 | $<1\times10^5$ | 6.41 | $2\times10^6$ |
| Dox+Dap | 23% | 6.03 | 5.91 | $3\times10^5$ | 7.31 | $6\times10^6$ |
| Dox+CefP | 56% | 6.35 | 6.01 | $7.5\times10^5$ | 6.87 | $4\times10^6$ |
| Dap | 55% | 5.85 | 7.01 | $3.1\times10^6$ | 7.11 | $5\times10^6$ |
| CefP | 61% | 6.60 | 6.13 | $1.5\times10^6$ | 6.95 | $5\times10^6$ |

a. Seven-day-old *B. burgdorferi* culture ($1\times10^7$ spirochetes/mL) (500 μL) was treated with 10 μg/mL drugs alone or drug combinations for 7 days. Then, 50 μL of washed bacterial cells was subcultured in 1 mL fresh BSK-H medium for 7 days and 15 days.
b. Abbreviations: G/R ratio, Green/Red fluorescence ratio; Dox, doxycycline; CefP, cefoperazone; Cfz, clofazimine; Dap, daptomycin; Smx, sulfamethoxazole.
c. Residual viable *B. burgdorferi* was assayed by epifluorescence microscope counting.
d. Green/Red fluorescence ratios were obtained by microplate reader after SYBR Green I/PI staining. Each value is the mean of three replicates.
e. The number of spirochetes was evaluated by microscope counting.

## Discussion

In the presently disclosed subject matter, the first *in vitro* drug combination study using persister active drugs was conducted (Feng, Wang, Shi, et al., 2014) in combination with the currently recommended Lyme antibiotics, such as doxycycline or amoxicillin or other antibiotics, to achieve more effective eradication of *B. burgdorferi* persisters. It was found that it is more effective to kill *B. burgdorferi* persisters by drug combination than single antibiotic, but bacteriocidal activity depended on the particular antibiotics used (Table 3). It is interesting to note that despite the persister active antibiotics, such as the lipopeptide daptomycin and beta-lactam cefoperazone themselves, were quite active

against planktonic *B. burgdorferi* persisters (both spirochetal and round body forms), they were unable to eradicate the more resistant microcolony form when used alone or even in combination (FIG. 17). Previous studies showed that tinadazole, metronidazole, and tigecycline were more active against *B. burgdorferi* round body and microcolonies than doxycycline and amoxicillin, but they could not completely kill the microcolonies even at high concentrations of antibiotics (Sapi et al., 2011), indicating the limited activity of these antibiotics used singly against *B. burgdorferi* persisters. Although tigecycline was the most active antibiotic against the round body form compared with tinadazole and metronidazole in that study (Sapi et al., 2011), it was found that by itself tigecycline was not very effective at killing the biofilm-like microcolonies (Table 3).

Remarkably, it was found that daptomycin in combination with doxycycline and cefoperazone or carbencillin was able to completely eradicate the most resistant microcolonies (FIG. 17), and this was further confirmed by subculture studies, which showed lack of any growth (FIG. 18). While various drug combinations showed improved activity against stationary phase *B. burgdorferi* persisters, daptomycin combinations had the best activity among drug combinations against persisters (Table 3). The only non-daptomycin regimens that were close to daptomycin combinations contained cefoperazone (FIG. 17, Table 3). Unexpectedly, other antibiotics, such as sulfamethoxazole, clofazimine and miconazole, also had more activity against stationary phase *B. burgdorferi* persisters in combination with doxycycline and cefoperazone. These drugs are not currently used as antibiotics for treatment of Lyme disease clinically (CDC, Post-Treatment Lyme Disease Syndrome, 2014; Hunfeld and Brade, 2006). Although sulfa drugs are bacteriostatic when used alone for growing bacteria, they could kill non-growing round body or aggregated microcolony form of *B. burgdorferi* during long-term treatment. Clofazimine with high anti-persister activity improved the combination with daptomycin or daptomycin plus doxycycline (Table 3), which may be due to its multiple mechanisms of action including membrane destabilization, reactive oxygen species production, and inhibition of membrane energy metabolism in *M. tuberculosis* (Xu et al., 2012). It also was found that miconazole, an imidazole antifungal drug, had high activity against *B. burgdorferi* persisters when combined with doxycycline and cefoperazone (Table 2). Miconazole has been shown to alter the integrity of lipid membrane (Vanden Bossche et al., 1989) and therefore may facilitate the penetration of other drugs, such as doxycycline and cefoperazone, for improved activity against *B. burgdorferi* persisters (Table 3).

The complete eradication of the *B. burgdorferi* biofilm-like microcolonies by the three drug combination of daptomycin+doxycycline+cefoperazone has not been achieved with any single, dual or even some three drug combinations in the presently disclosed subject matter or any other previous studies. The mechanism by which this three drug combination was able to achieve this remarkable activity is worth commenting. Without wishing to be bound to any one particular theory, doxycycline and cefoperazone inhibits protein synthesis and cell wall peptidoglycan synthesis respectively (Kersten et al., 1995). Either may be needed to kill the growing forms present in the *B. burgdorferi* microcolonies or those occasionally revert to growing forms from microcolonies, but these drugs are less effective against the round body or microcolony persisters themselves (Feng, Wang, Shi, et al., 2014; Brorson et al., 2009; Sapi et al., 2011). This inability could be because of the reduced drug penetration into the microcolony structure, efflux mechanism (Brorson et al., 2009; Casjens, 2000), or decreased protein or cell wall synthesis in persisters. The high efficacy of daptomycin against *B. burgdorferi* persisters could be due to its effect on membrane disruption or depolarization, resulting in a loss of membrane potential and inhibition of energy metabolism (Feng, Wang, Shi, et al., 2014; Pogliano et al., 2012), which is required for persister survival (Zhang, 2014). Prior studies have suggested that the combination of beta-lactams plus daptomycin increase effectiveness even with daptomycin resistant Gram-positive infections (Dhand et al., 2011). While drugs traditionally active against Gram-positive organisms are not thought to have activity against *B. burgdorferi*, *in vitro* studies have previously documented activity with drugs, such as vancomycin (Hall et al., 2014; Dever et al., 1993), but not teicoplanin or daptomycin, though this study was performed examining not persisters but log phase cultures. Though daptomycin is not used for Gram-negative pathogens, a drug, such as colistin, has been suggested to improve polyanionic lipopeptide activity due to outer membrane permeabilization (Morris et al., 1995). Regardless, these studies suggest that combined use of these agents that kill or inhibit both growing organisms (doxycycline and cefoperazone) and non-replicating organisms (daptomycin and cefoperazone) are important for good activity against the highly resistant microcolonies, which is consistent with the proposition to use drugs targeting both growing and non-growing microbial populations for improved treatment of persistent infections (Zhang, 2014).

It is worth noting that short term incubation in subculture studies of antibiotic treated *B. burgdorferi* is not sufficient to assess the stable eradication of persisters. This is shown by the 7-day subculture of *B. burgdorferi* persister cells treated with three drug combinations daptomycin+doxycycline+cefoperazone or Smx or Cfz, which all produced no detectable levels of any residual growth (Table 4). However, extended incubation to 15 days of subculture showed that only daptomycin, doxycycline and cefoperazone combination was able to completely eradicate biofilm-like microcolonies with no detectable spirochetes (FIG. 18i). These findings suggest that longer incubation to 15 days or more in post-antibiotic exposure may be needed to thoroughly assess the drug combinations that stably eradicate the persister forms without relapse. The subculture results do validate the SYBR Green I/PI viability assay and is a useful and more sensitive technique to assess the viability of *B. burgdorferi* persisters or microcolonies after drug treatment in identifying optimal drug combinations for killing more resistant persisters.

*B. burgdorferi* spirochetes could develop morphological variants as *in vitro* cultures age or are subjected to adverse conditions (Feng, Wang, Shi, et al., 2014; Brorson et al., 2009; Alban et al., 2000; Sapi et al., 2011; Murgia and Cinco, 2004). The proportions of these variants have not been well characterized over time in culture conditions. With careful measurement, the percentages of morphological variants were determined as they transitioned from spirochetes to progressively round body form to then microcolony form as log phase culture grew to stationary phase (7-10 days) (FIG. 13). Although previous studies reported the round body form or biofilm-like microcolony form is more resistant to antibiotics (Feng, Wang, Shi, et al., 2014; Brorson et al., 2009; Sapi et al., 2011), their relative resistance was not fully studied. Here, a hierarchy or varying levels of stationary phase *B. burgdorferi* persisters have been found in terms of their levels of persistence as the morphology of the variants changes from spirochetes, to round body, and to microcolony forms, with increasing antibiotic tolerance (Table 2).

The finding that persister frequencies are higher in stationary phase *B. burgdorferi* cultures than in log phase cultures is consistent with studies in other bacteria. However, the persister frequencies in *B. burgdorferi* log phase culture (5-10%) and stationary phase cultures (16-27%) determined by SYBR Green I/PI assay seem to be higher than those reported for *E. coli* (0.001% in log phase to 1% in stationary phase) (Keren et al., 2004). Given that the SYBR Green I/PI assay tended to overestimate the persister frequency by about 5 fold based on the *E. coli* data (4.4%/0.9%) (Table 2), the converted persister frequencies of 1-2% and 3-5% for *B. burgdorferi* log phase and stationary phase cultures would still suggest higher persister frequencies with *B. burgdorferi.* This could reflect differences in their ability to form persisters, the speed of growth of the organisms, the age of culture when antibiotic is added, and the dilution factor, which affects the number of persisters carried over during the subculture. In addition, it has been found that the persister frequencies vary according to the antibiotic used, with the more effective antibiotic ceftriaxone having a lower persister frequency than amoxicillin (Table 2), a finding that is consistent with previous studies (Zhang, 2014; Lu and Zhang, 2007). It remains to be determined if there are differences in persistence of *B. burgdorferi* strains and if the high persister frequencies in *B. burgdorferi* strains are associated with recalcitrance to antibiotic therapies.

In summary, it has been found that there is a hierarchy of *in vitro B. burgdorferi* persisters with increasing antibiotic tolerance as the culture ages from log phase to stationary phase with morphological changes from spirochetal form to round body and microcolony forms. Persister frequencies in log phase *B. burgdorferi* culture ranged 5.8-9.6% depending on the antibiotic as measured by SYBR Green I/propidium iodide (PI) viability stain and microscope counting, but the corrected log phase *B. burgdorferi* persister frequencies were at 1-2% using *E. coli* as a control. To more effectively eradicate these persister forms tolerant to doxycycline or amoxicillin, drug combinations were studied using previously identified drugs from an FDA-approved drug library with high activity against *B. burgdorferi* persisters. Using a SYBR Green/PI viability assay, daptomycin-containing drug combinations were the most effective at killing *B. burgdorferi* persisters. Of studied combinations, daptomycin was the common element in the most active regimens against persisters when used with doxycycline plus either beta-lactams (cefoperazone or carbenicillin) or energy inhibitor (clofazimine). Daptomycin plus doxycycline and cefoperazone eradicated the most resistant microcolony form of *B. burgdorferi* persisters and did not yield viable spirochetes upon subculturing, suggesting durable killing of these persisting forms, which was not achieved by any other two or three drug combinations. These findings may have implications for treatment of Lyme disease patients with stubborn ongoing symptoms or antibiotic-refractory arthritis, if persistent organisms or detritus are responsible for symptoms that do not resolve with conventional therapy.

## EXAMPLE 3

### FDA-approved Drugs Active Against the Round Body Form of *Borrelia burgdorferi* Persisters

### Materials and Methods

*Strain, media and culture: B. burgdorferi* strain B31 was obtained from American Type Tissue Collection. *B. burgdorferi* and was cultured in BSK-H media (HiMedia Laboratories Pvt. Ltd.), with 6% rabbit serum (Sigma-Aldrich, Co). All culture media were filter-sterilized by 0.2 μM filter. Cultures were incubated in sterile 50 mL closed conical tubes (BD Biosciences, California, USA) at 33°C without antibiotics.

*Induction of round body form of B. burgdorferi:* For induction of round body form of *B. burgdorferi, B. burgdorferi* spirochetes ($1 \times 10^5$ spirochetes/mL) were cultured in BKS-H medium for 6 days without shaking. After the 6-day incubation, amoxicillin at a final concentration of 50 μg/mL was added to the culture for round body form induction. After 72 h induction at 33°C, the round body forms of *B. burgdorferi* were examined by the microscopy. The round body cells (100 μL) were transferred to 96-well tissue culture microplates for evaluation of the effects of antibiotic treatment.

*Microscopy techniques:* Specimens were examined on a Nikon Eclipse E800 microscope equipped with differential interference contrast (DIC) and epi-fluorescence illumination, and recorded with a Spot slider color camera. Cell proliferation assays were performed by direct counting using a bacterial counting chamber (Hausser Scientific Partnership, PA, USA) and DIC microscopy. To assay the viability of *B. burgdorferi,* the SYBR Green I/PI assay (Feng, Wang,

Shi, et al., 2014) was performed. The ratio of live (green) and dead (red) *B. burgdorferi* was calculated by counting the cells using a bacterial counting chamber and epi-fluorescence microscopy.

*Antibiotics and FDA drug library:* Doxycycline, metronidazole, cefmetazole, roliteracycline, sulfachlorpyridazine, artemisinin, cefoperazone, daptomycin (Sigma-Aldrich) were dissolved in suitable solvents (Wikler and Ferraro, 2008) to form stock solutions. The antibiotic stocks were filter-sterilized by 0.2 μm filter. Then the stocks were pre-diluted into 500 μM pre-diluted stocks and stored at -20°C.

Each drug in the JHCCL FDA-approved drug library (Ricker et al., 2011) was made to 10 mM stock solutions with DMSO. The stock solutions were arrayed in a total of 24 96-well plates, leaving the first and the last columns in each plate as controls. Each solution in these master plates was diluted with PBS to make 500 μM pre-diluted working stock plates. The first and the last columns in each pre-diluted plate were set as blank controls, doxycycline control, and amoxicillin control. The pre-diluted drug stock plates were sealed and stored at -20°C.

*Antibiotic susceptibility test:* To qualitatively determine the effect of antibiotics, 10 μL of each compound (final concentration 50 μM) from the pre-diluted plate or pre-diluted stock was added to round body form or stationary phase *B. burgdorferi* culture in the 96-well plate. The final volume per well was adjusted to 100 μL. Plates were sealed and placed in a 33°C incubator for 7 days. The SYBR Green I/PI viability assay was used to assess the live and dead cells after antibiotic exposure as described (Feng, Wang, Shi, et al., 2014). Briefly, 10 μL of SYBR Green I (10,000 × stock, Invitrogen) was mixed with 30 μL propidium iodide (PI, 20 mM, Sigma-Aldrich) into 1.0 mL of sterile $dH_2O$. Then, 10 μL staining mixture was added to each well and mixed thoroughly. The plates were incubated at room temperature in the dark for 15 minutes followed by plate reading at excitation wavelength at 485 nm and the fluorescence intensity at 535 nm (green emission) and 635 nm (red emission) in microplate reader (HTS 7000 plus Bio Assay Reader, PerkinElmer Inc., USA). With least-square fitting analysis, the regression equation and regression curve of the relationship between percentage of live and dead bacteria as shown in green/red fluorescence ratios was obtained. The regression equation was used to calculate the percentage of live cells in each well of the 96-well plate.

## Results

*Induction of round body form of B. burgdorferi by amoxicillin:* Beta-lactam antibiotics are the most commonly used frontline drugs for the treatment of Lyme disease, but intriguingly could induce spirochetal *B. burgdorferi* to form round bodies which are resistant to Lyme antibiotics (Brorson et al., 2009; Sapi et al., 2011). In order to identify FDA-approved drugs active against the round body form of *B. burgdorferi,* the optimal conditions for induction of round body form were assessed. It was found that 6-day or older culture could not be induced to round body form completely with even 100 μg/mL amoxicillin (FIG. 19D). It was found that the best condition for producing the round body for use in a FDA drug library screen was 5-day *B. burgdorferi* culture treated with 50 μg/mL amoxicillin for 72 h. Microscopic examination showed that under the above inducing condition, up to about 96% of the *B. burgdorferi* spirochetes could be induced into round body form by amoxicillin (FIG. 19A). To confirm that the induced round body form was still viable, a subculture test in fresh BSK-H medium was performed. The round bodies (in 500 μL culture) were collected by centrifugation and rinsed with 1 mL fresh BSK-H medium followed by resuspension in 500 μL fresh BSK-H medium. Then, 50 μL of cell suspension was transferred to 1 mL fresh BSK-H medium for subculture at 33 °C for 5 days. Microscopy analysis revealed that the amoxicillin-induced round body form of *B. burgdorferi* could revert to spirochetes (up to 95%) in BSK-H medium after the 5-day subculture (FIG. 19), indicating that the round body form induced by and tolerant to amoxicillin treatment is fully viable.

To compare the antibiotic susceptibility of the round body form of *B. burgdorferi* with the spirochetal form, commonly used Lyme disease antibiotics doxycycline, cefuroxime, and ceftriaxone were tested on 5-day old spirochetes and the amoxicillin induced round body form of *B. burgdorferi.* The results showed that the round body form of *B. burgdorferi* was more tolerant or resistant to antibiotics than the spirochetal form (FIG. 20). The amoxicillin induced round body form was subsequently used for drug screens as described below.

*Screen for effective drugs against the round body form of B. burgdorferi:* In a previous study, a SYBR Green I/PI assay was developed which can be used as a high-throughput screening method for rapid viability assessment for *B. burgdorferi* (Feng, Wang, Shi, et al., 2014). In this study, this rapid SYBR Green I/PI assay was used to identify drugs which have activity against the round body form of *B. burgdorferi* persisters by using an FDA-approved drug library. Since metronidazole was shown to kill the round body form of *B. burgdorferi* (Amant et al., 2012), metronidazole and doxycycline were included as control drugs in the screen. In the initial screen, the effective hits were selected as having residual viable cell ratios below that of the amoxicillin control. Hit compounds were selected for further rescreens, followed by microscope counting to verify the screening results. Epi-fluorescence microscope counting further validated the effective drug candidates by the SYBR Green I/PI assay (data not shown). Of the 1582 FDA-approved drugs tested, 23 drugs were found to have higher activity against the round body form of *B. burgdorferi* than doxycycline (Table 5). Among the 23 hits that were more active than doxycycline, 11 drugs, daptomycin, artemisinin, ciprofloxacin, sulfacetamide, sulfamethoxypyridazine, nifuroxime, fosfomycin, chlortetracycline, sulfathiazole, clofazimine and cefmenoxime, in order

of decreasing activity, had better activity than the known round body active antibiotic metronidazole or tinidazole (Table 5). Antimalarial drug artemisinin showed high activity against the round body form of *B. burgdorferi.* Interestingly, ciprofloxacin (28% residual live cells) was the most active among quinolone drugs levofloxacin 41 %, norfloxacin 41 %, and moxifloxacin 49% (not shown). In addition, chlortetracycline, meclocycline and rolitetracycline were more active than doxycycline (42% residual live cells) against the round body forms (Table 5). On the other hand, some cell wall inhibitors such as vancomycin and macrolide antibiotic carbomycin, which had reasonable activity against stationary phase *B. burgdorferi* in the previous study (Feng, Wang, Shi, et al., 2014) showed relatively weak activity against the round body form of *B. burgdorferi* with 38% and 43% residual live cells, respectively (data not shown).

Table 5. Activity of top 23 active hits that had good activity against round body form of *B. burgdorferi*[a]

| Drugs (50 μM) | Residual viable cells[b] | Ratios of green/red fluorescence | | | |
|---|---|---|---|---|---|
| | | Primary screening | Rescreening | Rescreening | p-value[c] |
| Amoxicillin | 46% | 6.53 | 6.59 | 6.52 | 1.0000 |
| Doxycycline | 42% | 6.34 | 6.39 | 6.67 | 0.4915 |
| Penicillin G | 38% | 6.33 | 6.51 | 6.33 | 0.0767 |
| Cefuroxime | 34% | 6.29 | 6.28 | 6.31 | 0.0005 |
| Ceftriaxone | 36% | 6.37 | 6.29 | 6.39 | 0.0069 |
| Azithromycin | 47% | 6.79 | 6.52 | 6.42 | 0.8116 |
| Metronidazole | 33% | 6.23 | 6.30 | 6.31 | 0.0014 |
| Tinidazole | 33% | 6.24 | 6.21 | 6.36 | 0.0059 |
| **Daptomycin[d]** | **19%** | 5.90 | 6.09 | 5.93 | 0.0008 |
| **Artemisinin** | **24%** | 5.96 | 6.14 | 6.17 | 0.0028 |
| **Ciprofloxacin** | **28%** | 6.30 | 6.04 | 6.20 | 0.0108 |
| **Sulfacetamide** | **29%** | 6.26 | 6.14 | 6.20 | 0.0011 |
| **Sulfamethoxypyridazine** | **30%** | 6.20 | 6.07 | 6.34 | 0.0149 |
| **Nifuroxime** | **30%** | 6.10 | 6.32 | 6.22 | 0.0079 |
| **Fosfomycin** | **31%** | 6.39 | 6.12 | 6.16 | 0.0191 |
| **Chlortetracycline** | **31%** | 6.20 | 6.36 | 6.18 | 0.0078 |
| **Sulfathiazole** | **31%** | 6.38 | 6.18 | 6.17 | 0.0141 |
| **Clofazimine** | **32%** | 6.29 | 6.22 | 6.24 | 0.0008 |
| **Cefmenoxime** | **32%** | 6.18 | 6.33 | 6.28 | 0.0050 |
| Meclocycline | 33% | 6.52 | 6.23 | 6.09 | 0.1056 |
| Cefmetazole | 33% | 6.13 | 6.25 | 6.46 | 0.0530 |
| Loracarbef | 33% | 6.35 | 6.25 | 6.24 | 0.0036 |
| Sisomicin | 33% | 6.27 | 6.12 | 6.45 | 0.0562 |
| Sulfisoxazole | 33% | 6.53 | 6.15 | 6.18 | 0.1005 |
| Cefazolin | 34% | 6.23 | 6.34 | 6.29 | 0.0026 |
| Aztreonam | 34% | 6.16 | 6.38 | 6.33 | 0.0211 |
| Thymol | 34% | 6.15 | 6.36 | 6.36 | 0.0257 |
| Cefixime | 34% | 6.41 | 6.21 | 6.27 | 0.0169 |
| Sulfanilate | 34% | 6.49 | 6.03 | 6.40 | 0.1649 |
| Ceftazidime | 34% | 6.20 | 6.34 | 6.37 | 0.0126 |
| Rolitetracycline | 35% | 6.23 | 6.35 | 6.37 | 0.0085 |

[a]Round body form of *B. burgdorferi* from 7-day-old culture was treated with FDA-approved drugs (50 μM) for 7 days. The line above tinidazole refers to antibiotics used to treat Lyme disease.

[b]Residual viable *B. burgdorferi* was calculated according to the regression equation and ratios of Green/Red fluorescence obtained by SYBR Green I/PI assay.

[c]p-values of standard *t*-test were calculated for the select antibiotic treated samples in comparison with the amoxicillin treated sample as a control.

[d]Bold type indicates the 11 drug candidates that had better activity against the round body forms than metronidazole or tinidazole in order of decreasing activity.

*MIC values of round body active antibiotics:* In the previous study, it was found that the activity of antibiotics against non-growing persisters was not always correlated with their activity against growing *B. burgdorferi* (Feng, Wang, Shi, et al., 2014). Therefore, the MICs of artemisinin and ciprofloxacin that have excellent activity were tested against the round body form of *B. burgdorferi* using the SYBR Green I/PI assay and microscope counting. The MIC value of artemisinin was quite high at 50~100 μg/mL, indicating that artemisinin is much less active against growing *B. burgdorferi,* despite its high activity against the non-growing round body form of *B. burgdorferi* persisters. In contrast, ciprofloxacin was quite active against the growing *B. burgdorferi* with a low MIC (0.8~1.6 μg/mL), which is in agreement with a previous study (Kraiczy et al., 2001), indicating that it is quite active against both growing form and non-growing round body form of *B. burgdorferi.*

*Effect of drug combinations on the round body form and the stationary phase B. burgdorferi persisters:* In the previous study (Feng, Wang, Shi, et al., 2014), it was found that stationary phase cultures are enriched with morphological variants such as round body form and biofilm-like aggregated micro-colony form. These morphological variant forms of *B. burgdorferi* have different antibiotic susceptibilities (Brorson et al., 2009; Sapi et al., 2011), and the recent study showed that drug combinations are more effective at killing the *B. burgdorferi* persisters than single drugs (Feng et al., submitted). To identify the best drug combinations with the active hits from the above screens against the round body form of *B. burgdorferi,* some active hits were evaluated including artemisinin, cefmetazole, and sulfachlorpyridazine in combination with promising FDA-approved drugs daptomycin or cefoperazone from the previous persister screen (Feng, Wang, Shi, et al., 2014) and current Lyme antibiotic doxycycline on the round body form and also on a stationary phase *B. burgdorferi* culture. The results showed that the drug combinations were much more effective than each of these drugs alone (Table 6, FIG. 22). Overall, the round body forms were more susceptible to the tested drugs or drug combinations than the 10 day old stationary phase culture which was enriched with more resistant microcolony forms (Table 6). It is worth noting that antimalarial drug artemisinin highlighted itself as having among the best activity against the stationary phase *B. burgdorferi* persisters when combined with other drugs. For example, artemisinin in combination with doxycycline and cefoperazone showed remarkable activity against the stationary phase *B. burgdorferi* persisters (Table 6, FIG. 22o). Cefmenoxime and cefmetazole were the most effective of the few cephalosporin drugs tested against the round body form of *B. burgdorferi.* In addition, it was noted that the sulfa drug sulfachlorpyridazine when combined with daptomycin and doxycycline showed remarkable activity against *B. burgdorferi* persisters (FIG. 22n). Moreover, sulfachlorpyridazole combined with doxycycline and daptomycin showed the best activity against the round body form of *B. burgdorferi* persisters (Table 6).

Table 6. Effect of drug combinations on the round body form and stationary phase culture (10 day old) of *B. burgdorferi*

| | Live cell% | CefM | Scp | Art | Nft |
|---|---|---|---|---|---|
| Control | 50% (87%) | 34% (53%) | 38% (68%) | 28% (73%) | 33% (82%) |
| Dox | 49% (72%) | 31% (43%) | 29% (62%) | 26% (64%) | 30% (74%) |
| CefP | 30% (64%) | 31% (41%) | 30% (55%) | 25% (42%) | 25% (56%) |
| Dox+CefP | 29% (59%) | 28% (<u>41%</u>) | 25% (69%) | 23% (<u>**24%**</u>) | 23% (52%) |
| DAP | 17% (48%) | 16% (**20%**) | 15% (**27%**) | 24% (**29%**) | 16% (33%) |
| DAP+Dox | 16% (34%) | 16% (**16%**) | 8% (**21%**) | 15% (**19%**) | 17% (20%) |

Round body form and ten day old stationary phase (in brackets) *B. burgdorferi* culture was treated with 10 μg/mL drugs or their combinations for 7 days. Residual viable *B. burgdorferi* was calculated according to the regression equation and ratio of Green/Red fluorescence obtained by SYBR Green I/PI assay as described (Feng, Wang, Shi, et al., 2014). Direct microscopy counting was employed to rectify the results of the SYBR Green I/PI assay. Residual viable percentages less than 30% are shown in bold text and the best drug combinations without daptomycin are underlined. Abbreviation: Dox, doxcycline; CefP, cefoperazone; DAP, daptomycin; CefM, cefmetazole; Scp, sulfachlorpyridazine; Art, artemisinin; Nft, nitrofurantoin.

To confirm the drug combination results, subculture studies were performed in fresh BSK-H medium as described in the previous study (Feng, et al., in press) and it was found that drug-free round body controls and samples treated with single drugs grew in 10 day subculture (Table 7). Samples treated with two drug combinations grew more slowly (Table 7). However, after the 10 day subculture, the three drug combinations, e.g., doxycycline+daptomycin+ either cefoperazone or artemisinin or sulfachlorpyridazine did not show any sign of growth as no visible spirochetes were observed, whereas other drug combinations all had visible live spirochetes under the microscope (data not shown). After the 20-day subculture, there were about $8\times10^6$ spirochetes in the control sample and about $5\times10^6$ spirochetes in doxycycline-treated samples (Table 7). Daptomycin alone, or two drug combinations doxycycline+cefoperazone and doxycycline+daptomycin could not sterilize the round body form of *B. burgdorferi* persisters, as they all had visible spirochetes growing after the 20-day subculture (FIG. 23). However, doxycycline+daptomycin+artemisinin or sulfachlorpyridazine significantly reduced the number of spirochetes with very few spirochetes being visible after the 20-day subculture (FIG. 23). The daptomycin in combination with doxycycline and cefoperazone still showed the best activity which killed all round body form of *B. burgdorferi* persisters with no viable spirochetes observed after the 20-day subculture (FIG. 23).

Table 7. Subculture tests to assess the viability of drug-treated round body form of *B. burzdorferi*[a]

| Drugs[b] | Residual viable cells[c] | Spirochetes number After 10 day subculture[d] | Spirochetes number After 20 day subculture[d] |
|---|---|---|---|
| Control | 49% | $2\times10^6$ | $8\times10^6$ |
| Dox | 44% | $1\times10^6$ | $5\times10^6$ |
| Dox+Dap+CefP | 13% | $<1\times10^5$ | $<1\times10^5$[e] |
| Dox+Dap+Art | 15% | $<1\times10^5$ | $3\times10^5$ |
| Dox+Dap+Scp | 17% | $<1\times10^5$ | $6\times10^5$ |
| Dox+CefP | 31% | $7.5\times10^5$ | $4\times10^6$ |
| Dap | 18% | $5\times10^5$ | $5\times10^6$ |
| CefP | 33% | $9\times10^5$ | $4\times10^6$ |

a. Amoxicillin induced round body form *B. burgdorferi* culture (500 μL) was treated with 10 μg/mL drugs alone or drug combinations for 7 days. Then, 50 μL of washed bacterial cells was subcultured in 1 mL fresh BSK-H medium for 10 days and 20 days, respectively and examined by microscopy.
b. Abbreviations: Dox, doxycycline; CefP, cefoperazone; Dap, daptomycin; Art, artemisinin; Scp, sulfachlorpyridazine.
c. Green/Red fluorescence ratios were obtained by microplate reader after SYBR Green I/PI staining. Each value is the mean of three replicates.
d. The number of spirochetes was evaluated by microscope count.
e. Below detection limit as shown by lack of any visible spirochetes by microscopy.

Discussion

Previous *in vitro* and *in vivo* studies showed the round body form of *B. burgdorferi* as a persister form could survive in adverse conditions including antibiotic exposure *in vitro* and are found in chronic Lyme neuroborreliosis *in vivo* (Brorson and Brorson, 1998; Miklossy et al., 2008). As shown in previous studies (Brorson and Brorson, 1997; Murgia and Cinco, 2004; Brorson and Brorson, 1998) and also in this study, the round body form of *B. burgdorferi* could still reproduce and revert to spirochetes under suitable conditions upon removal of the stress during subculture. The *B. burgdorferi* round body form shows lower metabolic activity and is tolerant to antibiotics (Brorson et al., 2009; Kersten et al., 1995; Barthold et al., 2010). Although metronidazole, tinidazole and tigecycline were reported to have certain activity against the round body form, they were not able to completely eradicate these persister forms (Sapi et al., 2011). Thus, no good antibiotics against the round body form of *B. burgdorferi* are available (Brorson et al., 2009; Barthold et al., 2010). These studies demonstrate that it would be very difficult to kill the round body form of *B. burgdorferi* using current antibiotics.

In this study, this problem was addressed by first establishing an amoxicillin-induced round body model for *B. burgdorferi* persisters and then screening an FDA-approved drug library for activities against the round body form of *B. burgdorferi.* Eleven drug candidates were identified that have better activity against the round body form of *B. burgdorferi* (Table 5) than metronidazole or tinidazole, a control drug that is known to be active against the round body form (Sapi et al., 2011; Brorson and Brorson, 1999).

In a previous study, several drugs were identified that show excellent activity for stationary phase *B. burgdorferi* persisters from an FDA-approved drug library (Feng, Wang, Shi, et al., 2014). Some hits against the stationary phase *B. burgdorferi* persisters are also identified in this screen against round body form such as daptomycin, clofazimine and sulfa drugs, which validates the previous finding that these agents are active against the persister forms. Importantly, some active antibiotics against the round body form *B. burgdorferi* were found that did not show good activity in the previous drug screen against stationary phase *B. burgdorferi* persisters (Feng, Wang, Shi, et al., 2014). These include artemisinin, ciprofloxacin, nifuroxime, fosfomycin, tinidazole, loracarbef, and thymol that appear to be specifically active against the round body form. These candidate drugs are FDA approved and used for treatment of infections other than Lyme disease.

As in the previous study (Feng, Wang, Shi, et al., 2014), daptomycin still remains the most active drug against

the round body form of *B. burgdorferi* persisters. Daptomycin killed most planktonic round body form of *B. burgdorferi* (FIG. 21d). It is possible that daptomycin preferentially acts on the membrane of the round body form of *B. burgdorferi* that is different from the membrane of actively growing spirochetal form and thus making it particularly active for the persister forms. Daptomycin is known to disrupt the membrane structure and cause rapid depolarization thus depleting membrane energy that may be required for viability of the persisters.

An interesting finding of the study is the observation that the antimalarial drug artemisinin showed excellent activity against the round body form of *B. burgdorferi* persisters (FIG. 21 e). It is worth noting that artemisinin while having a high MIC (50-100 μg/mL) showed excellent activity against the round body form of *B. burgdorferi* compared with most commonly used antibiotics. Artemisinin is a commonly used antimalarial drug isolated from the plant *Artemisia annua,* a Chinese herbal medicine. The mechanism of action of artemisinin is not well understood. The antimalarial activity of artemisinin might involve endoperoxide activation by free ferrous iron from haemoglobin digestion by malaria parasites (Wells et al., 2009). However, the content of ferrous iron or haemoglobin is very low in the *B. burgdorferi* culture, so the activation of endoperoxide might not be the main mechanism of artemisinin activity against *B. burgdorferi* round body forms. The study in yeast is noted in which artemisinin impairs the membrane structure and causes depolarization of the mitochondrial membrane (Wang, Huang, et al., 2010; Li et al., 2005). In this respect, it is possible that artemisinin may have a similar mechanism of action of disrupting the bacterial membrane as the basis for its high activity against the round body form of *B. burgdorferi.* It is noteworthy that artemisinin has been used for treating Lyme co-infections and found to be effective clinically. The reason that artemisinin is effective was interpreted to be due to its action against *Babesia* co-infection, but it is quite likely that the clinical efficacy of artemisinin may at least partly be due to its activity against the round body form of *B. burgdorferi* persisters as shown in this study.

It was previously found that daptomycin combined with doxycycline and cefoperazone could best eliminate the most resistant microcolony form of persisters (Feng et al., submitted). In this study, it was found that artemisinin was the best substitute for daptomycin in the drug combination with doxycycline and cefoperazone and showed excellent activity against round body form of persisters (Table 6, FIG. 22m). Also, the lipophilic antibiotic clofazimine, which has complex antimicrobial activity including membrane disruption and depolarization (Van Rensburg et al., 1992; Cholo et al., 2012), showed good activity against both round body form and stationary phase persisters. Based on these findings on daptomycin, artemisinin and clofazimine, and without wishing to be bound to any one particular theory, it is proposed that membrane disruption may be a good approach to killing *B. burgdorferi* persisters.

Besides the top-ranked hits of screened drugs, many sulfonamide antibiotics, such as sulfacetamide, sulfamethoxypyridazine and sulfaquinoxaline, were found to be highly active against the round body form (Table 5). The sulfonamide antibiotics have also been identified in the previous drug screen against stationary phase persisters and showed low MICs (< 0.2 μg/mL) (Feng, Wang, Shi, et al., 2014). The sulfonamides inhibit utilization of PABA required for the synthesis of folic acid, which results in the blockade of several enzymes needed for synthesis of DNA and methionine, glycine, and formylmethionyl-transfer-RNA. It is worth noting that sulfachloropyridazine as the analogue of sulfamethoxypyridazine also showed good activity against stationary phase *B. burgdorferi* (residual viable cells is about 38%) and, when combined with daptomycin and doxycycline, showed remarkable activity against stationary phase *B. burgdorferi* (residual viable cells is about 8%) (Table 6). It is believed that further studies on metabolic changes of the round body form of *B. burgdorferi* could help understand the mechanism by which sulfonamide acts against *B. burgdorferi* persisters.

In addition to the previously found drugs (Feng, Wang, Zhang, et al., 2014), some novel drugs were discovered to be preferentially active against the round body form of *B. burgdorferi* in this study. Ciprofloxacin as a fluoroquinolone has shown activity against *B. burgdorferi in vitro* and could kill the inoculum with 16 μg/mL MBC (41.5 μM) after 72 h (Kraiczy et al., 2001). It has been presently disclosed that ciprofloxacin was the most active fluoroquinolone against the round body form of *B. burgdorferi* among other quinolones, but ciprofloxacin was not identified to have activity against *B. burgdorferi* stationary phase persisters in the previous screen (Feng, Wang, Shi, et al., 2014) as it was not in the old version of the FDA drug library. However, ciprofloxacin (50 μM) alone could not completely kill the round body form after 7 days. This result indicates that the round body form is more resistant or tolerant to antibiotics than multiplying *B. burgdorferi.* On the other hand, some drugs, such as nifuroxime and thymol, did not show activity in the previous drug screen on stationary phase *B. burgdorferi,* but showed good activity against the round body form in this study. This specific activity against the round body form could be related to the physiological difference of different morphological forms and/or the synergistic activity of these drugs with amoxicillin used to induce round forms used for drug screens. It is of interest to note that chlortetracycline was more active than doxycycline against the round body forms and that nitrofuran derivative nifuroxime was more active than metronidazole or tinidazole (Table 5). These findings could indicate the side chain involved in both cases may have conferred additional activity against the round body persisters. The drug combination test on the stationary phase *B. burgdorferi* using the nifuroxime analogue nitrofurantoin (residual live cell is 39%) showed that nitrofurantoin combined with cefoperazone was more effective than each drug alone (Table 6). Likewise, natural antimicrobial thymol combined with amoxicillin showed good activity (residual percentage is 34%) in the round body drug screen, but thymol alone did not work on the stationary phase *B. burgdorferi* (residual live cell

percentage is 82%) in the previous drug screen. Palaniappan et al. reported that thymol could reduce the resistance in *E. coli* and *S. aureus* to ampicillin and penicillin (2010). This synergistic activity between thymol and beta-lactams may explain its activity against the *B. burgdorferi* round body form. These results suggest that the drug combination could be an effective approach to fighting against *B. burgdorferi* persisters.

However, some drugs that had activity against stationary phase *B. burgdorferi*, such as beta-lactams, vancomycin, streptomycin, and amphotericin B (Feng, Wang, Shi, et al., 2014) did not show good activity against the round body form (Table 5). However, it was noted that two cephalosporins, cefmenoxime and cefmetazole, showed good activity against the round body form of *B. burgdorferi.* In the previous drug screen on stationary phase *B. burgdorferi,* cefoperazone, which was the best cephalosporin for killing stationary phase *B. burgdorferi,* also had certain activity against the round body form (not shown). Future studies are needed to further explore the mechanism of action of these cephalosporins that have activity against *B. burgdorferi* persisters which may involve targets outside the cell wall synthesis. Vancomycin is a glycopeptide antibiotic acting on the cell wall rather than acting on the cell membrane like daptomycin. Good activity of vancomycin was not found against amoxicillin treated round bodies, though it showed relatively good activity against stationary phase *B. burgdorferi* in the previous drug screen (Feng, Wang, Shi, et al., 2014). This might be due to cell wall deficiency of the round body form induced by amoxicillin.

In summary, this study represents the first high-throughput drug screens against the round body form of *B. burgdorferi* persisters and identified a number of FDA-approved antibiotics that show excellent activity against such forms. Despite some overlap in drugs active against both stationary phase persisters and round body form of persisters, some interesting drug candidates were identified that are preferentially active against the round body form of persisters, including artemisinin, ciprofloxacin, nifuroxime, fosfomycin, chlortetracycline, and some sulfa drugs which were found to be active against the round body form for the first time. These round body effective drugs in appropriate combinations can be used to eliminate the persistence phenomenon and improve the treatment of persistent forms of Lyme disease, including antibiotic refractory Lyme arthritis and PTLDS.

## EXAMPLE 4

### Identification of New Compounds with High Activity Against *Borrelia burgdorferi* Persisters from the NCI Compound Collection

### Materials and Methods

*Bacterial strain, media and culture: Borrelia burgdorferi* strain B31 (ATCC 35210) was obtained from American Type Tissue Collection. *B. burgdorferi* was cultured in BSK-H medium (HiMedia Laboratories Pvt. Ltd.), with 6% rabbit serum (Sigma-Aldrich). All culture media were filter-sterilized by 0.2 μm filter. Cultures were incubated in sterile 50 mL closed conical tubes (BD Biosciences, California, USA) at 33°C without antibiotics. Based on a previous study that demonstrated the antibiotic tolerance of the stationary phase cultures, 7 day old stationary phase *B. burgdorferi* cultures enriched in persisters were chosen for drug screens in 96-well microtiter plates as described (Feng, Wang, Shi, et al., 2014).

*Microscopy techniques:* Specimens were examined on a Zeiss AxioImager M2 microscope equipped with differential interference contrast (DIC) and epifluorescent illumination, and recorded with a Hamamatsu ORCA-$R^2$ C10600 camera. Cell proliferation assay was performed by direct counting using a bacterial counting chamber (Hausser Scientific Partnership, PA, USA) and DIC microscopy. SYBR Green I/PI assay was performed to assess the viability of *B. burgdorferi* as described (Feng, Wang, Zhang, et al., 2014). The ratio of live (green) and dead (red) *B. burgdorferi* was calculated by counting these cells using a bacterial counting chamber and epi-fluorescence microscopy as previously described (Feng, Wang, Zhang, et al., 2014).

*Antibiotics and the NCI chemical compound library:* Antibiotics including doxycycline, amoxicillin, and daptomycin were purchased from Sigma-Aldrich and dissolved in appropriate solvents (Clinical and Laboratory Standards Institute, 2007) to form stock solutions. All the antibiotic stocks were filter-sterilized by 0.2 μm filter. Then the stocks were diluted into 500 μM pre-diluted stocks and stored at -20°C.

The NCI compound library collection, consisting of diversity set V (Moody et al., 1978), mechanistic diversity set II (DTP-Mechanistic Set Information, 2015) and the natural products set III (DTP-Natural Products Set Information, 2015), was kindly supplied by National Cancer Institute Developmental Therapeutic Program's Open Compound Repository. These NCI compound libraries were prepared in 1 mM stock solutions with DMSO in 96-well plates leaving the first and the last columns in each plate for controls, which included DMSO blank controls, doxycycline control, and amoxicillin control. The pre-diluted drug plates were sealed and stored at -20°C.

*Screening NCI compound libraries against B. burgdorferi stationary phase persisters:* To qualitatively determine the effect of compounds on *B. burgdorferi* persisters, each compound (5 μL) from the pre-diluted stocks was added to a 7 day old *B. burgdorferi* stationary phase culture in 96-well microtiter plates. The final volume per well was adjusted

to 100 μL to achieve a final drug library concentration of 50 μM in the drug screen. The plates were sealed and placed in a 33°C incubator for 7 days when the viability of the bacteria was assessed by SYBR Green I/PI assay as described in a previous study (Feng, Wang, Zhang, et al., 2014). With the excitation wavelength at 485 nm, the fluorescence intensities at 535 nm (green emission) and 615 nm (red emission) were measured for each well of the screening plate using SpectraMax M2 Microplate Reader (Molecular Devices Inc., USA). Some effective candidates were further confirmed by epifluorescence microscopy as described (Feng, Wang, Zhang, et al., 2014).

*MIC determination:* The standard microdilution method was used to determine the minimum inhibitory concentration (MIC) that would inhibit visible growth of *B. burgdorferi* after a 72 hours incubation period (Sapi et al., 2011; Dever et al., 1992; Boerner et al., 1995). *B. burgdorferi* cells ($1 \times 10^5$) were inoculated into each well of a 96-well microplate containing 90 μL fresh BSK-H medium per well. Each diluted compound (10 μL) was added to the culture. All experiments were run in triplicate. The 96-well plate was sealed and placed in an incubator at 33°C for 5 days. Cell proliferation was assessed using the SYBR Green I/PI assay and a bacterial counting chamber after the incubation as described (Feng, Wang, Shi, et al., 2014).

## Introduction

To identify drugs that can more effectively kill *B. burgdorferi* persisters, a new viability assay using SYBR Green I/propidium iodide (PI) dyes was recently developed (Feng, Wang, Zhang, et al., 2014), which allowed screening of a FDA-approved drug library against stationary phase *B. burgdorferi* persisters (Feng, Wang, Zhang, et al., 2014). Using this high-throughput assay, a number of interesting drug candidates were identified, such as daptomycin, clofazimine, cefoperazone, carbomycin, which have excellent activity against *in vitro B. burgdorferi* persisters (Feng, Wang, Shi, et al., 2014). In the previous study, daptomycin was found to have the highest activity against *B. burgdorferi* persisters among all the candidate drugs. Although daptomycin could almost eradicate *B. burgdorferi* persisters at 50 μM, this drug concentration is quite high for clinical use, and in addition, daptomycin generally has to be used intravenously, which is not convenient to administer.

To identify new and more effective drugs than daptomycin in killing *B. burgdorferi* persisters, in this example, new drug screens were performed on stationary phase *B. burgdorferi* persisters using the chemical repository collection of the National Cancer Institute (NCI compound library collection). This NCI compound library collection has three compound libraries: the diversity set IV compound library (1593 compounds), the mechanistic set II library (816 compounds), and the natural product set III library (117 compounds), for a total of 2526 compounds. These compounds are chosen based on structural diversity from more than 250,000 natural products and synthetic compounds (Open Repository Collection of Synthetics and Pure Natural Products, 2014). By screening this NCI compound library collection, new anti-persister compounds were identified that were not found in the previous screens (Feng, Wang, Shi, et al., 2014). These new persister active hits can be used for a treatment for Lyme disease.

## Results

*Screening NCI compound Library to identify effective drugs active against dormant B. burgdorferi persisters:* The SYBR Green I/PI assay was used as a high-throughput screening method for rapid viability assessment for *B. burgdorferi* after exposure to the compound libraries (Feng, Wang, Shi, et al., 2014). Based on a previous study, some red colored compounds caused interference to the SYBR Green I/PI assay, which could make the background red and cause false positive results. Thus, in the presently disclosed subject matter, microscopic counting rescreens were done to examine the hit compounds in SYBR Green I/PI assay.

To identify effective chemical compounds that have activity against *B. burgdorferi* persisters, stationary phase *B. burgdorferi* was used as a persistence model to screen the NCI compound libraries. Meanwhile, the currently used Lyme disease antibiotics doxycycline and amoxicillin were included as control drugs. Consistent with previous results (Feng, Wang, Shi, et al., 2014), the currently used Lyme antibiotics had poor activity against the stationary phase *B. burgdorferi* persisters, and the bacteria treated with the two antibiotics still had 75% and 76% viable cells remaining, respectively, compared with 93% viable cells in drug-free control (Table 8).

Of the 2526 compounds in the NCI compound library collection tested, 237 were found to have higher activity against *B. burgdorferi* persisters than doxycycline and amoxicillin in the primary screen. The 237 candidates were rescreened by microscope counting with the SYBR Green I/PI viability assay. After the rescreen by microscopy, the top 30 active hits that had less than 50% residual viable cells after treatment were confirmed (Table 8, FIG. 24). Among the 30 active hits, 22 compounds were found in Mechanistic Set II, 9 compounds in Diversity Set IV, and 3 compounds in Natural Product Set III. Nanaomycin and dactinomycin showed up in both Mechanistic Set II and Natural Product Set III and NSC311153 and NSC637578 in both Mechanistic Set II and Diversity Set IV. It is interesting to note that all of them are aromatic compounds. Several clinically used drugs were identified that had excellent activity against stationary phase *B. burgdorferi* persisters. Anti-persister activities of some drugs were significantly higher than that of frontline

antibiotics doxycycline or amoxicillin and even more active than daptomycin, the best antibiotic against *B. burgdorferi* persisters in a previous study (Table 8, FIG. 24). Six anthraquinone antibiotics and compounds, daunomycin 3-oxime, dimethyldaunomycin, daunomycin, NSC299187, NSC363998 and nogalamycin, showed the highest activities (residual viable cells from 6% to 15%) against stationary phase *B. burgdorferi* persisters. These six compounds showed higher activity than daptomycin (18% residual viable cells). In addition, another five anthraquinone compounds, pyrromycin, rhodomycin A, NCS316157, emodin, and NSC156516, also showed good activity against stationary phase *B. burgdorferi* persisters (residual viable cells 21% to 50%). Following the six anthraquinones, pyronin B, a xanthene compound highlighted itself as having a good activity (residual viable cells 19%) against stationary phase *B. burgdorferi* persisters. Seven nitrogen-containing aromatic compounds, NSC343783 (residual viable cells 20%), Prodigiosin (24% residual viable cells), NSC637578, NSC 678917, NSC118832, NSC617570 and NSC96932, were found to be among the 30 most active compounds. Moreover, chaetochromin, a bis-naphtho-γ-pyrone compound, showed good activity with 22% residual viable cells. Mitomycin, an aziridine-containing benzoquinone antitumour drug, showed reasonably good activity with 25% residual viable cells. Three 1,4-naphthoquinones, nanaomycin (residual viable cells 26%), NCS659997 and NCS224124, had relatively good activity against stationary phase *B. burgdorferi* persisters. A polypeptide antibiotic dactinomycin also had relatively high activity against *B. burgdorferi* persisters (residual viable cells 30%). Besides 11 clinically used drugs (daunomycin 3-oxime, dimethyldaunomycin, daunomycin, nogalamycin, pyrromycin, chaetochromin, prodigiosin, mitomycin, nanaomycin and dactinomycin), 19 non-medicinal compounds also were found that showed good activity against stationary phase *B. burgdorferi* persisters to varying levels (Table 8, FIG. 24).

Table 8. Activity of top 30 active hits that had good activity (better than current clinical drugs) against *stationary* phase *B. burgdorferi* persisters[a]

| NSC[b] | Compounds (50 μM) | Chemical structure | Residual viable cells[c] | Residual viable cells[d] |
|---|---|---|---|---|
| | Control | - | 93% | 95% |
| | Amoxicillin | - | 71% | 77% |
| | Doxycycline | - | 68% | 77% |
| | Daptomycin | - | 23% | 18% |
| 143491 | Daunomycin 3-oxime hydrochloride | 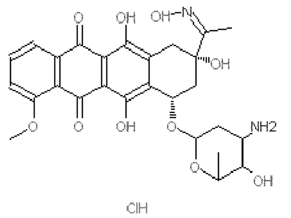 | 0% | 6% |
| 258812 | Dimethyldaunomycin hydrochloride | 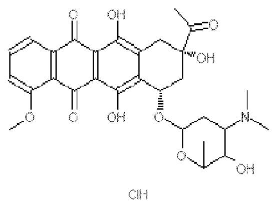 | 0% | 10% |
| 82151 | Daunorubicin hydrochloride | 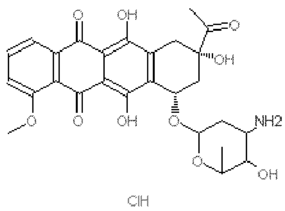 | 0% | 10% |
| 299187 | 9,10-Anthracenedione, 1-hydroxy-4-[[2-[(2-hydroxyethyl)amino] ethyl ]amino]- | 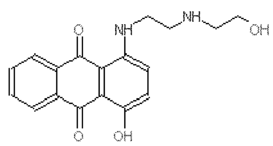 | 5% | 13% |

(continued)

| NSC[b] | Compounds (50 μM) | Chemical structure | Residual viable cells[c] | Residual viable cells[d] |
|---|---|---|---|---|
| 363998 | Anthracene-9,10-dione, 1,5-bis[3-[[(2-hydroxyethyl)amino] propyl] amino]-9,10-dihydro-, dihydrochloride | 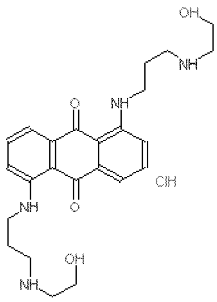 | 0% | 13% |
| 70845 | Nogalamycin | 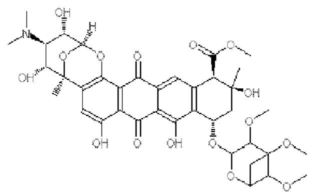 | 0% | 15% |
| 44690 | Pyronin B | 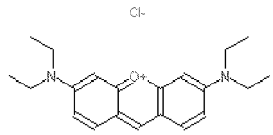 | 0% | 19% |
| 343783 | N-Allyl-2-(methylthio) [1,3]thiazolo[ 5,4-d] pyrimidin-7-amine | 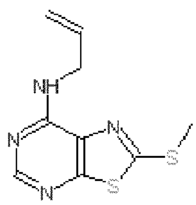 | 67% | 20% |
| 267229 | Pyrromycin | 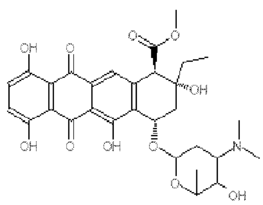 | 0% | 21% |
| 136044 | Rhodomycin A | 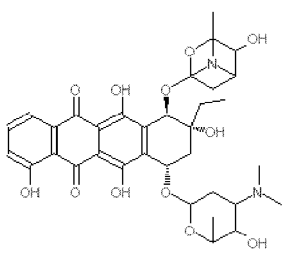 | 0% | 22% |

(continued)

| NSC[b] | Compounds (50 μM) | Chemical structure | Residual viable cells[c] | Residual viable cells[d] |
|---|---|---|---|---|
| 345647 | Chaetochromin | 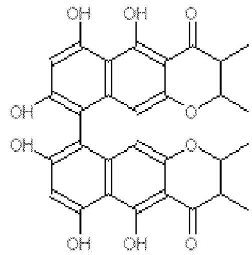 | 8% | 22% |
| 316157 | 9,10-Anthracenedione, 1,4-dihydroxy-2-[[2-[(2-hydroxyethyl)amino] ethyl ]amino]- | 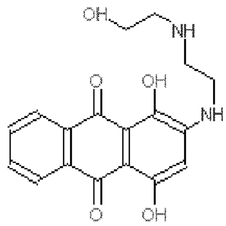 | 0% | 23% |
| 47147 | Prodigiosin | 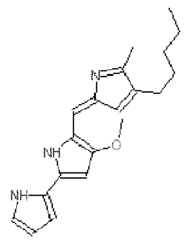 | 0% | 24% |
| 26980 | Mitomycin | 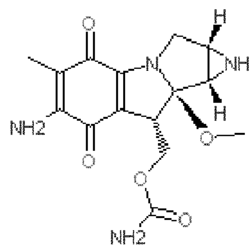 | 20% | 25% |
| 267461 | Nanaomycin | 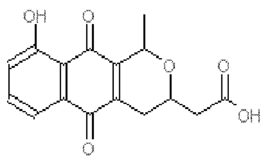 | 34% | 26% |
| 311153 | 9-Hydroxy-2-(2-piperidinylethyl)ellipticini um acetate | 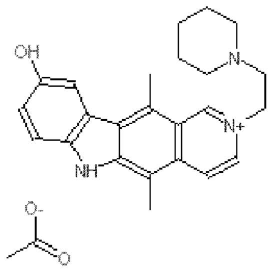 | 45% | 26% |

(continued)

| NSC[b] | Compounds (50 μM) | Chemical structure | Residual viable cells[c] | Residual viable cells[d] |
|---|---|---|---|---|
| 637578 | N-[3-(2-Pyridyl)isoquinolin-1-yl]-2-pyridinecarboxamidine | 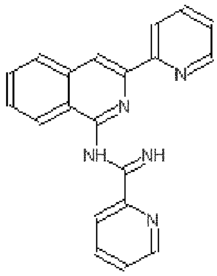 | 46% | 26% |
| 659997 | Naphthalene-1,4-dione, 2-chloro-5,8-dihydroxy- 3-(2-methoxyethoxy)- | 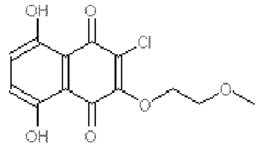 | 1% | 28% |
| 317003 | 9H-Thioxanthen-9-one, 1-[[2-(dimethylamino) ethyl] ami no]-7-hydroxy-4-methyl-,monohydriodide | 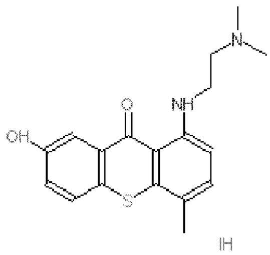 | 29% | 30% |
| 3053 | Dactinomycin | 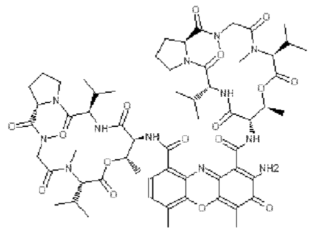 | 37% | 30% |
| 408120 | Emodin | 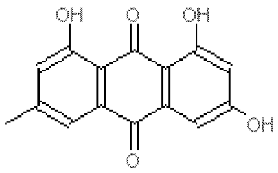 | 18% | 31% |
| 224124 | (5,8-dihydroxy-1,4-dioxo-1,4-dihydronaphthalene-2,3-diyl)dimethanediyl dicarbamate | 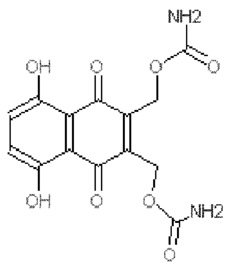 | 32% | 31% |

(continued)

| NSC[b] | Compounds (50 μM) | Chemical structure | Residual viable cells[c] | Residual viable cells[d] |
|---|---|---|---|---|
| 678917 | 1-Phenazinecarboxamide, N-[2-(dimethylamino)ethyl]-6,9-dimethoxy-, monohydrochloride | 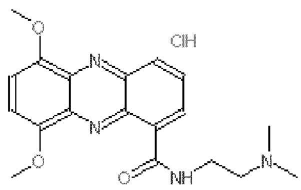 | 44% | 35% |
| 118832 | (5-phenyl-1,3-thiazol-2-yl)methanol | 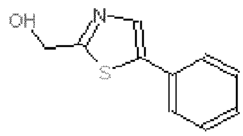 | 30% | 38% |
| 407010 | 2-(3,4-dihydroxyphenyl)-3,7-dihydroxy-4H-1-benzopyran-4-one | 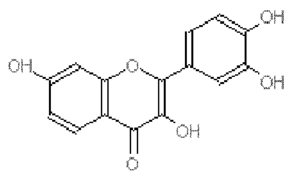 | 39% | 38% |
| 617570 | Benzoic acid, 2-hydroxy-, (2,6-pyridinediyldiethylidyne) dihydrazide, nickel complex | 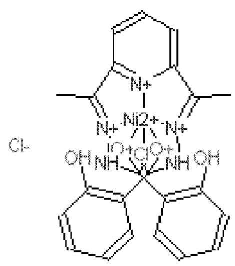 | 44% | 38% |
| 137399 | 1-(1,2-Dihydro-5-acenaphthylenyl)-N-hydroxy-1-phenylmethanimine | 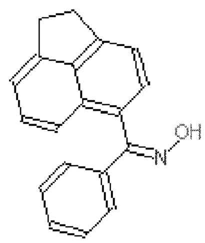 | 51% | 41% |
| 93739 | 2-Methyl-4,4'-[(4-imino-2,5-cyclohexadien-1-ylidene)methylene] dianili ne hydrochloride | 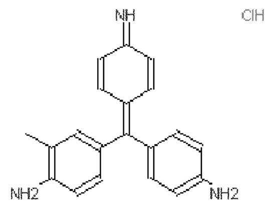 | 0% | 43% |
| 96932 | 3,3'-Diethyl-9-methylthiacarbocyanine iodide | 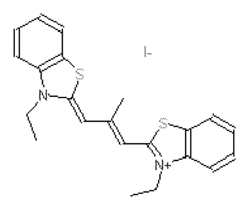 | 0% | 46% |

(continued)

| NSC[b] | Compounds (50 μM) | Chemical structure | Residual viable cells[c] | Residual viable cells[d] |
|---|---|---|---|---|
| 156516 | 1,8-Di(phenylthio)anthraquin one | 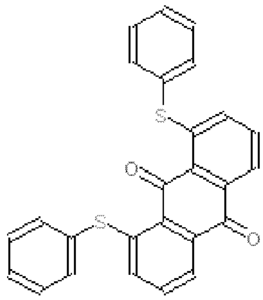 | 46% | 50% |

a. Seven day old stationary phase *B. burgdorferi* culture was treated with drugs or compounds (50 μM) for 7 days when the viability of the bacteria was determined as described (Feng, Wang, Shi, et al., 2014).
b. The NSC number is a numeric identifier for substances submitted to the National Cancer Institute (NCI).
c. Residual viable *B. burgdorferi* was calculated according to the regression equation and ratio of Green/Red fluorescence obtained by SYBR Green I/PI assay as described (Feng, Wang, Shi, et al., 2014).
d. Residual viable *B. burgdorferi* was assayed by epifluorescence microscope counting as described (Feng, Wang, Shi, et al., 2014).

*Relationship between MIC values and anti-persister activity:* Some compounds that have good activity against the non-growing stationary phase *B. burgdorferi* persisters were found (Table 8), but it is necessary to determine the MICs of these compounds against growing *B. burgdorferi* (Table 9). The standard microdilution method was used to determine the MIC as described in a previous study (Feng, Wang, Shi, et al., 2014). It was found that three anthracycline antibiotics, daunomycin 3-oxime, daunorubicin and pyrromycin, in addition to having good activity against stationary phase *B. burgdorferi* persisters, also were highly active against log phase growing *B. burgdorferi* with low MICs (<0.36, <0.36, 0.36-0.72 μg/mL, respectively). Another anthraquinone compound, NSC299187, showed relatively high MIC (3.26-6.52 μg/mL) although it had excellent anti-persister activity (residual viable cells 13%). It was also noted that prodigiosin (nitrogen-containing aromatic rings compound), mitomycin (aziridine-containing benzoquinone), nanaomycin (1,4-naphthoquinone) and dactinomycin (polypeptide antibiotic) had very good activity against replicating *B. burgdorferi* with low MICs (<0.2, ≤0.21, 0.76-1.57, <0.78 μg/mL, respectively). On the other hand, pyronin B and chaetochromin were less potent against growing *B. burgdorferi* with relatively high MICs (1.8-3.6, 2.74-5.47 μg/mL, respectively) but had excellent anti-persister activity.

Table 9. Comparison of the MIC values and anti-persister activity of some compounds for *B. burgdorferi*

| Antibiotics | MIC (μg/mL) | Activity against persisters[a] |
|---|---|---|
| Doxycycline[b] | ≤0.25 | 77% |
| Amoxicillin[b] | ≤0.25 | 77% |
| Daptomycin[b] | 12.5-25 | 18% |
| Daunomycin 3-oxime | ≤0.36 | 6% |
| Daunorubicin | ≤0.35 | 10% |
| NSC299187 | 3.26-6.52 | 13% |
| Pyronin B | 1.8-3.6 | 19% |
| Pyrromycin | 0.37-0.73 | 21% |
| Chaetochromin | 2.74-5.47 | 22% |
| Prodigiosin | ≤0.2 | 24% |
| Mitomycin | ≤0.21 | 25% |
| Nanaomycin | 0.76-1.57 | 26% |
| Dactinomycin | ≤0.78 | 30% |

a. Shown as residual viable cell percentage.
b. $C_{max}$ values are derived from the published literature.

*Comparison of anti-persister activity at low drug concentrations:* Although many highly effective hits were obtained from the NCI compound library with 50 μM compound screen, this drug concentration is likely too high for the *in vivo* experiments. Daptomycin at 50 μM has shown strong activity against stationary phase *B. burgdorferi* persisters in a previous study (Feng, Wang, Shi, et al., 2014), but it could not kill the microcolony form *B. burgdorferi* persisters at lower concentration such as 10 μg/mL (Feng et al. 2015, in press). To further compare the activity of hit compounds and daptomycin, the activity was tested against stationary phase *B. burgdorferi* persisters with 20 μM drug concentration (about 10 μg/mL for most compounds and 32 μg/mL for daptomycin). Most residual viable percentage of stationary phase *B. burgdorferi* increased with the decrease of drug concentration (Table 10, FIG. 25), but five anthracyclines, dimethyldaunomycin, NCS363998, nogalamycin, pyrromycin and Rhodomycin A, at 20 μM still showed as strong an activity against stationary phase *B. burgdorferi* persisters as 50 μM (Table 10, FIG. 25). Other non-anthracycline compounds showed relatively weaker activity than the daptomycin at 20 μM.

Table 10. Comparison of activity of some hit compounds at 20 μM and 50 μM against *stationary* phase *B. burgdorferi* persisters[a]

| NSC[d] | Compounds | Residual viable cells (%) | | |
|---|---|---|---|---|
| | | 20 μM[b] | 20 μM[c] | 50 μM[c] |
| | Control | 93% | 94% | 95% |
| | Amoxicillin | 77% | 77% | 77% |
| | Doxycycline | 76% | 77% | 77% |
| | Daptomycin | 32% | 25% | 18% |
| 258812 | Dimethyldaunomycin hydrochloride | 0% | 10% | 10% |
| 363998 | Anthracene-9,10-dione, 1,5-bis[3-[[(2-hydroxyethyl)amino] propyl]amino]-9,10-dihydro-, dihydrochloride | 22% | 14% | 13% |
| 70845 | Nogalamycin | 3% | 15% | 15% |
| 267229 | Pyrromycin | 6% | 20% | 21% |
| 136044 | Rhodomycin A | 5% | 21% | 21% |
| 345647 | Chaetochromin | 31% | 33% | 22% |
| 47147 | Prodigiosin | 0% | 45% | 24% |
| 267461 | Nanaomycin | 39% | 45% | 26% |
| 659997 | Naphthalene-1,4-dione, 2-chloro-5,8-dihydroxy-3-(2-methoxyethoxy)- | 40% | 50% | 28% |
| 224124 | (5,8-dihydroxy-1,4-dioxo-1,4-dihydronaphthalene-2,3-diyl) dimethanediyl dicarbamate | 54% | 77% | 31% |
| 617570 | Benzoic acid, 2-hydroxy-, (2,6-pyridinediyldiethylidyne) dihydrazide, nickel complex | 66% | 50% | 38% |
| 93739 | 2-Methyl-4,4'-[(4-imino-2,5-cyclohexadien-1-ylidene) methylene]dianiline hydrochloride | 0% | 77% | 43% |

a. Seven day old stationary phase *B. burgdorferi* culture was treated with drugs for 7 days.
b. Residual viable *B. burgdorferi* was calculated according to the regression equation and ratio of Green/Red fluorescence obtained by SYBR Green I/PI assay.
c. Residual viable *B. burgdorferi* was assayed by epifluorescence microscope counting.
d. The NSC number is a numeric identifier for substances submitted to the National Cancer Institute (NCI).

Discussion

A number of interesting drug candidates have recently been identified that have excellent activity against non-replicating *B. burgdorferi* persisters from the FDA-approved drug library (Feng, Wang, Shi, et al., 2014). The goal of this study was to identify new chemical compounds that have high activity against *B. burgdorferi* persisters using the NCI compound library collection. From the 2526 compounds in three NCI compound libraries, 237 compounds were found

to have higher activity against *B. burgdorferi* persisters than doxycycline or amoxicillin, from which the top 30 active hits were confirmed by microscopy rescreen. The use of the mechanistic compound library helped to identify the anthraquinone (anthracycline) class of drugs that have high activity against *B. burgdorferi* persisters. It is interesting to note that more than one third of the 30 most active compounds possess an anthraquinone (also called anthracenedione or dioxoanthracene) structure. The top 6 active compounds, daunomycin 3-oxime, dimethyldaunomycin, daunomycin (daunorubicin), NSC299187, NSC363998 and nogalamycin, are all anthraquinone derivatives, characterized by 3 aromatic rings linked together with benzoquinone in the center. Previously, the anti-persister activity of anthracycline antibiotic doxorubicin was noted (Feng, Wang, Shi, et al., 2014), but it was mistakenly excluded from the active drugs as it interfered with the SYBR Green I/PI staining. However, careful examination by microscopy confirmed the anti-persister activity by red colored anthraquinone drugs including doxorubicin. It is worth noting that not all red colored anthraquinone compounds have good anti-persister activity. For example, NCS156516 had weak anti-persister activity and showed 50% residual viable (green) cells (FIG. 24). Thus confirmation is needed in assessing compounds that have red color and have activity against *B. burgdorferi* persisters by careful microscopic examination, using low concentration of compounds and subculture studies.

It is of interest to note that the top six anthraquinone compounds with residual viable cells ranging from 6-15% seem to be even more active than daptomycin, which had 18% residual viable cells in the SYBR Green I/PI viability assay (Table 8). Nevertheless, since any single drug is unlikely to kill all bacterial populations and a drug combination using agents targeting both growing and non-growing persisters is required to more effectively kill heterogeneous bacterial populations *in vitro* and during persistent infection (Zhang, 2014), it would be necessary to evaluate the efficacy of an anthraquinone combination with current Lyme antibiotics against more resistant forms of *B. burgdorferi* persisters including microcolonies and biofilm-like growth. It has recently been shown that indeed daptomycin alone could not kill the aggregated form of *B. burgdorferi* persisters, such as microcolonies or biofilm-like structures, while daptomycin in combination with doxycycline and cefoperazone was able to completely eradicate the more resistant microcolonies or biofilm-like structures without any regrowth in subculture (Feng, et al., 2015, in press). Thus, further drug combinations and subculture studies are needed to confirm if the top six anthraquinone compounds are indeed more active than daptomycin against *B. burgdorferi* persisters *in vitro* and *in vivo*.

Anthraquinones are a class of naturally occurring phenolic compounds isolated from *Streptomyces* and have diverse medical uses including anti-cancer, antimalarial, and laxatives. Anthracycline antibiotics, such as daunomycin, nogalamycin, pyrromycin and rhodomycin A, were used in chemotherapy of some cancers, especially for several specific types of leukemia (Tan et al., 1967). It has been reported that anthracycline drugs have antibacterial activity against *S. aureus,* and the MICs of daunomycin and doxorubicin are 8-32 μg/mL and 0.12-0.5 μg/mL, respectively (Zhu et al., 2005). Daunomycin did not show bactericidal activity for Gram-negative bacteria *Pseudomonas aeruginosa, Klebsiella pneumoniae* and *E. coli* (Moody et al., 1978). This study is the first to demonstrate the activity of this class of compounds active against both growing and non-growing forms of Gram-negative *B. burgdorferi.* However, the mechanisms of action of this class of anthraquinone compounds against *B. burgdorferi* are unclear and remain to be determined. Anthracycline antibiotics could inhibit DNA and RNA synthesis by inserting into base pairs of the DNA/RNA strand (Mizuno et al., 1975). In addition, anthracycline antibiotics could stabilize the topoisomerase II complex and prevent dissociation of topoisomerase II from its nucleic acid substrate, leading to DNA damage and blocking DNA transcription and replication as well as producing reactive oxygen species, which could damage mitochondria and lead to cardiotoxicity as side effects (Jensen et al., 1993; Pommier et al., 2010). The sugar moiety of daunomycin plays a critical role in determining its anticancer activity (Zhu et al., 2005). In this study, it was found that anthracycline antibiotics with sugar structure and anthraquinone compounds without sugar structure (NCS299187 and NCS363998) all had good activity against *B. burgdorferi* persisters. These findings suggest that the mechanism of action of anthraquinone drugs may not be identical for its anti-cancer activity in eukaryotes and anti-persister activity in *B. burgdorferi.* Future studies are needed to identify the mechanism of action of anthracycline antibiotics against *B. burgdorferi* persisters, address the structure activity relationship of this class of compounds for *B. burgdorferi* persisters, and also to explore the possibility of utilizing the strong anti-persister activity of this class of compounds without untoward toxicity for the host cells.

Besides the anthracycline antibiotics, it was also found that some 1,4-naphthoquinones, such as nanaomycin, NCS659997 and NCS224124, showed high activity against stationary phase *B. burgdorferi* persisters. 1,4-naphthoquinone has an analogue molecular skeleton similar to anthraquinone. Nanaomycin may interfere with the function of the bacterial cell membrane and interact with the respiratory chain of bacteria (Hayashi et al., 1982), and such mode of action may explain its activity against *B. burgdorferi* persisters.

It was found that chaetochromin, a bis-naphtho-γ-pyrone produced by several species of chaetomium, also showed high activity against stationary phase *B. burgdorferi.* Bis-naphtho-γ-pyrones have a broad-range of biological activities such as inhibition of ATP synthesis in mitochondria, cells proliferation inhibition, triacylglycerol synthesis inhibition, and antimicrobial activity (Lu et al., 2014). Bis-naphtho-γ-pyrones were active against various bacteria such as *S. aureus, E. coli* and *M. tuberculosis,* with MIC values ranging from 2 to 50 μg/mL (Lu et al., 2014). Inhibition of ATP synthesis could explain the activity of bis-naphtho-γ-pyrone against *B. burgdorferi* persisters. Cephalochromin has been

shown to inhibit fatty acid biosynthesis (Campbell and Cronan, 2001). It is possible that fatty acid synthesis might play a role in *B. burgdorferi* persister formation, and future studies are needed to confirm this possibility.

In this study, it was found that some antibiotic compounds, such as prodigiosin, mitomycin, and dactinomycin, had decent activity against *B. burgdorferi* persisters, though their activities (24-30% residual viable cells) were not as strong as daptomycin (18% residual viable cells) (Table 8). Prodigiosin is a secondary metabolite of *Serratia marcescens* and is well known to have antibacterial, antifungal, antiprotozoal, antimalarial, immunosuppressive and anticancer activities (Williamson et al., 2007). Mitomycin shows its activity as a DNA crosslinker through its aziridine functional group and crosslinks the complementary strands of the DNA double helix to cause the death of a bacterial cell (Szybalski and Iyer, 1964; Tomasz, 1995). The activity of mitomycin against *B. burgdorferi* persisters may also be due to its DNA crosslinking activity. Dactinomycin is a polypeptide antitumor antibiotic isolated from soil bacteria *Streptomyces* (Hollstein, 1974) and is known to bind DNA and interfere with DNA replication (Hollstein, 1974), and also inhibit RNA transcription (Sobell, 1985).

In addition, some unstudied compounds, such as NSC343783 and NCS311153, were found to be effective against stationary phase *B. burgdorferi* persisters to a varying extent. These newfound interesting compounds have more anti-persister activity than current Lyme antibiotics and may be explored in the future as leads for further drug development and mechanism study for bacterial persistence.

In summary, the anthracycline class of compounds and antibiotics along with some other compounds, including prodigiosin, mitomycin, nanaomycin and dactinomycin, have been identified as having excellent activity against *B. burgdorferi* persisters. The structure activity relationship and mechanisms of action of the anthracycline/anthraquinone class of compounds against *B. burgdorferi* persisters should be addressed in future studies. Drug combination studies with the anthracycline/anthraquinone class of compounds and the current Lyme antibiotics to eradicate *B. burgdorferi* persisters *in vitro* and in animal models should be of value for improved treatment of Lyme disease.

EXAMPLE 5

<u>An Ultra Rapid Antibiotic Susceptibility Test in 30 minutes by SYBR Green/PI Assay</u> *Introduction*

Pathogens isolated from clinical specimens undergo antimicrobial drug susceptibility testing to help clinicians provide better antimicrobial therapy. It is important to identify drugs that can be used most effectively to cure the patient especially those who are infected with antibiotic resistant pathogens. For patients who are infected with bacteria that can cause life-threatening infections (*Staphylococcus, Neisseria, Acinetobacter, Pseudomonas, Stenotrophomonas, Haemophilus, Escherichia*), it is crucial that antimicrobial drug susceptibility tests be performed in a quick manner. Currently, the common drug susceptibility tests all rely on growth of the organisms and take at least one day for non-fastidious organisms (CLSI, 2016) and can be up to 1 month for fastidious organisms (CLSI, 2011) before any information can be determined about the pathogen, risking patients' lives during the waiting period for antibiotic susceptibility testing results, with potential worsened disease outcome and spread of antibiotic resistant organisms. Here, a rapid antibiotic susceptibility test is disclosed that does not rely on growth of the organism, but rather rely on detecting the viability of the organisms after drug exposure in a short time, which have been found to produce robust results in only 30 minutes - two hours for fast growing organisms such as *S. aureus* and *E. coli* and in 16 hrs for slow growing organisms, such as *M. tuberculosis*.

Antimicrobial activity is measured by determining the minimum inhibitory concentration (MIC), the lowest amount of antibiotics that is needed to inhibit the growth of the organism. Hence, the current tests are lengthy in time and depend on the growth of the pathogen. The disk diffusion (Kirby-Bauer Disk Diffusion) test consists of inoculating an agar plate with a known organism and then adding antibiotics (with concentrations recommended by the Clinical and Laboratory Standards Institute) onto filter-paper disks that are then placed onto the surface of the agar (CLSI, 2016). During incubation, which can take up to at least 16 hours (one day) even for fast growing organisms, the antibiotic will diffuse from the disk and into the agar. A zone of inhibition will form once the bacteria are grown up and the diameter of the zone of inhibition will be measured and used to extrapolate susceptibility categories based on zone size. The standards for comparison are provided by the Clinical and Laboratory Standards Institute (CLSI) (CLSI, 2016; CLSI, 2011). Similarly, the MIC determination procedure in liquid culture broth also takes a long time and is dependent on how fast the organism grows. Tubes or wells containing serial dilutions of drugs are inoculated with bacteria and growth in the presence of drug is observed by the turbidity of the culture, which also takes at least 16 hours for fast growing organisms to several weeks for slow growing organisms.

To expedite the process of antimicrobial susceptibility testing, the presently disclosed methods use SYBR Green/Propidium Iodide (PI) staining viability assay, which was initially developed for quantitating the viability of *Borrelia burgdorferi* (Feng, et al., 2014) and has since been adapted for viability assessment and antibiotic susceptibility testing for different bacteria as described herein. The SYBR Green I dye is commonly used in molecular biology to stain nucleic acids and was used for viability assessment for some bacteria by flow cytometry, (Barbesti, et al., 2000) but has not

been commonly used for bacterial viability assessment, until it was used for drug susceptibility testing for *B. burgdorferi* (Feng, et al., 2014). SYBR Green I is a permeable dye that stains all cells green and PI is an impermeant dye that stains only dead or damaged cells with a compromised cell membrane (Feng, et al., 2014). Hence, the live cells will be stained green by SYBR Green I while dead cells stained red by PI. One rapidly determines the viability of a bacterial population based on the ratio of green cells and red cells as measured by fluorescence microscopy or fluorescence microplate readers. Because the SYBR Green/PI stain conveniently measures the presence of live and dead cells by green versus red fluorescence ratio, this assay is not growth-dependent and eliminates a bottleneck in current antibiotic susceptibility tests - which depend on the lag period waiting for bacteria to grow as well as bacterial growth to certain numbers for detection by sensors in automated systems or visual measurements. It is important to note that aside from producing rapid results, the SYBR Green/PI staining assay also has other advantages. Compared to other colorimetric viability assays such as MTT and Alamar Blue, the SYBR Green/PI assay is more rapid and has lower detection limit, background, rate of error, is extremely cost-effective, and can be used in a high-throughput manner in 96-well or 384 well plates. In addition, the SYBR Green I/PI assay has been used for drug screens with compound libraries (Feng, et al., 2014; Feng, et al., Emerg Microbes Infect, 2014).

As provided herein, the SYBR Green/PI stain can successfully stain both live and dead populations of five different pathogens, Gram-positive *Staphylococcus aureus* (USA300), Gram-negative *Escherichia coli* (W3110), *Klebsiella pneumoniae* (Isolate 7), *Acinetobacter baumannii* and acid-fast *Mycobacterium tuberculosis* (H37Ra), as representatives for other bacteria, to demonstrate the applicability of this method for antibiotic susceptibility testing of different bacterial species. The SYBR Green/PI assay can detect the MIC of antibiotics that is concordant with results from the traditional broth dilution method and also, detect the amount of drug tolerant persisters that is concordant with results from traditional cell forming unit (CFU) enumeration. Further, the SYBR Green/PI assay also has been optimized to assign different strains of *S. aureus, E. coli,* and *K. pneumoniae* with different antibiotic sensitivity and resistance profiles into their respective drug susceptibility categories (as defined by CLSI standards) under 2 hours.

*Methods*

*Culture media, chemicals, and antibiotics*

*Staphylococcus aureus, Escherichia coli, Klebsiella pneumoniae, Acinetobacter baumannii* cultures were cultivated in tryptic soy broth (TSB) and tryptic soy agar (TSA) (Becton Dickinson) and *Mycobacterium tuberculosis* cultures were cultivated in 7H9 medium at 37°C with the appropriate antibiotics. *Staphylococcus aureus* strains USA300, CA-409, NY-315, CA-127, *Escherichia coli* strains W3110, UTI89, CFT073, and KTE181, *Klebsiella pneumoniae* (Isolate 7), *Acinetobacter baumannii* isolate and *M. tuberculosis* H37Ra, were obtained from ATCC (Manassas, Virginia). Stock solutions of the antibiotics ampicillin, chloramphenicol, gentamicin, erythromycin, trimethoprim, ciprofloxacin, streptomycin, ceftriaxone, and cefotaxime (Sigma-Aldrich Co.) were prepared and sterilized through filtration or autoclaved, if necessary, and used at indicated concentrations. Antibiotic susceptibility assays for different bacteria were performed using Mueller-Hinton broth and agar per CLSI methods as described below.

*Conventional antibiotic susceptibility tests*

The Kirby-Bauer disk diffusion assay was used to determine the susceptibility of the various strains to antibiotics. Instructions and also interpretation of zone diameters were made using the established standards as listed in the Clinical and Laboratory Standards Institute (CLSI). Briefly, bacterial cultures were adjusted to a 0.5 McFarland turbidity standard and spread onto Mueller-Hinton agar. Paper Disks were impregnated onto the agar and the antibiotics at concentrations recommended by CLSI were pipetted onto each blank disk and allowed to air dry for 10 minutes. The plates were incubated at 37°C overnight before the zone of inhibition was measured in millimeters. The protocol used to test for antibiotic susceptibility using a broth dilution method was based on the recommendations of CLSI. Briefly, bacterial cultures were adjusted to a 0.5 McFarland turbidity standard in Mueller-Hinton broth and drug concentrations of 2-fold dilutions beginning with 32 $\mu$g/mL down to 1 $\mu$g/mL were tested. The minimum inhibitory concentration was defined as the lowest drug concentration that completely inhibited visible bacterial growth.

*SYBR Green I/PI assay*

SYBR Green I (10,000X stock, Invitrogen) was mixed with propidium iodide (20 mM, Sigma) in distilled $H_2O$. The staining dye for *Staphylococcus aureus, Escherichia coli, Acinetobacter baumannii,* and *Mycobacterium tuberculosis* were made by mixing SYBR Green I to propidium iodide (1:3) in 100 $\mu$l distilled $H_2O$. The staining dye for *Klebsiella pneumoniae* was made by mixing SYBR Green I to propidium iodide (3:1) in 100 $\mu$l distilled $H_2O$. The SYBR Green/PI staining mix (10 $\mu$l) was added to each 100 $\mu$l of each sample. The sample was vortexed and incubated at room

temperature in the dark for 20 minutes. The green and red fluorescence intensity was detected using a Synergy H1 microplate reader by Bio Tek Instruments (VT, USA) at excitation wavelength of 485 nm, 538 nm (green emission), and 612 nm (red emission). The percentage of live cells in each sample was determined by a regression equation generated by a standard curve. To generate a standard curve, different proportions of live and isopropyl alcohol killed cells were made. The staining mixture was added to each sample and the green/red fluorescent ratios were measured as described above. The regression equation was generated using the least-square fitting analysis. Specimens of *S. aureus* and *K. pneumoniae* were examined on the Keyence BZ-X710 Fluorescence Microscope and images were recorded and processed using BZ-X Analyzer provided by Keyence (Osaka, Japan). Specimens of M. tuberculosis cells were examined on a Zeiss AxioImager M2 microscope equipped with epifluorescence illumination. Pictures were taken using ORCA-$R^2$ high resolution digital camera (HAMAMATSU, Japan). Image Pro-Plus software was used to quantitatively determine the fluorescence intensity.

*Isolation of spontaneous isoniazid-resistant mutants* of *M. tuberculosis*

*Mycobacterium tuberculosis* H37Ra was grown in 7H9 liquid medium (Difco) supplemented with 0.05% Tween 80 and 10% bovine serum albumin-dextrose-catalase (ADC) enrichment at 37°C for approximately 10-14 days (mid- to late-log phase). Pyrazinamide (PZA) (Sigma-Aldrich Co.) or isoniazid (INH) was dissolved in deionized water at a stock concentration of 10 mg/ml and filter-sterilized and incorporated into 7H11 agar plates containing ADC at concentrations of 100, 200, 300 μg/ml, pH6.0 for PZA, or at 0.2, 0.4, 1, and 2 μg/ml for INH. Mutants that grew on the PZA or INH containing plates after 3-4 week incubation at 37 °C were picked and grown in 7H9 liquid medium for confirming resistance phenotype. The PZA susceptibility testing of the PZA-resistant mutants was performed on 7H11 agar plates containing 100, 200, 300 μg/ml PZA (pH6.0) as described [Zhang S et al., 2013].

*SYBR Green I/PI viability staining for rapid antibiotic susceptibility testing*

Cultures of fast growing organisms (*S. aureus, E. coli,* and *K. pneumoniae*) were grown to stationary phase and diluted OD600 of 0.1 before treatment with antibiotics of indicated concentrations. After 30 minutes of incubation at 37°C, the cultures were stained with SYBR Green I/PI and the percentage of viable cells was determined as described above. For rapid drug susceptibility testing of *M. tuberculosis,* the culture of parent H37Ra (H37Ra WT) and INH-resistant mutant strains were respectively added into a 96-well plate, each well containing 80 μl of culture. INH in 20 μl 7H9 medium was added into 80 μl culture as the INH treated group; meanwhile, the untreated group was the control. The plate was sealed by aluminum seal foil and incubated at 37°C overnight (15 h), followed by the addition of 10 μl SYBR green I/PI staining mixture to the above treated and untreated cultures. The plate was incubated at room temperature in the dark for 45 minutes followed by fluorescence detection with a microplate reader as described above. Relative to the untreated group, the viability of INH treated H37Ra WT and INH resistant mutants were calculated according to the green and red fluorescence ratio. The difference of residual viability between H37Ra WT and INH resistant mutants were compared. For PZA susceptibility testing in *M. tuberculosis,* 100 μl of 20 day old cultures of *M. tuberculosis* H37Ra and PZA-resistant mutants (approx. $5x10^6$) was mixed with 4 μl PZA (50 mg/ml) alone or with enhancer (salicylic acid (40 μg/ml) or acetic acid (8 μM)). After overnight incubation, 10 μl SYBR green I/PI staining mixture was added to 100 μl PZA treated and untreated cultures. The plate was incubated at room temperature in the dark for 45 minutes and the percentage of viable cells was determined as described above. All untreated groups were used as controls. Statistical analyses were performed using Student's t-test. All experiments were performed in triplicates.

*SYBR Green/PI viability staining to determine antimicrobial susceptibility to antibiotics*

Stationary phase cultures were diluted to indicated concentrations before treatment with varying concentrations of antibiotics (0 μg/mL, 5 μg/mL, 30 μg/mL, 50 μg/mL, and 100 μg/mL). After 1.5 hr of incubation at 37 °C, the cultures were stained with SYBR Green/PI and the percentage of viable cells was determined as described above.

*Persisters assay*

Stationary phase cultures of *E. coli* UTI89 were treated with ofloxacin (5 μg/mL) for 4 hours to enrich for persisters, followed by drug exposure with different antibiotics including ampicillin (100 μg/mL), ofloxacin (5 μg/mL), polymycin B (10 μg/mL) and gentamicin (20 μg/mL). The bacterial viability was determined by CFU enumeration and SYBR Green/PI assay as described above.

Results

*SYBR Green/PI assay can be used for assessing the viability of various bacterial pathogens*

In order to develop a rapid test to evaluate antimicrobial susceptibility in the clinic, it is important to ensure that SYBR Green/PI staining can assay the viability for a range of organisms, especially those considered by the CDC as serious or urgent threats (CDC). To validate that the SYBR Green/PI stain can accurately measure the viability of different bacteria, the SYBR Green/PI assay was applied to generate a standard curve for Gram-positive bacteria using *S. aureus* as an example, Gram-negative bacteria using *K. pneumoniae, Acinetobacter baumannii,* and *E. coli* as examples, and acid-fast using using *M. tuberculosis* as an example. The SYBR Green/PI assay, which was developed for assessing the viability of *Borrelia burgdorferi* (Feng, et al., 2014), was adapted. First, the bacteria were grown in their normal culture media, and to generate dead cells, the bacteria were killed by treating the cells with 70% isopropyl alcohol for one hour. Mixtures were then made of live and dead cells in known proportions. After staining the cells with SYBR Green/PI, the green and red fluorescence intensities of the different samples were measured using a fluorescence microplate reader (BioTek Synergy HT) and a standard curve was generated. A linear relationship between the green/red fluorescence ratio and the percentages of live *S. aureus, Klebsiella pneumonia, E. coli, Acinetobacter* 0.9807, and *baumannii,* and *M. tuberculosis* with $R^2$ values of 0.9934, 0.9980, 0.9853, 0.9807, and 0.9561, respectively, were generated (FIG. 26A, FIG. 26B, FIG. 26C, FIG. 26D, and FIG. 26E).

The results indicate that SYBR Green/PI can successfully act as a viability stain for *S. aureus, E. coli, K. pneumoniae, A. baumannii,* and *M. tuberculosis.* Additionally, staining of varying viable proportions (0%, 50%, and 100%) of Gram-positive *S. aureus* (Fig. 26F) and Gram-negative *K. pneumoniae* samples (Fig. 26F) with SYBR Green/PI revealed that there is a good correlation between the amount of viable cells in the samples from fluorescence microscopy imaging and the actual known viable percentages (FIG. 27A, FIG. 27B, FIG. 27C, FIG. 27D, FIG. 27E, and FIG. 27F).

*Validation of the SYBR Green/PI assay for antibiotic susceptibility testing*

To assess if the SYBR Green/PI assay could be used for antimicrobial susceptibility testing, the SYBR Green/PI assay was tested to detect the MIC of chloramphenicol and ampicillin for *S. aureus* and *E. coli,* respectively. Following the protocols for antibiotic susceptibility testing (AST) method based on measuring the turbidity of bacterial growth as described by the CLSI (CLSI, 2016), the bacterial cultures were subjected to 2-fold dilutions of antibiotics ranging from (0 μg/mL to 32 μg/mL). After overnight incubation, no signs of turbidity was detected in the sample with 8 μg/mL of chloramphenicol for *S. aureus* (FIG. 28A) and 4 μg/mL of ampicillin for *E. coli* (FIG. 28B). No signs of turbidity were determined by OD600 readings of approximately 0.5, the read-out of the background signal. Similarly, the decrease and subsequent plateau of the live/dead ratio from SYBR Green/PI staining also reveals the same MIC for both bacteria and the respective drug, suggesting that SYBR Green/PI can be used to determine antibiotic susceptibility results that are similar to the currently accepted standard method.

*Development ofa rapid drug susceptibility test using SYBR Green/PI assay for fast growing organisms*

Because resistant strains are tougher to kill than susceptible strains, SYBR Green/PI assay will be able to distinguish resistant and susceptible categories based on the amount of residual viable cells in the culture after drug treatment. Since the SYBR Green/PI assay detects viability and killing (Feng, et al., 2014), this assay will eliminate the lag time needed for growth to occur, and produce antimicrobial susceptibility results in a shorter time than the traditional growth based methods.

To develop and validate the usage of SYBR Green/PI staining as a means for rapid antimicrobial testing, appropriate time needs to be identified where viability differences between resistant and sensitive strains can be observed. It had to be ensured that the assay can determine results that are in concordance with the current standards provided by CLSI. Thus, various antibiotic sensitive and resistant clinical isolates were obtained and we tested their susceptibility categories against drugs based on the traditional Kirby-Bauer disk diffusion assay. Then, we measured the green/red fluorescence ratio of both sensitive and resistant strains across the time course of 1.5 hours during drug exposure. For *S. aureus,* exposing the cultures to kanaymcin (100 μg/ml), a significant decrease in viability was observed as early as 30 minutes between the sensitive Newman strain and the resistant CA127 strain (two-way ANOVA, $p < 0.005$) (Fig. 28C). Similarly, for *E. coli,* we also observed a significant decrease in viability in sensitive W3110 and resistant KTE181 strains after exposure to ampicillin (100 μg/ml) in as early as 30 minutes (two-way ANOVA, $p < 0.005$) (Fig. 28D).

For *S. aureus* strains CA-409, NY-315, and CA-127, the percentages of live cells across the increasing drug concentration gradients of erythromycin, ciprofloxacin, gentamicin, and/or kanamycin determined by the green/red fluorescence ratio from SYBR Green/PI staining were higher than pan-susceptible Newman strain, a laboratory sensitive control strain, indicating that strains CA-409, NY-315, and CA-127 are resistant to the respective drugs (FIG. 30).

Similarly, the SYBR Green/PI staining also was able to detect the difference between strain USA300 and USA300 inserted with a chloramphenicol resistance (pRAB11) plasmid (FIG. 30). On the other hand, the strain USA300 showed equal to or a lower percentage of viable cells than Newman across the increasing concentrations of gentamicin, suggesting that USA300 is susceptible to gentamicin (FIG. 30). The antibiotic susceptibility determined by the SYBR Green/PI stain matched completely with the susceptibility results of the traditional Kirby-Bauer disk diffusion test but was performed in only 2 hours (FIG. 29 and FIG. 30).

To optimize the applicability of our SYBR Green/PI assay for AST, we exposed *S. aureus* strains to a panel of bactericidal and bacteriostatic drugs of various concentrations including gentamicin, kanamycin, erythromycin, and ciprofloxacin. To establish a quantitative cut-off to distinguish resistant and susceptible strains, a mathematical formula was used to determine the proportion of cells killed based on the green and red fluorescence values from microtiter plate readings. The proportion of cells killed is calculated by the formula:

$$(LD_{treated} - LD_{untreated}) / LD_{untreated}$$

where LD is equal to the ratio of live (green fluorescence) and dead (red fluorescence) cells. Upon calculating the respective killed proportions for all susceptible and resistant strains tested (FIG. 30), cut-offs distinguishing resistant and susceptible strains for the different antibiotics tested were established by averaging all the values (i.e., proportion of killed cells) from the respective susceptibility categories (FIG. 31). The cut-off for *S. aureus* strains susceptible and resistant to erythromycin (100 μg/mL) is -0.4 and -0.1, respectively; the cut-off for ciprofloxacin (100 μg/mL) susceptibility and resistance is -0.3 and -0.1, respectively; the cut-off for gentamicin (100 μg/mL) susceptibility and resistance is -0.3 and -0.2, respectively; the cut-off for kanamycin (100 μg/mL) susceptibility and resistance is -0.2 and -0.01, respectively; the cut-off for chloramphenicol (100 μg/mL) susceptibility and resistance is -0.5 and -0.1, respectively (FIG. 31).

To evaluate if the detection time of the presently disclosed SYBR Green/PI AST could be accelerated to under 2 hours, the inoculum size of the bacteria was increased before drug treatment. As opposed to diluting overnight cultures of bacteria to TSB medium 1:500 in the rapid 1.5 hr assay, overnight cultures were diluted to TSB 1:25. The data suggests that increasing the inoculum size of the culture can decrease the time of the AST down to 30 minutes. By generating a cut-off of 20% in proportion of killed cells, the presently disclosed AST can distinguish sensitive and resistant strains as determined by traditional Kirby-Bauer methods. After 30 minutes, kanamycin (25 μg/mL) killed only 5% of resistant strains CA-409, CA-127, NY-315 as opposed to 27% killing of the sensitive Newman strain. After erythromycin (400 μg/mL) treatment, resistant strains NY-315 had 17% killed as opposed to 50% killing in the sensitive Newman strain. Ciprofloxacin (50 μg/mL) killed 21% and 28% in resistant strains CA-409 and CA-127, respectively, while 58% was killed in sensitive Newman strain. Lastly, gentamicin (400 μg/mL) killed 10% in the resistant strain

To ensure that the SYBR Green/PI assay can be applied to different bacterial pathogens, the antimicrobial susceptibility testing was performed for various *E. coli* strains including K12- strain W3110, and uropathogenic clinical strains UTI89, CFT073, KTE181 and tested their susceptibility categories against bactericidal and bacteriostatic drugs using the traditional Kirby-Bauer disk diffusion assay as a reference control method (FIG. 33). After performing the protocol for rapid antimicrobial susceptibility testing as mentioned above, the strains KTE181 and CFT073 showed a higher percentage of live cells throughout increasing concentrations of ampicillin, trimethoprim, and streptomycin compared to W3110, the laboratory strain used as a susceptible control strain, suggesting that KTE181 is resistant to ampicillin, trimethoprim, and streptomycin (FIG. 34). At increasing concentrations of ampicillin, the strain UTI89 have an amount of live cells that was comparable to W3110, which indicates that KTE181 is sensitive to ampicillin (FIG. 34). Not only was the SYBR Green/PI viability assay able to distinguish between susceptible and resistant strains that correlate with the traditional Kirby-Bauer disk diffusion method, the assay also distinguished intermediate resistance as exemplified by findings for UTI89 in streptomycin testing. For streptomycin testing, strains KTE181 and CFT073 showed higher percentages of live cells across all drug concentrations compared to the susceptible control strain W3110, suggesting their resistance. However, while strain UTI89 showed higher percentages of live cells in all drug concentrations compared to W3110, the percentages of live cells were lower than the resistant strains, suggesting that UTI89 has intermediate resistance against streptomycin (FIG. 33 and FIG. 34).

The same formula as described above was used to establish cut-offs for susceptibility and resistance for *E. coli* AST. The cut-off for *E. coli* strains susceptible and resistant to ampicillin (100 μg/mL) is -0.5 and -0.1, respectively; the cut-off for trimethoprim (5 μg/mL) susceptibility and resistance is -0.5 and -0.2, respectively; the cut-off for streptomycin (100 μg/mL) susceptibility, intermediate resistance and resistance is -0.6, -0.5 and -0.1, respectively (FIG. 34 and FIG. 35).

Similar to *S. aureus,* whether AST can be performed in 30 minutes in *E. coli* was tested, as well. Using the same conditions of diluting overnight cultures to fresh TSB media 1:25, similar AST results as traditional Kirby-Bauer using the SYBR Green/ PI assay were observed. Upon ampicillin treatment (100 μg/mL), sensitive strains W3110 and UTI89 were killed 38% and 23%, respectively, while resistant strain KTE181 was killed only 1%. Trimethoprim treatment (25 μg/mL) killed 47% of sensitive strain W3110 but 22% and 1% of resistant strains CFT073 and KTE181, respectively.

Streptomycin treatment (25 μg/mL) killed 29% of sensitive strain W3110 but 0% in resistant strain KTE181. Similarly, the presently disclosed assay was tested in another Gram-negative organism, *K. pneumoniae.* Upon ceftriaxone and cefotaxime (25 μg/mL) treatment, sensitive strain W3110 was killed 37% and 24%, but the resistant *K. pneumoniae* strain was not killed at all. The presently disclosed AST produced findings in concordance with results from the Kirby-Bauer disk diffusion test for both *E. coli* and *K. pneumoniae.* (FIG. 36 and FIG. 37).

*Validation of the SYBR Green/PI assay in quantitating persister numbers*

While antibiotic resistance is a major public health problem, antibiotic persistence mediated by persister cells play a significant role in causing recurrent and relapsing infections (Zhang, 2014). Research and development in novel drug therapies to eradicate persistent infections such as persistent Lyme Disease (Feng, et al., Emerg Microbes Infect, 2014), biofilm infections from indwelling devices, endocarditis & osteomyelitis caused by *S. aureus* (Niu, et al., 2015) and urinary tract infections caused by *E. coli* (Niu, et al., Antibiotics, 2015) are of utmost importance. Currently, researchers are depending on serial dilutions to enumerate colonies forming on agar plates, which is also dependent on bacterial growth and hence, time-consuming. To test if SYBR Green/PI can be used to rapidly detect the presence of persisters in different drug treatments, persister-enriched cultures of *E. coli* were exposed to single drug treatment of ampicillin and ofloxacin, and a drug combination of gentamicin and polymycin B. Starting at Day 3, the decrease in the level of persisters after exposure with the combination of polymycin B and gentamicin can be detected by both CFU enumeration and also green/red fluorescence ratio from SYBR Green/PI staining. In the samples with a single drug treatment (ampicillin and ofloxacin) and the no drug treated sample (negative control), both CFU enumeration and SYBR Green/PI staining revealed that the amount of persisters stayed constant throughout the course of 7 days (FIG. 38). The presently disclosed findings suggest that the SYBR Green/PI assay can also be used to perform high-throughput drug screens as was demonstrated with *Borrelia burgdorferi* (Feng, et al., Emerg Microbes Infect, 2014; Feng, et al., 2015; Feng, et al., 2016) to search for better therapies forrecurrent infections by other bacterial pathogens.

*Demonstration that bacteriostatic drugs work as well as bacteriocidal drugs with SYBR Green I/PI assay in distingusihing sensitive and resistant strains*

By definition, bactericidal drugs kill the bacteria whereas bacteriostatic drugs inhibit the growth of the bacteria. Here we wanted to address if the SYBR Green I/PI assay would also work with bacteristatic drugs in addition to bactericidal drugs in detecting sensitive strains versus resistant strains. We tested the viability of both resistant and sensitive strains of *S. aureus* and *E. coli* to increasing concentrations of bactericidal and bacteriostatic drugs (Fig. 42). Our SYBR Green I/PI assay revealed that treatment with bactericidal drug kanamycin (100, 200, 400, and 800 μg/ml) for 30 minutes consistently killed 40% of the cells in the sensitive *S. aureus* Newman strain but not the resistant NY315 strain, as expected (Fig. 42A). For *E. coli,* the percentage of killed cells after treatment with bactericidal drug ampicillin (100, 200, 400, and 800 μg/ml) increased in a dose-dependent manner. A statistical significant difference between the sensitive strain W3110 and resistant strain KTE181 was evident using 100 μg/ml of ampicillin but a 10% higher of cells were killed using 200 μg/ml. Using 400 and 800 μg/ml of ampicillin resulted in killing activity above the limit of detection for the sensitive strain (Fig. 42B).

To test for killing activity by bacteriostatic drugs, we added increasing concentrations of bacteriostatic agent erythromycin (200, 400, 800, and 1000 μg/ml) to *S. aureus* strains (Fig. 42C) and bacteriostatic drug trimethoprim (50, 100, 150, and 300 μg/ml) to *E. coli* strains (Fig. 42D). In both cases, the killing activity increased in a dose-dependent manner. For both sensitive and resistant strains, a larger (and even above the detection limit) amount of killed cells were detected as the concentrations of drugs increased. Statistical significance between the amount of killed cells in sensitive *S. aureus* Newman and resistant NY315 strain (using 200 μg/ml erythromycin) and sensitive *E. coli* W3110 and resistant KTE181 strain (using 50 μg/ml trimethoprim) were observed at appropriate drug concentrations, though killing activity between the strains became insignificant when the highest concentration of drugs were administered. Our findings suggest that the SYBR Green I/PI assay also works for bacteriostatic drugs in distinguishing sensitive and resistant strains when used at appropriate concentrations.

**Implementation of SYBR Green I/PI assay for rapid drug susceptibility testing for *M. tuberculosis***

Having demonstrated that the SYBR Green/PI assay has worked successfully for fast growing bacteria above, we wanted to determine if this assay also works for slow growing *M. tuberculosis* which takes 2-6 weeks to get the drug susceptibility testing (DST) results. We first tested the utility of the SYBR Green/PI assay for detecting isoniazid (INH) resistance. Because INH mainly acts on growing cells of *M. tuberculosis* [Zhang and Yew 2009], we used a 7-10 day log phase *M. tuberculosis* H37Ra culture and INH-resistant H37Ra mutants (I2 and I4) to assess if the SYBR Green I/PI assay could be used for rapidly identifying drug resistance for *M. tuberculosis.* We treated the *M. tuberculosis* H37Ra

and INH resistant mutants with different INH concentrations (10, 100, 500 and 1000 μg/ml) in 96-well microtiter plate overnight, followed by SYBR Green I/PI assay to determine the percentage of cells killed (see Methods). We did not find significant differences between the proportion of killed H37Ra and INH resistant strains at lower concentrations of10 and 100 μg/ml INH in overnight drug treatment. However, at higher concentrations of INH (500 μg/ml and 1000 μg/ml), the INH-susceptible parent strain H37Ra was killed more effectively compared to the two INH resistant mutants I2 and I4 (Fig. 41A) with statistically significant difference ($P<0.005$, $P<0.05$, respectively). However, 1000 μg/ml INH also caused death of the INH resistant strains (Fig. 41A) and decreased the difference in residual viability between the susceptible parent strain H37Ra and the INH resistant mutants. Thus, these results indicate that the SYBR Green I/PI assay at the appropriate drug concentration (500 μg/ml) could rapidly detect INH resistance in *M. tuberculosis* in just 16 hr as compared to weeks with the current growth based DST methods.

We then went on to assess if the SYBR Green/PI assay could be used for detecting pyrazinamide (PZA) resistance, the most challenging DST of all TB drugs. Because PZA plays a critical role in shortening TB treatment from previously 9-12 months to 6 months and is used for treating both drug susceptible and drug resistant TB, accurate testing of PZA susceptibility is crucial for treatment outcome. Unfortunately, the current PZA susceptibility testing methods produce unreliable results and can take weeks due to reliance on growth of *M. tuberculosis* [6-8]. Also, a small percentage of PZA-resistant strains do not have mutations in *pncA* or *rpsA* involved in PZA's mechanisms of resistance, which limits the capacity of molecular testing for PZA susceptibility [Zhang Y et al., 2013]. Thus, there is a huge need for a rapid and reliable PZA DST for more effective clinical care. To evaluate if our rapid AST can detect PZA resistance, we treated the parental strain H37Ra (MIC<100 μg/ml PZA, pH5.9) and PZA resistant mutants (>200 μg/ml PZA, pH5.9) with different PZA concentrations (0.1, 0.5, 1 and 2 mg/ml) followed by viability staining using SYBR Green I/PI. We found that the residual viability difference between H37Ra and PZA resistant strains increased with higher PZA concentration after overnight (16 hr) treatment. After optimization, we found the residual viability of H37Ra decreased significantly as compared with the PZA resistant mutants with 2 mg/ml PZA treatment overnight (Fig. 41B), suggesting that our assay could identify PZA resistance in just 16 hr.

Since PZA acts differently from common antibiotics by killing nonreplicating *M. tuberculosis* persisters [Zhang Y et al., 2013], certain weak acids and acid pH are known to enhance the activity of PZA against *M. tuberculosis* [Zhang et al., 1999; Wade and Zhang 2006]. Therefore, we tested if weak acids salicylic acid (SA) and acetic acid (AA) when added to the PZA treatment could help show more significant difference in residual viability between susceptible parental strain H37Ra and PZA-resistant mutants. Indeed, we found that the 40 μg/ml salicylic acid or 8 μM acetic acid significantly enhanced the activity of PZA against parent strain H37Ra but not PZA resistant mutants at 2 mg/ml PZA in overnight (16 hr) treatment (Fig. 41B). Using the SYBR Green I/PI assay, the residual viability of H37Ra was significantly lower than the residual viability of the two PZA-resistant mutants P2 and P5 (Fig. 41B) ($p <0.05$). These results showed that the SYBR Green I/PI assay with weak acids could allow the difference in susceptibility to PZA between susceptible strain and PZA resistant mutants to show more clearly in the presence of the weak acids and quantitatively measure PZA resistance in *M. tuberculosis.* Thus, the new PZA DST using SYBR Green/PI assay which can be performed in less than 1 day has considerable advantage over the current growth based PZA DST which takes 2-6 weeks.

## Discussion

The presently disclosed subject matter provides an ultra-rapid antibiotic susceptibility testing (AST) method using SYBR Green I/PI viability assay that is growth independent and can be performed in hours rather than days and weeks for the current AST. The current conventional AST relies on the growth of the test organism in the presence of the antibiotic, which can be time consuming depending the growth rate of the test organism, ranging from days with fast growing bacteria to weeks for slow growing bacteria. The presently disclosed subject matter demonstrates that by using an optimized SYBR Green I/PI assay, the AST for representative Gram-positive, Gram-negative and acid fast bacteria using *S. aureus, E. coli* could be determined in less than 2 hrs, and for and for slow growing mycobacteria, *M. tuberculosis,* in 16 hrs rather than days or weeks as current tests. The presently method takes advantage of the rapid SYBR Green I/PI viability assay which was developed initially for rapid assessment of viability of *B. burgdorferi* (Feng, et al., 2014) and was used for high throughput drug screens against *B. burgdorferi* persisters (Feng, et al., Emerg Microbes Infect, 2014; Feng, et al., 2015; Feng, et al., 2016). Here, this SYBR Green I/PI viability assay was adapted and found to work very well for AST for different bacteria (FIG 29, FIG. 30, FIG. 31, FIG. 32, FIG. 33, and FIG. 34).

Antibiotics with different modes of action, including cell wall inhibitor ampicillin, protein synthesis inhibitor streptomycin, folate synthesis inhibitor sulfa drug trimethoprim, DNA gyrase inhibitors ciprofloxacin (quinolones) were tested in the SYBR Green I/PI assay and they all worked well and could determine the AST results in less than 2 hr for fast growing bacteria like *E. coli* and *S. aureus.* It is worth noting that the SYBR Green I/PI assay not only works for bactericidal antibiotics such as ampicillin, streptomycin, ciprofloxacin, gentamicin, and kanamycin and also bacteriostatic drugs such as sulfa drug trimethoprim, erythromycin, and chloramphenicol (Fig. 42).

The presently disclosed subject matter used liquid stationary phase cultures as inocula, which is more compatible

with the current blood culture system used commonly in the clinic lab setting. Previous studies have shown that flow cytometry can be used for AST testing for *M. tuberculosis* in 24 hr (Hughes, et al., 2005). However, the test still relies on detecting of growth by fluorescence diacetate, and the most significant drawbacks are the requirement for expensive instrumentation and lack of throughput by the flow cytometry method. Because of these shortcomings, the flow cytometry method has not been adopted by clinical or field settings. Although a luciferase phage system has been developed for rapid AST for RIF for mycobacteria in 2 days (Wilson, et al., 1997; Jacobs, et al., 1993), it has contamination issues that limit its use in field settings (Mole, et al., 2007).

Advantages of the new SYBR Green I/PI assay include: rapid AST results in less than 2 hrs for fast growers and 16 hrs for slow growers, low cost using plate reader without use of expensive instrumentation (mass spectrometry and flow cytometry), and high throughput capability in 96-well or 384 well plate format.

EXAMPLE 6

A Rapid Pyrazinamide Susceptibility Test in 16 Hours by SYBR Green/PI Assay

A rapid PZA drug susceptibility testing (DST) method was developed based on a modified SYBR Green I/PI assay for viability assessment of *M. tuberculosis.* Using this SYBR Green/PI assay, PZA resistance in resistant mutants could clearly be distinguished from PZA susceptible control strain. This SYBR Green/PI assay has allowed the time required for DST results to be reduced from weeks to less than 1 day. This is made possible using the SYBR Green I/PI viability assay by changing the concept of DST through converting a conventional time-consuming growth/culture-dependent DST to a viability based and growth/culture-independent DST. The presently disclosed subject matter demonstrates the feasibility and reliability of adapting the SYBR Green I/PI assay for rapidly identifying PZA resistance in *M. tuberculosis.* The SYBR Green I/PI assay overcomes not only the inefficiency of classic microbiology growth/culture based DST relying on growth of slow growing *M. tuberculosis* (which takes several weeks), but also the limitations of molecular testing methods (with compromised sensitivity compared with phenotype based DST as mutations are not all covered by molecular tests). The SYBR Green I/PI assay can be developed as a convenient, accurate and quantitative test for rapid DST for all TB drugs, with capability of high throughput, thus providing timely guidance on clinical treatment of drug resistant TB, with high efficiency and low cost that can be easily adapted for field application.

Background

Tuberculosis (TB) remains a leading infectious killer worldwide, with 9 million new TB cases and 1.5 million deaths annually (WHO, 2008). Drug-resistant tuberculosis (TB), especially multidrug-resistant TB (MDR-TB) (with bacillary resistance to at least isoniazid and rifampicin) and extensively drug-resistant TB (XDR-TB) poses an increasing challenge for TB control (WHO, 2008). The standard 6 month TB therapy recommended by WHO for treatment of drug susceptible TB consists of 2 months of daily treatment with isoniazid (INH), rifampicin (RIF), pyrazinamide (PZA) and ethambutol, followed by 4 months of daily or intermittent treatment with INH and RIF (WHO, 2008). The lengthy therapy makes patient compliance difficult and increases the risk of development of drug resistant TB.

PZA plays a unique role in shortening of the treatment from previously 9-12 months to 6 months and is used for treating both drug susceptible and drug resistant TB (Mitchison, 1985). The powerful sterilizing activity of PZA is due to its ability to kill a population of persister tubercle bacilli that are not killed by other TB drugs (Mitchison, 1985). However, PZA can have potential hepatotoxicity (Chang, et al., 2008), and should be avoided when there is bacillary resistance demonstrated. In a number of available studies, PZA resistance in MDR-TB ranges from 10% in Papua New Guinea (Simpson, et al., 2011), 25% in Turkey (Senol, et al., 2008), 49% in Thailand (Jonmalung, et al., 2010), to 52% in South Africa (Louw, et al., 2006), 53% in Japan (Ando, et al., 2010), 76.6% in Pakistan (Rao, et al., 2010); 85% in India (Shenau, et al., 2009). Bacillary resistance to PZA is even higher in XDR-TB cases (Velayati, et al., 2009). In view of the potential importance of PZA resistance on MDR-TB treatment outcome (Migliori, et al., 2007), it is critical to determine the prevalence of PZA resistance and susceptibility in MDR-TB strains.

However, the PZA drug susceptibility testing (DST) methods, such as Lowenstein-Jensen medium and 7H10/11 agar at pH 5.5, BACTEC 460 and MGIT 960 or BacT/ALERT systems at pH 6.0 (Aragon, et al., 2007), are not reliable and are subject to frequent false resistance problem (Zhang, et al., 2003; Hewlett, et al., 1995; Chedore, et al., 2010). In a recent study, the MGIT960 PZA susceptibility testing method showed even less reliable results than the discontinued radioactive BACTEC 460 method causing more false resistance problem presumably due to higher inoculum size used in the MGIT method (Chedore, et al., 2010). These phenotype based PZA DST methods rely on growth of slow growing *M. tuberculosis,* which can take a lengthy period of 2-6 weeks to obtain the DST results.

Since mutation in the *pncA* gene encoding pyrazinamidase/nicotinamidase (Scorpio, et al., 1996) is the major mechanism for PZA resistance in *M. tuberculosis* (Scorpio, et al., 1996; Scorpio, et al., 1997; Cheng, et al., 2000) and in view of the good correlation of *pncA* mutations and PZA resistance (Scorpio, et al., 1997; Cheng, et al., 2000; Somoskovi,

et al., 2007), detection of *pncA* mutations by rapid molecular tests such as DNA sequencing is considered a good correlate of PZA resistance and can circumvent the problem of phenotype based PZA DST (Zhang, et al., 2012). However, a small percentage of PZA-resistant strains do not have *pncA* or *rpsA* mutations but due to other unknown resistance mechanisms (Zhang, et al., Microbiol Spectrum, 2014), therefore, molecular test detecting *pncA* mutations cannot replace phenotypic PZA susceptibility testing.

A SYBR Green I/PI viability assay for rapid assessment of *Borrelia burgdorferi* viability was recently developed (Feng, et al., 2014) and this method has successfully been used for high throughput drug screens against *B. burgdorferi* persisters (Feng, et al., Emerg Microbes Infect, 2014; Feng, et al., 2016). The presently disclosed subject matter provides the successful adaptation of this SYBR Green I/PI viability assay for rapid PZA DST in *M. tuberculosis* in 16 hrs.

## Methods

*Isolation of spontaneous PZA-resistant mutants of M. tuberculosis*

*Mycobacterium tuberculosis* H37Ra was grown in 7H9 liquid medium (Difco) supplemented with 0.05% Tween 80 and 10% bovine serum albumin-dextrose-catalase (ADC) enrichment at 37°C for approximately 10-14 days (mid- to late-log phase). Pyrazinamide (Sigma-Aldrich Co.) was dissolved in deionized water at a stock concentration of 10 mg/mL and filter-sterilized and incorporated into 7H11 agar plates containing ADC at concentrations of 100, 200, 300 μg/mL, pH6.0. Mutants that grew on the PZA containing plates after 3-4 week incubation at 37 °C were picked and grown in 7H9 liquid medium for confirming PZA resistance phenotype by repeated PZA susceptibility testing. The PZA susceptibility testing of the PZA-resistant mutants was performed on 7H11 agar plates containing 100, 200, 300 μg/mL PZA (pH6.0) as described (Zhang, et al., 2013).

*Microscopy techniques*

Specimens of *M. tuberculosis* cultures or cells were examined on a Zeiss AxioImager M2 microscope equipped with epifluorescence illumination. Pictures were taken using ORCA-$R^2$ high resolution digital camera (HAMAMATSU, Japan). A SYBR Green I/PI assay was performed as previously described to assess cell viability using the ratio of green:red fluorescence to determine the live:dead cell ratio (Feng, et al., 2014). This residual cell viability was confirmed by analyzing three representative images of the bacterial culture using epifluorescence microscopy. Image Pro-Plus software was used to quantitatively determine the fluorescence intensity.

*Establishing a rapid viability test of M. tuberculosis using SYBR Green I/PI assay.*

For assaying the live and dead cells in 96-well plates, SYBR Green I and PI were used for double staining of nucleic acids. SYBR Green I (10,000 × stock, Invitrogen) (10 μL) was mixed with 30 μL propidium iodide (20 mM, Sigma) into 1.0 mL of sterile $dH_2O$ and vortexed thoroughly. The staining mixture (10 μL) was added to each well (100 μL) and mixed thoroughly. The plate was incubated at room temperature in the dark for 45 minutes. With excitation wavelength at 485 nm, the fluorescence intensities at 538 nm (green emission) and 612 nm (red emission) were measured for each well of the plate using Synergy H1 microplate reader (BioTek Inc., USA).

Meanwhile the *M. tuberculosis* suspensions (live and 70% isopropyl alcohol killed) in five different proportions of live:dead cells (0:10, 2:8, 5:5, 8:2, 10:0) was mixed in wells of 96-well plate. The SYBR Green I/PI was added to each well and the green/red fluorescence ratios were measured for each proportion of live/dead *M. tuberculosis* using Synergy H1 microplate reader. With least-square fitting analysis, the regression equation and regression curve of the relationship between percentage of live bacteria and green/red fluorescence ratios were obtained. The regression equation was used to calculate the percentage of live cells in each well.

*SYBR Green I/PI assay for rapidly testing PZA susceptibility*

The culture of wild type H37Ra (H37Ra WT) and PZA (Pyrazinamide) resistant mutants were respectively added into a 96-well plate, each well contains 100 μL culture (20 day old, $5 \times 10^6$). 4 μL PZA (50 mg/mL) alone or with enhancer (salicylic acid 40 μg/mL, or acetic acid 8 μM) was added into 100 μL culture as PZA treated group; meanwhile untreated group was set as control. After overnight incubation, 10 μL SYBR green I/PI staining mixture was added to 100 μL PZA treated and untreated cultures. The plate was incubated at room temperature in the dark for 45 minutes. The fluorescence intensities at 538 nm (green emission) and 612 nm (red emission) were measured for each well of the plate using Synergy H1 microplate reader (BioTek Inc., USA). Relative to the untreated group, the viability of PZA treated H37Ra WT and PZA resistant mutants were calculated according to the green and red fluorescence ratio. The difference in residual viability between H37Ra WT and PZA resistant mutants was compared by standard *t*-test.

Results and Discussion

*Isolation of spontaneous PZA-resistant mutants of M. tuberculosis H37Ra*

The parent *M. tuberculosis* strain H37Ra was susceptible to 100 μg/mL PZA (pH5.9). To isolate PZA-resistant spontaneous mutants, early stationary phase cultures of *M. tuberculosis* H37Ra were plated on 7H11 agar plates containing 200 μg/mL PZA (pH5.9). Four PZA resistant mutants were selected for developing the rapid PZA DST method by SYBR Green/PI assay as below.

*A rapid viability test for M. tuberculosis by SYBR Green I/PI assay*

SYBR Green I dye is commonly used for staining nucleic acids in gels and RT-PCR in molecular biology techniques. In previous studies with *Borrelia burgdorferi,* it was found that SYBR Green I/PI assay showed more sensitivity, more reliability and low background, than other viability assays such as MTT and XTT assays, FDA assay, commercial LIVE/DEAD BacLight kit, and the Sytox Green/Hoechst 33342 assay (Feng, et al., 2014). In the presently disclosed subject matter, the SYBR Green I/PI assay was adapted to be used as a rapid viability assay for *M. tuberculosis.*

For *M. tuberculosis* H37Ra, the SYBR Green I/PI viability assay was optimized with a range of concentrations of SYBR Green I and PI dyes and tested with serial dilutions of *M. tuberculosis* culture. A linear relationship between the fluorescence ratio and the number of *M. tuberculosis* cells was found when the concentration of bacteria was higher than $1 \times 10^6$ bacteria/mL. To validate the SYBR Green I/PI viability assay for *M. tuberculosis,* a mixture of live and dead *M. tuberculosis* cells in known proportions was used as standards. Live and 70% isopropyl alcohol killed *M. tuberculosis* cells were prepared in five different proportions ($10^8$ bacteria/mL) in wells of the 96-well plate for the SYBR Green I/PI assay. The ratios of the integrated intensity of the portion of each spectrum at 538 nm (green) and 612 nm (red) for each bacterial suspension were calculated. The results are consistent with the results of fluorescence microscope counting, showing that the percentages of live bacteria correlated well with the ratio of green fluorescence to red fluorescence in a linear relationship for the SYBR Green/PI assay (FIG. 39).

*SYBR Green I/PI assay for rapid PZA susceptibility testing*

The major impetus for this study is because there is no reliable PZA drug susceptibility testing (DST) method available. Thus there is a need to develop such a rapid PZA DST test for identifying PZA resistance for more effective clinical care. Since the SYBR Green I/PI assay was successfully adapted for rapid viability testing of *M. tuberculosis* (FIG. 1), whether the newly adapted SYBR Green I/PI assay could be used as a rapid PZA DST for detecting PZA resistance was evaluated.

To do so, the residual viability of H37Ra wild type (WT) and the PZA resistant H37Ra mutants were compared after PZA treatment. The H37Ra WT and PZA resistant mutants were treated with different PZA concentrations (0.1, 0.5, 1 and 2 mg/mL) in 96-well microtiter plate, the SYBR Green I/PI assay was then used to determine the residual viability of the mycobacteria (see Methods). It was found that the residual viability difference between H37Ra WT and PZA resistant strains increased with higher PZA concentration in overnight (16 hr) treatment. Higher PZA concentration (2 mg/mL PZA) was more effective to kill *M. tuberculosis* H37Ra WT in a short time span while rapidly distinguishing PZA resistant mutants from drug susceptible H37Ra WT. After optimization, the residual viability of H37Ra WT was found to be obviously decreased as compared with the PZA resistant mutants after 2 mg/mL PZA overnight treatment (FIG. 40 and FIG. 41). This result provided the possibility to rapidly identify PZA resistance in less than one day.

Previous studies showed that PZA acts differently from common antibiotics by killing nonreplicating *M. tuberculosis* persisters (Zhang, et al., Microbiol Spectrum, 2014). Some weak acids and acid pH could enhance the activity of PZA against *M. tuberculosis in vitro* (Wade, et al., 2006; Zhang, et al., 1999). Therefore adding salicylic acid (SA) and acetic acid (AA) was tested in the PZA treatment to see if they help to show more significant difference in residual viability between parent drug susceptible strain H37Ra and PZA resistant mutants. It was found that the 40 μg/mL salicylic acid or 8 μM acetic acid could indeed enhance the activity of PZA against H37Ra WT but not PZA resistant mutants at 2 mg/mL PZA in overnight treatment (FIG. 40 and FIG. 41). Using the SYBR Green I/PI assay, the residual viability of H37Ra WT was significantly lower than the residual viability of the two PZA resistant mutants P5, P2 (FIG. 41), and their difference achieved statistical significance ($P<0.05$). These results showed that the SYBR Green I/PI assay with weak acids could quantitatively assay the PZA resistance in *M. tuberculosis* in less than 1 day. This provides proof of principle test for performing DST with the newly developed SYBR Green I/PI assay for PZA and all other TB drugs in less than one day, thus revolutionizing TB drug susceptibility testing, which currently takes weeks to complete and often with expensive instrumentation.

Previous studies have shown that flow cytometry can be used for PZA DST for *M. tuberculosis* in 24 hr (Fredericks, et al., 2006). However, this method still relies on detecting of growth by fluoroscein diacetate, and the most

significant drawbacks are the requirement for expensive instrumentation of flow cytometer and lack of high throughput. Because of these shortcomings, the flow cytometry method has not been adopted by clinical or field settings.

In summary, the SYBR Green I/PI assay was adapted for viability assessment of *M. tuberculosis* and developed a rapid PZA DST, reducing the time required for DST results from previously weeks to less than 1 day. This is made possible using the SYBR Green I/PI viability assay by converting a conventional growth/culture-dependent DST to a viability based and growth/culture-independent DST. The presently disclosed subject matter is the first to demonstrate the feasibility and reliability of the adapted SYBR Green I/PI assay for rapidly identifying PZA resistant *M. tuberculosis* mutants. The SYBR Green I/PI assay overcomes the inefficiency of classic microbiology growth/culture based DST relying on growth of slow growing *M. tuberculosis* (which takes several weeks) and the limitations of molecular testing methods (with compromised sensitivity compared with phenotype based DST as mutations are not all covered by molecular tests). The SYBR Green I/PI assay can be developed as a convenient and accurate test for rapid TB DST for all TB drugs and thus provide timely feedback for guiding clinical treatment.

## EXAMPLE 7

### Extremely Rapid Growth-Independent Drug Combination Screens for Non-replicating Persisters of Slow Growing M. tuberculosis Achieved in 30 minutes by SYBR Green I/PI Assay

### Materials and Methods

*Antibiotics and FDA drug library*

Each drug in the JHCCL FDA-approved drug library version 1.32 was pre-diluted in 1 mM stock solutions with DMSO. The first and the last columns in each pre-diluted plate were set as DMSO drug free controls and PZA alone control. The pre-diluted drug stock plates were sealed and stored at -20°C. PZA was dissolved in dimethylsulfoxide (DMSO) to form 20 mM stock.

*Rapid drug screens to identify drug candidates which may enhance PZA activity against M. tuberculosis using the SYBR Green/PI assay*

*M. tuberculosis* H37Ra was cultured in 7H9 medium with 10% albumin-dextrose-catalase (ADC) at 37°C for 20 days as stationary phase culture in sterile 50 mL closed conical tubes. The stationary phase cells were collected by centrifugation and resuspended in pH 5.5 7H9 medium without ADC. Glass beads were added and the tube was vortexed to break up clumps and aggregates. Culture was allowed to settle for 10 minuites and the supernatant containing dispersed cell suspension was kept. Cell numbers were counted using a counting chamber (Hausser Scientific Partnership, PA, USA). The culture was diluted to $10^7$ cells/mL with pH 5.5 7H9 medium without ADC for the drug screen. PZA at a final concentration of 200 μM was added to the cell suspesion for PZA drug combination screen. The culture was transferred to 96-well microplates for high-throughput drug screen.

To qualitatively determine the effect of antibiotics, 5 μl of each compound (final concentration 50 μM) from the pre-diluted stock was added to the *M. tuberculosis* H37Ra culture containing 200 μM PZA in the 96-well plate. The final volume per well was adjusted to 100 μl. Plates were sealed and placed in 37°C incubator for 7 days for the drug screen. The SYBR Green I/ PI viability assay was used to assess the live and dead cells after drug exposure as described (Feng et al., 2014) with some modifications. Briefly, 10 μl of SYBR Green I (10,000 × stock, Invitrogen) was mixed with 30 μl propidium iodide (PI, 20 mM, Sigma) into 1.0 ml of sterile $dH_2O$. Then 10 μl staining mixture was added to each well and mixed thoroughly. The plates were incubated at room temperature in the dark for 30 minutes followed by plate reading at excitation wavelength at 485 nm and the fluorescence intensity at 538 nm (green emission) and 612 nm (red emission) in microplate reader Synergy H1 Multi-Mode plate reader (BioTek Inc., USA). With least-square fitting analysis, the regression equation and regression curve of the relationship between percentage of live and dead bacteria as shown in green/red fluorescence ratios was obtained. The regression equation was used to calculate the percentage of live cells in each well of the 96-well plate.

*Microscopy techniques*

The 96 well microplates were directly examined using BZ-X710 All-in-One fluorescence microscope (KEYENCE, Inc.). The SYBR Green I/PI viability assay was performed to assess cell viability using the ratio of green/red fluorescence to determine the live:dead cell ratio, respectively, as described previously [Feng et al., 2014]. This residual cell viability reading was confirmed by analyzing representative images of the bacterial culture using epifluorescence microscopy. BZ-X Analyzer and Image Pro-Plus software were used to quantitatively determine the fluorescence intensity.

Results

Using this SYBR Green/PI assay, we were able to rapidly identify hit compounds from the FDA-approved drug libray a range of drug candidates that potentially enhanced the activity of the frontiline tuberculosis drug pyrazinamide (PZA) (Table 11). The time needed to obtain the screen results was just 30 minutes required for the staining of the drug treated cells by the SYBR Green/PI assay, followed by detection of green (live cells) versus red (dead cells) fluorescence in the 96-well plates by a fluorescence plate reader. This is in contrast to the growth based CFU assay that would take 4 weeks for slow growing *M. tuberculosis* to grow. Another advantage of the SYBR Green/PI assay is that it does not require washing of the 96 well-plates after drug incubation to obtain results as the plates can be read directly without the tedious washing step, which not only saves time but also prevents loss of small number of cells during the washing step.

Here as examples, we list the top 100 hits identified from the drug combination screen (Table 11). In addition, we show the images of some top hits identified by ratio of green and red fluorescence determined from the plate reader but verified by fluorescence microscopy stained by SYBR Green/PI assay, where red cells indicate dead cells and green cells live cells (see Fig. 43), including rifamixin, cyacetacide, toltrazuril, chlortetracycline, nitroxoline, piroxicam, nifuroxazide, rifampicin, and clofazimine, identified from the drug screen.

Many of the identified drug candidate hits are the same as those identified in our previous conventional growth based drug screens, where after the 7 day drug exposure as the above condition, an additional 4 weeks were required to allow residual viable cells after drug exposure to grow and form colony forming units (CFU) on 7H11 agar plates (paper under review). Thus, the good correlation of the results by the two different screen methods, i.e., SYB Green/PI assay and the conventional CFU based growth assay further validates the utility and relevance of the new and much more rapid SYB Green/PI assay for drug screens for slow growing organisms like *M. tuberculosis.*

**Table 11. Top 100 hits from FDA drug library that synergized with PZA activity against *M. tuberculosis* in drug combination screen using the rapid SYBR Green I/PI assay.**

| Common name | Indication | SYBR Green I/PI ratio |
|---|---|---|
| Rifamixin, RIFAXIMIN | Antibiotic, Antibacterial | 0.958 |
| Formylrifamycin, 3-formyl Rifamycin | Antibacterial (tuberculostatic) | 0.983 |
| CYACETACIDE, Cyacetacide (cyanoacetohydrazide) | Antibacterial | 1.049 |
| Toltrazuril | Antibacterial | 1.068 |
| Domiphen bromide | Antibacterial | 1.098 |
| Cetalkonium Chloride, Cetalkonium chloride (Benzyldimethylhexadecylammonium chloride) | Antibacterial | 1.099 |
| CHLORTETRACYCLINE, Chlortetracycline Hydrochloride | Antibiotic | 1.116 |
| NITROXOLINE, Nitroxoline (8-hydroxy 5-nitroquinoline) | Antibiotic | 1.117 |
| PIROXICAM | Antiinflammatory | 1.118 |
| NIFUROXAZIDE | Antibiotic | 1.120938 |
| RIFAMYCIN, Rifamycin sv | Antibiotic | 1.132 |
| Thiostrepton | Antibiotic | 1.162 |
| MONENSIN, Monensin sodium salt, MONENSIN SODIUM (monensin A is shown), MONENSIN SODIUM | Antibiotic | 1.183 |
| SILVER NITRATE | Antibacterial | 1.25 |
| Diclazuril | Antibacterial | 1.252 |
| Cloxyquin, Cloxyquin (5-chloro-8-hydroxyquinoline) | Antibacterial | 1.267 |
| DIHYDROSTREPTOMYCIN, Dihydrostreptomycin Sulfate | Antibacterial | 1.27 |
| BIFONAZOLE | Antifungal | 1.278 |
| CICLOPIROX OLAMINE, Ciclopirox ethanolamine | Antifungal | 1.282 |

(continued)

| Common name | Indication | SYBR Green I/PI ratio |
|---|---|---|
| ROXITHROMYCIN | Antibiotic | 1.283 |
| SILVER SULFADIAZINE 1 %, Silver Sulfadiazine, Silver (I) sulfadiazine, 98% | Antibacterial | 1.284 |
| TENOXICAM | Antiinflammatory | 1.285 |
| DICLOFENAC, Diclofenac Sodium, Diclofenac sodium salt, SODIUM DICHLOFENAC | Antiinflammatory | 1.293 |
| PYRITHIONE, Pyrithione Zinc, Pyrithione zinc (1-Hydroxypyridine-2-Thione Zinc Salt), 2-Mercaptopyridine N-oxide | Antibacterial | 1.295 |
| RIFAMPIN, Rifampicin, Rifampicin (Rifampin) | Antibacterial | 1.295 |
| Erythromycin Ethylsuccinate | Antibiotic | 1.297 |
| Climbazole | Antifungal | 1.329 |
| Flufenamic acid | Antiinflammatory | 1.335 |
| Meclofenamic Acid, MECLOFENAMATE, Meclofenamate Sodium, Meclofenamic acid sodium salt monohydrate, Meclofenamic acid sodium salt, SODIUM MECLOFENAMATE | Antiinflammatory | 1.34 |
| CLIOQUINOL | Antibacterial | 1.351 |
| CLOFAZIMINE | Antibacterial | 1.354 |
| ECONAZOLEEconazole Nitrate | Antifungal | 1.386 |
| KANAMYCIN, KANAMYCIN acid, Kanamycin Sulfate, Kanamycin A sulfate | Antibiotic | 1.386 |
| IODOFORM | Antibacterial | 1.388 |
| MELOXICAM, MELOXICAM SODIUM | Antiinflammatory | 1.388 |
| TOLFENAMIC ACID | Antiinflammatory | 1.404 |
| CHLORQUINALDOL, Chlorquinaldol (5,7-Dichloro-2-methyl-8-quinolinol) | Antibacterial | 1.421 |
| PRIMAQUINE, Primaquine Phosphate, Primaquine diphosphate | Antimalarial | 1.424 |
| TIOCONAZOLE | Antifungal | 1.431 |
| oxacillin, Oxacillin Sodium | Antibiotic | 1.431 |
| Pazufloxacin | Antibiotic | 1.435 |
| STREPTOMYCIN SULFATE | Antibacterial, Antibiotic | 1.447 |
| OXICONAZOLE, Oxiconazole Nitrate | Antifungal | 1.449 |
| Meclocycline, Meclocycline Sulfosalicylate, Meclocycline sulfosalicylate salt | Antibacterial | 1.449 |
| amikacin, Amikacin hydrate | Antibiotic | 1.45 |
| Penicillin G, Penicillin G Potassium, Penicillin G potassium salt, Penicillin G potassium (Benzyl penicillin potassium) | Antibacterial | 1.45 |
| ATOVAQUONE | Antimalarial | 1.458 |
| MICONAZOLE, MICONAZOLE NITRATE | Antifungal | 1.464 |
| BUTOCONAZOLE, Butoconazole Nitrate | Antifungal | 1.485 |

(continued)

| Common name | Indication | SYBR Green I/PI ratio |
|---|---|---|
| NEOMYCIN, Neomycin Sulfate | Antibiotic | 1.485 |
| Crospovidone, Povidone Iodine, Povidone, Povidone-Iodine, Povidone iodine (Polyvinylpyrrolidone-iodine complex) | Antibacterial, Phamaceutic aid, Antiseptic | 1.485 |
| NIFLUMIC-ACID, Niflumic Acid | Antiinflammatory | 1.485 |
| Enrofloxacin | Antibiotic | 1.491 |
| CLOTRIMAZOLE | Antifungal | 1.523 |
| KANAMYCIN, KANAMYCIN acid, Kanamycin Sulfate, Kanamycin A sulfate | Antibiotic | 1.523 |
| SULCONAZOLE, Sulconazole Nitrate | Antifungal | 1.549 |
| MINOCYCLINE, Minocycline hydrochloride | Antibiotic | 1.549 |
| Clinafloxacin, Clinafloxacin Hydrochloride, Clinafloxacin HCl | Antibiotic | 1.607 |
| ACEMETACIN | Antiinflammatory | 1.635 |
| INDOMETHACIN | Antiinflammatory | 1.667 |
| FENTICLOR, Fenticlor (Bis(2-hydroxy-5-chlorophenyl) sulfide) | Antibacterial | 1.734 |
| Nebramycin | Antibacterial | 1.740375 |
| SULFAQUINOXALINE, Sulfaquinoxaline sodium salt, SULFAQUINOXALINE SODIUM | Antiprotozoal | 1.766 |
| Tosufloxacin | Antibiotic | 1.823 |
| Piperine | Antibacterial | 1.921 |
| AMINOSALICYLIC ACID, 5 Aminosalicylic acid, 4-Aminosalicylic acid, p-Aminosalicylic acid | Antibacterial | 1.961 |
| ISOXICAM | Antiinflammatory | 2.004 |
| FLUMEQUINE | Antibacterial | 2.311 |
| Sarafloxacin, Sarafloxacin Hydrochloride, Sarafloxacin HCl | Antibiotic | 2.33 |
| ISONIAZID, Isoniazid (Isonicotinic acid Hydrazide) | Antibacterial, Antibiotic | 2.356 |
| CEFMENOXIME, cefmenoxime hydrochloride | Antibiotic | 2.366 |
| SULFAMERAZINE | Antibiotic | 2.366 |
| Dicloxacillin, Dicloxacillin Sodium, Dicloxacillin sodium salt | Antibiotic | 2.38 |
| PROTIONAMIDE, Prothionamide (Protionamide) | Antibacterial | 2.457 |
| CLOPIDOL | Antibacterial | 2.565 |
| Amithiozone, Thiacetazone, Thiacetazone (Amithiozone) | Antibacterial | 2.611 |
| Artemisinin, Artemesinin, ARTEMISIN | Antimalarial | 2.619 |
| AMINOPYRINE, Amidopyrine, Aminopyrine (4 Dimethylamino antipyrene) | Antiinflammatory | 2.624 |
| MEPARTRICIN | Antifungal | 2.709 |
| PHENYLBUTAZONE | Antiinflammatory | 2.765 |
| Clindamycin, Clindamycin hydrochloride | Antibiotic | 2.808188 |

(continued)

| Common name | Indication | SYBR Green I/PI ratio |
|---|---|---|
| Florfenicol | Antibiotic | 2.811 |
| Doxycycline Hyclate, Doxycycline hydrochloride | Antibiotic | 2.954 |
| CARBADOX | Antibacterial | 3.026 |
| cefdinir | Antibiotic | 3.058 |
| SULFABENZAMIDE | Antibiotic, Antibacterial | 3.058 |
| Cephalosporin Cephalosporin C zinc salt | Antibiotic | 3.094 |
| AMINOSALICYLIC ACID, 5 Aminosalicylic acid, 4-Aminosalicylic acid, p-Aminosalicylic acid | Antibacterial | 3.127 |
| SULFADIMETHOXINE | Antibiotic | 3.135 |
| SPIRAMYCIN | Antibiotic | 3.142 |
| BUTOXYPHENYLACETHYDROXAMATE, Bufexamac, PHENYLACETOHYDROXAMIC ACID (Bufexamac) | Antiinflammatory | 3.15 |
| Fleroxacin | Antibiotic | 3.201 |
| CEFOTAXIME, Cefotaxime Sodium, Cefotaxime sodium salt | Antibiotic | 3.253 |
| FENOPROFEN, Fenoprofen calcium salt dihydrate, Fenoprofen calcium salt hydrate | Antiinflammatory | 3.381 |
| 21-ACETOXYPREGNENOLONE POTENCY NOT GIVEN, 21-Acetoxypregnenolone | Antiinflammatory | 3.391 |
| SULFAMETHIZOLE | Antibiotic | 3.412 |
| cloxacillin, Cloxacillin Sodium, Cloxacillin sodium salt | Antibiotic | 3.654 |
| CLOFOCTOL | Antibiotic | 3.751 |
| NIMESULIDE | Antiinflammatory | 3.77 |
| METAMPICILLIN, Metampicillin sodium salt | Antibiotic | 3.852 |

REFERENCES

All publications, patent applications, patents, and other references mentioned in the specification are indicative of the level of those skilled in the art to which the presently disclosed subject matter pertains. All publications, patent applications, patents, and other references are herein incorporated by reference to the same extent as if each individual publication, patent application, patent, and other reference was specifically and individually indicated to be incorporated by reference. It will be understood that, although a number of patent applications, patents, and other references are referred to herein, such reference does not constitute an admission that any of these documents forms part of the common general knowledge in the art. In case of a conflict between the specification and any of the incorporated references, the specification (including any amendments thereof, which may be based on an incorporated reference), shall control. Standard art-accepted meanings of terms are used herein unless indicated otherwise. Standard abbreviations for various terms are used herein.

Alban, P.S., et al., Serum-starvation-induced changes in protein synthesis and morphology of Borrelia burgdorferi. Microbiology 2000, 146 (Pt 1): 119-127.

Amant, F., et al., (2012) Chemotherapy during pregnancy. Curr. Opin. Oncol. 2012, 24: 580-586.

Ando H, Mitarai S, Kondo Y, Suetake T, Sekiguchi JI, et al. (2010) Pyrazinamide resistance in multidrug-resistant Mycobacterium tuberculosis isolates in Japan. Clin Microbiol Infect 16: 1164-1168.

Aragon LM, Garrigo M, Moreno C, Espanol M, Coll P (2007) Evaluation of the BacT/ALERT PZA kit in comparison with the BACTEC 460TB PZA for testing Mycobacterium tuberculosis susceptibility to pyrazinamide. J Antimicrob

Chemother 60: 655-657.
Arbiser, J.L. and Moschella, S.L., Clofazimine: a review of its medical uses and mechanisms of action. J. Am. Acad Dermatol. 1995, 32(2 Pt 1): p. 241-7.
Armed Forces Health Surveillance, Center, Surveillance snapshot: Lyme disease among beneficiaries of the Military Health System, 2001-2012. MSMR 2013, 20(8): p. 23.
Barbesti, S., et al., Two and three-color fluorescence flow cytometric analysis of immunoidentified viable bacteria. Cytometry 2000, 40:214-8.
Bacon, R.M., et al., Surveillance for Lyme disease--United States, 1992-2006. MMWR Surveill. Summ. 2008, 57(10): p. 1-9.
Barthold, S.W., et al., Ineffectiveness of tigecycline against persistent Borrelia burgdorferi. Antimicrobial Agents and Chemotherapy 2010, 54: 643-651.
Bergström, S. and Zückert, W.R., Structure, function and biogenesis of the Borrelia cell envelope, in Borrelia: Molecular Biology, Host Interaction and Pathogenesis. Caister Academic Press 2010, p. 139-166.
Berndtson, K., Review of evidence for immune evasion and persistent infection in Lyme disease. Int. J. Gen. Med. 2013, 6:291-306.
Bockenstedt, L.K., et al., Spirochete antigens persist near cartilage after murine Lyme borreliosis therapy. J. Clin. Invest. 2012, 122: 2652-2660.
Boerner, J., et al., In vitro antimicrobial susceptibility testing of Borrelia burgdorferi: influence of test conditions on minimal inhibitory concentration (MIC) values. Zentralbl Bakteriol 1995, 283(1): p. 49-60.
Boulos, L., et al., LIVE/DEAD BacLight : application of a new rapid staining method for direct enumeration of viable and total bacteria in drinking water. J. Microbiol. Methods 1999, 37:77-86.
Brorson, O., and Brorson, S.H. Transformation of cystic forms of Borrelia burgdorferi to normal, mobile spirochetes. Infection 1997, 25: 240-246.
Brorson, O. and Brorson, S.H. In vitro conversion of Borrelia burgdorferi to cystic forms in spinal fluid, and transformation to mobile spirochetes by incubation in BSK-H medium. Infection 1998, 26: 144-150.
Brorson, O. and Brorson, S.H. An in vitro study of the susceptibility of mobile and cystic forms of Borrelia burgdorferi to metronidazole. APMIS 1999, 107(6): p. 566-76.
Brorson, O., et al., Destruction of spirochete Borrelia burgdorferi round-body propagules (RBs) by the antibiotic tigecycline. PNAS 2009, 106(44): p. 18656-61.
Brouqui, P., et al., Eucaryotic cells protect Borrelia burgdorferi from the action of penicillin and ceftriaxone but not from the action of doxycycline and erythromycin. Antimicrob. Agents Chemother. 1996, 40(6): p. 1552-1554.
Campbell, J.W. and Cronan, J.E., Jr., Bacterial fatty acid biosynthesis: targets for antibacterial drug discovery. Annu. Rev. Microbiol. 2001;55:305-32.
Casjens, S. (2000) Borrelia genomes in the year 2000. J. Mol. Microbiol. Biotechnol. 2000, 2: 401-410.
Centers for Disease Control (**2012** [cited by 2014 February]) Lyme disease data.
Centers for Disease Control (**2014**) Post-Treatment Lyme Disease Syndrome.
Chang KC, Leung CC, Yew WW, Lau TY, Tam CM (2008) Hepatotoxicity of pyrazinamide: cohort and case-control analyses. American Journal of Respiratory and Critical Care Medicine 177: 1391-1396.
Chedore P, Bertucci L, Wolfe J, Sharma M, Jamieson F (2010) Potential for erroneous results indicating resistance when using the Bactec MGIT 960 system for testing susceptibility of Mycobacterium tuberculosis to pyrazinamide. Journal of Clinical Microbiology 48: 300-301.
Cheng SJ, Thibert L, Sanchez T, Heifets L, Zhang Y (2000) pncA mutations as a major mechanism of pyrazinamide resistance in Mycobacterium tuberculosis: spread of a monoresistant strain in Quebec, Canada. Antimicrob Agents Chemother 44: 528-532.
Cholo, M.C., et al., Clofazimine: current status and future prospects. J. Antimicrob. Chemother. 2012, 67: 290-298.
Chong, C.R., et al., A clinical drug library screen identifies astemizole as an antimalarial agent. Nat. Chem. Biol. 2006, 2(8): p. 415-6.
Clinical and Laboratory Standards Institute, Performance Standards for Antimicrobial Susceptibility Testing; Seventeenth Informational Supplement. CLSI document M100-S17 2007, 27(1): p. 154-161.
CLSI (2011) Susceptibility Testing of Mycobacteria, Nocardiae, and Other Aerobic Actinomycetes; Approved Standard-Second Edition. CLSI document M24-A2. Wayne, PA: Clinical and Laboratory Standards Institute.
CLSI (2016) Performance Standards for Antimicrobial Performance Standards for Antimicrobial Susceptibility Testing. 26th ed. CLSI supplement M100S. Wayne, PA: Clinical and Laboratory Standards Institute.
Dever, L.L., et al., In vitro antimicrobial susceptibility testing of Borrelia burgdorferi: a microdilution MIC method and time-kill studies. J. Clin. Microbiol. 1992, 30(10): p. 2692-7.
Dever, L.L., et al., In vitro activity of vancomycin against the spirochete Borrelia burgdorferi. Antimicrob Agents Chemother. 1993, 37: 1115-1121.
Dhand, A., et al., Use of antistaphylococcal beta-lactams to increase daptomycin activity in eradicating persistent

bacteremia due to methicillin-resistant Staphylococcus aureus: role of enhanced daptomycin binding. Clin. Infect. Dis. 2011, 53: 158-163.

Diterich, I., et al., Borrelia burgdorferi-induced tolerance as a model of persistence via immunosuppression. Infect. Immun. 2003, 71(7): p. 3979-87.

DTP- Mechanistic Set Information. 2015 [cited 2015].

DTP- Natural Products Set information. 2015 [cited 2015].

Embers, M.E., et al. Persistence of Borrelia burgdorferi in Rhesus Macaques following Antibiotic Treatment of Disseminated Infection. PLoS One 2012, 7(1):e29914.

Fallon, B.A., et al., A randomized, placebo-controlled trial of repeated IV antibiotic therapy for Lyme encephalopathy. Neurology 2008, 70: 992-1003.

Feng J, Shi W, Zhang S, Zhang Y (2015) Identification of new compounds with high activity against stationary phase Borrelia burgdorferi from the NCI compound collection. Emerg Microbes Infect 4: e31.

Feng J, Shi W, Zhang S, Sullivan D, Auwaerter PG, et al. (2016) A Drug Combination Screen Identifies Drugs Active against Amoxicillin-Induced Round Bodies of In Vitro Borrelia burgdorferi Persisters from an FDA Drug Library. Front Microbiol 7: 743.

Feng, J., Wang, T., Shi, W., et al., Identification of novel activity against Borrelia Burgdorferi persisters using an FDA approved drug library. Emerging Microbes and Infections 2014, e49.

Feng, J., Wang, T., Zhang, S., et al., An Optimized SYBR Green I/PI Assay for Rapid Viability Assessment and Antibiotic Susceptibility Testing for Borrelia burgdorferi. PLoS One 2014, 9: e111809.

Fredricks BA, DeCoster DJ, Kim Y, Sparks N, Callister SM, et al. (2006) Rapid pyrazinamide susceptibility testing of Mycobacterium tuberculosis by flow cytometry. J Microbiol Methods 67: 266-272.

Gabrielson, J., et al., Evaluation of redox indicators and the use of digital scanners and spectrophotometer for quantification of microbial growth in microplates. J Microbiol Methods 2002, 50:63-73.

Gregori, G., et al., Resolution of viable and membrane-compromised bacteria in freshwater and marine waters based on analytical flow cytometry and nucleic acid double staining. Appl. Environ. Microbiol. 2001, 67:4662-70.

Hall Snyder, A., et al., Evaluation of the novel combination of daptomycin plus ceftriaxone against vancomycin-resistant enterococci in an in vitro pharmacokinetic/pharmacodynamic simulated endocardial vegetation model. The Journal of Antimicrobial Chemotherapy 2014, 69: 2148-2154.

Hayashi, M., et al., The mode of action of nanaomycins D and A on a gram-negative marine bacterium Vibrio alginolyticus. J. of Antibiotics. 1982;35(8):1078-85.

Hewlett D, Jr., Horn DL, Alfalla C (1995) Drug-resistant tuberculosis: inconsistent results of pyrazinamide susceptibility testing. JAMA 273: 916-917.

Hodzic, E., et al., Resurgence of Persisting Non-Cultivable Borrelia burgdorferi following Antibiotic Treatment in MicePLoS One. 2014, 9: e86907.

Hodzic, E., et al., Persistence of Borrelia burgdorferi following antibiotic treatment in mice. Antimicrobial Agents and Chemotherapy 2008, 52:1728-36.

Hollstein, U. Actinomycin. Chemistry and mechanism of action. Chemical Reviews. 1974;74(6):625-52.

Hughes AJ, Hutchinson P, Gooding T, Freezer NJ, Holdsworth SR, et al. (2005) Diagnosis of Mycobacterium tuberculosis infection using ESAT-6 and intracellular cytokine cytometry. Clin Exp Immunol 142: 132-139.

Hunfeld, K.P. and Brade, V., Antimicrobial susceptibility of Borrelia burgdorferi sensu lato: what we know, what we don't know, and what we need to know. Wien Klin Wochenschr. 2006, 118(21-22): p. 659-68.

Jacobs WR, Jr., Barletta RG, Udani R, Chan J, Kalkut G, et al. (1993) Rapid assessment of drug susceptibilities of Mycobacterium tuberculosis by means of luciferase reporter phages. Science 260: 819-822.

Jensen, P.B., et al., Different modes of anthracycline interaction with topoisomerase II. Separate structures critical for DNA-cleavage, and for overcoming topoisomerase II-related drug resistance. Biochem. Pharmacol. 1993;45(10):2025-35.

Jonmalung J, Prammananan T, Leechawengwongs M, Chaiprasert A (2010) Surveillance of pyrazinamide susceptibility among multidrug-resistant Mycobacterium tuberculosis isolates from Siriraj Hospital, Thailand. BMC Microbiol 10: 223.

Keren, I., et al., Persister cells and tolerance to antimicrobials. FEMS Microbiol. Lett. 2004, 230:13-18.

Kersten, A., et al., Effects of penicillin, ceftriaxone, and doxycycline on morphology of Borrelia burgdorferi. Antimicrob. Agents Chemother. 1995, 39(5): p. 1127-33.

Klempner, M.S. et al., Treatment trials for post-Lyme disease symptoms revisited. Am. J. Med. 2013, 126: 665-669.

Kraiczy, P., et al., In vitro activities of fluoroquinolones against the spirochete Borrelia burgdorferi. Antimicrob. Agents Chemother. 2001, 45: 2486-2494.

Krupp, L.B., et al., Study and treatment of post Lyme disease (STOP-LD): a randomized double masked clinical trial. Neurology 2003, 60: 1923-1930.

Lantos, P.M., et al. A systematic review of Borrelia burgdorferi morphologic variants does not support a role in

chronic Lyme disease. Clin. Infect. Dis. 2014, 58: 663-671.
Li, W., et al., Yeast model uncovers dual roles of mitochondria in action of artemisinin. PLoS Genet 2005, 1: e36.
Li, Y. and Zhang, Y. PhoU is a persistence switch involved in persister formation and tolerance to multiple antibiotics and stresses in Escherichia coli. Antimicrob. Agents Chemother. 2007, 51: 2092-2099.
Louw GE, Warren RM, Donald PR, Murray MB, Bosman M, et al. (2006) Frequency and implications of pyrazinamide resistance in managing previously treated tuberculosis patients. Int J Tuberc Lung Dis 10: 802-807.
Lu, S., et al., Bis-naphtho-gamma-pyrones from fungi and their bioactivities. Molecules. 2014;19(6):7169-88.
Marques, A., et al., Xenodiagnosis to detect Borrelia burgdorferi Infection: A first-in-Human Study. Clinical Infectious Diseases 2014, 58 (7): 937-945.
Meek, J.I., et al., Underreporting of Lyme disease by Connecticut physicians, 1992. J. Public Health. Pract. 1996, 2(4): p. 61-65.
Migliori GB, Besozzi G, Girardi E, Kliiman K, Lange C, et al. (2007) Clinical and operational value of the extensively drug-resistant tuberculosis definition. European Respiratory Journal 30: 623-626.
Miklossy, J., et al., Persisting atypical and cystic forms of Borrelia burgdorferi and local inflammation in Lyme neuroborreliosis. J. of Neuroinflammation 2008, 5:40.
Mitchison DA (1985) The action of antituberculosis drugs in short course chemotherapy. Tubercle 66: 219-225.
Mizuno, N.S., et al., Binding of daunomycin to DNA and the inhibition of RNA and DNA synthesis. Cancer Res. 1975;35(6):1542-6.
Mole R, Trollip A, Abrahams C, Bosman M, Albert H (2007) Improved contamination control for a rapid phage-based rifampicin resistance test for Mycobacterium tuberculosis. J Med Microbiol 56: 1334-1339.
Moody, M.R., et al., Effect of two cancer chemotherapeutic agents on the antibacterial activity of three antimicrobial agents. Antimicrob. Agents Chemother. 1978;14(5):737-42.
Morris, C.M., et al., Effect of polymyxin B nonapeptide on daptomycin permeability and cell surface properties in Pseudomonas aeruginosa, Escherichia coli, and Pasteurella multocida. J Antibiot. (Tokyo) 1995, 48: 67-72.
Mshana, R. N., et al., Use of 3-(4,5-dimethylthiazol-2-yl)-2,5-diphenyl tetrazolium bromide for rapid detection of rifampin-resistant Mycobacterium tuberculosis. J. Clin. Microbiol. 1998, 36:1214-9.
Murgia, R. and Cinco, M. Induction of cystic forms by different stress conditions in Borrelia burgdorferi. APMIS : acta pathologica, microbiologica, et immunologica Scandinavica 2004, 112: 57-62.
Niu H, Peng Cui, Rebecca Yee, Wanliang Shi, Shuo Zhang, Jie Feng, David Sullivan, Wenhong Zhang, Bingdong Zhu, Ying Zhang (2015) A Clinical Drug Library Screen Identifies Tosufloxacin as Being Highly Active against Staphylococcus aureus Persisters. Antibiotics 4: 329-336.
Niu H, Cui P, Shi W, Zhang S, Feng J, et al. (2015) Identification of Anti-Persister Activity against Uropathogenic Escherichia coli from a Clinical Drug Library. Antibiotics (Basel) 4: 179-187.
Open Repository Collection of Synthetics and Pure Natural Products. 2014 [cited 2015 Mar 15].
Orloski, K.A., et al., Surveillance for Lyme disease--United States, 1992-1998. MMWR CDC Surveill Summ. 2000, 49(3): p. 1-11.
Palaniappan, K., and Holley, R.A. Use of natural antimicrobials to increase antibiotic susceptibility of drug resistant bacteria. International Journal of Food Microbiology 2010, 140: 164-168.
Pogliano, J., et al., Daptomycin-mediated reorganization of membrane architecture causes mislocalization of essential cell division proteins. J. Bacteriol. 2012, 194(17): p. 4494-504.
Pommier, Y., et al., DNA topoisomerases and their poisoning by anticancer and antibacterial drugs. Chemistry & Biology. 2010;17(5):421-33.
Radolf, J.D., et al., Of ticks, mice and men: understanding the dual-host lifestyle of Lyme disease spirochaetes. Nat. Rev. Microbiol. 2012, 10: 87-99.
Rao NA, Irfan M, Soomro MM, Mehfooz Z (2010) Drug resistance pattern in multidrug resistance pulmonary tuberculosis patients. J Coll Physicians Surg Pak 20: 262-265.
Ricker, L.J., et al. Persistent subfoveal fluid and increased preoperative foveal thickness impair visual outcome after macula-off retinal detachment repair. Retina 2011,31: 1505-1512.
Sapi, E., et al., Characterization of biofilm formation by Borrelia burgdorferi in vitro. Plos One 2012, 7(10): p. e48277.
Sapi, E., et al., Evaluation of in-vitro antibiotic susceptibility of different morphological forms of Borrelia burgdorferi. Infect. Drug Resist. 2011, 4: p. 97-113.
Scorpio A, Lindholm-Levy P, Heifets L, Gilman R, Siddiqi S, et al. (1997) Characterization of pncA mutations in pyrazinamide-resistant Mycobacterium tuberculosis. Antimicrob Agents Chemother 41: 540-543.
Scorpio A, Zhang Y (1996) Mutations in pncA, a gene encoding pyrazinamidase/nicotinamidase, cause resistance to the antituberculous drug pyrazinamide in tubercle bacillus. Nat Med 2: 662-667.
Senol G, Coskun M, Gunduz AT, Bicmen C, Gayaf M, et al. (2008) [Investigation of pyrazinamide resistance in multidrug-resistant tuberculosis cases in Hospital of Pulmonary Diseases, Izmir, Turkey]. Mikrobiyoloji Bulteni 42: 591-597.

Shenai S, Rodrigues C, Sadani M, Sukhadia N, Mehta A (2009) Comparison of phenotypic and genotypic methods for pyrazinamide susceptibility testing. Indian J Tuberc 56: 82-90.

Shopov, A., et al., Improvements in image analysis and fluorescence microscopy to discriminate and enumerate bacteria and viruses in aquatic samples. Aquatic Microbial Ecology 2000, 22:103-110.

Simpson G, Coulter C, Weston J, Knight T, Carter R, et al. (2011) Resistance patterns of multidrug-resistant tuberculosis in Western Province, Papua New Guinea. Int J Tuberc Lung Dis 15: 551-552.

Sobell, H.M. Actinomycin and DNA transcription. Proceedings of the National Academy of Sciences. 1985;82(16):5328-31.

Somoskovi A, Dormandy J, Parsons LM, Kaswa M, Goh KS, et al. (2007) Sequencing of the pncA gene in members of the Mycobacterium tuberculosis complex has important diagnostic applications: Identification of a species-specific pncA mutation in "Mycobacterium canettii" and the reliable and rapid predictor of pyrazinamide resistance. J Clin Microbiol 45: 595-599.

Steere, A.C. and Glickstein, L., Elucidation of Lyme arthritis. Nat. Rev. Immunol. 2004, 4: 143-152.

Straubinger, R.K., et al., Persistence of Borrelia burgdorferi in experimentally infected dogs after antibiotic treatment. Journal of Clinical Microbiology 1997, 35: 111-116.

Stricker, R.B. and L. Johnson, Lyme disease: the next decade. Infect. Drug Resist. 2011, 4: p. 1-9.

Sun, Z. and Zhang, Y., Spent culture supernatant of Mycobacterium tuberculosis H37Ra improves viability of aged cultures of this strain and allows small inocula to initiate growth. Journal of Bacteriology 1999, 181:7626-7628.

Szybalski, W. and Iyer, V.N. Crosslinking of DNA by Enzymatically or Chemically Activated Mitomycins and Porfiromycins, Bifunctionally "Alkylating" Antibiotics. Federation Proceedings. 1964;23:946-57.

Tan, C., et al., Daunomycin, an antitumor antibiotic, in the treatment of neoplastic disease. Clinical evaluation with special reference to childhood leukemia. Cancer 1967;20(3):333-53.

Taylor, J. P., et al., Comparison of microbial numbers and enzymatic activities in surface soils and subsoils using various techniques. Soil Biology & Biochemistry 2002, 34:387-401.

Tomasz, M., Mitomycin C: small, fast and deadly (but very selective). Chem. Biol. 1995;2(9):575-9.

Vanden Bossche, H., et al., Biochemical approaches to selective antifungal activity. Focus on azole antifungals. Mycoses 1989, 32 Suppl 1: 35-52.

Van Rensburg, C.E., et al., Antimicrobial activities of clofazimine and B669 are mediated by lysophospholipids. Antimicrob. Agents Chemother. 1992, 36: 2729-2735.

Velayati AA, Masjedi MR, Farnia P, Tabarsi P, Ghanavi J, et al. (2009) Emergence of new forms of totally drug-resistant tuberculosis bacilli: super extensively drug-resistant tuberculosis or totally drug-resistant strains in iran. Chest 136: 420-425.

Wade MM, Zhang Y (2006) Effects of weak acids, UV and proton motive force inhibitors on pyrazinamide activity against Mycobacterium tuberculosis in vitro. J Antimicrob Chemother 58: 936-941.

Wang, H., Cheng, H., et al., An improved 3-(4,5-dimethylthiazol-2-yl)-2,5-diphenyl tetrazolium bromide (MTT) reduction assay for evaluating the viability of Escherichia coli cells. J Microbiol Methods 2010, 82:330-3.

Wang, J., Huang, L., et al. Artemisinin directly targets malarial mitochondria through its specific mitochondrial activation. Plos One 2010, 5: e9582.

Welch, H., The antibiotic saga. The University of Michigan 1960, p. 96.

Wells, T.N., et al., New medicines to improve control and contribute to the eradication of malaria. Nat. Rev. Drug Discov. 2009, 8: 879-891.

WHO (2008) Anti-tuberculosis Drug Resistance in the World, Report No. 4.

Wikler, M.A., and Ferraro, M.J., Correction of a reference to clinical laboratory standards institute interpretive criteria. Clin. Infect. Dis. 2008, 46: 1798; author reply 1798-1799.

Williamson, N.R., et al., Anticancer and immunosuppressive properties of bacterial prodiginines. Future Microbiol. 2007;2(6):605-18.

Wilson SM, al-Suwaidi Z, McNerney R, Porter J, Drobniewski F (1997) Evaluation of a new rapid bacteriophage-based method for the drug susceptibility testing of Mycobacterium tuberculosis. Nat Med 3: 465-468.

Wormser, G.P., et al., The clinical assessment, treatment, and prevention of Lyme disease, human granulocytic anaplasmosis, and babesiosis: clinical practice guidelines by the Infectious Diseases Society of America. Clin. Infect. Dis. 2006, 43: 1089-1134.

Xu, J., et al., In vitro and in vivo activity of clofazimine against Mycobacterium tuberculosis persisters. Int. J. Tuberc. Lung Dis. 2012, 16: 1119-1125.

Zhang, Y., Persisters, persistent infections and the Yin-Yang Model. Emerging Microbes & Infections 2014, 3, e3.

Zhu, L., et al., Syntheses and biological activities of daunorubicin analogs with uncommon sugars. Bioorganic & Medicinal Chemistry. 2005;13(23):6381-7.

Zhang S, Chen J, Shi W, Liu W, Zhang WH, et al. (2013) Mutations in panD encoding aspartate decarboxylase are associated with pyrazinamide resistance in Mycobacterium tuberculosis. Emerging Microbes & Infections 2: e34;

doi:10.1038/emi.2013.1038.

Zhang Y, Chang K, Leung C, Yew W, Gicquel G, et al. (2012) "ZS-MDR-TB" versus "ZR-MDR-TB": Improving Treatment of MDR-TB by Identifying Pyrazinamide Susceptibility. Emerging Microbes and Infections (Nature Publishing Group) 1: e5; doi:10.1038/emi.2012.1018.

Zhang Y, Mitchison D (2003) The curious characteristics of pyrazinamide: a review. Int J Tuberc Lung Dis 7: 6-21.

Zhang Y, Scorpio A, Nikaido H, Sun Z (1999) Role of acid pH and deficient efflux of pyrazinoic acid in unique susceptibility of Mycobacterium tuberculosis to pyrazinamide. J Bacteriol 181: 2044-2049.

Zhang Y, Shi W, Zhang W, Mitchison D (2014) Mechanisms of Pyrazinamide Action and Resistance. Microbiol Spectrum, ASM Press 2(4): July 2014, 2012:2012.2014.2003. doi:2010.1128/microbiolspec.MGM2012-0023-2013.

Although the foregoing subject matter has been described in some detail by way of illustration and example for purposes of clarity of understanding, it will be understood by those skilled in the art that certain changes and modifications can be practiced to include other microbes within the scope of the appended claims.

## Claims

**1.** A method for assessing the susceptibility of cells to at least one drug or test agent, the method comprising:

(a) obtaining or establishing a cell culture comprising said cells;
(b) incubating the cell culture with a staining mixture comprising: (i) a first agent which emits fluorescence of a first color that is indicative of live cells in the culture, and (ii) a second agent which emits fluorescence of a second color that contrasts from the first color and is indicative of dead cells in the culture;
(c) calculating a ratio of the intensity of emitted fluorescence of the first color to the intensity of emitted fluorescence of the second color; and
(d) assessing the viability or susceptibility of the cells in the culture to said at least one drug or test agent, wherein the ratio calculated in (c) is indicative of the percentage of live cells and dead cells in the culture.

**2.** A method according to claim 1 wherein the method is a method for assessing the susceptibility of cells to at least one drug or test agent to thereby identify at least one agent that is capable of inhibiting the growth or survival of the cells wherein the cells may be growing cells or non-growing cells.

**3.** A method according to claim 1 or 2 wherein the method is a method for assessing the susceptibility of cells to at least one drug wherein step (b) comprises:

(b)(i) incubating the cells with at least one dose of said at least one drug for a period of exposure without the need for growth of said cells; and
(b)(ii) incubating the drug-treated cells with said staining mixture.

**4.** A method according to claim 1 or 2 wherein the method is a method for assessing susceptibility of cells to at least one test agent wherein step (b) comprises:

(b)(i) incubating the cells with at least one dose of said at least one test agent for a period of exposure without the need for growth of said cells; and
(b)(ii) incubating the test agent-treated cells with said staining mixture.

**5.** A method according to any one of the preceding claims wherein:

(i) the method is a method according to claim 3 or 4 wherein (b)(i) and (b)(ii) are performed simultaneously;
(ii) the method is a method according to claim 3 or 4 wherein (b)(i) and (b)(ii) are performed separately or sequentially;
(iii) the method is a method according to claim 3 or claim 5 part (i) or (ii) wherein the method comprises determining a minimum inhibitory concentration breakpoint or cut-off for at least one drug or agent in comparison to known sensitive and known resistant control cells;
(iv) the method comprises incubating the cells in the cell culture at an appropriate cell density, optionally. in the range of $10^{1-9}$ cells/ml;
(v)the method is a method according to claim 3 or 4 wherein the susceptibility (or resistance) of the cells is assessed by comparing the viability of the cells in the culture in the presence of the at least one drug or test

agent with the viability of cells in a control culture of known sensitive and resistant organisms or cells which lacks, or has not been exposed to, the at least one drug or test agent;
(vi) the method is a method according to claim 3 or 4 wherein the cells are assessed as susceptible to the at least one drug or test agent according to whether the ratio in step (b) remains the same or decreases after the period of exposure to the at least one dose of the at least one drug or test agent; and wherein the cells are assessed as resistant to the at least one drug or test agent according to whether the ratio in step (b) remains the same or decreases after the period of exposure to the at least one dose of the at least one drug or test agent in comparison to known sensitive and resistant organisms or cells as controls;
(vii) the culture comprises a stationary phase culture optionally comprising non-replicating dormant or persister cells; and/or
(viii) the testing can be done in a real-time continuous manner to obtain real time data.

**6.** A method according to any one of the preceding claims wherein the period of incubation of the cells with the at least one drug or test agent is is less than about 17 hours, 16 hours, 15 hours, 14 hours, 13 hours, 12 hours, 11 hours, 10 hours, 9 hours, 8 hours, 7 hours, 6 hours, 5 hours, 4 hours, 3 hours, 2 hours or 1 hour but is optionally at least about 5, 10, 15, 20, 25, 30, 35, 40, 45 or 50 minutes.

**7.** A method according to claim 6 wherein the period of incubation of the cells with the at least one drug or test agent is is less than about 17 hours, 16 hours, 15 hours, 14 hours, 13 hours, 12 hours, 11 hours, 10 hours, 9 hours, 8 hours, 7 hours, 6 hours, 5 hours, 4 hours, 3 hours, 2 hours or 1 hour but at least about 5, 10, 15, 20, 25, 30, 35, 40, 45 or 50 minutes.

**8.** A method according to any one of the preceding claims wherein:

(i) the method comprises incubating the cells with at least one dose of at least one drug or test agent at a concentration range from 10-10,000 fold of conventional growth based minimum inhibitory concentration (MIC); and/or
(ii) the method is used for real-time and continuous monitoring and identification of drug candidates at different time points in a continuous manner.

**9.** A method according to any one of the preceding claims wherein the method is used for real-time and continuous monitoring, and wherein optionally the period of incubation is a real-time continuous period from about 5 minutes to 16 hours.

**10.** A method according to any one of the preceding claims wherein the method is performed in a high-throughput format.

**11.** A method or kit for rapid drug screensof at least one candidate agent that is capable of inhibiting growth or survival of cells of eukaryotic or prokaryotic origin wherein the kit comprises:

(a) optionally a population of growing or non-growing cells of eukaryotic or prokaryotic origin or a culture thereof;
(b) exposure of the cells with test compounds
(c) a staining mixture comprising: (i) a first agent which emits fluorescence of a first color that is indicative of live cells(e.g. SYBR Green I), and (ii) a second agent (e.g. propidium iodide) which emits fluorescence of a second color that contrasts from the first color and is indicative of dead cells, wherein when the staining mixture is incubated with the cells or culture thereof a calculated ratio of the intensity of emitted fluorescence of the first color to the intensity of emitted fluorescence the second color is indicative of the percentage of live cells in the population or culture thereof; and
(d) optionally instructions, such as instructions for using the cells in (a) and the staining mixture in (b) to screen for at least one candidate agent that is capable of inhibiting growth or survival of the cells.
(e) the method is performed in the absence of a washing step after drug incubation and prior to the reading of the fluorescence results;
(f) the cells are not recultured or regrown after drug incubation; and/or
(g) the method does not comprise a period of growing the cells with the at least one drug or test agent.
(h) the method is used for real-time and continuous monitoring and identification of drug candidates at different time points in continuous manner.

**12.** A kit according to claim 11 wherein the kit comprises at least one drug or test agent to screen or test for its ability to inhibit cell growth or survival.

**13.** A method or kit according to any one of the preceding claims wherein:

(i) the first fluorescent agent is SYBR Green I and the second fluorescent agent is propidium iodide;
(ii) the cells are selected from the group consisting of: prokaryotic cells; eukaryotic cells; bacteria; Gram-positive bacteria such as *Staphylococcus aureus*; Gram-negative bacteria such as *Escherichia coli, Klebsiella pneumoniae,* and *Acinetobacter baumannii*; mycobacteria such as *Mycobacterium tuberculosis*; bacteria from the *Borrelia* genus (e.g., replicating and/or non-replicating persister forms of *B. burgdorferi);* bacteria from a selected genus and wherein the selected genus is optionally selected from the group consisting of *Borrelia, Staphylococcus, Escherichia, Klebsiella, Acinetobacter,* and *Mycobacterium*; microbial cells of eukaryotic or origin; eukaryotic cells such as fungal or yeast cells, plant cells, parasite cells, cancer cells; animal cells including but not limited to vertebrate cells and invertebrate cells, mammalian cells, mouse cells, rabbit cells, rat cells, guinea pig cells, fish cells, amphibians cells, reptile cells, bird cells, monkey cells, hamster cells, non-human primate (e.g. monkeys, apes, gibbons, chimpanzees, orangutans, macaques and the like) cells, and human cells; cancerous or infected animal cells; bovine cells (e.g., cattle, oxen, and the like); ovine cells (e.g., sheep and the like), caprine cells (e.g. goats and the like); porcine cells (e.g., pigs, hogs, and the like); equine cells (e.g., horses, donkeys, zebras, and the like); feline, canine, rodent or lagomorph cells; cells from animal disease models (e.g., rats or mice used in experiments, and the like); tumor cells; cells from a human or non-human animal suffering from a cell proliferative disorder e.g. a neoplastic disorder; cells of a pathogen, e.g., a multicellular pathogen or microorganism such as a pathogenic bacterium, pathogenic fungus, or a pathogenic protist (e.g., a Plasmodium cell); cells derived from a cell line e.g. CHO cells, and HeLa cells; and/or
(iii) the cells being assayed are growing or non-growing cells.

**14.** A method or kit according to any one of the preceding claims wherein the cells are selected from the group consisting of: replicating cells; non-replicating persister or dormant cells; cancerous cells (e.g. breast cancer, prostate cancer, colon cancer, pancreatic cancer, lung cancer, leukemia etc), diseased, infected (e.g. with an antibiotic or antimicrobial refractory infection), precancerous cells; cell-lines, stem cells, pluripotent, multipotent or oligopotent cells, cancer stem cells, cancer cells from cancer tissues of patients, fastidious cells; and transformed cells.

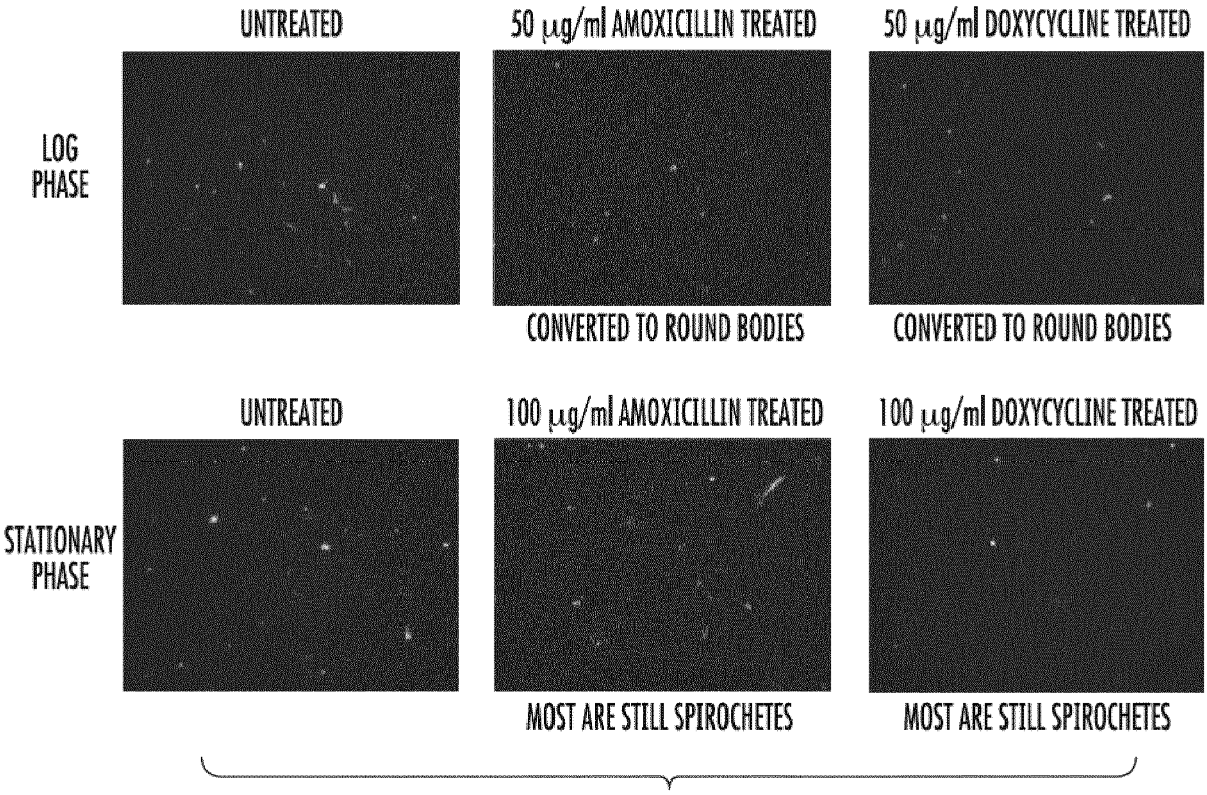
UNTREATED
50 μg/ml AMOXICILLIN TREATED
50 μg/ml DOXYCYCLINE TREATED
LOG PHASE
CONVERTED TO ROUND BODIES
CONVERTED TO ROUND BODIES
UNTREATED
100 μg/ml AMOXICILLIN TREATED
100 μg/ml DOXYCYCLINE TREATED
STATIONARY PHASE
MOST ARE STILL SPIROCHETES
MOST ARE STILL SPIROCHETES

*Fig. 1*

| PERCENT OF LIVE CELLS | MTT($OD_{565}$) | XTT($OD_{450}$) | FDA($\lambda$em) | LIVE/DEAD ($RATIO_{G/R}$) | SYBR GREEN /PI($RATIO_{G/R}$) |
|---|---|---|---|---|---|
| 0% | 0.131 | 2.894 | 26069 | 10.81 | 3.49 |
| 20% | 0.132 | 2.894 | 25938 | 10.13 | 4.15 |
| 50% | 0.147 | 3.024 | 26134 | 11.35 | 5.48 |
| 80% | 0.161 | 3.051 | 29136 | 10.75 | 7.17 |
| 100% | 0.157 | 2.978 | 30158 | 11.63 | 8.03 |
| BSK-H | 0.152 | 2.892 | 26984 | 11.41 | 2.03 |

PI: PROPIDIUM IODIDE: FDA: FLOURESCEIN DIACETATE
RATIO G/R READ IN HTS 7000 PERKIN ELMER BIO ASSAY READER

*Fig. 2*

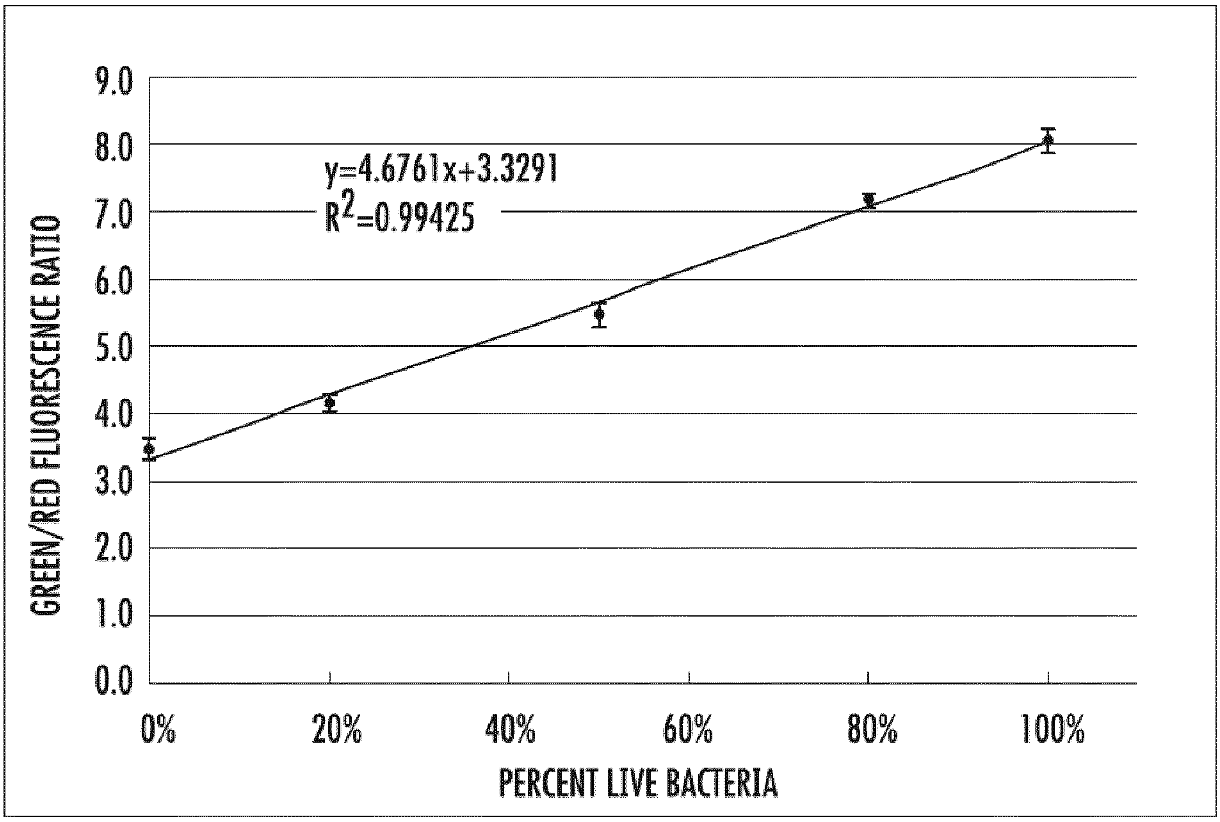
9.0
8.0
7.0
6.0
5.0
4.0
3.0
2.0
1.0
0.0
y=4.6761x+3.3291
R2=0.99425
GREEN/RED FLUORESCENCE RATIO
0%
20%
40%
60%
80%
100%
PERCENT LIVE BACTERIA

*Fig. 3*

| | MTT | XTT | FDA | LIVE/DEAD (RATIOG/R) | LIVE/DEAD RATIO G/R (WASH) 1 | SYBR GREEN/P1 | MICROSCOPE COUNTING |
|---|---|---|---|---|---|---|---|
| DETECTION LIMIT (100 μL) | $>10^8$ | ND | $10^7$ | ND | $5 \times 10^5$ | $10^5$ | $2.5 \times 10^4$ |
| ERROR | ND | ND | 30%~40% | ND | 20%~30% | <10% | 10%~20% |
| TIME (FOR 96-WELL PLATE) | 3~5 h | 2.5 h | 1 h | 20 min | 50 min | 20 min | ~48 h |

*Fig. 4*

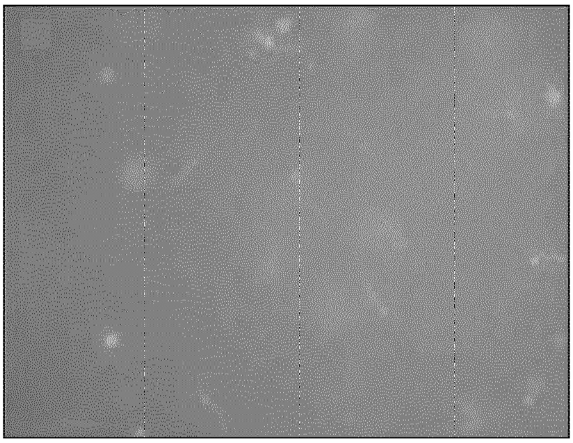

Fig. 5A

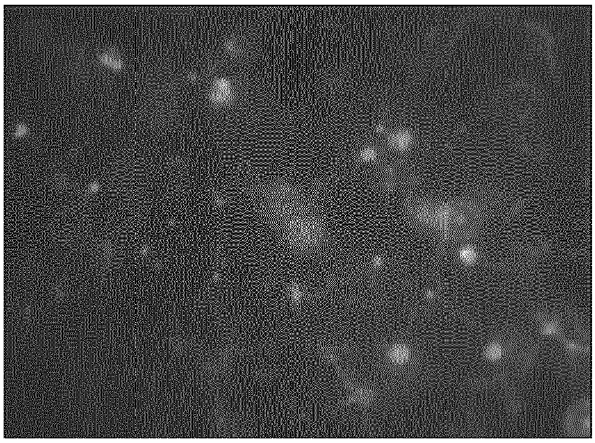

Fig. 5B

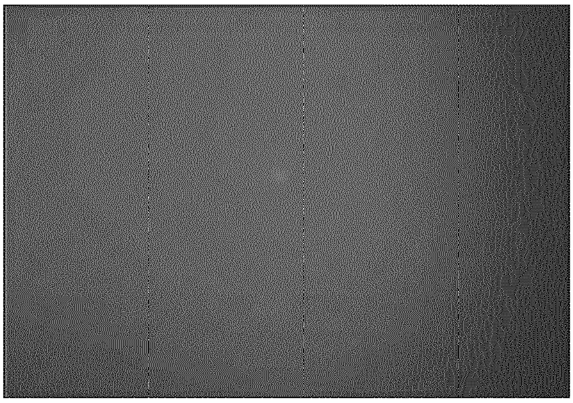

Fig. 5C

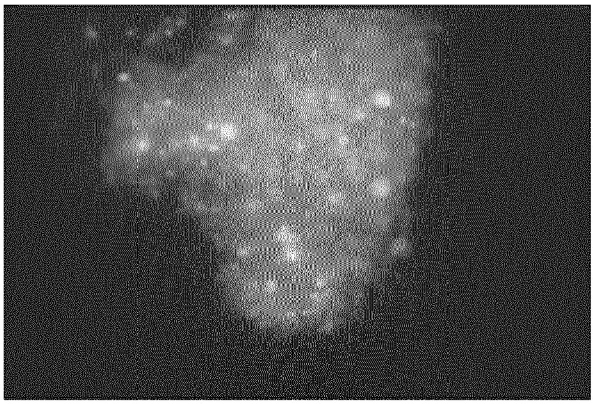

Fig. 5D

| ANTIBIOTICS | LIVE/DEAD ($RATIO_{G/R}$) | SYBR GREEN/P1 ($RATIO_{G/R}$) | MICROSCOPE COUNTING (LIVE %)[1] |
|---|---|---|---|
| DOXYCYCLINE | 9.83 | 4.35 | 35% |
| AMOXICILLIN | 10.18 | 5.76 | 66% |
| METRONIDAZOLE | 10.34 | 7.12 | 81% |
| CONTROL | 10.32 | 8.85 | 95% |

*Fig. 6*

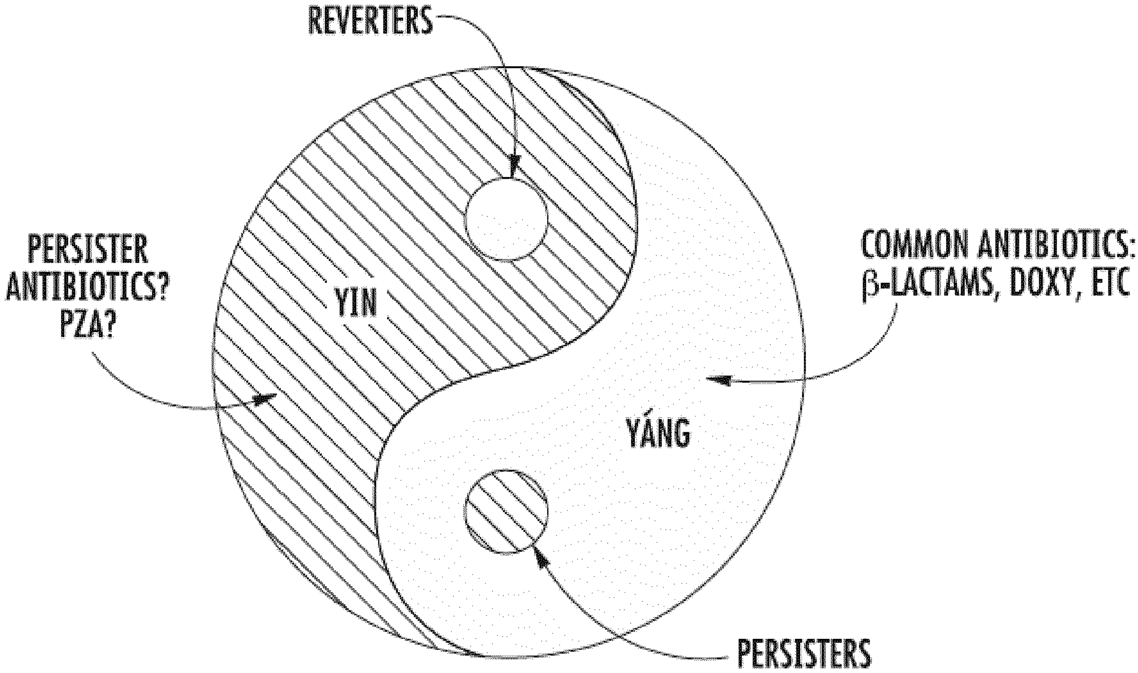
REVERTERS
PERSISTER ANTIBIOTICS? PZA?
YIN
COMMON ANTIBIOTICS: β-LACTAMS, DOXY, ETC
YÁNG
PERSISTERS

*Fig. 7*

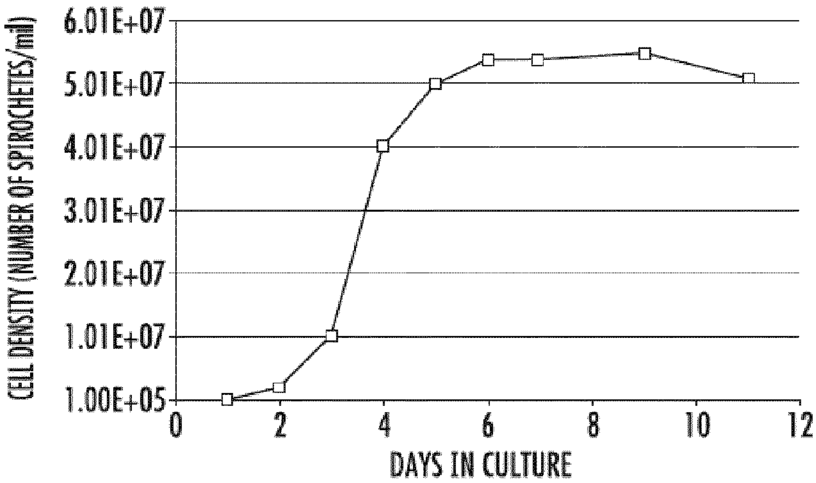


*Fig. 8A*

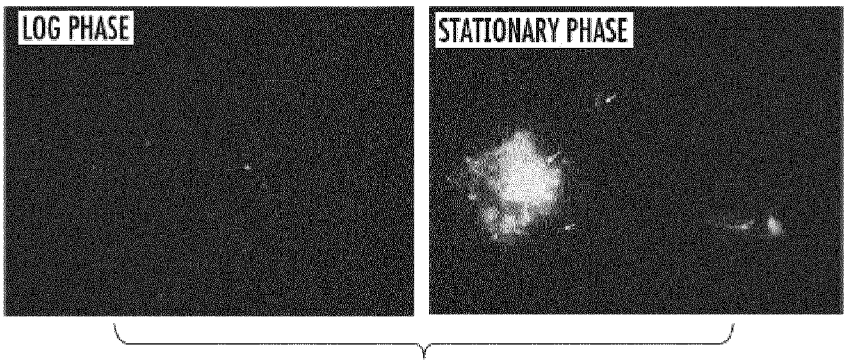


*Fig. 8B*

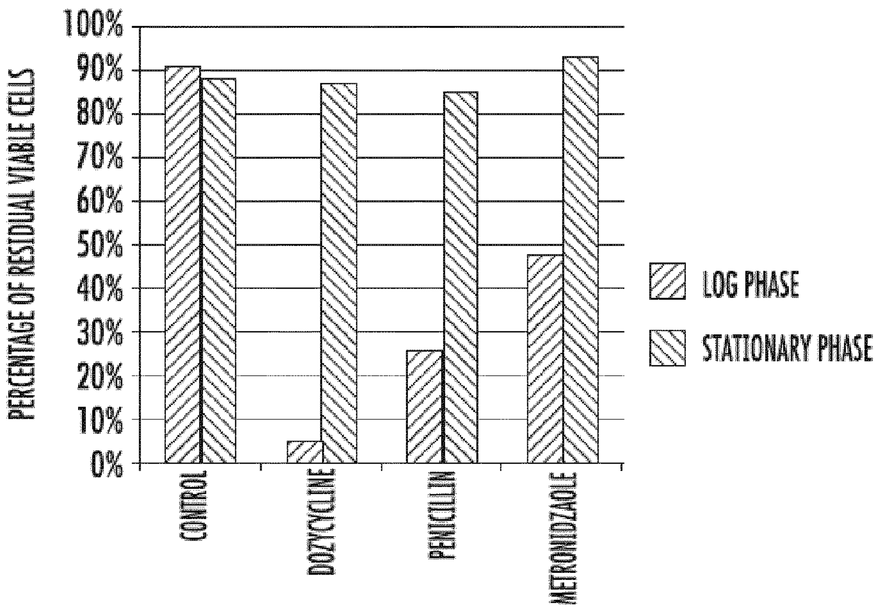


*Fig. 8C*

| DRUGS (50 μM) | RESIDUAL VIABLE CELLS[a] | RESIDUAL VIABLE CELLS[b] | RATIO OF GREEN/RED FLUORESCE PRIMARY SCRENNING | RESCREENING | RESCREENING |
|---|---|---|---|---|---|
| CONTROL[c] | 93% | | 8.67 | | |
| DOXYCYCLINE[c] | 75% | | 7.62 | | |
| AMOXICILLIN[c] | 76% | | 7.98 | | |
| DAPTOMYCIN | 35% | 28% | 6.10 | 6.20 | 6.09 |
| CEFOPERAZONE | 37% | 34% | 6.54 | 6.32 | 6.23 |
| CARBOMYCIN | 41% | 37% | 6.37 | 6.81 | 6.32 |
| CEFOTIAM | 42% | 43% | 6.41 | 7.55 | 6.21 |
| CEFMETAZOLE | - | 43% | 6.80 | 7.38 | 6.00 |
| CEFEPIME | - | 44% | 6.67 | 7.16 | 6.45 |
| CLOFAZIMINE | 45% | 32% | 6.56 | 6.23 | 6.02 |
| AMODIAQUIN | - | 45% | 6.79 | - | - |
| STREPTOMYCIN | - | 45% | 6.72 | 6.93 | 6.76 |
| TICARCILLIN | - | 46% | 6.82 | 6.72 | 6.93 |
| CEFONICID SODIUM | - | 46% | 6.86 | 7.54 | 6.07 |
| PIPERACILLIN-TAZOBACTAM | 47% | 47% | 7.18 | 6.47 | 6.98 |
| CEFDINIR | - | 48% | 6.88 | 7.51 | 6.29 |
| CEFORANIDE | - | 48% | 6.89 | 7.49 | 6.33 |
| CEFMENOXIME | - | 48% | 6.82 | 7.59 | 6.32 |
| CEFTIZOXIME | - | 49% | 6.94 | 6.83 | 7.03 |
| BISMUTH | - | 48% | 6.94 | 6.82 | 6.92 |
| CEPHALOTHIN | 49% | 40% | 6.74 | 6.49 | 6.55 |
| CEFUROXIME | 49% | 43% | 6.59 | 6.84 | 6.67 |
| AMPHOTERICIN B | - | 50% | 7.14 | 6.88 | 6.87 |
| CEFAMONDOLE | - | 50% | 6.71 | 7.73 | 6.52 |
| QUININE HYDROBROMIDE | - | 50% | 7.00 | - | - |
| CEFTIBUTEN | 51% | 49% | 6.81 | 6.78 | 7.27 |
| CYCLACILLIN | 51% | 53% | 6.81 | 6.88 | 6.64 |
| CINCHONINE | - | 52% | 7.07 | - | - |
| CALCIUM PROPIONATE | - | 52% | 7.27 | 7.44 | 6.50 |
| CEFOXITIN | - | 53% | 7.33 | 7.60 | 6.33 |

[a]RESIDUAL VIABLE B. BURGDORFERI WAS ASSAYED BY EPI- FLUORESCENT MICROSCOPE COUNTING.
[b]RESIDUAL VIABLE B. BURGDORFERI WAS CALCULATED ACCORDING TO THE REGRESSION EQUATION AND RATIO OF GREEN/RED FLUORESCENCE OBTAINED BY SYBR GREEN I/PI ASSAY.
[c]DATA FROM TRIPLICATE EXPERIMENTS.

*Fig. 9*

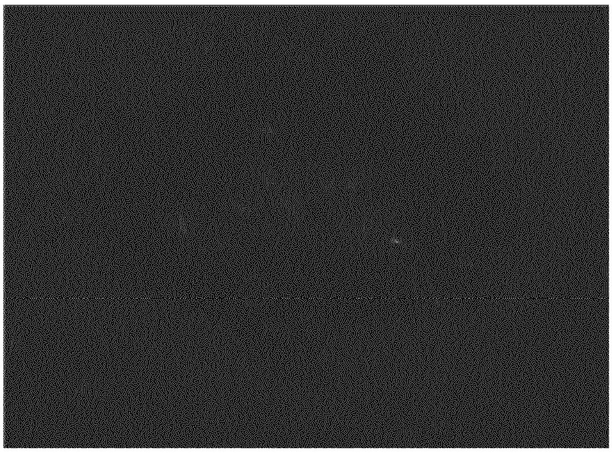

Fig. 10A

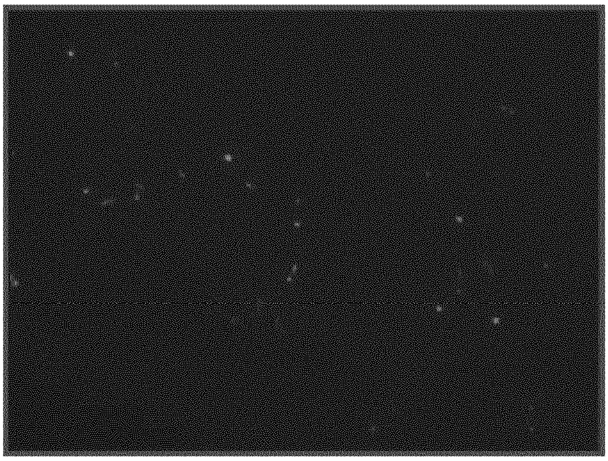

Fig. 10B

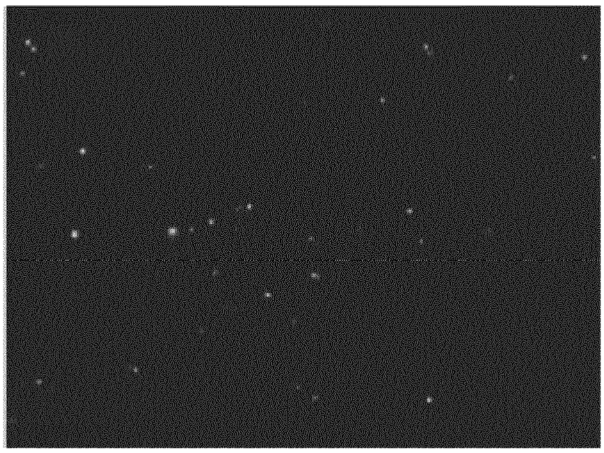

Fig. 10C

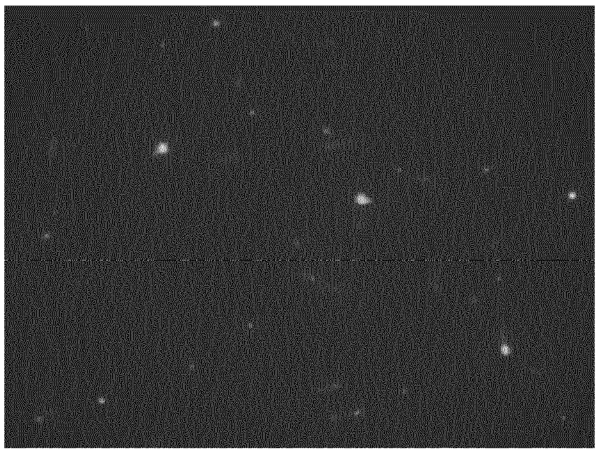

Fig. 10D

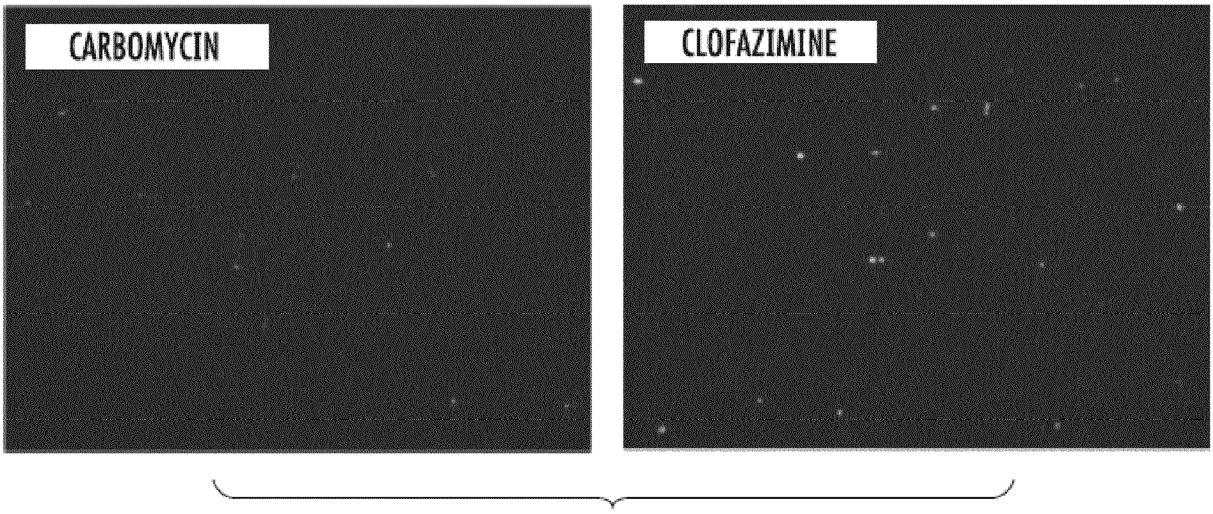


*Fig. 11*

| ANTIBIOTICS | MIC (μg/ml) | ANTIBIOTICS | MIC (μg/ml) |
|---|---|---|---|
| DOXYCYCLINE | ≤ 0.25 | CEFOPERAZONE | ≤ 0.25 |
| AMOXICILLIN | ≤ 0.25 | CEFOTIAM | ≤ 0.25 |
| METRONIDAZOLE | 25 | TAZOBACTAM | 12.5 |
| CLOFAZIMINE | 6.25 | SULFAMETHOXAZOLE | ≤0.25 |
| CARBOMYCIN | ≤ 0.25 | KETOCONAZOLE | 50 |

*Fig. 12*

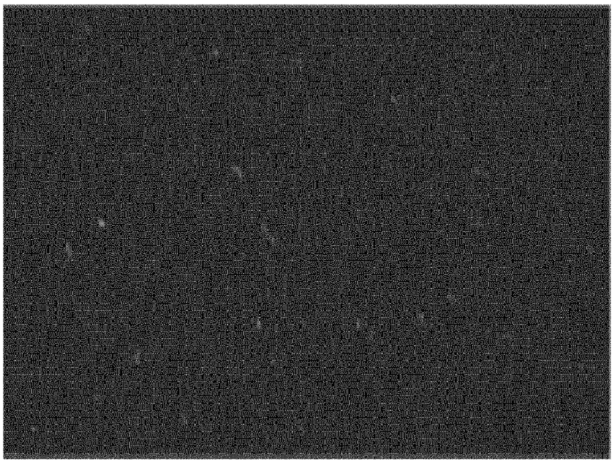

*Fig. 13A*

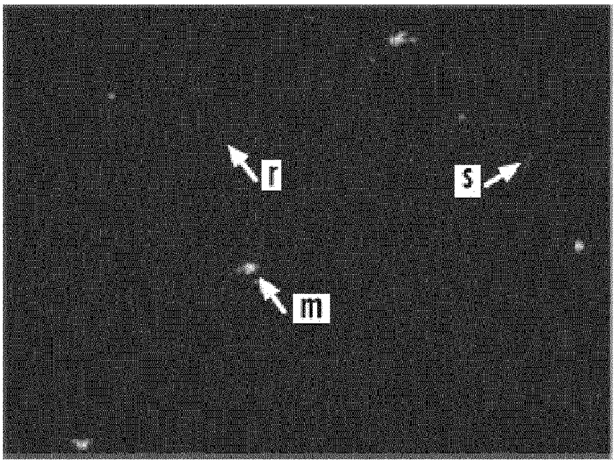


*Fig. 13B*

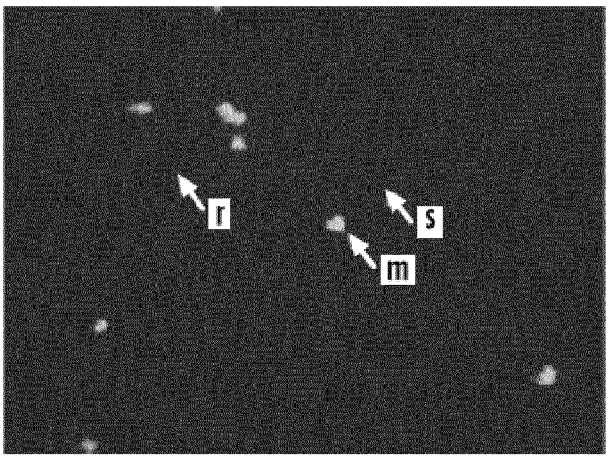


*Fig. 13C*

| G/R | - | PMB | CFZ | SMX | MTZ | CARBOMYCIN | ERYTHROMYCIN | DAPTOMYCIN | MCZ |
|---|---|---|---|---|---|---|---|---|---|
| - | 7.8 (80%) | 7.3 | 6.8 | 7.1 | 7.0 | 7.3 | 7.6 | 5.6 | 7.1 |
| DOX | 7.3 (60%) | 6.3 (30%) | 5.9 (25%) | 5.8 (26%) | 7.1 | 6.4 (33%) | 6.6 | 5.3 (15%) | 6.5 (28%) |
| AMOX | 7.9 | 6.6 | 6.1 | 7.0 | 7.3 | 6.6 (33%) | 7.9 | 5.4 (15%) | 6.8 (30%) |
| CEF-P | 7.1 | 6.6 | 5.9 (25%) | 6.6 (30%) | 7.3 | 6.8 | - | 5.5 | - |
| CEF-T | 7.4 | 7.1 | 6.3 | 7.1 | 7.3 | 7.2 | - | 5.4 | - |

*Fig. 14*

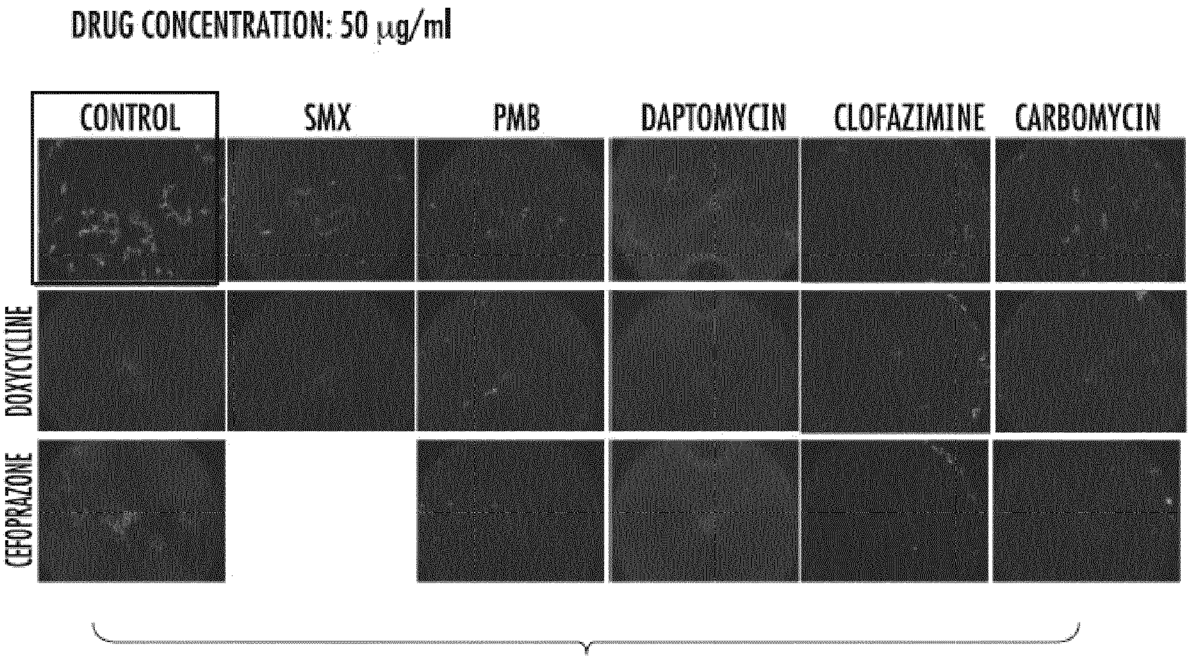
DRUG CONCENTRATION: 50 μg/ml
CONTROL
SMX
PMB
DAPTOMYCIN
CLOFAZIMINE
CARBOMYCIN
DOXYCYCLINE
CEFOPRAZONE

*Fig. 15*

| | | CONTROL | SMX | PMB | DAPTOMYCIN | CLOFAZIMINE | CARBOMYCIN |
|---|---|---|---|---|---|---|---|
| CONTROL | RELATIVE AREA | 159349 | 73270 | 54268 | 37053 | 45721 | 53901 |
| | GREEN FLUORESCENCE % | 95% | 41% | 43% | 40% | 44% | 50% |
| DOXYCYCLINE | RELATIVE AREA | 79048 | 27263 | 43111 | 12721 | 51965 | 47264 |
| | GREEN FLUORESCENCE % | 48% | 25% | 22% | 2% | 24% | 37% |
| CEFOPERAZONE | RELATIVE AREA | 60119 | | 35699 | 17515 | 52809 | 28280 |
| | GREEN FLUORESCENCE % | 59% | | 41% | 5% | 29% | 39% |

*Fig. 16*

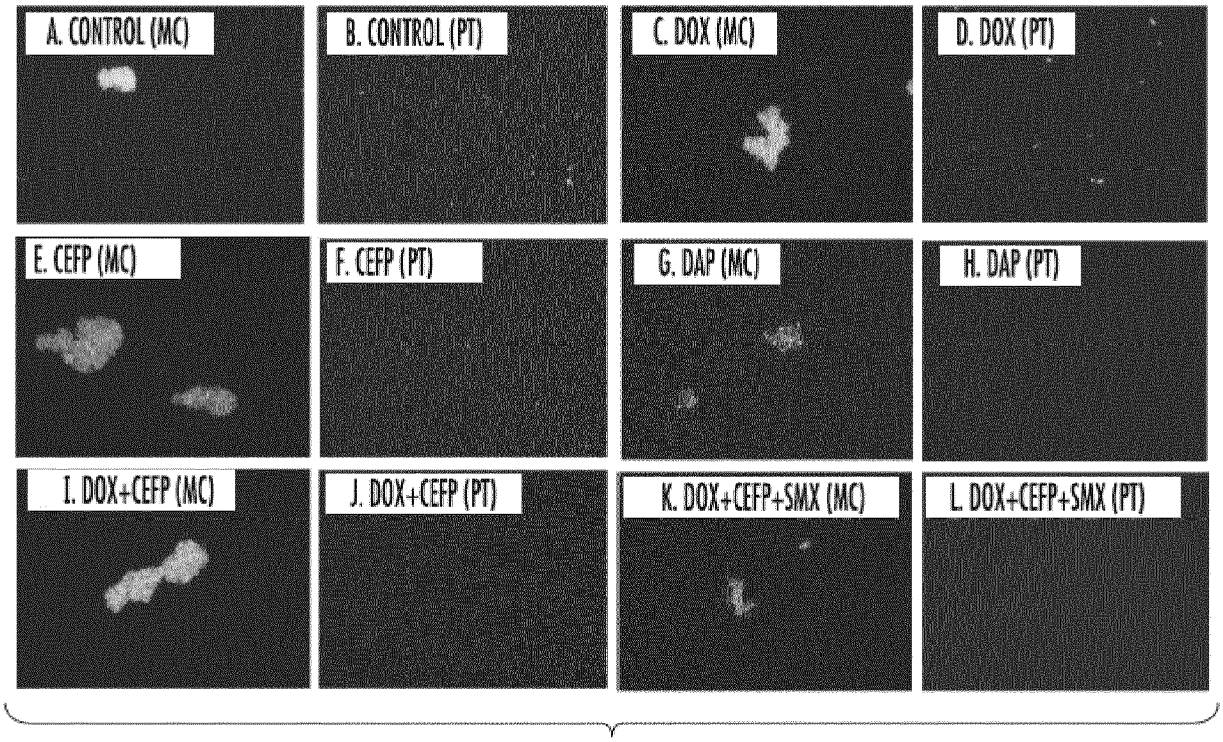


*Fig. 17A*

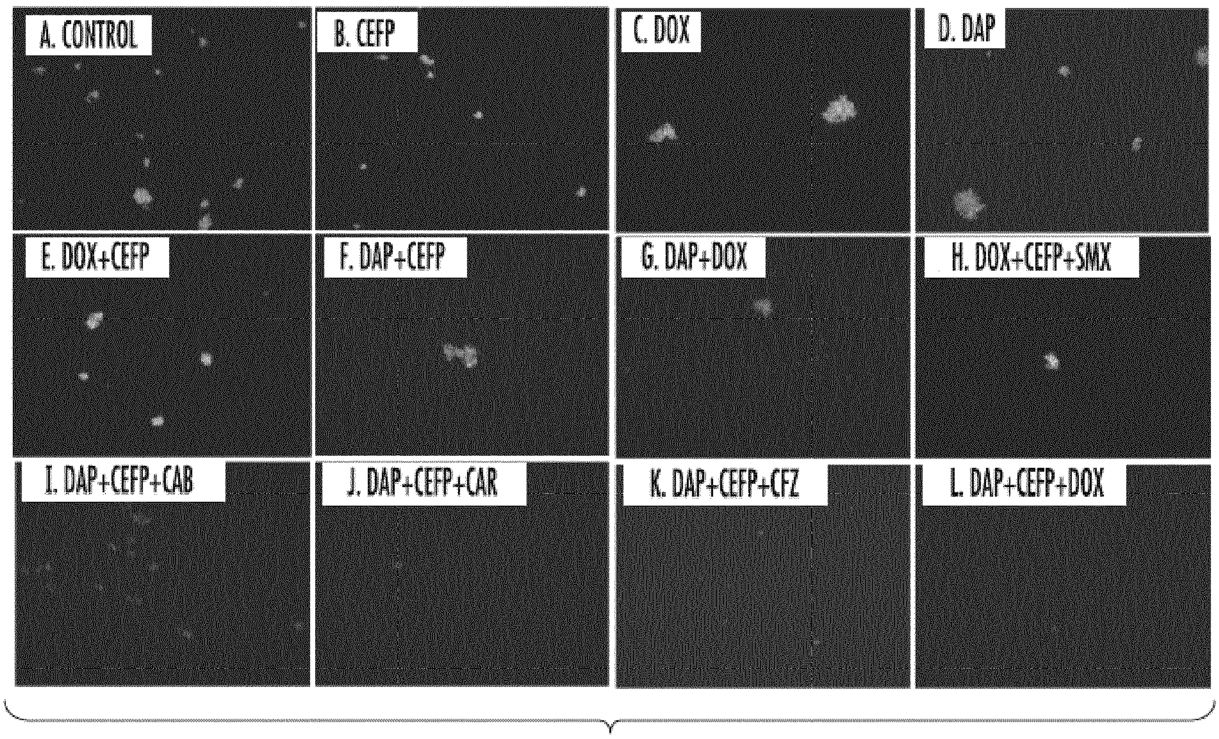


*Fig. 17B*

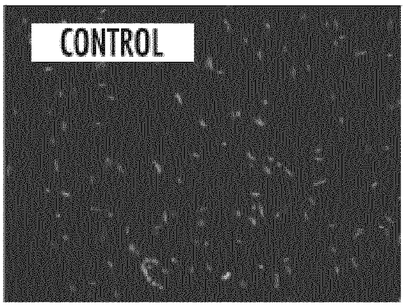


*Fig. 18A*

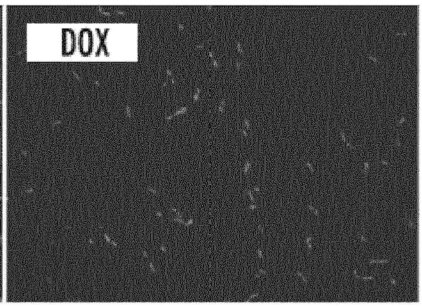


*Fig. 18B*

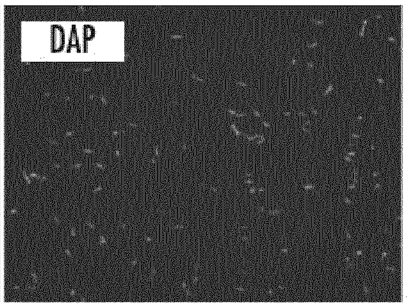


*Fig. 18C*

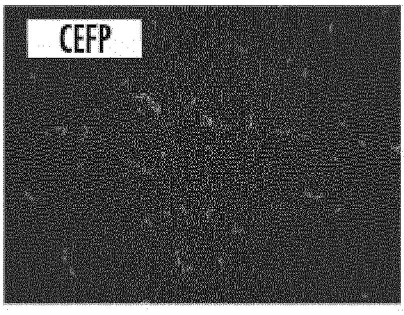


*Fig. 18D*

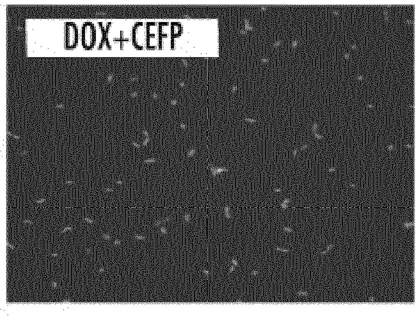


*Fig. 18E*

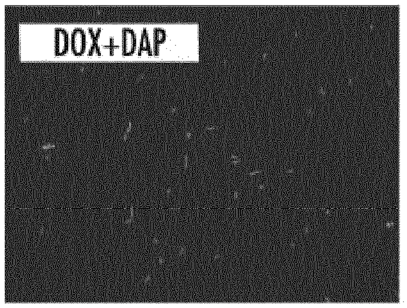


*Fig. 18F*

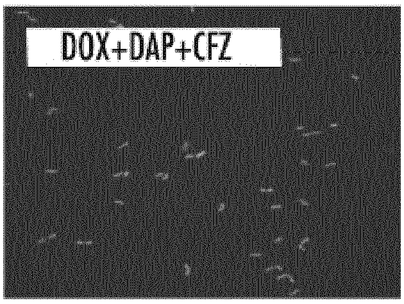


*Fig. 18G*

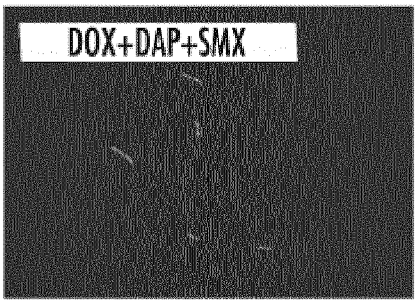


*Fig. 18H*

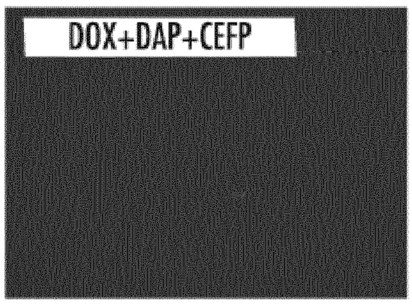


*Fig. 18I*

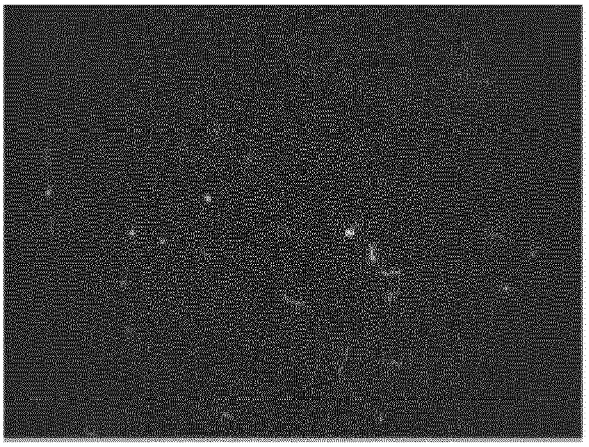

Fig. 19A

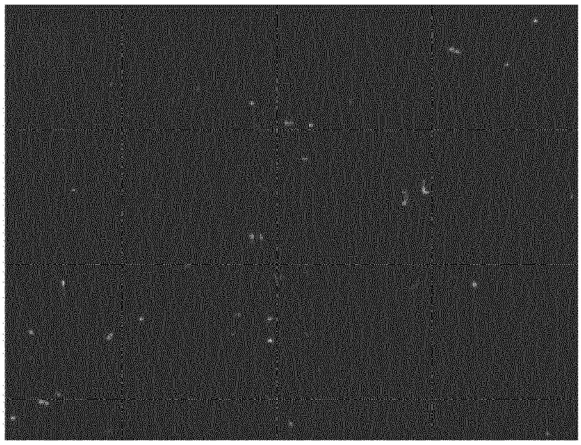

Fig. 19B

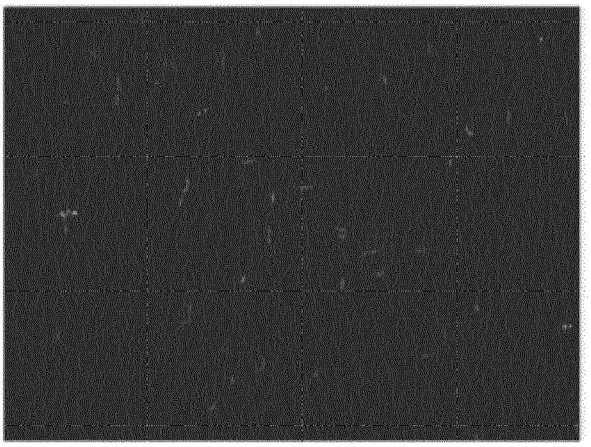

Fig. 19C

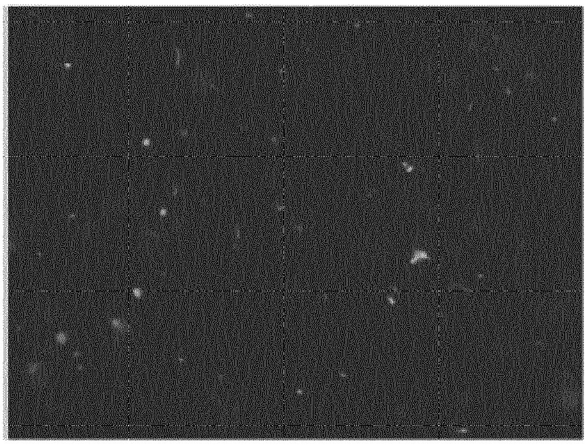

Fig. 19D

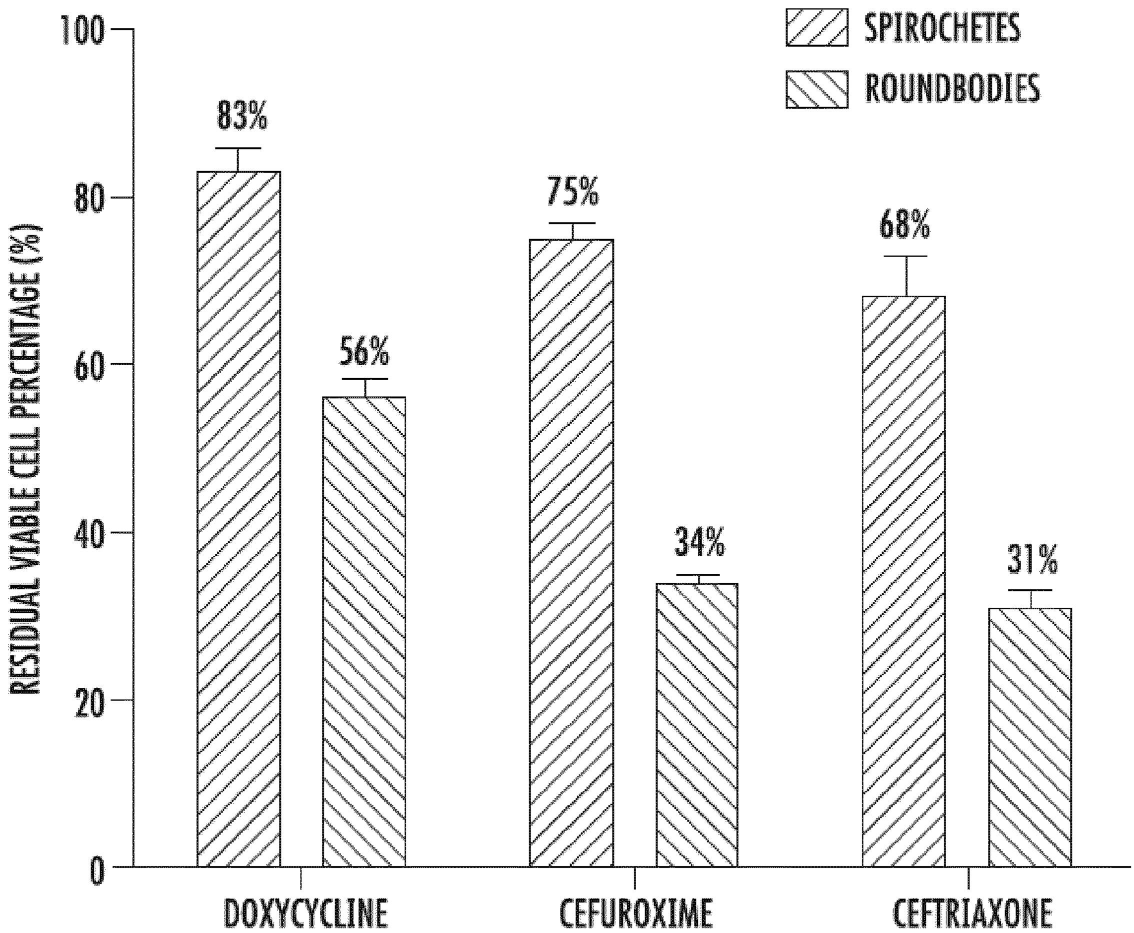
SPIROCHETES
ROUNDBODIES
100
80
60
40
20
0
RESIDUAL VIABLE CELL PERCENTAGE (%)
83%
56%
75%
34%
68%
31%
DOXYCYCLINE
CEFUROXIME
CEFTRIAXONE

*Fig. 20*

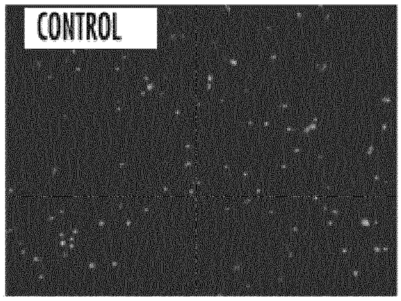


Fig. 21A

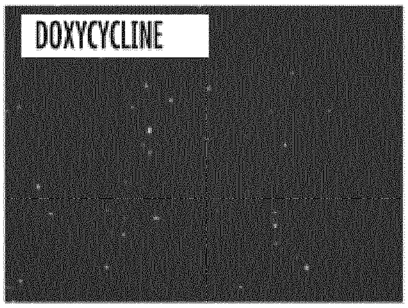


Fig. 21B

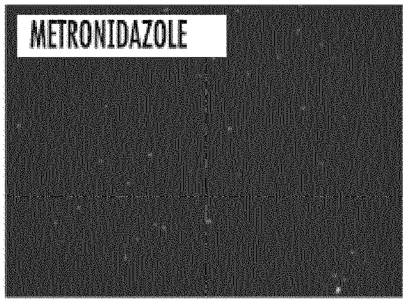


Fig. 21C

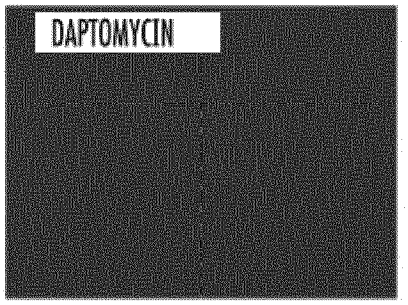


Fig. 21D

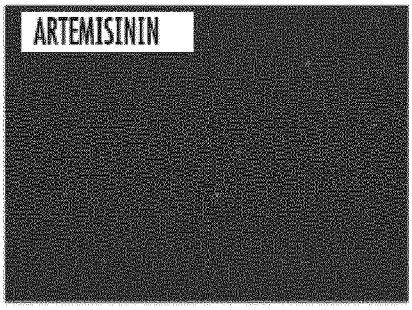


Fig. 21E

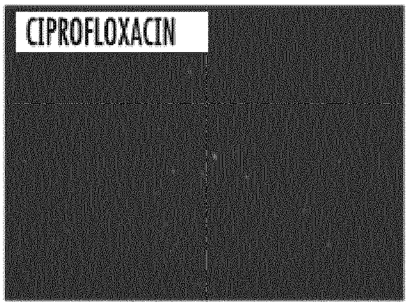


Fig. 21F

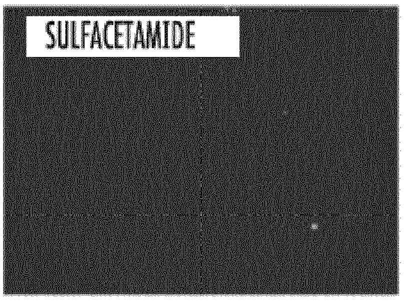


Fig. 21G

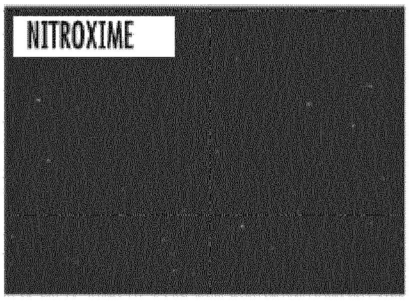


Fig. 21H

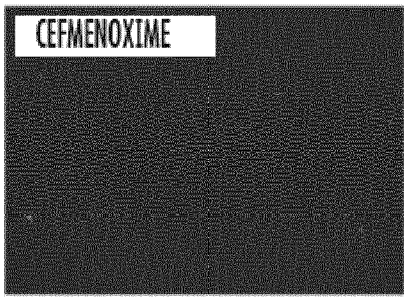


Fig. 21I

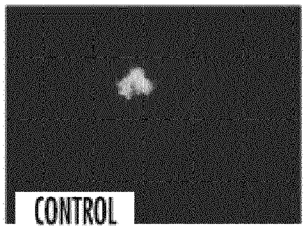


*Fig. 22A*

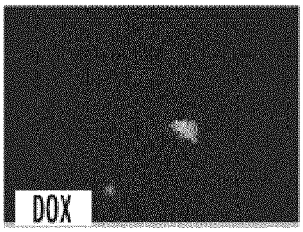


*Fig. 22B*

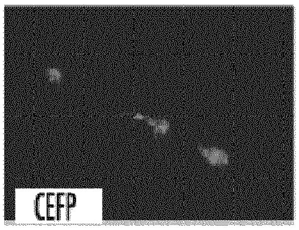


*Fig. 22C*

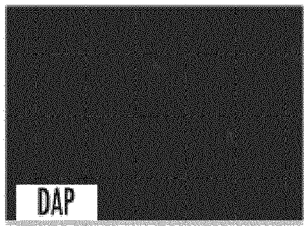


*Fig. 22D*

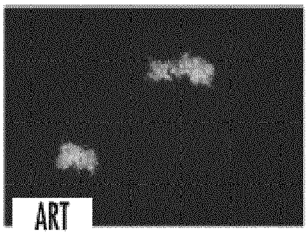


*Fig. 22E*

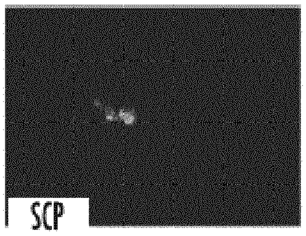


*Fig. 22F*

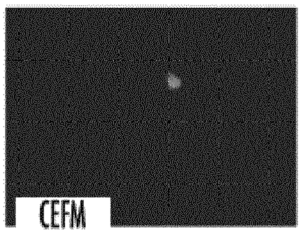


*Fig. 22G*

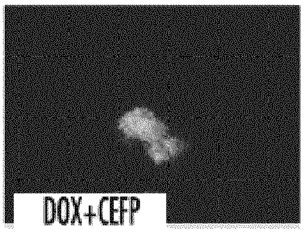


*Fig. 22H*

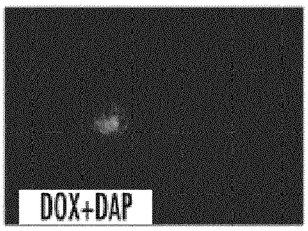


*Fig. 22I*

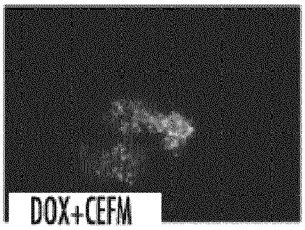


*Fig. 22J*

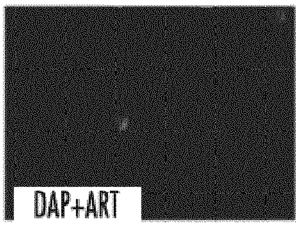


*Fig. 22K*

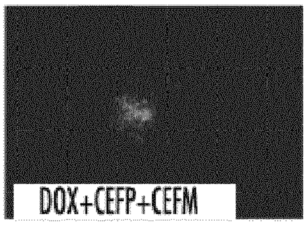


*Fig. 22L*

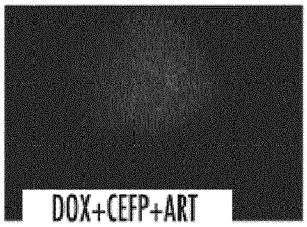


*Fig. 22M*

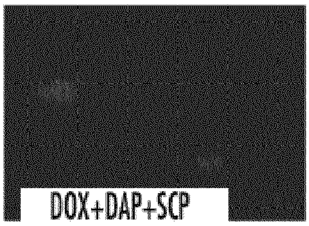


*Fig. 22N*

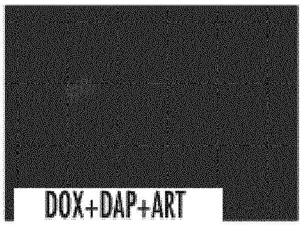


*Fig. 22O*

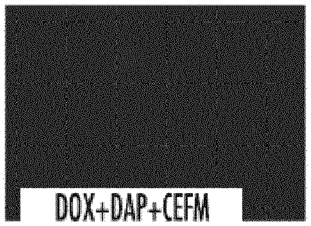


*Fig. 22P*

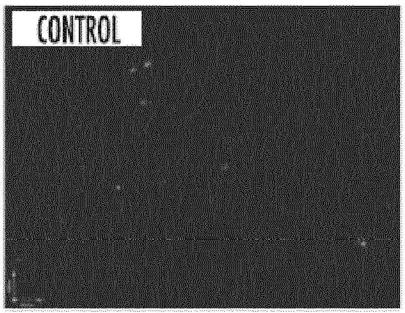


*Fig. 23A*

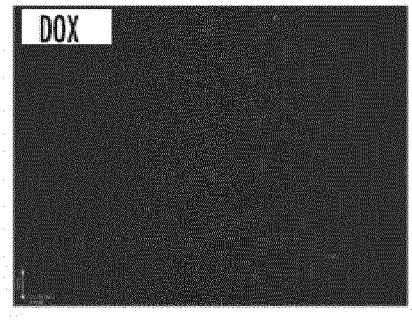


*Fig. 23B*

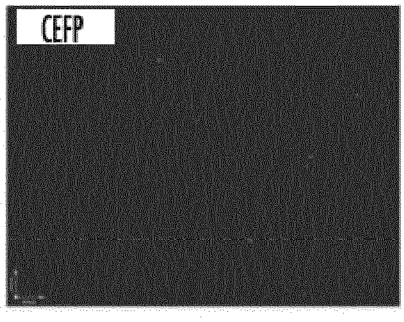


*Fig. 23C*

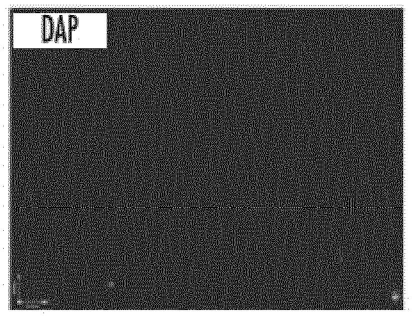


*Fig. 23D*

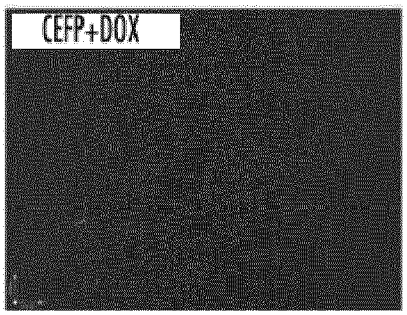


*Fig. 23E*

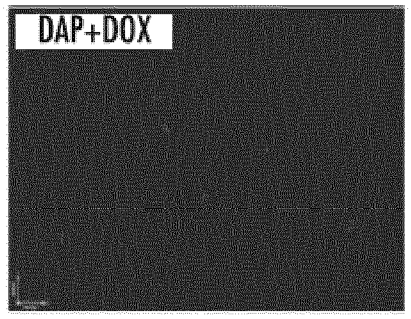


*Fig. 23F*

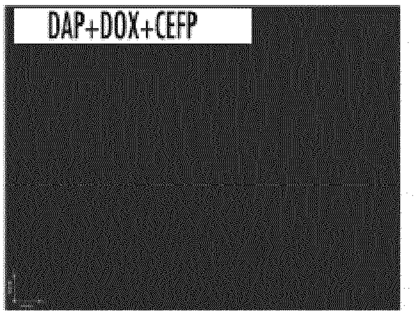


*Fig. 23G*

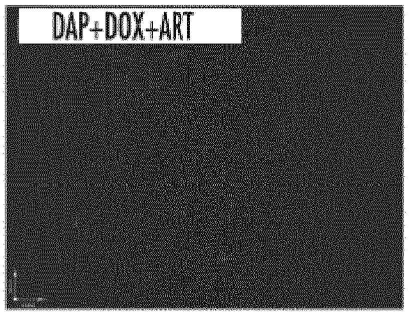


*Fig. 23H*

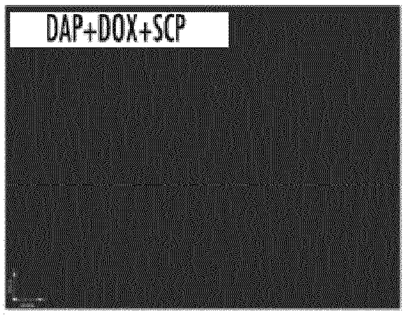


*Fig. 23I*

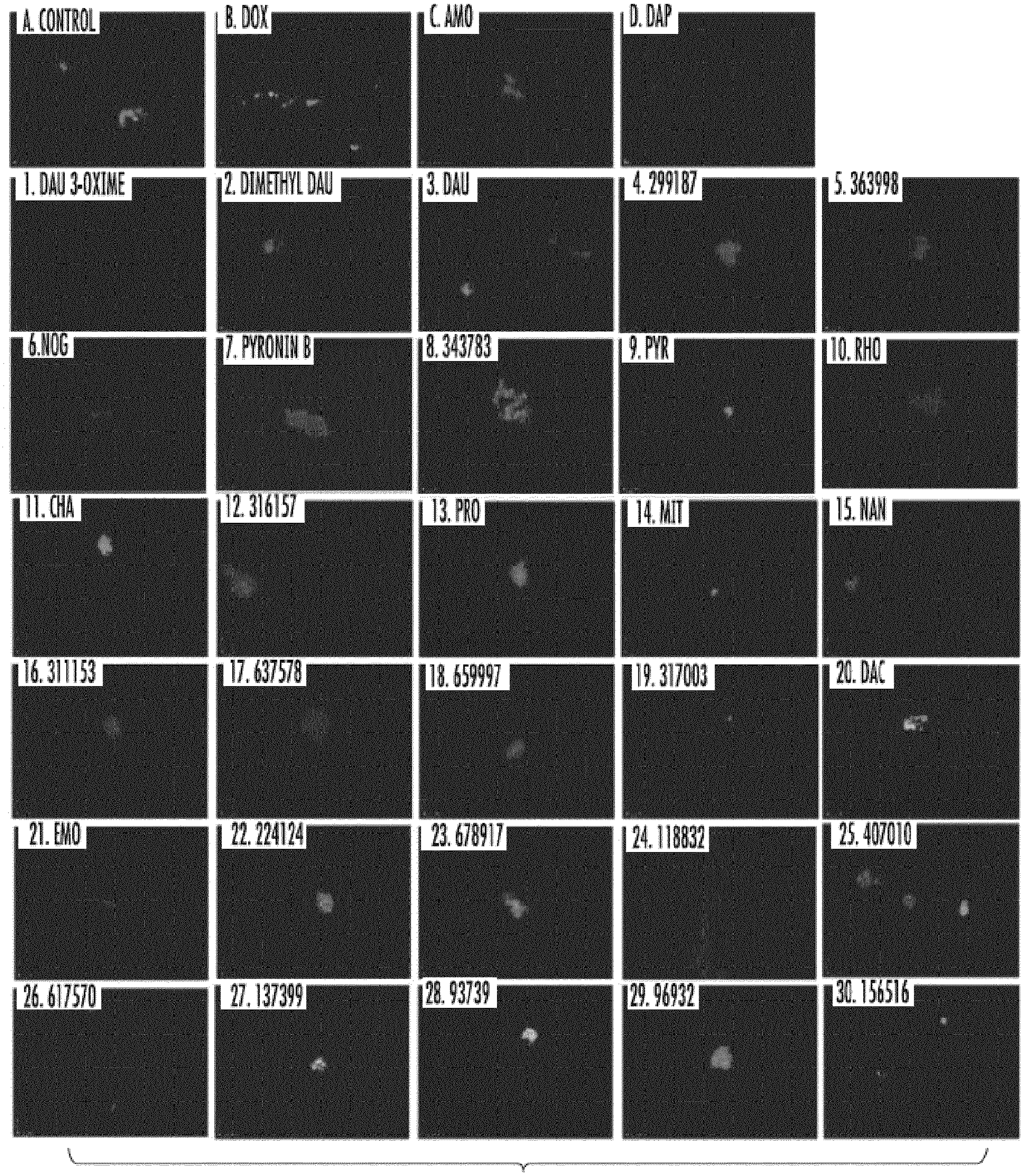
A. CONTROL
B. DOX
C. AMO
D. DAP
1. DAU 3-OXIME
2. DIMETHYL DAU
3. DAU
4. 299187
5. 363998
6.NOG
7. PYRONIN B
8. 343783
9. PYR
10. RHO
11. CHA
12. 316157
13. PRO
14. MIT
15. NAN
16. 311153
17. 637578
18. 659997
19. 317003
20. DAC
21. EMO
22. 224124
23. 678917
24. 118832
25. 407010
26. 617570
27. 137399
28. 93739
29. 96932
30. 156516

*Fig. 24*

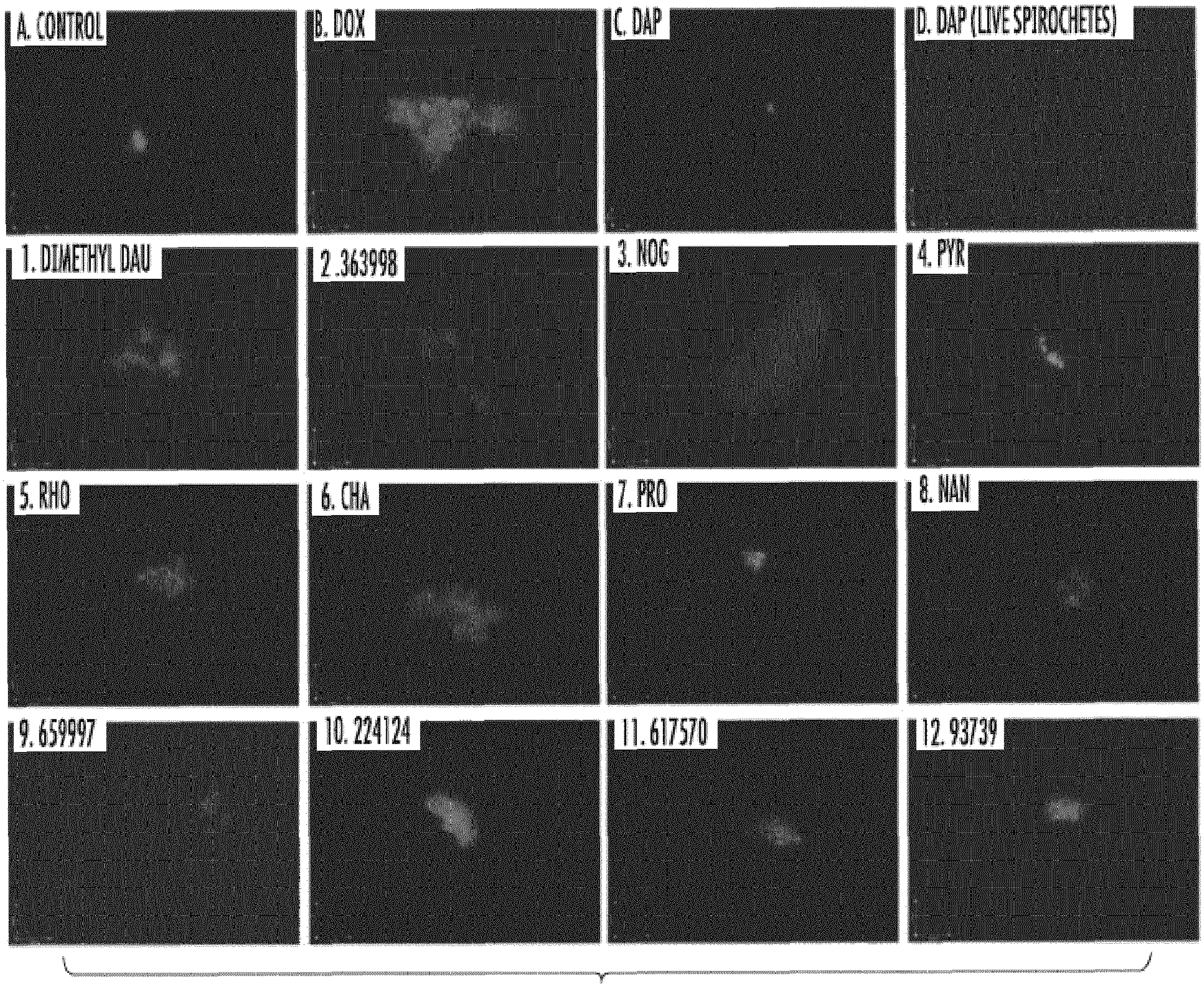
A. CONTROL
B. DOX
C. DAP
D. DAP (LIVE SPIROCHETES)
1. DIMETHYL DAU
2 .363998
3. NOG
4. PYR
5. RHO
6. CHA
7. PRO
8. NAN
9. 659997
10. 224124
11. 617570
12. 93739

*Fig. 25*

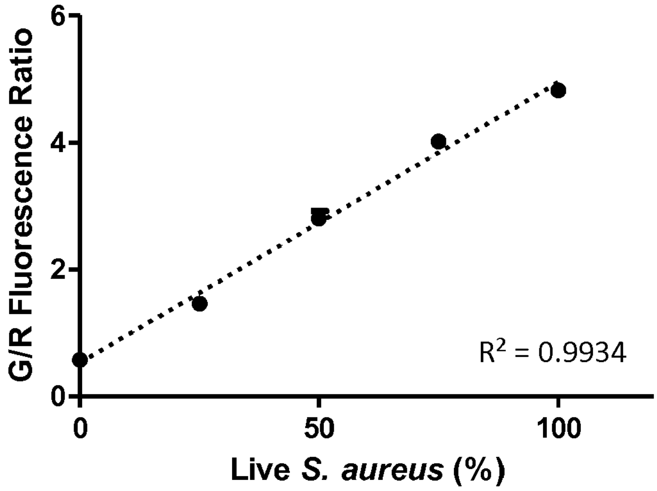


*Fig. 26A*

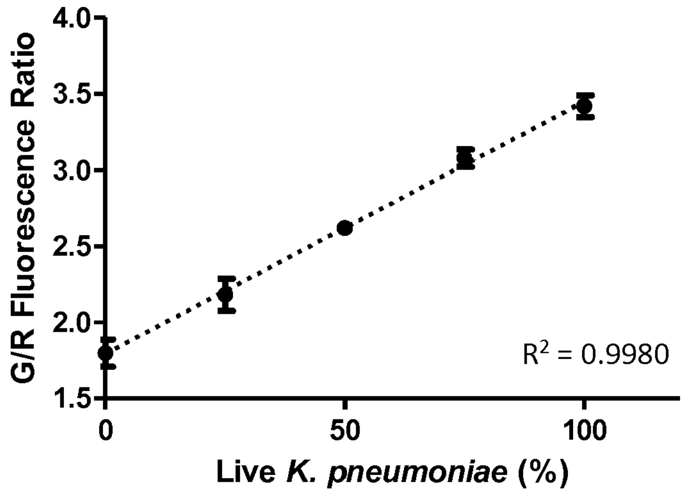


*Fig. 26B*

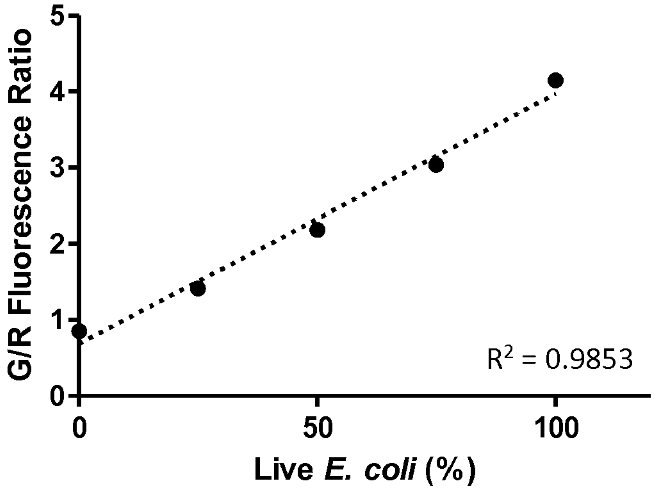


*Fig. 26C*

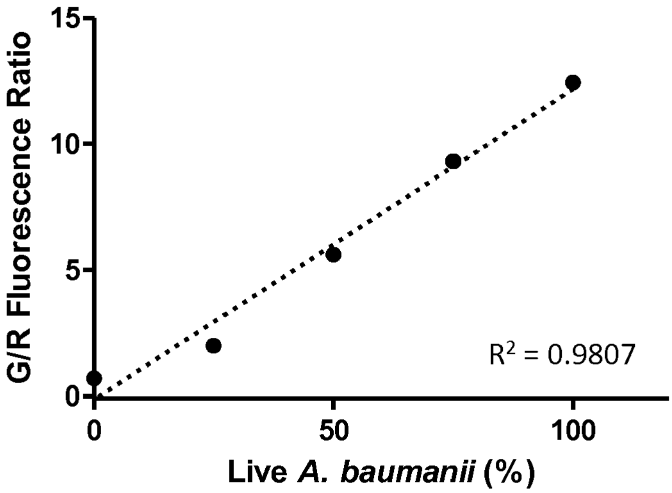


*Fig. 26D*

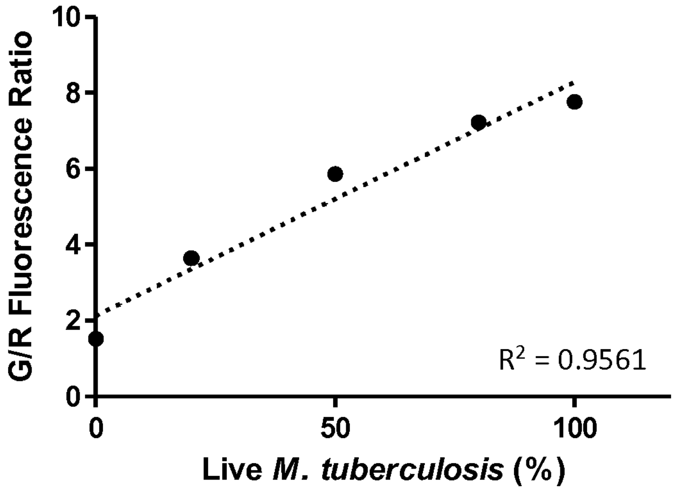


*Fig. 26E*

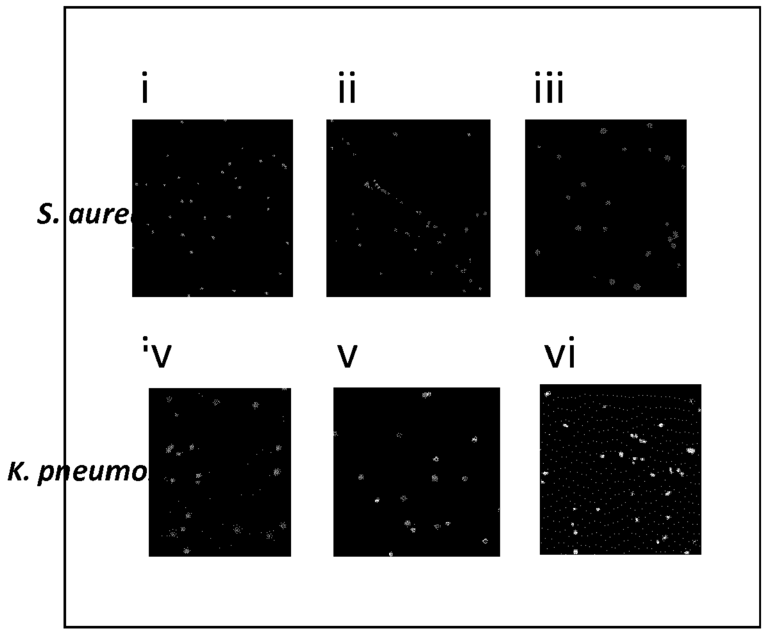


*Fig. 26F*

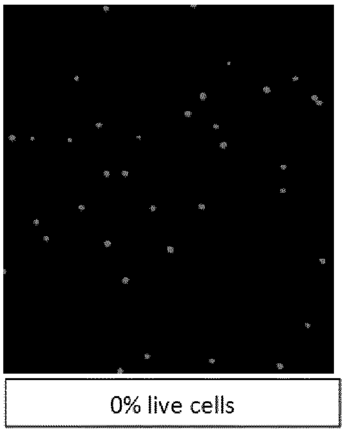

0% live cells

*Fig. 27A*

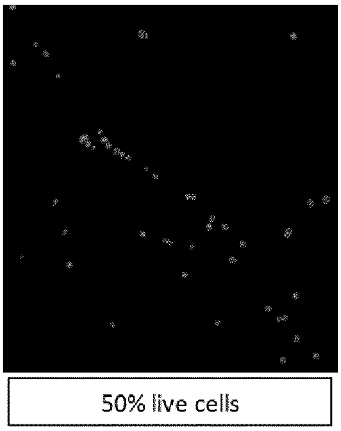

50% live cells

*Fig. 27B*

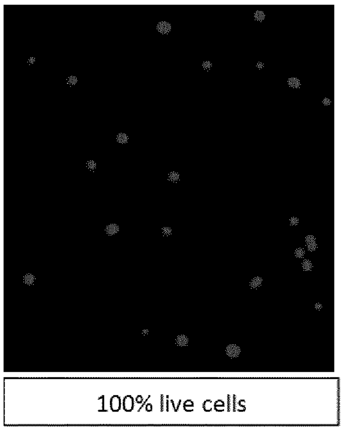

100% live cells

*Fig. 27C*

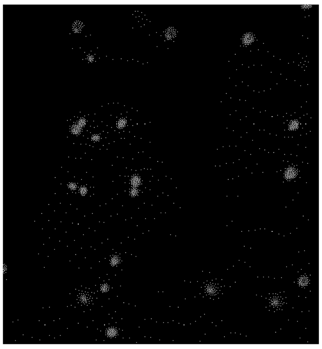

0% live cells

*Fig. 27D*

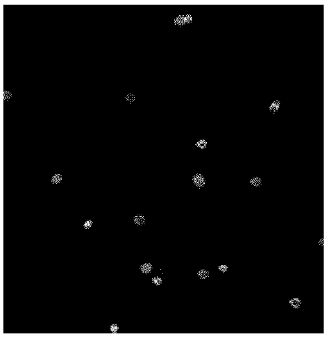

50% live cells

*Fig. 27E*

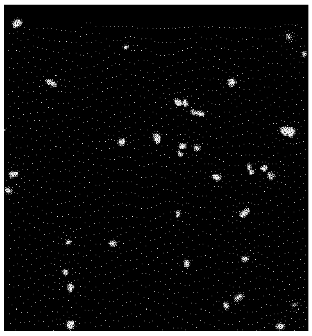

100% live cells

*Fig. 27F*

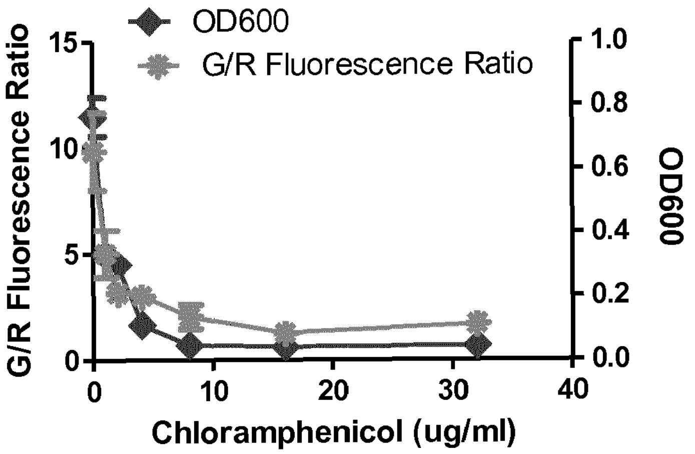


*Fig. 28A*

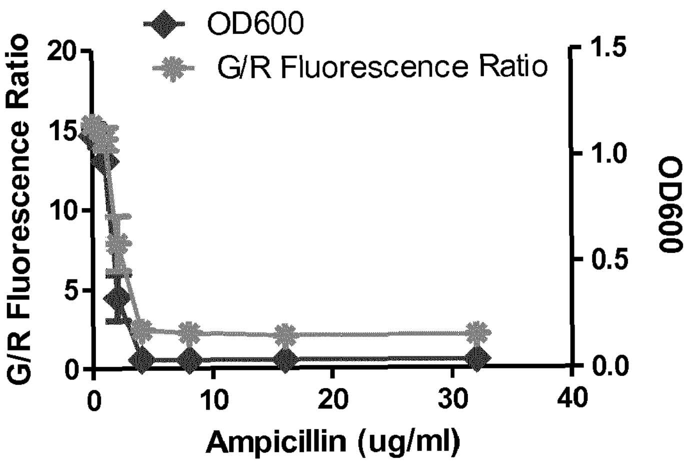


*Fig. 28B*

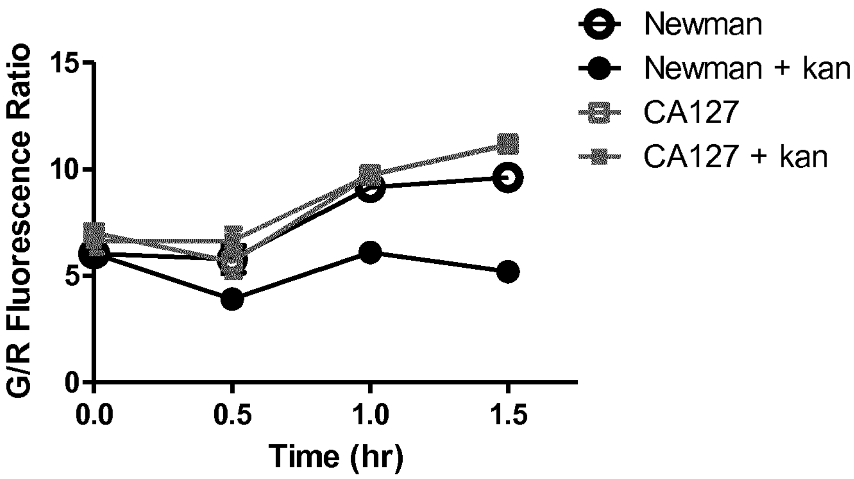


*Fig. 28C*

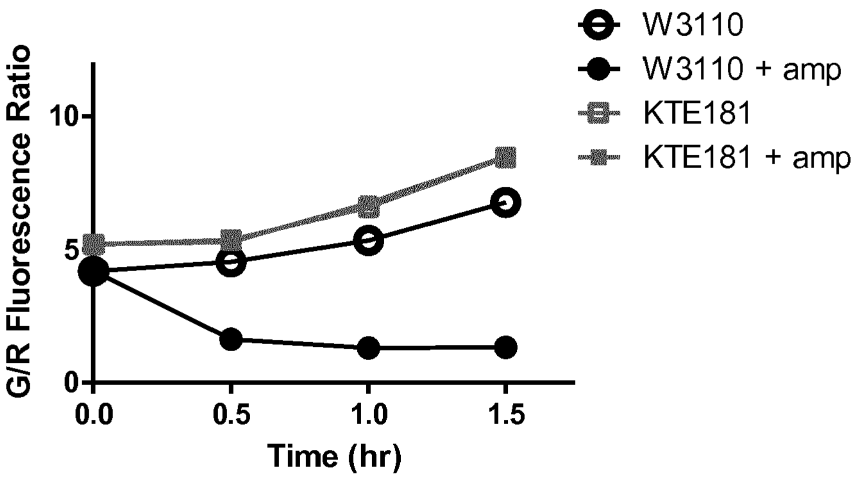


*Fig. 28D*

| Strain name | Gen | Kan | Tet | Ery | Rif | Cip | Cm |
|---|---|---|---|---|---|---|---|
| Newman | S | S | S | S | S | S | S |
| USA300 | S | R | S | IR | -- | -- | -- |
| CA-409 | R | R | S | R | S | R | S |
| CA-127 | S | R | S | IR | S | R | S |
| NY-315 | S | R | S | R | -- | -- | -- |

*Fig. 29*

| Erythromycin | µg /ml | 0 | 5 | 30 | 50 | 100 | Kirby - Bauer | Prop. Killed (100 ug/ml drug) |
|---|---|---|---|---|---|---|---|---|
| % live | Newman | 100 | 43 | 48 | 52 | 50 | S | -0.42 |
| | CA-409 | 100 | 87 | 82 | 84 | 80 | R | -0.14 |
| | NY-315 | 100 | 89 | 83 | 70 | 81 | R | -0.07 |

| Ciprofloxacin | µg /ml | 0 | 5 | 30 | 50 | 100 | Kirby - Bauer | Prop. Killed (100 ug/ml drug) |
|---|---|---|---|---|---|---|---|---|
| % live | Newman | 100 | 20 | 33 | 24 | 36 | S | -0.32 |
| | CA-409 | 100 | 109 | 86 | 88 | 90 | R | -0.09 |

| Gentamicin | µg /ml | 0 | 5 | 30 | 50 | 100 | Kirby - Bauer | Prop. Killed ( 100 ug/ml drug) |
|---|---|---|---|---|---|---|---|---|
| % live | Newman | 99 | 68 | 60 | 61 | 64 | S | -0.22 |
| | USA300 | 100 | 33 | 35 | 36 | 38 | S | -0.45 |
| | CA-409 | 100 | 86 | 88 | 87 | 88 | R | -0.15 |

| Kanamycin | µg /ml | 0 | 5 | 30 | 50 | 100 | Kirby- Bauer | Prop. Killed ( 100 ug/ml drug) |
|---|---|---|---|---|---|---|---|---|
| % live | Newman | 99 | 55 | 48 | 44 | 54 | S | -0.16 |
| | CA-409 | 100 | 86 | 97 | 96 | 100 | R | -0.04 |
| | CA-127 | 100 | 86 | 92 | 85 | 88 | R | -0.06 |

| Chloramphenicol | µg /ml | 0 | 5 | 30 | 50 | 100 | Prop. Killed ( 100 ug/ml drug) |
|---|---|---|---|---|---|---|---|
| % live | USA300 | 100 | 14 | 14 | 22 | 24 | -0.50 |
| | USA300 + CmR (pRAB11 plasmid) | 100 | 72 | 62 | 68 | 29 | -0.13 |

***Fig. 30***

| Drug (100 ug/ml) | Susceptible | Resistant |
|---|---|---|
| Erythromycin | -0.4 | -0.1 |
| Ciprofloxacin | -0.3 | -0.1 |
| Gentamicin | -0.3 | -0.2 |
| Kanamycin | -0.2 | -0.01 |
| Chloramphenicol | -0.5 | -0.1 |

*Fig. 31*

| **Kanamycin** | **µg/ml** | **0** | **25** | **50** | **100** | **Kirby-Bauer** |
|---|---|---|---|---|---|---|
| **% killed** | **Newman** | 0 | 27 | 31 | 32 | S |
| | **CA-409** | 0 | 5 | 6 | 8 | R |
| | **CA-127** | 0 | 5 | 7 | 6 | R |
| | **NY-315** | 0 | 5 | 6 | 16 | R |

| **Erythromycin** | **µg/ml** | **0** | **100** | **200** | **400** | **Kirby-Bauer** |
|---|---|---|---|---|---|---|
| **% killed** | **Newman** | 0 | 32 | 40 | 50 | S |
| | **NY-315** | 0 | 8 | 10 | 17 | R |

| **Ciprofloxacin** | **µg/ml** | **0** | **25** | **50** | **100** | **Kirby-Bauer** |
|---|---|---|---|---|---|---|
| **% killed** | **Newman** | 0 | 46 | 58 | 59 | S |
| | **CA-409** | 0 | 17 | 21 | 22 | R |
| | **CA-127** | 0 | 23 | 28 | 30 | R |

| **Gentamicin** | **µg/ml** | **0** | **100** | **200** | **400** | **Kirby-Bauer** |
|---|---|---|---|---|---|---|
| **% killed** | **Newman** | 0 | 32 | 32 | 27 | S |
| | **CA-409** | 0 | 18 | 18 | 10 | R |

***Fig. 32***

| Strain name | Cm | Cip | Trim | Gen | Kan | Tet | Amp | Sm |
|---|---|---|---|---|---|---|---|---|
| W3110 | S | S | S | S | S | S | S | S |
| UTI89 | S | S | S | S | S | S | S | IR |
| CFT073 | S | S | R | S | S | S | R | R |
| KTE181 | S | S | R | R | S | S | R | R |

*Fig. 33*

| Ampicillin | µg /ml | 0 | 5 | 30 | 50 | 100 | Kirby- Bauer | Prop. Killed (100 ug/ml drug) |
|---|---|---|---|---|---|---|---|---|
| % live | W3110 | 100 | 1 | 2 | 2 | 6 | S | -0.56 |
| | KTE181 | 100 | 100 | 100 | 100 | 81 | R | -0.14 |
| | UTI89 | 100 | 100 | 23 | 10 | 4 | S | -0.50 |

| Trimethoprim | µg /ml | 0 | 5 | 30 | 50 | 100 | Kirby-Bauer | Prop. Killed ( 5 ug/ml drug) |
|---|---|---|---|---|---|---|---|---|
| % live | W3110 | 100 | 8 | 8 | 10 | 13 | S | -0.50 |
| | KTW181 | 100 | 68 | 54 | 51 | 48 | R | -0.02 |
| | CFT073 | 100 | 42 | 27 | 27 | 25 | R | -0.29 |

| Streptomycin | µg /ml | 0 | 5 | 30 | 50 | 100 | Kirby-Bauer | Prop. Killed (100 ug/ml drug) |
|---|---|---|---|---|---|---|---|---|
| % live | W3110 | 100 | 39 | 6 | 5 | 6 | S | -0.55 |
| | UTI89 | 100 | 78 | 20 | 19 | 19 | IR | -0.45 |
| | CFT073 | 100 | 93 | 32 | 27 | 28 | R | -0.36 |
| | KTE181 | 100 | 98 | 94 | 94 | 96 | R | -0.05 |

***Fig. 34***

| Drug | Susceptible | Intermediate Resistant | Resistant |
|---|---|---|---|
| **Ampicillin** (100 ug/ml) | -0.5 | N.D. | -0.1 |
| **Trimethoprim** (5 ug/ml) | -0.5 | N.D. | -0.2 |
| **Streptomycin** (100 ug/ml) | -0.6 | -0.5 | -0.2 |

***Fig. 35***

| Ampicillin | µg/ml | 0 | 25 | 50 | 100 | 200 | Kirby-Bauer |
|---|---|---|---|---|---|---|---|
| % killed | W3110 | 0 | 17 | 34 | 38 | 68 | S |
| | UTI88 | 0 | 7 | 1 | 23 | 60 | S |
| | KTE181 | 0 | 2 | 1 | 1 | 5 | R |

| Trimethoprim | µg/ml | 0 | 25 | 50 | 100 | 200 | Kirby-Bauer |
|---|---|---|---|---|---|---|---|
| % killed | W3110 | 0 | 47 | 47 | 47 | 46 | S |
| | CFT073 | 0 | 22 | 25 | 25 | 26 | R |
| | KTE181 | 0 | 1 | 2 | 5 | 7 | R |

| Streptomycin | µg/ml | 0 | 25 | 50 | 100 | 200 | Kirby-Bauer |
|---|---|---|---|---|---|---|---|
| % killed | W3110 | 0 | 29 | 32 | 32 | 32 | S |
| | KTE181 | 0 | 0 | 0 | 0 | 0 | R |

***Fig. 36***

| Ceftriaxone | µg/ml | 0 | 25 | 50 | 100 | 200 | Kirby-Bauer |
|---|---|---|---|---|---|---|---|
| % killed | W3110 | 0 | 37 | 51 | 58 | 61 | S |
| | Isolate 7 | 0 | 0 | 0 | 0 | 0 | R |

| Cefotaxime | µg/ml | 0 | 25 | 50 | 100 | 200 | Kirby-Bauer |
|---|---|---|---|---|---|---|---|
| % killed | W3110 | 0 | 24 | 42 | 52 | 65 | S |
| | Isolate 7 | 0 | 0 | 0 | 0 | 0 | R |

*Fig. 37*

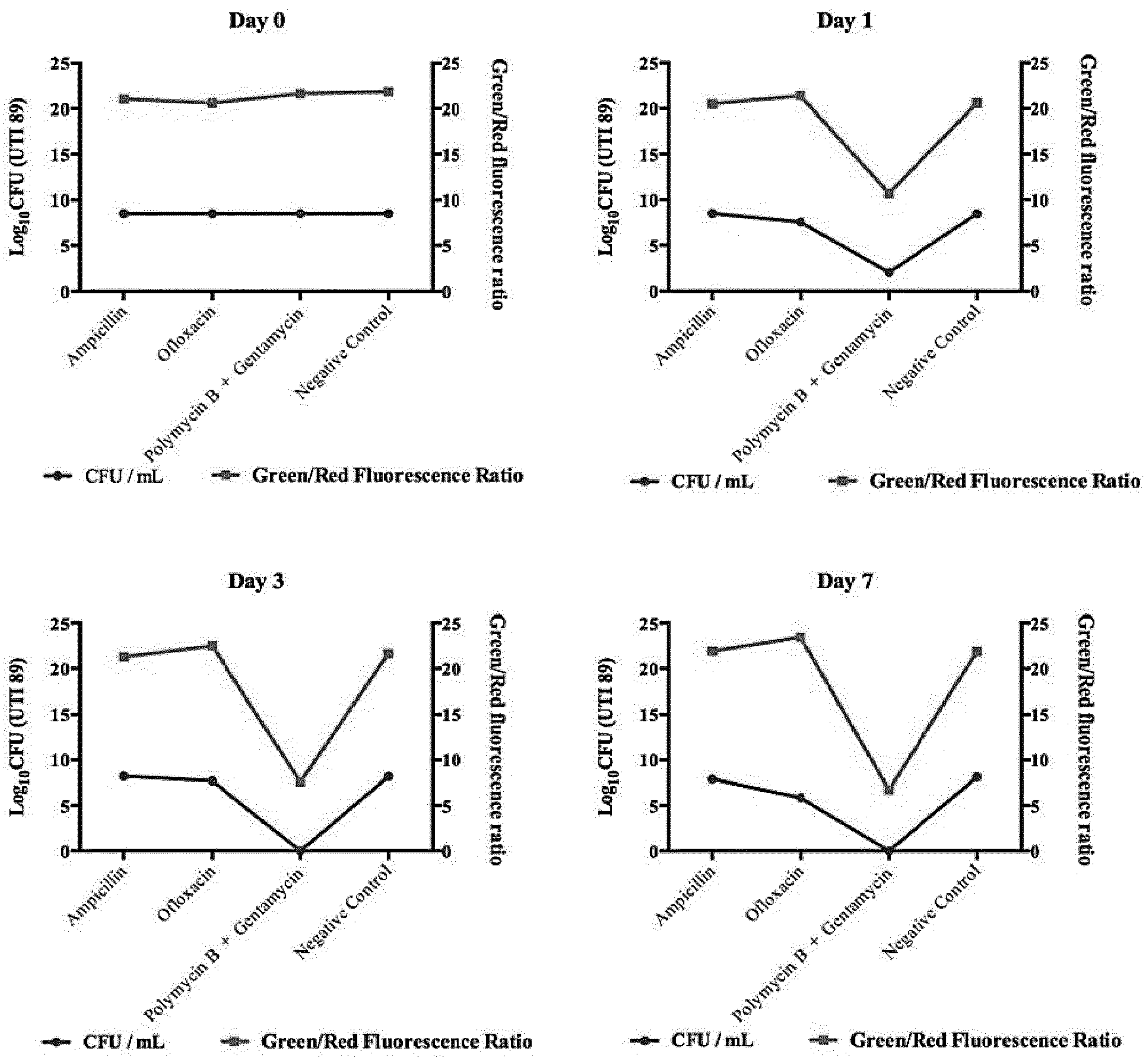
Day 0
Day 1
Day 3
Day 7
Log10CFU (UTI 89)
Green/Red fluorescence ratio
0
5
10
15
20
25
Ampicillin
Ofloxacin
Polymycin B + Gentamycin
Negative Control
CFU / mL
Green/Red Fluorescence Ratio

*Fig. 38*

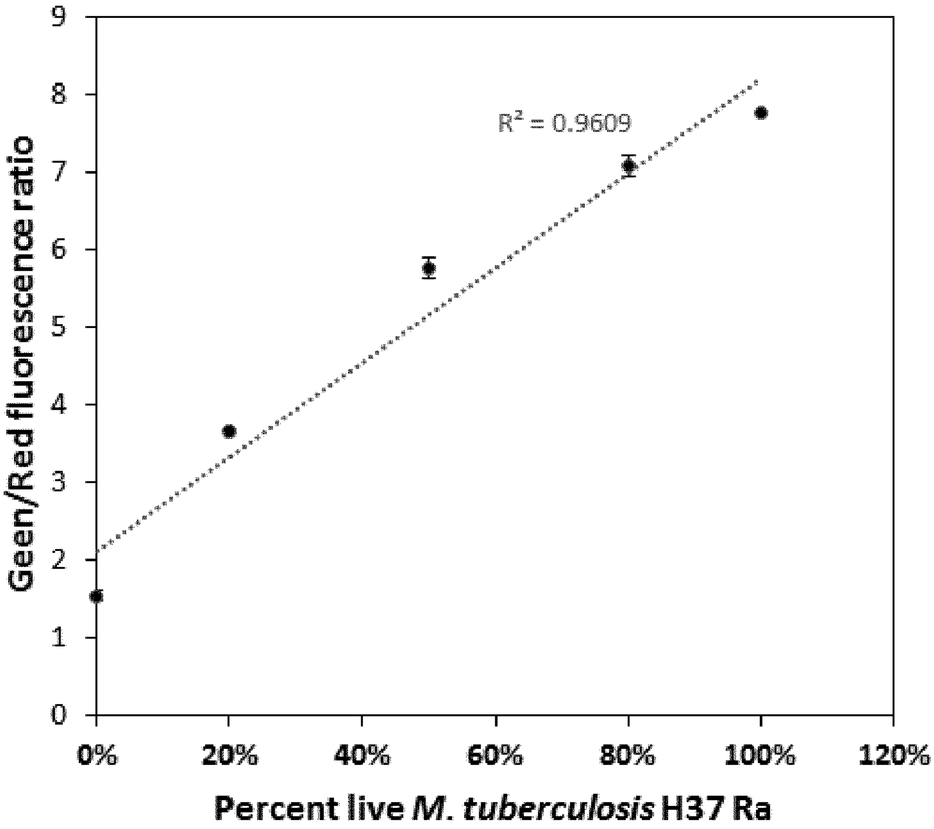
R² = 0.9609
Geen/Red fluorescence ratio
0
1
2
3
4
5
6
7
8
9
0%
20%
40%
60%
80%
100%
120%
Percent live *M. tuberculosis* H37 Ra

*Fig. 39*

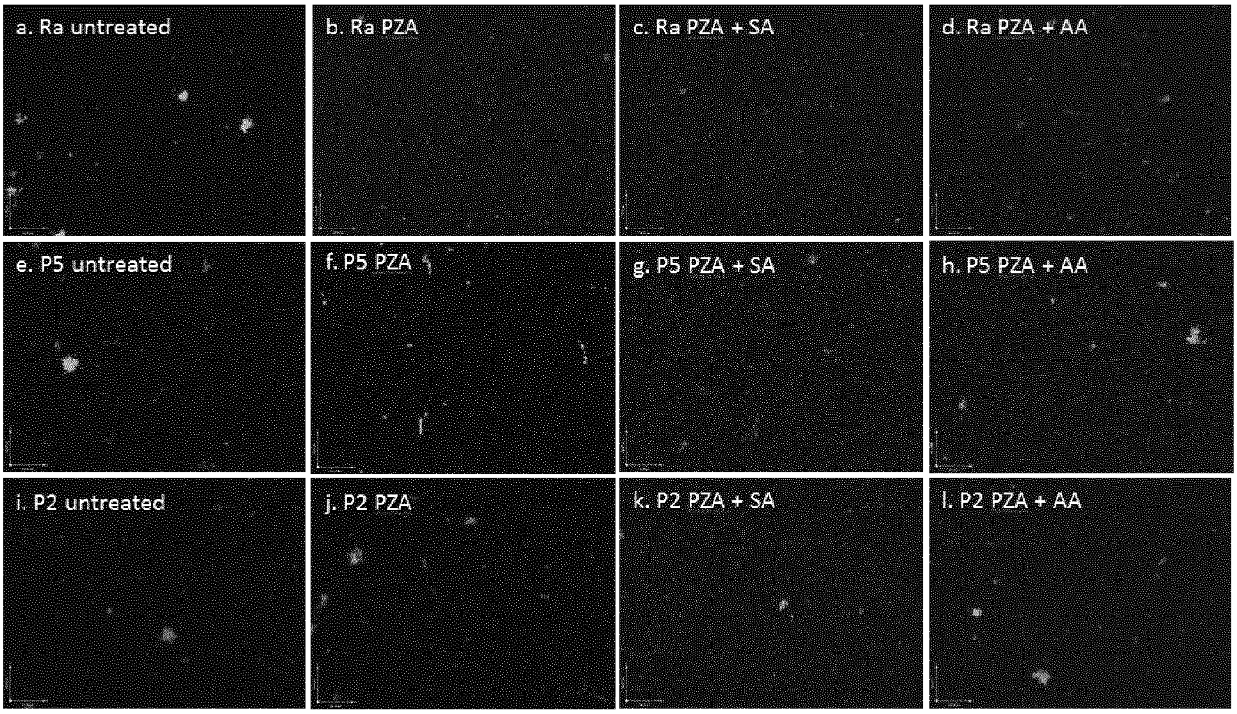
a. Ra untreated
b. Ra PZA
c. Ra PZA + SA
d. Ra PZA + AA
e. P5 untreated
f. P5 PZA
g. P5 PZA + SA
h. P5 PZA + AA
i. P2 untreated
j. P2 PZA
k. P2 PZA + SA
l. P2 PZA + AA

*Fig. 40*

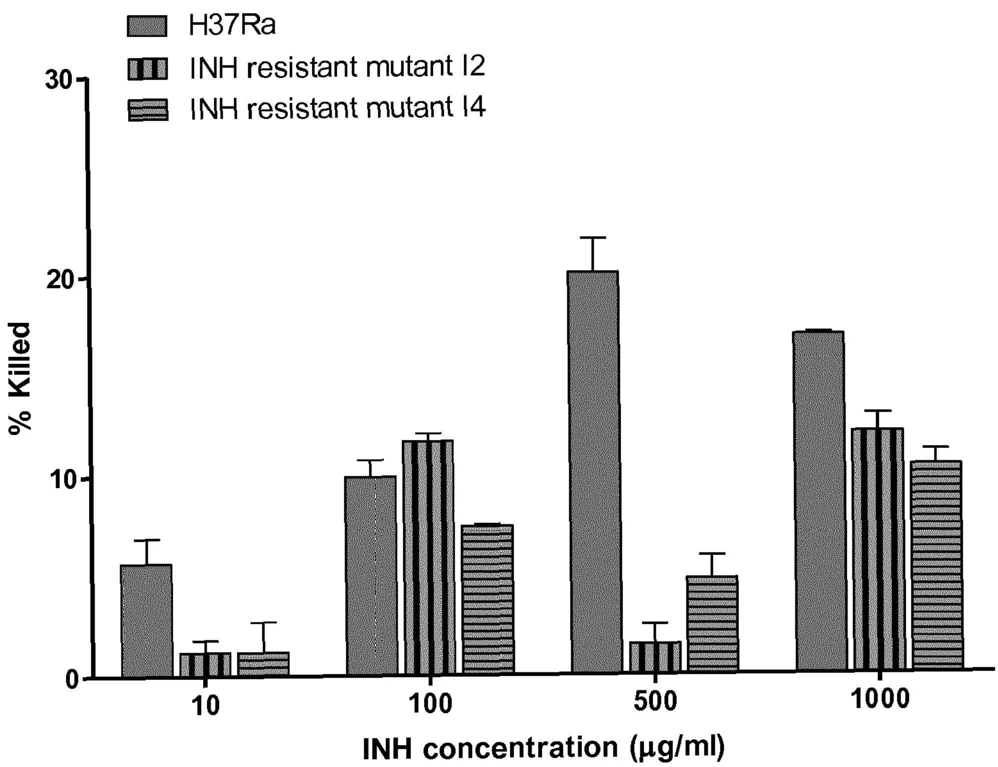


*Fig. 41A*

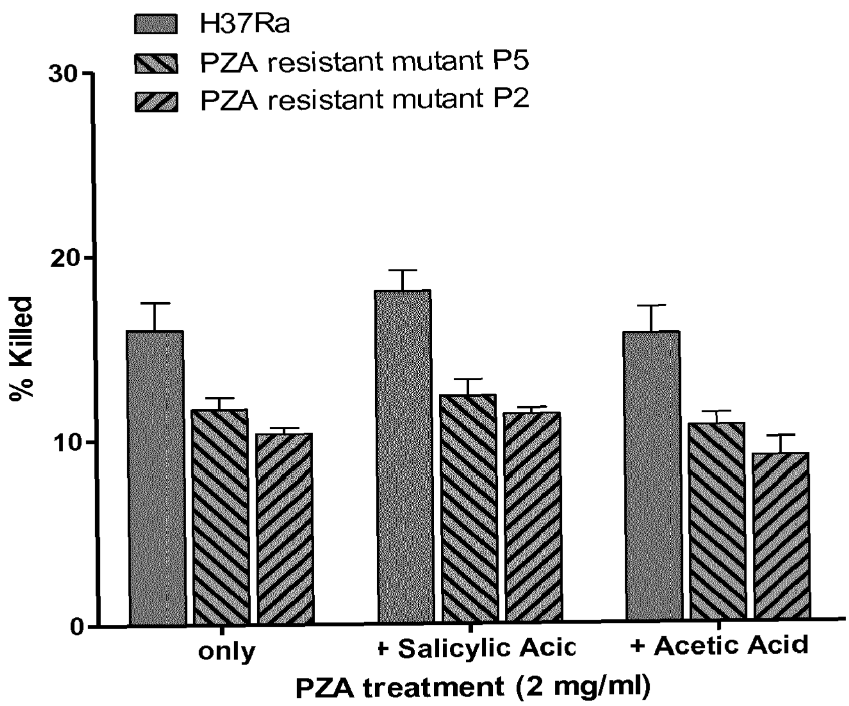


*Fig. 41B*

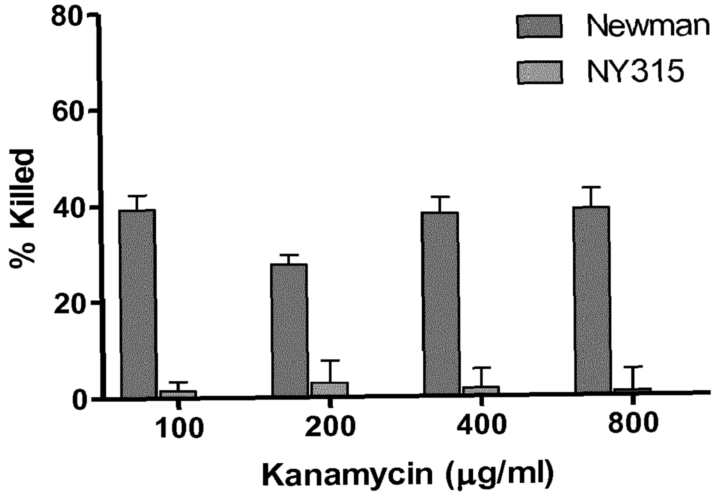


*Fig. 42A*

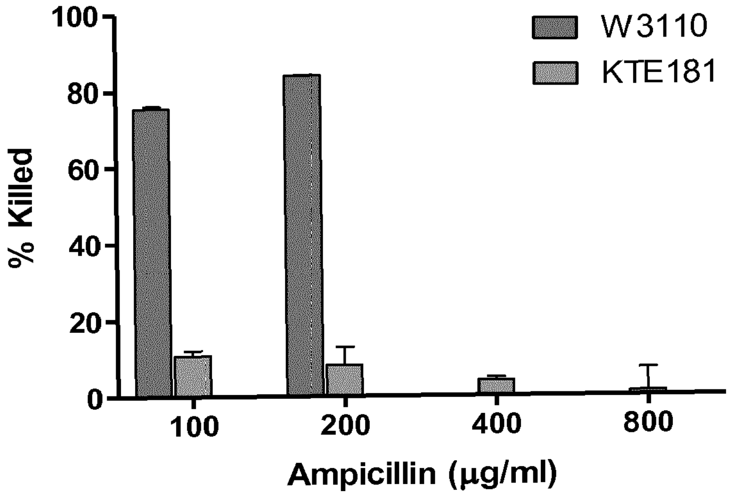


*Fig. 42B*

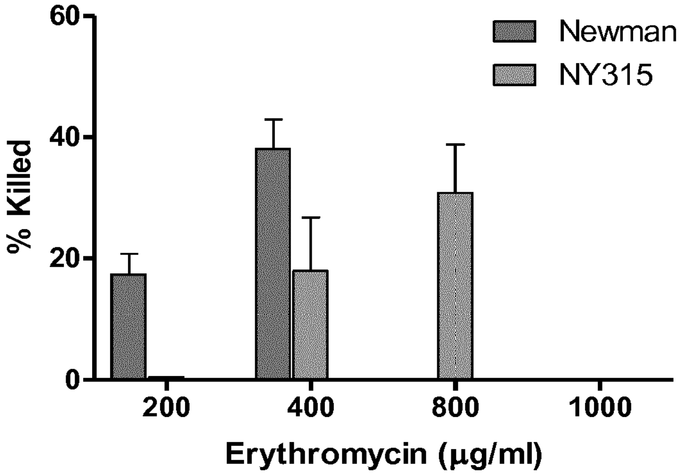


*Fig. 42 C*

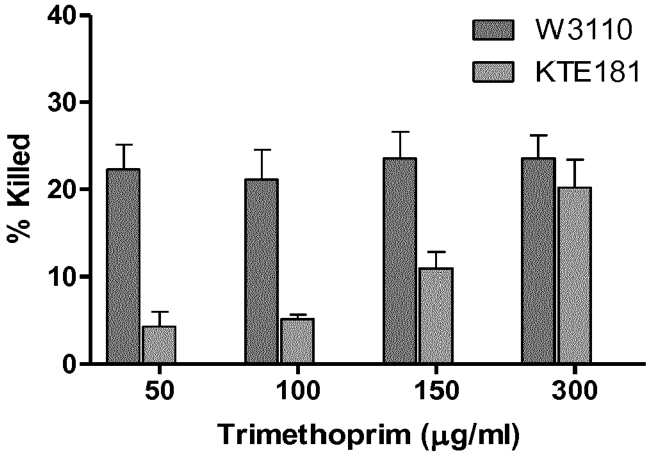


*Fig. 42D*

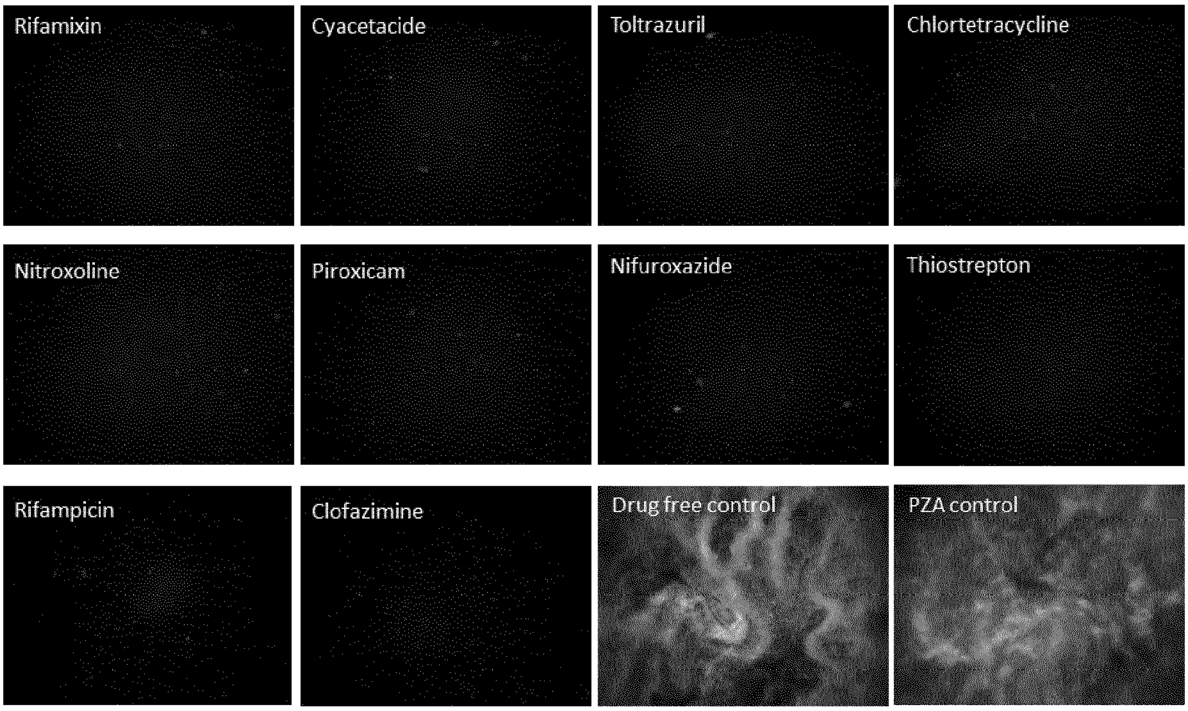
Rifamixin
Cyacetacide
Toltrazuril
Chlortetracycline
Nitroxoline
Piroxicam
Nifuroxazide
Thiostrepton
Rifampicin
Clofazimine
Drug free control
PZA control

*Fig. 43*

Europäisches Patentamt
European Patent Office
Office européen des brevets

# EUROPEAN SEARCH REPORT

Application Number
EP 17 15 9022

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br>Y | JIE FENG ET AL: "An Optimized SYBR Green I/PI Assay for Rapid Viability Assessment and Antibiotic Susceptibility Testing for Borrelia burgdorferi", PLOS ONE, vol. 9, no. 11, 3 November 2014 (2014-11-03), page e111809, XP055387549, DOI: 10.1371/journal.pone.0111809<br>* the whole document *<br>* in particular: *<br>* Materials and Methods; page 2 - page 3 *<br>* page 5; figure 3 *<br>----- | 1-5,8, 10-14<br><br><br><br><br><br><br><br>6,7,9 | INV.<br>C12Q1/18<br>G01N33/50 |
| X<br><br><br><br><br><br><br><br><br>Y | JIE FENG ET AL: "A Drug Combination Screen Identifies Drugs Active against Amoxicillin-Induced Round Bodies of In Vitro Borrelia burgdorferi Persisters from an FDA Drug Library", FRONTIERS IN MICROBIOLOGY, vol. 7, 23 May 2016 (2016-05-23), XP055387543, DOI: 10.3389/fmicb.2016.00743<br>* the whole document *<br>-----<br>-/-- | 1-5,8, 10-14<br><br><br><br><br><br><br><br><br>6,7,9 | TECHNICAL FIELDS SEARCHED (IPC)<br>C12Q<br>G01N |

The present search report has been drawn up for all claims

1

| Place of search | Date of completion of the search | Examiner |
|---|---|---|
| The Hague | 4 July 2017 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 15 9022

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2011/033290 A2 (UNIV ABERYSTWYTH [GB]; PEAK EMILY [GB]; HOFFMANN KARL FRANCIS [GB]) 24 March 2011 (2011-03-24)<br>* the whole document *<br>* in particular: *<br>* abstract *<br>* page 8, last paragraph - page 9, paragraph 1 *<br>* page 11, line 3 - line 9 *<br>* page 14, last paragraph *<br>* page 15, line 14 - line 29 *<br>* page 39, line 5 - page 40, line 13 *<br>* claims 1-39 *<br>----- | 6,7,9 | |
| X | JIE FENG ET AL: "Drug Combinations against Borrelia burgdorferi Persisters In Vitro: Eradication Achieved by Using Daptomycin, Cefoperazone and Doxycycline. article e0117207",<br>PLOS ONE,<br>vol. 10, no. 3,<br>1 January 2015 (2015-01-01), pages 1-15,<br>XP055235667,<br>ISSN: 1932-6203, DOI: 10.1371/journal.pone.0117207 | 1-5,8, 10-14 | |
| Y | * the whole document *<br>----- | 6,7,9 | TECHNICAL FIELDS SEARCHED (IPC) |
| X | JIE FENG ET AL: "Identification of novel activity against Borrelia burgdorferi persisters using an FDA approved drug library",<br>EMERGING MICROBES & INFECTIONS,<br>vol. 3, no. 7, 2 July 2014 (2014-07-02),<br>pages 1-8, XP055235665,<br>DOI: 10.1038/emi.2014.53 | 1-5,8, 10-14 | |
| Y | * the whole document *<br>----- | 6,7,9 | |
| | -/-- | | |

1

The present search report has been drawn up for all claims

| Place of search | Date of completion of the search | Examiner |
|---|---|---|
| The Hague | 4 July 2017 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 17 15 9022

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 92/15700 A1 (ANTICANCER INC [US]) 17 September 1992 (1992-09-17) * page 17, line 15 - page 18, line 3 * * claims 11-28 * ----- | 6,7,9 | |
| Y | S. TAMBURINI ET AL: "Accurate flow cytometric monitoring of Escherichia coli subpopulations on solid food treated with high pressure carbon dioxide", JOURNAL OF APPLIED MICROBIOLOGY., vol. 117, no. 2, 16 May 2014 (2014-05-16), pages 440-450, XP055387765, GB ISSN: 1364-5072, DOI: 10.1111/jam.12528 * abstract * * page 441, left-hand column, last paragraph - right-hand column, paragraph 1 * ----- | 6,7,9 | |
| | | | TECHNICAL FIELDS SEARCHED (IPC) |

The present search report has been drawn up for all claims

1

| Place of search | Date of completion of the search | Examiner |
|---|---|---|
| The Hague | 4 July 2017 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 17 15 9022

This annex lists the patent family members relating to the patent documents cited in the above-mentioned European search report. The members are as contained in the European Patent Office EDP file on
The European Patent Office is in no way liable for these particulars which are merely given for the purpose of information.

04-07-2017

| Patent document cited in search report | Publication date | Patent family member(s) | Publication date |
|---|---|---|---|
| WO 2011033290 A2 | 24-03-2011 | EP 2478370 A2 | 25-07-2012 |
| | | US 2012225019 A1 | 06-09-2012 |
| | | WO 2011033290 A2 | 24-03-2011 |
| WO 9215700 A1 | 17-09-1992 | AT 175242 T | 15-01-1999 |
| | | DE 69228055 D1 | 11-02-1999 |
| | | DE 69228055 T2 | 20-05-1999 |
| | | EP 0573606 A1 | 15-12-1993 |
| | | JP 2950519 B2 | 20-09-1999 |
| | | JP H06505636 A | 30-06-1994 |
| | | US 5849579 A | 15-12-1998 |
| | | WO 9215700 A1 | 17-09-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ZHANG et al.** *Microbiol Spectrum,* 2014
- **FENG et al.** *Emerg Microbes Infect,* 2014
- **ALBAN, P.S. et al.** Serum-starvation-induced changes in protein synthesis and morphology of Borrelia burgdorferi. *Microbiology,* 2000, vol. 146, 119-127
- **AMANT, F. et al.** Chemotherapy during pregnancy. *Curr. Opin. Oncol. 2012,* 2012, vol. 24, 580-586
- **ANDO H ; MITARAI S ; KONDO Y ; SUETAKE T ; SEKIGUCHI JI et al.** Pyrazinamide resistance in multidrug-resistant Mycobacterium tuberculosis isolates in Japan. *Clin Microbiol Infect,* 2010, vol. 16, 1164-1168
- **ARAGON LM ; GARRIGO M ; MORENO C ; ESPANOL M ; COLL P.** Evaluation of the BacT/ALERT PZA kit in comparison with the BACTEC 460TB PZA for testing Mycobacterium tuberculosis susceptibility to pyrazinamide. *J Antimicrob Chemother,* 2007, vol. 60, 655-657
- **ARBISER, J.L. ; MOSCHELLA, S.L.** Clofazimine: a review of its medical uses and mechanisms of action. *J. Am. Acad Dermatol.,* 1995, vol. 32, 241-7
- Armed Forces Health Surveillance, Center, Surveillance snapshot: Lyme disease among beneficiaries of the Military Health System, 2001-2012. *MSMR,* 2013, vol. 20 (8), 23
- **BARBESTI, S. et al.** Two and three-color fluorescence flow cytometric analysis of immunoidentified viable bacteria. *Cytometry,* 2000, vol. 40, 214-8
- **BACON, R.M. et al.** Surveillance for Lyme disease--United States, 1992-2006. *MMWR Surveill. Summ.,* 2008, vol. 57 (10), 1-9
- **BARTHOLD, S.W. et al.** Ineffectiveness of tigecycline against persistent Borrelia burgdorferi. *Antimicrobial Agents and Chemotherapy,* 2010, vol. 54, 643-651
- Structure, function and biogenesis of the Borrelia cell envelope. **BERGSTRÖM, S. ; ZÜCKERT, W.R.** Borrelia: Molecular Biology, Host Interaction and Pathogenesis. Caister Academic Press, 2010, 139-166
- **BERNDTSON, K.** Review of evidence for immune evasion and persistent infection in Lyme disease. *Int. J. Gen. Med.,* 2013, vol. 6, 291-306
- **BOCKENSTEDT, L.K. et al.** Spirochete antigens persist near cartilage after murine Lyme borreliosis therapy. *J. Clin. Invest.,* 2012, vol. 122, 2652-2660
- **BOERNER, J. et al.** In vitro antimicrobial susceptibility testing of Borrelia burgdorferi: influence of test conditions on minimal inhibitory concentration (MIC) values. *Zentralbl Bakteriol,* 1995, vol. 283 (1), 49-60
- **BOULOS, L. et al.** LIVE/DEAD BacLight : application of a new rapid staining method for direct enumeration of viable and total bacteria in drinking water. *J. Microbiol. Methods,* 1999, vol. 37, 77-86
- **BRORSON, O. ; BRORSON, S.H.** Transformation of cystic forms of Borrelia burgdorferi to normal, mobile spirochetes. *Infection,* 1997, vol. 25, 240-246
- **BRORSON, O. ; BRORSON, S.H.** In vitro conversion of Borrelia burgdorferi to cystic forms in spinal fluid, and transformation to mobile spirochetes by incubation in BSK-H medium. *Infection,* 1998, vol. 26, 144-150
- **BRORSON, O. ; BRORSON, S.H.** An in vitro study of the susceptibility of mobile and cystic forms of Borrelia burgdorferi to metronidazole. *APMIS,* 1999, vol. 107 (6), 566-76
- **BRORSON, O. et al.** Destruction of spirochete Borrelia burgdorferi round-body propagules (RBs) by the antibiotic tigecycline. *PNAS,* 2009, vol. 106 (44), 18656-61
- **BROUQUI, P. et al.** Eucaryotic cells protect Borrelia burgdorferi from the action of penicillin and ceftriaxone but not from the action of doxycycline and erythromycin. *Antimicrob. Agents Chemother.,* 1996, vol. 40 (6), 1552-1554
- **CAMPBELL, J.W. ; CRONAN, J.E., JR.** Bacterial fatty acid biosynthesis: targets for antibacterial drug discovery. *Annu. Rev. Microbiol.,* 2001, vol. 55, 305-32
- **CASJENS, S.** Borrelia genomes in the year 2000. *J. Mol. Microbiol. Biotechnol. 2000,* 2000, vol. 2, 401-410
- **CHANG KC ; LEUNG CC ; YEW WW ; LAU TY ; TAM CM.** Hepatotoxicity of pyrazinamide: cohort and case-control analyses. *American Journal of Respiratory and Critical Care Medicine,* 2008, vol. 177, 1391-1396

- **CHEDORE P ; BERTUCCI L ; WOLFE J ; SHARMA M ; JAMIESON F.** Potential for erroneous results indicating resistance when using the Bactec MGIT 960 system for testing susceptibility of Mycobacterium tuberculosis to pyrazinamide. *Journal of Clinical Microbiology,* 2010, vol. 48, 300-301
- **CHENG SJ ; THIBERT L ; SANCHEZ T ; HEIFETS L ; ZHANG Y.** pncA mutations as a major mechanism of pyrazinamide resistance in Mycobacterium tuberculosis: spread of a monoresistant strain in Quebec, Canada. *Antimicrob Agents Chemother,* 2000, vol. 44, 528-532
- **CHOLO, M.C. et al.** Clofazimine: current status and future prospects. *J. Antimicrob. Chemother.,* 2012, vol. 67, 290-298
- **CHONG, C.R. et al.** A clinical drug library screen identifies astemizole as an antimalarial agent. *Nat. Chem. Biol.,* 2006, vol. 2 (8), 415-6
- Clinical and Laboratory Standards Institute, Performance Standards for Antimicrobial Susceptibility Testing; Seventeenth Informational Supplement. *CLSI,* 2007, vol. 27 (1), 154-161
- CLSI (2011) Susceptibility Testing of Mycobacteria, Nocardiae, and Other Aerobic Actinomycetes; Approved Standard-Second Edition. CLSI document M24-A2. Clinical and Laboratory Standards Institute
- CLSI (2016) Performance Standards for Antimicrobial Performance Standards for Antimicrobial Susceptibility Testing. CLSI supplement M100S. Clinical and Laboratory Standards Institute
- **DEVER, L.L. et al.** In vitro antimicrobial susceptibility testing of Borrelia burgdorferi: a microdilution MIC method and time-kill studies. *J. Clin. Microbiol.,* 1992, vol. 30 (10), 2692-7
- **DEVER, L.L. et al.** In vitro activity of vancomycin against the spirochete Borrelia burgdorferi. *Antimicrob Agents Chemother.,* 1993, vol. 37, 1115-1121
- **DHAND, A. et al.** Use of antistaphylococcal beta-lactams to increase daptomycin activity in eradicating persistent bacteremia due to methicillin-resistant Staphylococcus aureus: role of enhanced daptomycin binding. *Clin. Infect. Dis.,* 2011, vol. 53, 158-163
- **DITERICH, I. et al.** Borrelia burgdorferi-induced tolerance as a model of persistence via immunosuppression. *Infect. Immun.,* 2003, vol. 71 (7), 3979-87
- *DTP- Mechanistic Set Information,* 2015
- *DTP- Natural Products Set information,* 2015
- **EMBERS, M.E. et al.** Persistence of Borrelia burgdorferi in Rhesus Macaques following Antibiotic Treatment of Disseminated Infection. *PLoS One,* 2012, vol. 7 (1), e29914
- **FALLON, B.A. et al.** A randomized, placebo-controlled trial of repeated IV antibiotic therapy for Lyme encephalopathy. *Neurology,* 2008, vol. 70, 992-1003
- **FENG J ; SHI W ; ZHANG S ; ZHANG Y.** Identification of new compounds with high activity against stationary phase Borrelia burgdorferi from the NCI compound collection. *Emerg Microbes Infect,* 2015, vol. 4, e31
- **FENG J ; SHI W ; ZHANG S ; SULLIVAN D ; AUWAERTER PG et al.** A Drug Combination Screen Identifies Drugs Active against Amoxicillin-Induced Round Bodies of In Vitro Borrelia burgdorferi Persisters from an FDA Drug Library. *Front Microbiol,* 2016, vol. 7, 743
- **FENG, J. ; WANG, T. ; SHI, W. et al.** Identification of novel activity against Borrelia Burgdorferi persisters using an FDA approved drug library. *Emerging Microbes and Infections,* 2014, e49
- **FENG, J. ; WANG, T. ; ZHANG, S. et al.** An Optimized SYBR Green I/PI Assay for Rapid Viability Assessment and Antibiotic Susceptibility Testing for Borrelia burgdorferi. *PLoS One,* 2014, vol. 9, e111809
- **FREDRICKS BA ; DECOSTER DJ ; KIM Y ; SPARKS N ; CALLISTER SM et al.** Rapid pyrazinamide susceptibility testing of Mycobacterium tuberculosis by flow cytometry. *J Microbiol Methods,* 2006, vol. 67, 266-272
- **GABRIELSON, J. et al.** Evaluation of redox indicators and the use of digital scanners and spectrophotometer for quantification of microbial growth in microplates. *J Microbiol Methods,* 2002, vol. 50, 63-73
- **GREGORI, G. et al.** Resolution of viable and membrane-compromised bacteria in freshwater and marine waters based on analytical flow cytometry and nucleic acid double staining. *Appl. Environ. Microbiol.,* 2001, vol. 67, 4662-70
- **HALL SNYDER, A. et al.** Evaluation of the novel combination of daptomycin plus ceftriaxone against vancomycin-resistant enterococci in an in vitro pharmacokinetic/pharmacodynamic simulated endocardial vegetation model. *The Journal of Antimicrobial Chemotherapy,* 2014, vol. 69, 2148-2154
- **HAYASHI, M. et al.** The mode of action of nanaomycins D and A on a gram-negative marine bacterium Vibrio alginolyticus. *J. of Antibiotics.,* 1982, vol. 35 (8), 1078-85
- **HEWLETT D, JR. ; HORN DL ; ALFALLA C.** Drug-resistant tuberculosis: inconsistent results of pyrazinamide susceptibility testing. *JAMA,* 1995, vol. 273, 916-917
- **HODZIC, E. et al.** Resurgence of Persisting Non-Cultivable Borrelia burgdorferi following Antibiotic Treatment. *MicePLoS One,* 2014, vol. 9, e86907

- **HODZIC, E. et al.** Persistence of Borrelia burgdorferi following antibiotic treatment in mice. *Antimicrobial Agents and Chemotherapy,* 2008, vol. 52, 1728-36
- **HOLLSTEIN, U.** Actinomycin. Chemistry and mechanism of action. *Chemical Reviews,* 1974, vol. 74 (6), 625-52
- **HUGHES AJ ; HUTCHINSON P ; GOODING T ; FREEZER NJ ; HOLDSWORTH SR et al.** Diagnosis of Mycobacterium tuberculosis infection using ESAT-6 and intracellular cytokine cytometry. *Clin Exp Immunol,* 2005, vol. 142, 132-139
- **HUNFELD, K.P. ; BRADE, V.** Antimicrobial susceptibility of Borrelia burgdorferi sensu lato: what we know, what we don't know, and what we need to know. *Wien Klin Wochenschr,* 2006, vol. 118 (21-22), 659-68
- **JACOBS WR, JR. ; BARLETTA RG ; UDANI R ; CHAN J ; KALKUT G et al.** Rapid assessment of drug susceptibilities of Mycobacterium tuberculosis by means of luciferase reporter phages. *Science,* 1993, vol. 260, 819-822
- **JENSEN, P.B. et al.** Different modes of anthracycline interaction with topoisomerase II. Separate structures critical for DNA-cleavage, and for overcoming topoisomerase II-related drug resistance. *Biochem. Pharmacol.,* 1993, vol. 45 (10), 2025-35
- **JONMALUNG J ; PRAMMANANAN T ; LEECHAWENGWONGS M ; CHAIPRASERT A.** Surveillance of pyrazinamide susceptibility among multidrug-resistant Mycobacterium tuberculosis isolates from Siriraj Hospital, Thailand. *BMC Microbiol,* 2010, vol. 10, 223
- **KEREN, I. et al.** Persister cells and tolerance to antimicrobials. *FEMS Microbiol. Lett.,* 2004, vol. 230, 13-18
- **KERSTEN, A. et al.** Effects of penicillin, ceftriaxone, and doxycycline on morphology of Borrelia burgdorferi. *Antimicrob. Agents Chemother,* 1995, vol. 39 (5), 1127-33
- **KLEMPNER, M.S. et al.** Treatment trials for post-Lyme disease symptoms revisited. *Am. J. Med.,* 2013, vol. 126, 665-669
- **KRAICZY, P. et al.** In vitro activities of fluoroquinolones against the spirochete Borrelia burgdorferi. *Antimicrob. Agents Chemother,* 2001, vol. 45, 2486-2494
- **KRUPP, L.B. et al.** Study and treatment of post Lyme disease (STOP-LD): a randomized double masked clinical trial. *Neurology,* 2003, vol. 60, 1923-1930
- **LANTOS, P.M. et al.** A systematic review of Borrelia burgdorferi morphologic variants does not support a role in chronic Lyme disease. *Clin. Infect. Dis.,* 2014, vol. 58, 663-671
- **LI, W. et al.** Yeast model uncovers dual roles of mitochondria in action of artemisinin. *PLoS Genet,* 2005, vol. 1, e36
- **LI, Y. ; ZHANG, Y.** PhoU is a persistence switch involved in persister formation and tolerance to multiple antibiotics and stresses in Escherichia coli. *Antimicrob. Agents Chemother.,* 2007, vol. 51, 2092-2099
- **LOUW GE ; WARREN RM ; DONALD PR ; MURRAY MB ; BOSMAN M et al.** Frequency and implications of pyrazinamide resistance in managing previously treated tuberculosis patients. *Int J Tuberc Lung Dis,* 2006, vol. 10, 802-807
- **LU, S. et al.** Bis-naphtho-gamma-pyrones from fungi and their bioactivities. *Molecules,* 2014, vol. 19 (6), 7169-88
- **MARQUES, A. et al.** Xenodiagnosis to detect Borrelia burgdorferi Infection: A first-in-Human Study. *Clinical Infectious Diseases,* 2014, vol. 58 (7), 937-945
- **MEEK, J.I. et al.** Underreporting of Lyme disease by Connecticut physicians, 1992. *J. Public Health. Pract.,* 1996, vol. 2 (4), 61-65
- **MIGLIORI GB ; BESOZZI G ; GIRARDI E ; KLIIMAN K ; LANGE C et al.** Clinical and operational value of the extensively drug-resistant tuberculosis definition. *European Respiratory Journal,* 2007, vol. 30, 623-626
- **MIKLOSSY, J. et al.** Persisting atypical and cystic forms of Borrelia burgdorferi and local inflammation in Lyme neuroborreliosis. *J. of Neuroinflammation,* 2008, vol. 5, 40
- **MITCHISON DA.** The action of antituberculosis drugs in short course chemotherapy. *Tubercle,* 1985, vol. 66, 219-225
- **MIZUNO, N.S. et al.** Binding of daunomycin to DNA and the inhibition of RNA and DNA synthesis. *Cancer Res.,* 1975, vol. 35 (6), 1542-6
- **MOLE R ; TROLLIP A ; ABRAHAMS C ; BOSMAN M ; ALBERT H.** Improved contamination control for a rapid phage-based rifampicin resistance test for Mycobacterium tuberculosis. *J Med Microbiol,* 2007, vol. 56, 1334-1339
- **MOODY, M.R. et al.** Effect of two cancer chemotherapeutic agents on the antibacterial activity of three antimicrobial agents. *Antimicrob. Agents Chemother.,* 1978, vol. 14 (5), 737-42
- **MORRIS, C.M. et al.** Effect of polymyxin B nonapeptide on daptomycin permeability and cell surface properties in Pseudomonas aeruginosa, Escherichia coli, and Pasteurella multocida. *J Antibiot.,* 1995, vol. 48, 67-72
- **MSHANA, R. N. et al.** Use of 3-(4,5-dimethylthiazol-2-yl)-2,5-diphenyl tetrazolium bromide for rapid detection of rifampin-resistant Mycobacterium tuberculosis. *J. Clin. Microbiol.,* 1998, vol. 36, 1214-9
- **MURGIA, R. ; CINCO, M.** Induction of cystic forms by different stress conditions in Borrelia burgdorferi. *APMIS : acta pathologica, microbiologica, et immunologica Scandinavica,* 2004, vol. 112, 57-62

- **NIU H ; PENG CUI ; REBECCA YEE ; WANLIANG SHI ; SHUO ZHANG ; JIE FENG ; DAVID SULLIVAN ; WENHONG ZHANG ; BINGDONG ZHU ; YING ZHANG.** A Clinical Drug Library Screen Identifies Tosufloxacin as Being Highly Active against Staphylococcus aureus Persisters. *Antibiotics,* 2015, vol. 4, 329-336
- **NIU H ; CUI P ; SHI W ; ZHANG S ; FENG J et al.** Identification of Anti-Persister Activity against Uropathogenic Escherichia coli from a Clinical Drug Library. *Antibiotics (Basel),* 2015, vol. 4, 179-187
- *Open Repository Collection of Synthetics and Pure Natural Products. 2014,* 15 March 2015
- **ORLOSKI, K.A. et al.** Surveillance for Lyme disease--United States, 1992-1998. *MMWR CDC Surveill Summ.,* 2000, vol. 49 (3), 1-11
- **PALANIAPPAN, K. ; HOLLEY, R.A.** Use of natural antimicrobials to increase antibiotic susceptibility of drug resistant bacteria. *International Journal of Food Microbiology,* 2010, vol. 140, 164-168
- **POGLIANO, J. et al.** Daptomycin-mediated reorganization of membrane architecture causes mislocalization of essential cell division proteins. *J. Bacteriol.,* 2012, vol. 194 (17), 4494-504
- **POMMIER, Y. et al.** DNA topoisomerases and their poisoning by anticancer and antibacterial drugs. *Chemistry & Biology,* 2010, vol. 17 (5), 421-33
- **RADOLF, J.D. et al.** Of ticks, mice and men: understanding the dual-host lifestyle of Lyme disease spirochaetes. *Nat. Rev. Microbiol.,* 2012, vol. 10, 87-99
- **RAO NA ; IRFAN M ; SOOMRO MM ; MEHFOOZ Z.** Drug resistance pattern in multidrug resistance pulmonary tuberculosis patients. *J Coll Physicians Surg Pak,* 2010, vol. 20, 262-265
- **RICKER, L.J. et al.** Persistent subfoveal fluid and increased preoperative foveal thickness impair visual outcome after macula-off retinal detachment repair. *Retina,* 2011, vol. 31, 1505-1512
- **SAPI, E. et al.** Characterization of biofilm formation by Borrelia burgdorferi in vitro. *Plos One,* 2012, vol. 7 (10), e48277
- **SAPI, E. et al.** Evaluation of in-vitro antibiotic susceptibility of different morphological forms of Borrelia burgdorferi. *Infect. Drug Resist.,* 2011, vol. 4, 97-113
- **SCORPIO A ; LINDHOLM-LEVY P ; HEIFETS L ; GILMAN R ; SIDDIQI S et al.** Characterization of pncA mutations in pyrazinamide-resistant Mycobacterium tuberculosis. *Antimicrob Agents Chemother,* 1997, vol. 41, 540-543
- **SCORPIO A ; ZHANG Y.** Mutations in pncA, a gene encoding pyrazinamidase/nicotinamidase, cause resistance to the antituberculous drug pyrazinamide in tubercle bacillus. *Nat Med,* 1996, vol. 2, 662-667
- **SENOL G ; COSKUN M ; GUNDUZ AT ; BICMEN C ; GAYAF M et al.** Investigation of pyrazinamide resistance in multidrug-resistant tuberculosis cases in Hospital of Pulmonary Diseases, Izmir, Turkey. *Mikrobiyoloji Bulteni,* 2008, vol. 42, 591-597
- **SHENAI S ; RODRIGUES C ; SADANI M ; SUKHADIA N ; MEHTA A.** Comparison of phenotypic and genotypic methods for pyrazinamide susceptibility testing. *Indian J Tuberc,* 2009, vol. 56, 82-90
- **SHOPOV, A. et al.** Improvements in image analysis and fluorescence microscopy to discriminate and enumerate bacteria and viruses in aquatic samples. *Aquatic Microbial Ecology,* 2000, vol. 22, 103-110
- **SIMPSON G ; COULTER C ; WESTON J ; KNIGHT T ; CARTER R et al.** Resistance patterns of multidrug-resistant tuberculosis in Western Province, Papua New Guinea. *Int J Tuberc Lung Dis,* 2011, vol. 15, 551-552
- **SOBELL, H.M.** Actinomycin and DNA transcription. *Proceedings of the National Academy of Sciences,* 1985, vol. 82 (16), 5328-31
- **SOMOSKOVI A ; DORMANDY J ; PARSONS LM ; KASWA M ; GOH KS et al.** Sequencing of the pncA gene in members of the Mycobacterium tuberculosis complex has important diagnostic applications: Identification of a species-specific pncA mutation in "Mycobacterium canettii" and the reliable and rapid predictor of pyrazinamide resistance. *J Clin Microbiol,* 2007, vol. 45, 595-599
- **STEERE, A.C. ; GLICKSTEIN, L.** Elucidation of Lyme arthritis. *Nat. Rev. Immunol.,* 2004, vol. 4, 143-152
- **STRAUBINGER, R.K. et al.** Persistence of Borrelia burgdorferi in experimentally infected dogs after antibiotic treatment. *Journal of Clinical Microbiology,* 1997, vol. 35, 111-116
- **STRICKER, R.B. ; L. JOHNSON.** Lyme disease: the next decade. *Infect. Drug Resist.,* 2011, vol. 4, 1-9
- **SUN, Z. ; ZHANG, Y.** Spent culture supernatant of Mycobacterium tuberculosis H37Ra improves viability of aged cultures of this strain and allows small inocula to initiate growth. *Journal of Bacteriology,* 1999, vol. 181, 7626-7628
- **SZYBALSKI, W. ; IYER, V.N.** Crosslinking of DNA by Enzymatically or Chemically Activated Mitomycins and Porfiromycins, Bifunctionally "Alkylating" Antibiotics. *Federation Proceedings,* 1964, vol. 23, 946-57
- **TAN, C. et al.** Daunomycin, an antitumor antibiotic, in the treatment of neoplastic disease. Clinical evaluation with special reference to childhood leukemia. *Cancer,* 1967, vol. 20 (3), 333-53

- **TAYLOR, J. P. et al.** Comparison of microbial numbers and enzymatic activities in surface soils and subsoils using various techniques. *Soil Biology & Biochemistry,* 2002, vol. 34, 387-401
- **TOMASZ, M. ; MITOMYCIN C.** small, fast and deadly (but very selective). *Chem. Biol.,* 1995, vol. 2 (9), 575-9
- **VANDEN BOSSCHE, H. et al.** Biochemical approaches to selective antifungal activity. Focus on azole antifungals. *Mycoses,* 1989, vol. 32 (1), 35-52
- **VAN RENSBURG, C.E. et al.** Antimicrobial activities of clofazimine and B669 are mediated by lysophospholipids. *Antimicrob. Agents Chemother,* 1992, vol. 36, 2729-2735
- **VELAYATI AA ; MASJEDI MR ; FARNIA P ; TABARSI P ; GHANAVI J et al.** Emergence of new forms of totally drug-resistant tuberculosis bacilli: super extensively drug-resistant tuberculosis or totally drug-resistant strains in iran. *Chest,* 2009, vol. 136, 420-425
- **WADE MM ; ZHANG Y.** ffects of weak acids, UV and proton motive force inhibitors on pyrazinamide activity against Mycobacterium tuberculosis in vitro. *J Antimicrob Chemother,* 2006, vol. 58, 936-941
- **WANG, H. ; CHENG, H. et al.** An improved 3-(4,5-dimethylthiazol-2-yl)-2,5-diphenyl tetrazolium bromide (MTT) reduction assay for evaluating the viability of Escherichia coli cells. *J Microbiol Methods,* 2010, vol. 82, 330-3
- **WANG, J. ; HUANG, L. et al.** Artemisinin directly targets malarial mitochondria through its specific mitochondrial activation. *Plos One,* 2010, vol. 5, e9582
- **WELCH, H.** The antibiotic saga. The University of Michigan, 1960, 96
- **WELLS, T.N. et al.** New medicines to improve control and contribute to the eradication of malaria. *Nat. Rev. Drug Discov.,* 2009, vol. 8, 879-891
- **WHO.** *Anti-tuberculosis Drug Resistance in the World,* 2008
- **WIKLER, M.A. ; FERRARO, M.J.** Correction of a reference to clinical laboratory standards institute interpretive criteria. *Clin. Infect. Dis.,* 2008, vol. 46, 1798
- **WILLIAMSON, N.R. et al.** Anticancer and immunosuppressive properties of bacterial prodiginines. *Future Microbiol.,* 2007, vol. 2 (6), 605-18
- **WILSON SM ; AL-SUWAIDI Z ; MCNERNEY R ; PORTER J ; DROBNIEWSKI F.** Evaluation of a new rapid bacteriophage-based method for the drug susceptibility testing of Mycobacterium tuberculosis. *Nat Med,* 1997, vol. 3, 465-468
- **WORMSER, G.P. et al.** The clinical assessment, treatment, and prevention of Lyme disease, human granulocytic anaplasmosis, and babesiosis: clinical practice guidelines by the Infectious Diseases Society of America. *Clin. Infect. Dis.,* 2006, vol. 43, 1089-1134
- **XU, J. et al.** In vitro and in vivo activity of clofazimine against Mycobacterium tuberculosis persisters. *Int. J. Tuberc. Lung Dis.,* 2012, vol. 16, 1119-1125
- **ZHANG, Y.** Persisters, persistent infections and the Yin-Yang Model. *Emerging Microbes & Infections,* 2014, vol. 3, e3
- **ZHU, L. et al.** Syntheses and biological activities of daunorubicin analogs with uncommon sugars. *Bioorganic & Medicinal Chemistry,* 2005, vol. 13 (23), 6381-7
- **ZHANG S ; CHEN J ; SHI W ; LIU W ; ZHANG WH et al.** Mutations in panD encoding aspartate decarboxylase are associated with pyrazinamide resistance in Mycobacterium tuberculosis. *Emerging Microbes & Infections,* 2013, vol. 2, e34
- ZS-MDR-TB" versus "ZR-MDR-TB": Improving Treatment of MDR-TB by Identifying Pyrazinamide Susceptibility. **ZHANG Y ; CHANG K ; LEUNG C ; YEW W ; GICQUEL G et al.** Emerging Microbes and Infections. Nature Publishing Group, 2012, vol. 1, e5
- **ZHANG Y ; MITCHISON D.** The curious characteristics of pyrazinamide: a review. *Int J Tuberc Lung Dis,* 2003, vol. 7, 6-21
- **ZHANG Y ; SCORPIO A ; NIKAIDO H ; SUN Z.** Role of acid pH and deficient efflux of pyrazinoic acid in unique susceptibility of Mycobacterium tuberculosis to pyrazinamide. *J Bacteriol,* 1999, vol. 181, 2044-2049
- Mechanisms of Pyrazinamide Action and Resistance. **ZHANG Y ; SHI W ; ZHANG W ; MITCHISON D.** Microbiol Spectrum. ASM Press, 2014, vol. 2